# EUROPEAN PATENT APPLICATION

(11) **EP 1 935 428 A1**
(43) Date of publication of application: **25.06.2008**
(21) Application number: 06127100.3
(22) Date of filing: 22.12.2006
(51) Int. Cl.: A61K 38/19

(54) **Oligonucleotide-polymer conjugates**

(71) Applicant: Antisense Pharma GmbH, 93053 Regensburg (DE)
(72) Inventor: Schlingensiepen, Karl-Hermann, Dr., 93093 Donaustauf (DE); Schlingensiepen, Reimar, Dr., 93051 Regensburg (DE)
(74) Representative: Schreiber, Christoph

(57) **Abstract**

A compound comprising an immunostimulator linked with at least one polymer.

### Annex to the application documents - subsequently filed sequences listing

SEQUENCE LISTING

<110>  Antisense Pharma GmbH

<120>  Oligonucleotide-polymer conjugates

<130>  063007ep

<160>  2451

<170>  PatentIn version 3.3

<210>  1
<211>  18
<212>  DNA
<213>  Artificial

<220>
<223>  Antisense nucleotide

<400>  1
cggcatgtct attttgta                                                    18

<210>  2
<211>  20
<212>  DNA
<213>  Artificial

<220>
<223>  Antisense nucleotide

<400>  2
gctttcacca aattggaagc                                                  20

<210>  3
<211>  14
<212>  DNA
<213>  Artificial

<220>
<223>  Antisense nucleotide

<400>  3
ctggcttttg ggtt                                                        14

<210>  4
<211>  14
<212>  DNA
<213>  Artificial

<220>
<223>  Antisense nucleotide

<400>  4
cacacagtag tgca                                                        14

<210>  5
<211>  14
<212>  DNA
<213>  Artificial

<220>
<223>  Antisense nucleotide

<400>  5
gcacacagta gtgc                                                          14


<210>  6
<211>  17
<212>  DNA
<213>  Artificial

<220>
<223>  Antisense nucleotide

<400>  6
gcttgctcag gatctgc                                                       17


<210>  7
<211>  14
<212>  DNA
<213>  Artificial

<220>
<223>  Antisense nucleotide

<400>  7
tactcttcgt cgct                                                          14


<210>  8
<211>  14
<212>  DNA
<213>  Artificial

<220>
<223>  Antisense nucleotide

<400>  8
cttggcgtag tact                                                          14


<210>  9
<211>  14
<212>  DNA
<213>  Artificial

<220>
<223>  Antisense nucleotide

<400>  9
gtaaacctcc ttgg                                                          14


<210>  10
<211>  19
<212>  DNA
<213>  Artificial

<220>
<223>  Antisense nucleotide

<400>  10
   gtctattttg taaacctcc                                                      19


<210>  11
<211>  20
<212>  DNA
<213>  Artificial

<220>
<223>  Antisense nucleotide

<400>  11
gcatgtctat tttgtaaacc                                                    20


<210>  12
<211>  14
<212>  DNA
<213>  Artificial

<220>
<223>  Antisense nucleotide

<400>  12
ggcatcaagg tacc                                                          14


<210>  13
<211>  14
<212>  DNA
<213>  Artificial

<220>
<223>  Antisense nucleotide

<400>  13
ctgtagaaag tggg                                                          14


<210>  14
<211>  18
<212>  DNA
<213>  Artificial

<220>
<223>  Antisense nucleotide

<400>  14
acaattctga agtagggt                                                      18


<210>  15
<211>  18
<212>  DNA
<213>  Artificial

<220>
<223>  Antisense nucleotide

<400>  15
tcaccaaatt ggaagcat                                                      18


<210>  16
<211>  18
<212>  DNA
<213>  Artificial

<220>
<223>  Antisense nucleotide

<400>  16
tctgatatag ctcaatcc                                                      18

<210>  17
<211>  18
<212>  DNA
<213>  Artificial

<220>
<223>  Antisense nucleotide

<400>  17
tcctagtgga ctttatag                                                    18


<210>  18
<211>  16
<212>  DNA
<213>  Artificial

<220>
<223>  Antisense nucleotide

<400>  18
tttttcctag tggact                                                      16


<210>  19
<211>  19
<212>  DNA
<213>  Artificial

<220>
<223>  Antisense nucleotide

<400>  19
caattatcct gcacatttc                                                   19


<210>  20
<211>  16
<212>  DNA
<213>  Artificial

<220>
<223>  Antisense nucleotide

<400>  20
gcaattatcc tgcaca                                                      16


<210>  21
<211>  16
<212>  DNA
<213>  Artificial

<220>
<223>  Antisense nucleotide

<400>  21
gcagcaatta tcctgc                                                      16


<210>  22
<211>  14
<212>  DNA
<213>  Artificial

<220>
<223>  Antisense nucleotide

<400>  22
tggcattgta ccct                                                         14


<210>  23
<211>  14
<212>  DNA
<213>  Artificial

<220>
<223>  Antisense nucleotide

<400>  23
tgtgctgagt gtct                                                         14


<210>  24
<211>  16
<212>  DNA
<213>  Artificial

<220>
<223>  Antisense nucleotide

<400>  24
cctgctgtgc tgagtg                                                       16


<210>  25
<211>  14
<212>  DNA
<213>  Artificial

<220>
<223>  Antisense nucleotide

<400>  25
cttgggtgtt ttgc                                                         14


<210>  26
<211>  18
<212>  DNA
<213>  Artificial

<220>
<223>  Antisense nucleotide

<400>  26
tttagctgca tttgcaag                                                     18


<210>  27
<211>  14
<212>  DNA
<213>  Artificial

<220>
<223>  Antisense nucleotide

<400>  27
gccacttttc caag                                                         14


<210>  28
<211>  18
<212>  DNA
<213>  Artificial

<220>
<223>  Antisense nucleotide

<400>  28
ctgatgtgtt gaagaaca                                                    18


<210>  29
<211>  14
<212>  DNA
<213>  Artificial

<220>
<223>  Antisense nucleotide

<400>  29
cgatagtctt gcag                                                        14


<210>  30
<211>  14
<212>  DNA
<213>  Artificial

<220>
<223>  Antisense nucleotide

<400>  30
gtcgatagtc ttgc                                                        14


<210>  31
<211>  14
<212>  DNA
<213>  Artificial

<220>
<223>  Antisense nucleotide

<400>  31
cttggacagg atct                                                        14


<210>  32
<211>  14
<212>  DNA
<213>  Artificial

<220>
<223>  Antisense nucleotide

<400>  32
ccaggaattg ttgc                                                        14


<210>  33
<211>  14
<212>  DNA
<213>  Artificial

<220>
<223>  Antisense nucleotide

<400>  33
cctcaatttc ccct                                                        14

<210>  34
<211>  14
<212>  DNA
<213>  Artificial

<220>
<223>  Antisense nucleotide

<400>  34
gatgtccact tgca                                                          14


<210>  35
<211>  14
<212>  DNA
<213>  Artificial

<220>
<223>  Antisense nucleotide

<400>  35
ctccaaatgt aggg                                                          14


<210>  36
<211>  14
<212>  DNA
<213>  Artificial

<220>
<223>  Antisense nucleotide

<400>  36
accttgctgt actg                                                          14


<210>  37
<211>  14
<212>  DNA
<213>  Artificial

<220>
<223>  Antisense nucleotide

<400>  37
gtagtacacg atgg                                                          14


<210>  38
<211>  14
<212>  DNA
<213>  Artificial

<220>
<223>  Antisense nucleotide

<400>  38
cacgtagtac acga                                                          14


<210>  39
<211>  14
<212>  DNA
<213>  Artificial

<220>
<223>  Antisense nucleotide

<400>  39
catgttggac agct                                                          14


<210>  40
<211>  14
<212>  DNA
<213>  Artificial

<220>
<223>  Antisense nucleotide

<400>  40
gcacgatcat gttg                                                          14


<210>  41
<211>  16
<212>  DNA
<213>  Artificial

<220>
<223>  Antisense nucleotide

<400>  41
tgtactctgc ttgaac                                                        16


<210>  42
<211>  16
<212>  DNA
<213>  Artificial

<220>
<223>  Antisense nucleotide

<400>  42
ctctgatgtg ttgaag                                                        16


<210>  43
<211>  16
<212>  DNA
<213>  Artificial

<220>
<223>  Antisense nucleotide

<400>  43
ggaagtcaat gtacag                                                        16


<210>  44
<211>  18
<212>  DNA
<213>  Artificial

<220>
<223>  Antisense nucleotide

<400>  44
catgtcgata gtcttgca                                                      18


<210>  45
<211>  18
<212>  DNA
<213>  Artificial

<220>
<223>  Antisense nucleotide

<400>  45
agctgaagca atagttgg                                                    18


<210>  46
<211>  18
<212>  DNA
<213>  Artificial

<220>
<223>  Antisense nucleotide

<400>  46
gtcatagatt tcgttgtg                                                    18


<210>  47
<211>  18
<212>  DNA
<213>  Artificial

<220>
<223>  Antisense nucleotide

<400>  47
ctccactttt aacttgag                                                    18


<210>  48
<211>  18
<212>  DNA
<213>  Artificial

<220>
<223>  Antisense nucleotide

<400>  48
tgctgtattt ctggtaca                                                    18


<210>  49
<211>  14
<212>  DNA
<213>  Artificial

<220>
<223>  Antisense nucleotide

<400>  49
tcgagcttcc ccca                                                        14


<210>  50
<211>  14
<212>  DNA
<213>  Artificial

<220>
<223>  Antisense nucleotide

<400>  50
ccccgagccc aagg                                                        14

<210>  51
<211>  13
<212>  DNA
<213>  Artificial

<220>
<223>  Antisense nucleotide

<400>  51
cccgacgagc cgg                                                          13


<210>  52
<211>  14
<212>  DNA
<213>  Artificial

<220>
<223>  Antisense nucleotide

<400>  52
acgcaccaag gcga                                                         14


<210>  53
<211>  15
<212>  DNA
<213>  Artificial

<220>
<223>  Antisense nucleotide

<400>  53
cgggttgtcg agccc                                                        15


<210>  54
<211>  13
<212>  DNA
<213>  Artificial

<220>
<223>  Antisense nucleotide

<400>  54
cggcagtgcc ccg                                                          13


<210>  55
<211>  14
<212>  DNA
<213>  Artificial

<220>
<223>  Antisense nucleotide

<400>  55
cgcaattctg ctcg                                                         14


<210>  56
<211>  14
<212>  DNA
<213>  Artificial

<220>
<223>  Antisense nucleotide

<400>  56
ttcgttgtgc tccc                                                          14


<210>  57
<211>  14
<212>  DNA
<213>  Artificial

<220>
<223>  Antisense nucleotide

<400>  57
attccgactc ggtg                                                          14


<210>  58
<211>  17
<212>  DNA
<213>  Artificial

<220>
<223>  Antisense nucleotide

<400>  58
acgtgcgtca tcaccgt                                                       17


<210>  59
<211>  10
<212>  DNA
<213>  Artificial

<220>
<223>  Antisense nucleotide

<400>  59
ccaagaagcc                                                               10


<210>  60
<211>  14
<212>  DNA
<213>  Artificial

<220>
<223>  Antisense nucleotide

<400>  60
cctaatgcct tcca                                                          14


<210>  61
<211>  14
<212>  DNA
<213>  Artificial

<220>
<223>  Antisense nucleotide

<400>  61
tcagcagggc cagg                                                          14


<210>  62
<211>  17
<212>  DNA
<213>  Artificial

<220>
<223>  Antisense nucleotide

<400>  62
gcaaagttca gcagggc                                                    17


<210>  63
<211>  16
<212>  DNA
<213>  Artificial

<220>
<223>  Antisense nucleotide

<400>  63
ggcaaagttc agcagg                                                     16


<210>  64
<211>  18
<212>  DNA
<213>  Artificial

<220>
<223>  Antisense nucleotide

<400>  64
gtggcaaagt tcagcagg                                                   18


<210>  65
<211>  14
<212>  DNA
<213>  Artificial

<220>
<223>  Antisense nucleotide

<400>  65
gtggcaaagt tcag                                                       14


<210>  66
<211>  18
<212>  DNA
<213>  Artificial

<220>
<223>  Antisense nucleotide

<400>  66
gaccgtggca aagttcag                                                   18


<210>  67
<211>  15
<212>  DNA
<213>  Artificial

<220>
<223>  Antisense nucleotide

<400>  67
agagaggctg accgt                                                      15

<210>  68
<211>  17
<212>  DNA
<213>  Artificial

<220>
<223>  Antisense nucleotide

<400>  68
gagagagaga ggctgac                                                    17


<210>  69
<211>  15
<212>  DNA
<213>  Artificial

<220>
<223>  Antisense nucleotide

<400>  69
acagagagag gctga                                                      15


<210>  70
<211>  15
<212>  DNA
<213>  Artificial

<220>
<223>  Antisense nucleotide

<400>  70
gtggacagag agagg                                                      15


<210>  71
<211>  18
<212>  DNA
<213>  Artificial

<220>
<223>  Antisense nucleotide

<400>  71
caactggaca gagagagg                                                   18


<210>  72
<211>  16
<212>  DNA
<213>  Artificial

<220>
<223>  Antisense nucleotide

<400>  72
tcttcttgat gtggcc                                                     16


<210>  73
<211>  17
<212>  DNA
<213>  Artificial

<220>
<223>  Antisense nucleotide

<400>  73
ccctcttctt cttgatg                                                    17


<210>  74
<211>  14
<212>  DNA
<213>  Artificial

<220>
<223>  Antisense nucleotide

<400>  74
caccctcttc ttct                                                       14


<210>  75
<211>  17
<212>  DNA
<213>  Artificial

<220>
<223>  Antisense nucleotide

<400>  75
atggatttct ttggcat                                                    17


<210>  76
<211>  13
<212>  DNA
<213>  Artificial

<220>
<223>  Antisense nucleotide

<400>  76
ggatttcttt ggc                                                        13


<210>  77
<211>  18
<212>  DNA
<213>  Artificial

<220>
<223>  Antisense nucleotide

<400>  77
aagttggact ctcttctc                                                   18


<210>  78
<211>  18
<212>  DNA
<213>  Artificial

<220>
<223>  Antisense nucleotide

<400>  78
taagttggac tctcttct                                                   18


<210>  79
<211>  16
<212>  DNA
<213>  Artificial

<220>
<223>  Antisense nucleotide

<400>  79
taggagtggt tgaggc                                                    16


<210>  80
<211>  17
<212>  DNA
<213>  Artificial

<220>
<223>  Antisense nucleotide

<400>  80
gtgtaggagt ggttgag                                                   17


<210>  81
<211>  14
<212>  DNA
<213>  Artificial

<220>
<223>  Antisense nucleotide

<400>  81
ctgtgtagga gtgg                                                      14


<210>  82
<211>  14
<212>  DNA
<213>  Artificial

<220>
<223>  Antisense nucleotide

<400>  82
cccacatgcc tgtg                                                      14


<210>  83
<211>  14
<212>  DNA
<213>  Artificial

<220>
<223>  Antisense nucleotide

<400>  83
cgatgaacaa cgag                                                      14


<210>  84
<211>  16
<212>  DNA
<213>  Artificial

<220>
<223>  Antisense nucleotide

<400>  84
ctggcgatga acaacg                                                    16

<210>  85
<211>  14
<212>  DNA
<213>  Artificial

<220>
<223>  Antisense nucleotide

<400>  85
cgctggcgat gaac                                                          14


<210>  86
<211>  15
<212>  DNA
<213>  Artificial

<220>
<223>  Antisense nucleotide

<400>  86
gagctagtcc cgttg                                                         15


<210>  87
<211>  15
<212>  DNA
<213>  Artificial

<220>
<223>  Antisense nucleotide

<400>  87
gcgaagagct agtcc                                                         15


<210>  88
<211>  17
<212>  DNA
<213>  Artificial

<220>
<223>  Antisense nucleotide

<400>  88
ccagttatgc gaagagc                                                       17


<210>  89
<211>  16
<212>  DNA
<213>  Artificial

<220>
<223>  Antisense nucleotide

<400>  89
ccccagttat gcgaag                                                        16


<210>  90
<211>  14
<212>  DNA
<213>  Artificial

<220>
<223>  Antisense nucleotide

<400>  90
cggccgcggt gtgt                                                          14


<210>  91
<211>  17
<212>  DNA
<213>  Artificial

<220>
<223>  Antisense nucleotide

<400>  91
cgggaatgct tccgccg                                                       17


<210>  92
<211>  17
<212>  DNA
<213>  Artificial

<220>
<223>  Antisense nucleotide

<400>  92
cggctcaccg cctcggc                                                       17


<210>  93
<211>  14
<212>  DNA
<213>  Artificial

<220>
<223>  Antisense nucleotide

<400>  93
cacgtctgcg gatc                                                          14


<210>  94
<211>  18
<212>  DNA
<213>  Artificial

<220>
<223>  Antisense nucleotide

<400>  94
ccccgcatcg catcaggg                                                      18


<210>  95
<211>  14
<212>  DNA
<213>  Artificial

<220>
<223>  Antisense nucleotide

<400>  95
cgccttgcaa cgcg                                                          14


<210>  96
<211>  14
<212>  DNA
<213>  Artificial

<220>
<223>  Antisense nucleotide

<400>  96
ccgaccgggg ccgg                                                          14


<210>  97
<211>  14
<212>  DNA
<213>  Artificial

<220>
<223>  Antisense nucleotide

<400>  97
gttcatggtt tcgg                                                          14


<210>  98
<211>  15
<212>  DNA
<213>  Artificial

<220>
<223>  Antisense nucleotide

<400>  98
gcagaaagtt catgg                                                         15


<210>  99
<211>  14
<212>  DNA
<213>  Artificial

<220>
<223>  Antisense nucleotide

<400>  99
gctgatagac atcc                                                          14


<210>  100
<211>  14
<212>  DNA
<213>  Artificial

<220>
<223>  Antisense nucleotide

<400>  100
gcgctgatag acat                                                          14


<210>  101
<211>  16
<212>  DNA
<213>  Artificial

<220>
<223>  Antisense nucleotide

<400>  101
gtagctgcgc tgatag                                                        16

<210>  102
<211>  14
<212>  DNA
<213>  Artificial

<220>
<223>  Antisense nucleotide

<400>  102
ctcgatctca tcag                                                         14


<210>  103
<211>  17
<212>  DNA
<213>  Artificial

<220>
<223>  Antisense nucleotide

<400>  103
atgtactcga tctcatc                                                      17


<210>  104
<211>  16
<212>  DNA
<213>  Artificial

<220>
<223>  Antisense nucleotide

<400>  104
gaagatgtac tcgatc                                                       16


<210>  105
<211>  16
<212>  DNA
<213>  Artificial

<220>
<223>  Antisense nucleotide

<400>  105
cttgaagatg tactcg                                                       16


<210>  106
<211>  14
<212>  DNA
<213>  Artificial

<220>
<223>  Antisense nucleotide

<400>  106
gcatcgcatc aggg                                                         14


<210>  107
<211>  14
<212>  DNA
<213>  Artificial

<220>
<223>  Antisense nucleotide

<400>  107
ccgcatcgca tcag                                                          14


<210>  108
<211>  18
<212>  DNA
<213>  Artificial

<220>
<223>  Antisense nucleotide

<400>  108
catttgttgt gctgtagg                                                      18


<210>  109
<211>  18
<212>  DNA
<213>  Artificial

<220>
<223>  Antisense nucleotide

<400>  109
ggtctgcatt cacatttg                                                      18


<210>  110
<211>  15
<212>  DNA
<213>  Artificial

<220>
<223>  Antisense nucleotide

<400>  110
ctttggtctg cattc                                                         15


<210>  111
<211>  15
<212>  DNA
<213>  Artificial

<220>
<223>  Antisense nucleotide

<400>  111
ctttctttgg tctgc                                                         15


<210>  112
<211>  17
<212>  DNA
<213>  Artificial

<220>
<223>  Antisense nucleotide

<400>  112
gctctatctt tctttgg                                                       17


<210>  113
<211>  17
<212>  DNA
<213>  Artificial

<220>
<223>  Antisense nucleotide

<400>  113
gtcttgctct atctttc                                                    17


<210>  114
<211>  16
<212>  DNA
<213>  Artificial

<220>
<223>  Antisense nucleotide

<400>  114
cttgtcttgc tctatc                                                     16


<210>  115
<211>  18
<212>  DNA
<213>  Artificial

<220>
<223>  Antisense nucleotide

<400>  115
catctgcaag tacgttcg                                                   18


<210>  116
<211>  18
<212>  DNA
<213>  Artificial

<220>
<223>  Antisense nucleotide

<400>  116
cacatctgca agtacgtt                                                   18


<210>  117
<211>  18
<212>  DNA
<213>  Artificial

<220>
<223>  Antisense nucleotide

<400>  117
gtcacatctg caagtacg                                                   18


<210>  118
<211>  14
<212>  DNA
<213>  Artificial

<220>
<223>  Antisense nucleotide

<400>  118
catctgcaag tacg                                                       14

<210>  119
<211>  15
<212>  DNA
<213>  Artificial

<220>
<223>  Antisense nucleotide

<400>  119
cacatctgca agtac                                                        15


<210>  120
<211>  14
<212>  DNA
<213>  Artificial

<220>
<223>  Antisense nucleotide

<400>  120
gtcacatctg caag                                                         14


<210>  121
<211>  14
<212>  DNA
<213>  Artificial

<220>
<223>  Antisense nucleotide

<400>  121
cttgtcacat ctgc                                                         14


<210>  122
<211>  16
<212>  DNA
<213>  Artificial

<220>
<223>  Antisense nucleotide

<400>  122
ggcttgtcac atctgc                                                       16


<210>  123
<211>  16
<212>  DNA
<213>  Artificial

<220>
<223>  Antisense nucleotide

<400>  123
ctcggcttgt cacatc                                                       16


<210>  124
<211>  14
<212>  DNA
<213>  Artificial

<220>
<223>  Antisense nucleotide

<400>  124
ctccttcctc ctgc                                                          14


<210>  125
<211>  18
<212>  DNA
<213>  Artificial

<220>
<223>  Antisense nucleotide

<400>  125
gcttgaagat gtacctcg                                                      18


<210>  126
<211>  15
<212>  DNA
<213>  Artificial

<220>
<223>  Antisense nucleotide

<400>  126
cgttgctctc cgacg                                                         15


<210>  127
<211>  18
<212>  DNA
<213>  Artificial

<220>
<223>  Antisense nucleotide

<400>  127
cttcttttgc aagtctgt                                                      18


<210>  128
<211>  18
<212>  DNA
<213>  Artificial

<220>
<223>  Antisense nucleotide

<400>  128
tgagctgtgc atgccttc                                                      18


<210>  129
<211>  15
<212>  DNA
<213>  Artificial

<220>
<223>  Antisense nucleotide

<400>  129
agtcaggagg accag                                                         15


<210>  130
<211>  15
<212>  DNA
<213>  Artificial

<220>
<223>  Antisense nucleotide

<400>  130
tgggtgccct ggcct                                                        15


<210>  131
<211>  15
<212>  DNA
<213>  Artificial

<220>
<223>  Antisense nucleotide

<400>  131
catgttaggc aggtt                                                        15


<210>  132
<211>  17
<212>  DNA
<213>  Artificial

<220>
<223>  Antisense nucleotide

<400>  132
aggcatctcg gagatct                                                      17


<210>  133
<211>  16
<212>  DNA
<213>  Artificial

<220>
<223>  Antisense nucleotide

<400>  133
aaagtcttca ctctgc                                                       16


<210>  134
<211>  17
<212>  DNA
<213>  Artificial

<220>
<223>  Antisense nucleotide

<400>  134
aacaagttgt ccagctg                                                      17


<210>  135
<211>  18
<212>  DNA
<213>  Artificial

<220>
<223>  Antisense nucleotide

<400>  135
gtaaaactgg atcatctc                                                     18

<210>  136
<211>  15
<212>  DNA
<213>  Artificial

<220>
<223>  Antisense nucleotide

<400>  136
catcacctcc tccag                                                        15


<210>  137
<211>  18
<212>  DNA
<213>  Artificial

<220>
<223>  Antisense nucleotide

<400>  137
gggtcttcag gttctccc                                                     18


<210>  138
<211>  18
<212>  DNA
<213>  Artificial

<220>
<223>  Antisense nucleotide

<400>  138
cacggccttg ctcttgtt                                                     18


<210>  139
<211>  18
<212>  DNA
<213>  Artificial

<220>
<223>  Antisense nucleotide

<400>  139
ttattaaagg cattcttc                                                     18


<210>  140
<211>  18
<212>  DNA
<213>  Artificial

<220>
<223>  Antisense nucleotide

<400>  140
aagatgtcaa actcactc                                                     18


<210>  141
<211>  18
<212>  DNA
<213>  Artificial

<220>
<223>  Antisense nucleotide

<400>  141
gtagttgatg aagatgtc                                                    18


<210>  142
<211>  16
<212>  DNA
<213>  Artificial

<220>
<223>  Antisense nucleotide

<400>  142
gattttggag acctct                                                      16


<210>  143
<211>  16
<212>  DNA
<213>  Artificial

<220>
<223>  Antisense nucleotide

<400>  143
tcagctatcc cagagc                                                      16


<210>  144
<211>  15
<212>  DNA
<213>  Artificial

<220>
<223>  Antisense nucleotide

<400>  144
ggctgggtca gctat                                                       15


<210>  145
<211>  18
<212>  DNA
<213>  Artificial

<220>
<223>  Antisense nucleotide

<400>  145
aaatcgttca cagagaag                                                    18


<210>  146
<211>  18
<212>  DNA
<213>  Artificial

<220>
<223>  Antisense nucleotide

<400>  146
tctttctaaa tcgttcac                                                    18


<210>  147
<211>  14
<212>  DNA
<213>  Artificial

<220>
<223>  Antisense nucleotide

<400>  147
ttcatgtctg tgcc                                                          14


<210>  148
<211>  14
<212>  DNA
<213>  Artificial

<220>
<223>  Antisense nucleotide

<400>  148
gtaggtgagt tcca                                                          14


<210>  149
<211>  14
<212>  DNA
<213>  Artificial

<220>
<223>  Antisense nucleotide

<400>  149
gttgtgagcg atga                                                          14


<210>  150
<211>  18
<212>  DNA
<213>  Artificial

<220>
<223>  Antisense nucleotide

<400>  150
catagttgtc ctcaaaga                                                      18


<210>  151
<211>  14
<212>  DNA
<213>  Artificial

<220>
<223>  Antisense nucleotide

<400>  151
ggcatagttg tcct                                                          14


<210>  152
<211>  14
<212>  DNA
<213>  Artificial

<220>
<223>  Antisense nucleotide

<400>  152
cattgtctag cacg                                                          14

<210>  153
<211>  14
<212>  DNA
<213>  Artificial

<220>
<223>  Antisense nucleotide

<400>  153
ctccattgtc tagc                                                         14


<210>  154
<211>  14
<212>  DNA
<213>  Artificial

<220>
<223>  Antisense nucleotide

<400>  154
gtattgttca gcgg                                                         14


<210>  155
<211>  16
<212>  DNA
<213>  Artificial

<220>
<223>  Antisense nucleotide

<400>  155
tcaagatctc tgtgag                                                       16


<210>  156
<211>  16
<212>  DNA
<213>  Artificial

<220>
<223>  Antisense nucleotide

<400>  156
cacaaaatcg tgtcct                                                       16


<210>  157
<211>  16
<212>  DNA
<213>  Artificial

<220>
<223>  Antisense nucleotide

<400>  157
tccttccaca aaatcg                                                       16


<210>  158
<211>  14
<212>  DNA
<213>  Artificial

<220>
<223>  Antisense nucleotide

<400>  158
gtggaagatg tcct                                                         14


<210>  159
<211>  16
<212>  DNA
<213>  Artificial

<220>
<223>  Antisense nucleotide

<400>  159
tcttgtggaa gatgtc                                                       16


<210>  160
<211>  16
<212>  DNA
<213>  Artificial

<220>
<223>  Antisense nucleotide

<400>  160
tctatcagtg tgagag                                                       16


<210>  161
<211>  14
<212>  DNA
<213>  Artificial

<220>
<223>  Antisense nucleotide

<400>  161
ggttggtgtc tatc                                                         14


<210>  162
<211>  14
<212>  DNA
<213>  Artificial

<220>
<223>  Antisense nucleotide

<400>  162
acatcggaga acag                                                         14


<210>  163
<211>  14
<212>  DNA
<213>  Artificial

<220>
<223>  Antisense nucleotide

<400>  163
ccttacacat cgga                                                         14


<210>  164
<211>  16
<212>  DNA
<213>  Artificial

<220>
<223>  Antisense nucleotide

<400>  164
acaatcctca gaactc                                                       16


<210>  165
<211>  14
<212>  DNA
<213>  Artificial

<220>
<223>  Antisense nucleotide

<400>  165
gctctgacaa tcct                                                         14


<210>  166
<211>  14
<212>  DNA
<213>  Artificial

<220>
<223>  Antisense nucleotide

<400>  166
tggttgaagt ggag                                                         14


<210>  167
<211>  14
<212>  DNA
<213>  Artificial

<220>
<223>  Antisense nucleotide

<400>  167
ctgtggttga agtg                                                         14


<210>  168
<211>  14
<212>  DNA
<213>  Artificial

<220>
<223>  Antisense nucleotide

<400>  168
gttgtaggtg acca                                                         14


<210>  169
<211>  14
<212>  DNA
<213>  Artificial

<220>
<223>  Antisense nucleotide

<400>  169
ctgtgttgta ggtg                                                         14

<210>  170
<211>  14
<212>  DNA
<213>  Artificial

<220>
<223>  Antisense nucleotide

<400>  170
gactcaaacg tgtc                                                          14


<210>  171
<211>  14
<212>  DNA
<213>  Artificial

<220>
<223>  Antisense nucleotide

<400>  171
catggactca aacg                                                          14


<210>  172
<211>  14
<212>  DNA
<213>  Artificial

<220>
<223>  Antisense nucleotide

<400>  172
cgaatgtata ccgg                                                          14


<210>  173
<211>  14
<212>  DNA
<213>  Artificial

<220>
<223>  Antisense nucleotide

<400>  173
ccgaatgtat accg                                                          14


<210>  174
<211>  14
<212>  DNA
<213>  Artificial

<220>
<223>  Antisense nucleotide

<400>  174
gccgaatgta tacc                                                          14


<210>  175
<211>  14
<212>  DNA
<213>  Artificial

<220>
<223>  Antisense nucleotide

<400>  175
gtagttgtag ggac                                                          14


<210>  176
<211>  18
<212>  DNA
<213>  Artificial

<220>
<223>  Antisense nucleotide

<400>  176
tagaaaggta gttgtagg                                                      18


<210>  177
<211>  18
<212>  DNA
<213>  Artificial

<220>
<223>  Antisense nucleotide

<400>  177
gtagaaaggt agttgtag                                                      18


<210>  178
<211>  16
<212>  DNA
<213>  Artificial

<220>
<223>  Antisense nucleotide

<400>  178
cgtagaaagg tagttg                                                        16


<210>  179
<211>  14
<212>  DNA
<213>  Artificial

<220>
<223>  Antisense nucleotide

<400>  179
ccgtagaaag gtag                                                          14


<210>  180
<211>  14
<212>  DNA
<213>  Artificial

<220>
<223>  Antisense nucleotide

<400>  180
gaccatagca cact                                                          14


<210>  181
<211>  14
<212>  DNA
<213>  Artificial

<220>
<223>  Antisense nucleotide

<400>  181
ggatattggc actg                                                         14


<210>  182
<211>  14
<212>  DNA
<213>  Artificial

<220>
<223>  Antisense nucleotide

<400>  182
cctggatatt ggca                                                         14


<210>  183
<211>  14
<212>  DNA
<213>  Artificial

<220>
<223>  Antisense nucleotide

<400>  183
gctcccaaag atct                                                         14


<210>  184
<211>  14
<212>  DNA
<213>  Artificial

<220>
<223>  Antisense nucleotide

<400>  184
cccatcaaag ctct                                                         14


<210>  185
<211>  14
<212>  DNA
<213>  Artificial

<220>
<223>  Antisense nucleotide

<400>  185
caaacacttg gagc                                                         14


<210>  186
<211>  16
<212>  DNA
<213>  Artificial

<220>
<223>  Antisense nucleotide

<400>  186
gtctcaaaca cttgga                                                       16

<210>  187
<211>  16
<212>  DNA
<213>  Artificial

<220>
<223>  Antisense nucleotide

<400>  187
gagtctcaaa cacttg                                                        16


<210>  188
<211>  16
<212>  DNA
<213>  Artificial

<220>
<223>  Antisense nucleotide

<400>  188
gtaacctgtg atctct                                                        16


<210>  189
<211>  14
<212>  DNA
<213>  Artificial

<220>
<223>  Antisense nucleotide

<400>  189
ggtaacctgt gatc                                                          14


<210>  190
<211>  16
<212>  DNA
<213>  Artificial

<220>
<223>  Antisense nucleotide

<400>  190
gtataggtaa cctgtg                                                        16


<210>  191
<211>  18
<212>  DNA
<213>  Artificial

<220>
<223>  Antisense nucleotide

<400>  191
tgagatgtat aggtaacc                                                      18


<210>  192
<211>  16
<212>  DNA
<213>  Artificial

<220>
<223>  Antisense nucleotide

<400>  192
tgctgagatg tatagg                                                    16


<210>  193
<211>  14
<212>  DNA
<213>  Artificial

<220>
<223>  Antisense nucleotide

<400>  193
ccatgctgag atgt                                                      14


<210>  194
<211>  14
<212>  DNA
<213>  Artificial

<220>
<223>  Antisense nucleotide

<400>  194
ggattacttg cagg                                                      14


<210>  195
<211>  16
<212>  DNA
<213>  Artificial

<220>
<223>  Antisense nucleotide

<400>  195
tgttatggtg gatgag                                                    16


<210>  196
<211>  14
<212>  DNA
<213>  Artificial

<220>
<223>  Antisense nucleotide

<400>  196
ggtgttatgg tgga                                                      14


<210>  197
<211>  14
<212>  DNA
<213>  Artificial

<220>
<223>  Antisense nucleotide

<400>  197
gcagttgaca cact                                                      14


<210>  198
<211>  14
<212>  DNA
<213>  Artificial

<220>
<223>  Antisense nucleotide

<400>  198
agtactcggc attc                                                          14


<210>  199
<211>  16
<212>  DNA
<213>  Artificial

<220>
<223>  Antisense nucleotide

<400>  199
cattcacata ctccct                                                        16


<210>  200
<211>  16
<212>  DNA
<213>  Artificial

<220>
<223>  Antisense nucleotide

<400>  200
tccaaaacag gtcact                                                        16


<210>  201
<211>  14
<212>  DNA
<213>  Artificial

<220>
<223>  Antisense nucleotide

<400>  201
ggtccttata gtgg                                                          14


<210>  202
<211>  14
<212>  DNA
<213>  Artificial

<220>
<223>  Antisense nucleotide

<400>  202
cagaatgcca acca                                                          14


<210>  203
<211>  14
<212>  DNA
<213>  Artificial

<220>
<223>  Antisense nucleotide

<400>  203
acgagaatgc caac                                                          14

<210>  204
<211>  14
<212>  DNA
<213>  Artificial

<220>
<223>  Antisense nucleotide

<400>  204
gatcccaaag acca                                                          14


<210>  205
<211>  14
<212>  DNA
<213>  Artificial

<220>
<223>  Antisense nucleotide

<400>  205
tcgcttgatg agga                                                          14


<210>  206
<211>  14
<212>  DNA
<213>  Artificial

<220>
<223>  Antisense nucleotide

<400>  206
catcgtgtac ttcc                                                          14


<210>  207
<211>  14
<212>  DNA
<213>  Artificial

<220>
<223>  Antisense nucleotide

<400>  207
gcatcgtgta cttc                                                          14


<210>  208
<211>  14
<212>  DNA
<213>  Artificial

<220>
<223>  Antisense nucleotide

<400>  208
actgtgccaa aagc                                                          14


<210>  209
<211>  14
<212>  DNA
<213>  Artificial

<220>
<223>  Antisense nucleotide

<400>  209
cttgtagact gtgc                                                          14


<210>  210
<211>  14
<212>  DNA
<213>  Artificial

<220>
<223>  Antisense nucleotide

<400>  210
cccttgtaga ctgt                                                          14


<210>  211
<211>  16
<212>  DNA
<213>  Artificial

<220>
<223>  Antisense nucleotide

<400>  211
tcaacacttt gatggc                                                        16


<210>  212
<211>  14
<212>  DNA
<213>  Artificial

<220>
<223>  Antisense nucleotide

<400>  212
ccctcaacac tttg                                                          14


<210>  213
<211>  16
<212>  DNA
<213>  Artificial

<220>
<223>  Antisense nucleotide

<400>  213
gtgttttccc tcaaca                                                        16


<210>  214
<211>  16
<212>  DNA
<213>  Artificial

<220>
<223>  Antisense nucleotide

<400>  214
gtatgcttcg tctaag                                                        16


<210>  215
<211>  14
<212>  DNA
<213>  Artificial

<220>
<223>  Antisense nucleotide

<400>  215
cgtatgcttc gtct                                                          14


<210>  216
<211>  14
<212>  DNA
<213>  Artificial

<220>
<223>  Antisense nucleotide

<400>  216
ccatcacgta tgct                                                          14


<210>  217
<211>  14
<212>  DNA
<213>  Artificial

<220>
<223>  Antisense nucleotide

<400>  217
gcataagctg tgtc                                                          14


<210>  218
<211>  14
<212>  DNA
<213>  Artificial

<220>
<223>  Antisense nucleotide

<400>  218
catggtctaa gagg                                                          14


<210>  219
<211>  16
<212>  DNA
<213>  Artificial

<220>
<223>  Antisense nucleotide

<400>  219
caatctgcat acacca                                                        16


<210>  220
<211>  14
<212>  DNA
<213>  Artificial

<220>
<223>  Antisense nucleotide

<400>  220
ggcaatctgc atac                                                          14

<210>  221
<211>  14
<212>  DNA
<213>  Artificial

<220>
<223>  Antisense nucleotide

<400>  221
ctgtctcgtc aatg                                                          14


<210>  222
<211>  16
<212>  DNA
<213>  Artificial

<220>
<223>  Antisense nucleotide

<400>  222
cataactcca cacatc                                                        16


<210>  223
<211>  16
<212>  DNA
<213>  Artificial

<220>
<223>  Antisense nucleotide

<400>  223
agtcacacca taactc                                                        16


<210>  224
<211>  16
<212>  DNA
<213>  Artificial

<220>
<223>  Antisense nucleotide

<400>  224
acagtcacac cataac                                                        16


<210>  225
<211>  14
<212>  DNA
<213>  Artificial

<220>
<223>  Antisense nucleotide

<400>  225
ccccaaaagt catc                                                          14


<210>  226
<211>  14
<212>  DNA
<213>  Artificial

<220>
<223>  Antisense nucleotide

<400>  226
tcgtaaggtt tggc                                                         14


<210>  227
<211>  14
<212>  DNA
<213>  Artificial

<220>
<223>  Antisense nucleotide

<400>  227
gatcccatcg taag                                                         14


<210>  228
<211>  14
<212>  DNA
<213>  Artificial

<220>
<223>  Antisense nucleotide

<400>  228
caatggtgca gatg                                                         14


<210>  229
<211>  14
<212>  DNA
<213>  Artificial

<220>
<223>  Antisense nucleotide

<400>  229
gacatcaatg gtgc                                                         14


<210>  230
<211>  16
<212>  DNA
<213>  Artificial

<220>
<223>  Antisense nucleotide

<400>  230
gtagacatca atggtg                                                       16


<210>  231
<211>  18
<212>  DNA
<213>  Artificial

<220>
<223>  Antisense nucleotide

<400>  231
catgatcatg tagacatc                                                     18


<210>  232
<211>  16
<212>  DNA
<213>  Artificial

<220>
<223>  Antisense nucleotide

<400>  232
ccatgatcat gtagac                                                        16


<210>  233
<211>  18
<212>  DNA
<213>  Artificial

<220>
<223>  Antisense nucleotide

<400>  233
catttgacca tgatcatg                                                      18


<210>  234
<211>  16
<212>  DNA
<213>  Artificial

<220>
<223>  Antisense nucleotide

<400>  234
ccaacatttg accatg                                                        16


<210>  235
<211>  18
<212>  DNA
<213>  Artificial

<220>
<223>  Antisense nucleotide

<400>  235
tcatccaaca tttgacca                                                      18


<210>  236
<211>  18
<212>  DNA
<213>  Artificial

<220>
<223>  Antisense nucleotide

<400>  236
gagtcaatca tccaacat                                                      18


<210>  237
<211>  16
<212>  DNA
<213>  Artificial

<220>
<223>  Antisense nucleotide

<400>  237
cagagtcaat catcca                                                        16

<210>  238
<211>  14
<212>  DNA
<213>  Artificial

<220>
<223>  Antisense nucleotide

<400>  238
ccgacattca gagt                                                         14


<210>  239
<211>  16
<212>  DNA
<213>  Artificial

<220>
<223>  Antisense nucleotide

<400>  239
gaattcagac accaac                                                       16


<210>  240
<211>  14
<212>  DNA
<213>  Artificial

<220>
<223>  Antisense nucleotide

<400>  240
gatgaccaca aagc                                                         14


<210>  241
<211>  16
<212>  DNA
<213>  Artificial

<220>
<223>  Antisense nucleotide

<400>  241
ccatcaaata catcgg                                                       16


<210>  242
<211>  18
<212>  DNA
<213>  Artificial

<220>
<223>  Antisense nucleotide

<400>  242
tcaccatcaa atacatcg                                                     18


<210>  243
<211>  14
<212>  DNA
<213>  Artificial

<220>
<223>  Antisense nucleotide

<400>  243
caacgtagcc atca                                                          14


<210>  244
<211>  14
<212>  DNA
<213>  Artificial

<220>
<223>  Antisense nucleotide

<400>  244
acgtctttga cgac                                                          14


<210>  245
<211>  18
<212>  DNA
<213>  Artificial

<220>
<223>  Antisense nucleotide

<400>  245
caaaaacgtc tttgacga                                                      18


<210>  246
<211>  16
<212>  DNA
<213>  Artificial

<220>
<223>  Antisense nucleotide

<400>  246
ggcaaaaacg tctttg                                                        16


<210>  247
<211>  16
<212>  DNA
<213>  Artificial

<220>
<223>  Antisense nucleotide

<400>  247
caaaggcaaa aacgtc                                                        16


<210>  248
<211>  14
<212>  DNA
<213>  Artificial

<220>
<223>  Antisense nucleotide

<400>  248
gtgtcaagta ctcg                                                          14


<210>  249
<211>  16
<212>  DNA
<213>  Artificial

<220>
<223>  Antisense nucleotide

<400>  249
gtaatagagg ttgtcg                                                    16


<210>  250
<211>  14
<212>  DNA
<213>  Artificial

<220>
<223>  Antisense nucleotide

<400>  250
cccagtaata gagg                                                      14


<210>  251
<211>  14
<212>  DNA
<213>  Artificial

<220>
<223>  Antisense nucleotide

<400>  251
catggtgctc actg                                                      14


<210>  252
<211>  14
<212>  DNA
<213>  Artificial

<220>
<223>  Antisense nucleotide

<400>  252
gtgcctgtac gtac                                                      14


<210>  253
<211>  14
<212>  DNA
<213>  Artificial

<220>
<223>  Antisense nucleotide

<400>  253
tcggactata ctgc                                                      14


<210>  254
<211>  16
<212>  DNA
<213>  Artificial

<220>
<223>  Antisense nucleotide

<400>  254
cagttcggac tatact                                                    16

<210>  255
<211>  14
<212>  DNA
<213>  Artificial

<220>
<223>  Antisense nucleotide

<400>  255
aagcctaaga cgca                                                          14


<210>  256
<211>  14
<212>  DNA
<213>  Artificial

<220>
<223>  Antisense nucleotide

<400>  256
gcccaagttc aaca                                                          14


<210>  257
<211>  14
<212>  DNA
<213>  Artificial

<220>
<223>  Antisense nucleotide

<400>  257
tgaaaagtcg cggt                                                          14


<210>  258
<211>  18
<212>  DNA
<213>  Artificial

<220>
<223>  Antisense nucleotide

<400>  258
ggttaattaa gatgcctc                                                      18


<210>  259
<211>  14
<212>  DNA
<213>  Artificial

<220>
<223>  Antisense nucleotide

<400>  259
tctctaagag cgca                                                          14


<210>  260
<211>  16
<212>  DNA
<213>  Artificial

<220>
<223>  Antisense nucleotide

<400>  260
acgtgaggtt agtttg                                                        16


<210>  261
<211>  14
<212>  DNA
<213>  Artificial

<220>
<223>  Antisense nucleotide

<400>  261
cacgtgaggt tagt                                                          14


<210>  262
<211>  14
<212>  DNA
<213>  Artificial

<220>
<223>  Antisense nucleotide

<400>  262
catagaacag tccg                                                          14


<210>  263
<211>  16
<212>  DNA
<213>  Artificial

<220>
<223>  Antisense nucleotide

<400>  263
cagtcataga acagtc                                                        16


<210>  264
<211>  18
<212>  DNA
<213>  Artificial

<220>
<223>  Antisense nucleotide

<400>  264
ctttgcagtc atagaaca                                                      18


<210>  265
<211>  14
<212>  DNA
<213>  Artificial

<220>
<223>  Antisense nucleotide

<400>  265
tgcagtcata gaac                                                          14


<210>  266
<211>  14
<212>  DNA
<213>  Artificial

<220>
<223>  Antisense nucleotide

<400>  266
ggtcgtttcc atct                                                          14


<210>  267
<211>  16
<212>  DNA
<213>  Artificial

<220>
<223>  Antisense nucleotide

<400>  267
catagaaggt cgtttc                                                        16


<210>  268
<211>  14
<212>  DNA
<213>  Artificial

<220>
<223>  Antisense nucleotide

<400>  268
cgtcatagaa ggtc                                                          14


<210>  269
<211>  15
<212>  DNA
<213>  Artificial

<220>
<223>  Antisense nucleotide

<400>  269
catcgtcata gaagg                                                         15


<210>  270
<211>  24
<212>  DNA
<213>  Artificial

<220>
<223>  Antisense nucleotide

<400>  270
ggacgggagg aacgaggcgt tgag                                               24


<210>  271
<211>  14
<212>  DNA
<213>  Artificial

<220>
<223>  Antisense nucleotide

<400>  271
tagccataag gtcc                                                          14

<210>  272
<211>  14
<212>  DNA
<213>  Artificial

<220>
<223>  Antisense nucleotide

<400>  272
ggttactgta gcca                                                          14


<210>  273
<211>  14
<212>  DNA
<213>  Artificial

<220>
<223>  Antisense nucleotide

<400>  273
ggttactgta gcca                                                          14


<210>  274
<211>  30
<212>  DNA
<213>  Artificial

<220>
<223>  Antisense nucleotide

<400>  274
cagggtcatg ctctgtttca ggatcttggg                                         30


<210>  275
<211>  18
<212>  DNA
<213>  Artificial

<220>
<223>  Antisense nucleotide

<400>  275
agttcttggc gcggaggt                                                      18


<210>  276
<211>  18
<212>  DNA
<213>  Artificial

<220>
<223>  Antisense nucleotide

<400>  276
aggtgaggag gtccgagt                                                      18


<210>  277
<211>  16
<212>  DNA
<213>  Artificial

<220>
<223>  Antisense nucleotide

<400>  277
tggactggat tatcag                                                    16


<210>  278
<211>  18
<212>  DNA
<213>  Artificial

<220>
<223>  Antisense nucleotide

<400>  278
gtggtggtga tgtgcccg                                                  18


<210>  279
<211>  14
<212>  DNA
<213>  Artificial

<220>
<223>  Antisense nucleotide

<400>  279
tgtcacgttc ttgg                                                      14


<210>  280
<211>  14
<212>  DNA
<213>  Artificial

<220>
<223>  Antisense nucleotide

<400>  280
ctcatctgtc acgt                                                      14


<210>  281
<211>  18
<212>  DNA
<213>  Artificial

<220>
<223>  Antisense nucleotide

<400>  281
cgaagccctc ggcgaacc                                                  18


<210>  282
<211>  24
<212>  DNA
<213>  Artificial

<220>
<223>  Antisense nucleotide

<400>  282
gcgtgttctg gctgtgcagt tcgg                                           24


<210>  283
<211>  14
<212>  DNA
<213>  Artificial

<220>
<223>  Antisense nucleotide

<400>  283
ctgccccgtt gacc                                                          14


<210>  284
<211>  14
<212>  DNA
<213>  Artificial

<220>
<223>  Antisense nucleotide

<400>  284
aggtttgcgt agac                                                          14


<210>  285
<211>  14
<212>  DNA
<213>  Artificial

<220>
<223>  Antisense nucleotide

<400>  285
ggttgaagtt gctg                                                          14


<210>  286
<211>  14
<212>  DNA
<213>  Artificial

<220>
<223>  Antisense nucleotide

<400>  286
ctgggttgaa gttg                                                          14


<210>  287
<211>  24
<212>  DNA
<213>  Artificial

<220>
<223>  Antisense nucleotide

<400>  287
tgctggggtt gcgcgggaaa ggcc                                               24


<210>  288
<211>  20
<212>  DNA
<213>  Artificial

<220>
<223>  Antisense nucleotide

<400>  288
tgctgcacgg gcatctgctg                                                    20

<210>  289
<211>  27
<212>  DNA
<213>  Artificial

<220>
<223>  Antisense nucleotide

<400>  289
ggcactgtct gaggctcctc cttcagg                                          27


<210>  290
<211>  14
<212>  DNA
<213>  Artificial

<220>
<223>  Antisense nucleotide

<400>  290
actccatgtc gatg                                                        14


<210>  291
<211>  16
<212>  DNA
<213>  Artificial

<220>
<223>  Antisense nucleotide

<400>  291
ctctccgcct tgatcc                                                      16


<210>  292
<211>  16
<212>  DNA
<213>  Artificial

<220>
<223>  Antisense nucleotide

<400>  292
gttcctcatg cgcttc                                                      16


<210>  293
<211>  14
<212>  DNA
<213>  Artificial

<220>
<223>  Antisense nucleotide

<400>  293
ctgagctttc aagg                                                        14


<210>  294
<211>  26
<212>  DNA
<213>  Artificial

<220>
<223>  Antisense nucleotide

<400>  294
gcgattctct ccagcttcct ttttcg                                          26


<210>  295
<211>  30
<212>  DNA
<213>  Artificial

<220>
<223>  Antisense nucleotide

<400>  295
ctgagctttc aaggttttca ctttttcctc                                      30


<210>  296
<211>  14
<212>  DNA
<213>  Artificial

<220>
<223>  Antisense nucleotide

<400>  296
tccctgagca tgtt                                                       14


<210>  297
<211>  16
<212>  DNA
<213>  Artificial

<220>
<223>  Antisense nucleotide

<400>  297
tctgtttaag ctgtgc                                                     16


<210>  298
<211>  18
<212>  DNA
<213>  Artificial

<220>
<223>  Antisense nucleotide

<400>  298
ctttctgttt aagctgtg                                                   18


<210>  299
<211>  16
<212>  DNA
<213>  Artificial

<220>
<223>  Antisense nucleotide

<400>  299
ggttcatgac tttctg                                                     16


<210>  300
<211>  14
<212>  DNA
<213>  Artificial

<220>
<223>  Antisense nucleotide

<400>  300
cgtggttcat gact                                                          14


<210>  301
<211>  16
<212>  DNA
<213>  Artificial

<220>
<223>  Antisense nucleotide

<400>  301
actgttaacg tggttc                                                        16


<210>  302
<211>  14
<212>  DNA
<213>  Artificial

<220>
<223>  Antisense nucleotide

<400>  302
ccactgttaa cgtg                                                          14


<210>  303
<211>  14
<212>  DNA
<213>  Artificial

<220>
<223>  Antisense nucleotide

<400>  303
cccactgtta acgt                                                          14


<210>  304
<211>  14
<212>  DNA
<213>  Artificial

<220>
<223>  Antisense nucleotide

<400>  304
agcatgagtt ggca                                                          14


<210>  305
<211>  14
<212>  DNA
<213>  Artificial

<220>
<223>  Antisense nucleotide

<400>  305
gcgttagcat gagt                                                          14

<210>  306
<211>  14
<212>  DNA
<213>  Artificial

<220>
<223>  Antisense nucleotide

<400>  306
gtttgcaact gctg                                                          14


<210>  307
<211>  18
<212>  DNA
<213>  Artificial

<220>
<223>  Antisense nucleotide

<400>  307
caaaatgttt gcaactgc                                                      18


<210>  308
<211>  14
<212>  DNA
<213>  Artificial

<220>
<223>  Antisense nucleotide

<400>  308
tcgtagaagg tcgt                                                          14


<210>  309
<211>  14
<212>  DNA
<213>  Artificial

<220>
<223>  Antisense nucleotide

<400>  309
agggttactg tagc                                                          14


<210>  310
<211>  14
<212>  DNA
<213>  Artificial

<220>
<223>  Antisense nucleotide

<400>  310
gtagtggtga tgtg                                                          14


<210>  311
<211>  14
<212>  DNA
<213>  Artificial

<220>
<223>  Antisense nucleotide

<400>  311
cgtcgtagaa ggtc                                                          14


<210>  312
<211>  14
<212>  DNA
<213>  Artificial

<220>
<223>  Antisense nucleotide

<400>  312
cgagaacatc atcg                                                          14


<210>  313
<211>  14
<212>  DNA
<213>  Artificial

<220>
<223>  Antisense nucleotide

<400>  313
gtagtctgcg ttga                                                          14


<210>  314
<211>  20
<212>  DNA
<213>  Artificial

<220>
<223>  Antisense nucleotide

<400>  314
gctgcagcgg gaggatgacg                                                    20


<210>  315
<211>  16
<212>  DNA
<213>  Artificial

<220>
<223>  Antisense nucleotide

<400>  315
agtaagagag gctatc                                                        16


<210>  316
<211>  14
<212>  DNA
<213>  Artificial

<220>
<223>  Antisense nucleotide

<400>  316
gtagtaagag aggc                                                          14


<210>  317
<211>  14
<212>  DNA
<213>  Artificial

<220>
<223>  Antisense nucleotide

<400>  317
ggtagtaaga gagg                                                          14


<210>  318
<211>  16
<212>  DNA
<213>  Artificial

<220>
<223>  Antisense nucleotide

<400>  318
gtgagtggta gtaaga                                                        16


<210>  319
<211>  18
<212>  DNA
<213>  Artificial

<220>
<223>  Antisense nucleotide

<400>  319
gtccgtgcag aagtcctg                                                      18


<210>  320
<211>  16
<212>  DNA
<213>  Artificial

<220>
<223>  Antisense nucleotide

<400>  320
gaatgaagtt ggcact                                                        16


<210>  321
<211>  14
<212>  DNA
<213>  Artificial

<220>
<223>  Antisense nucleotide

<400>  321
ggaatgaagt tggc                                                          14


<210>  322
<211>  14
<212>  DNA
<213>  Artificial

<220>
<223>  Antisense nucleotide

<400>  322
gggaatgaag ttgg                                                          14

<210>  323
<211>  30
<212>  DNA
<213>  Artificial

<220>
<223>  Antisense nucleotide

<400>  323
gctgcaccag ccactgcagg tccggactgg                                        30


<210>  324
<211>  30
<212>  DNA
<213>  Artificial

<220>
<223>  Antisense nucleotide

<400>  324
ctggtctgcg atggggccac agaggagacg                                        30


<210>  325
<211>  16
<212>  DNA
<213>  Artificial

<220>
<223>  Antisense nucleotide

<400>  325
tcatggtctt cacaac                                                       16


<210>  326
<211>  30
<212>  DNA
<213>  Artificial

<220>
<223>  Antisense nucleotide

<400>  326
caatgctctg cgctcggcct cctgtcatgg                                        30


<210>  327
<211>  14
<212>  DNA
<213>  Artificial

<220>
<223>  Antisense nucleotide

<400>  327
ctagagttcc tcac                                                         14


<210>  328
<211>  14
<212>  DNA
<213>  Artificial

<220>
<223>  Antisense nucleotide

<400>  328
gagtacgcta gagt                                                          14


<210>  329
<211>  14
<212>  DNA
<213>  Artificial

<220>
<223>  Antisense nucleotide

<400>  329
gaagagtacg ctag                                                          14


<210>  330
<211>  28
<212>  DNA
<213>  Artificial

<220>
<223>  Antisense nucleotide

<400>  330
ctgcttccca cccagccccc acattccc                                           28


<210>  331
<211>  20
<212>  DNA
<213>  Artificial

<220>
<223>  Antisense nucleotide

<400>  331
ttcatcctct gtactgggct                                                    20


<210>  332
<211>  14
<212>  DNA
<213>  Artificial

<220>
<223>  Antisense nucleotide

<400>  332
gttacggatg tgca                                                          14


<210>  333
<211>  14
<212>  DNA
<213>  Artificial

<220>
<223>  Antisense nucleotide

<400>  333
cagttacgga tgtg                                                          14


<210>  334
<211>  14
<212>  DNA
<213>  Artificial

<220>
<223>  Antisense nucleotide

<400>  334
ccagttacgg atgt                                                          14


<210>  335
<211>  14
<212>  DNA
<213>  Artificial

<220>
<223>  Antisense nucleotide

<400>  335
agagtctgag ttgg                                                          14


<210>  336
<211>  14
<212>  DNA
<213>  Artificial

<220>
<223>  Antisense nucleotide

<400>  336
gtgagactca gagt                                                          14


<210>  337
<211>  14
<212>  DNA
<213>  Artificial

<220>
<223>  Antisense nucleotide

<400>  337
tcttagggtg agac                                                          14


<210>  338
<211>  16
<212>  DNA
<213>  Artificial

<220>
<223>  Antisense nucleotide

<400>  338
gagagtactt cttagg                                                        16


<210>  339
<211>  18
<212>  DNA
<213>  Artificial

<220>
<223>  Antisense nucleotide

<400>  339
ggaagaaact atgagagt                                                      18

<210>  340
<211>  18
<212>  DNA
<213>  Artificial

<220>
<223>  Antisense nucleotide

<400>  340
cttagggaag aaactatg                                                    18


<210>  341
<211>  16
<212>  DNA
<213>  Artificial

<220>
<223>  Antisense nucleotide

<400>  341
cggtaagaaa cttagg                                                      16


<210>  342
<211>  14
<212>  DNA
<213>  Artificial

<220>
<223>  Antisense nucleotide

<400>  342
agcatgcggt aaga                                                        14


<210>  343
<211>  14
<212>  DNA
<213>  Artificial

<220>
<223>  Antisense nucleotide

<400>  343
gtctgaaagc atgc                                                        14


<210>  344
<211>  16
<212>  DNA
<213>  Artificial

<220>
<223>  Antisense nucleotide

<400>  344
agaacaaaga agagcc                                                      16


<210>  345
<211>  18
<212>  DNA
<213>  Artificial

<220>
<223>  Antisense nucleotide

<400>  345
caagagaaca aagaagag                                                    18


<210>  346
<211>  16
<212>  DNA
<213>  Artificial

<220>
<223>  Antisense nucleotide

<400>  346
cagcaagaga acaaag                                                      16


<210>  347
<211>  14
<212>  DNA
<213>  Artificial

<220>
<223>  Antisense nucleotide

<400>  347
tcctcagcaa gaga                                                        14


<210>  348
<211>  14
<212>  DNA
<213>  Artificial

<220>
<223>  Antisense nucleotide

<400>  348
aggtgtgact tgca                                                        14


<210>  349
<211>  16
<212>  DNA
<213>  Artificial

<220>
<223>  Antisense nucleotide

<400>  349
gaataggtgt gacttg                                                      16


<210>  350
<211>  16
<212>  DNA
<213>  Artificial

<220>
<223>  Antisense nucleotide

<400>  350
cagaataggt gtgact                                                      16


<210>  351
<211>  14
<212>  DNA
<213>  Artificial

<220>
<223>  Antisense nucleotide

<400>  351
gcagaatagg tgtg                                                          14


<210>  352
<211>  16
<212>  DNA
<213>  Artificial

<220>
<223>  Antisense nucleotide

<400>  352
cagttgcaga ataggt                                                        16


<210>  353
<211>  16
<212>  DNA
<213>  Artificial

<220>
<223>  Antisense nucleotide

<400>  353
gaaaccattt ctgacc                                                        16


<210>  354
<211>  18
<212>  DNA
<213>  Artificial

<220>
<223>  Antisense nucleotide

<400>  354
tgtgaaacca tttctgac                                                      18


<210>  355
<211>  18
<212>  DNA
<213>  Artificial

<220>
<223>  Antisense nucleotide

<400>  355
cactgtgaaa ccatttct                                                      18


<210>  356
<211>  14
<212>  DNA
<213>  Artificial

<220>
<223>  Antisense nucleotide

<400>  356
ccactgtgaa acca                                                          14

<210> 357
<211> 30
<212> DNA
<213> Artificial

<220>
<223> Antisense nucleotide

<400> 357
agaactggct cctgcagctt ccctgcttcc 30


<210> 358
<211> 15
<212> DNA
<213> Artificial

<220>
<223> Antisense nucleotide

<400> 358
cacctccatt caccc 15


<210> 359
<211> 16
<212> DNA
<213> Artificial

<220>
<223> Antisense nucleotide

<400> 359
cagtaaaagt gtctgc 16


<210> 360
<211> 18
<212> DNA
<213> Artificial

<220>
<223> Antisense nucleotide

<400> 360
cgacattcag taaaagtg 18


<210> 361
<211> 14
<212> DNA
<213> Artificial

<220>
<223> Antisense nucleotide

<400> 361
gaccgacatt cagt 14


<210> 362
<211> 18
<212> DNA
<213> Artificial

<220>
<223> Antisense nucleotide

<400>  362
cttctggaga taactaga                                                    18


<210>  363
<211>  18
<212>  DNA
<213>  Artificial

<220>
<223>  Antisense nucleotide

<400>  363
catcttattc ctttccct                                                    18


<210>  364
<211>  16
<212>  DNA
<213>  Artificial

<220>
<223>  Antisense nucleotide

<400>  364
cagccatctt attcct                                                      16


<210>  365
<211>  16
<212>  DNA
<213>  Artificial

<220>
<223>  Antisense nucleotide

<400>  365
tgcagccatc ttattc                                                      16


<210>  366
<211>  15
<212>  DNA
<213>  Artificial

<220>
<223>  Antisense nucleotide

<400>  366
gagtgtatca gtcag                                                       15


<210>  367
<211>  14
<212>  DNA
<213>  Artificial

<220>
<223>  Antisense nucleotide

<400>  367
ggagtgtatc agtc                                                        14


<210>  368
<211>  16
<212>  DNA
<213>  Artificial

<220>
<223>  Antisense nucleotide

<400>  368
cttggagtgt atcagt                                                       16


<210>  369
<211>  14
<212>  DNA
<213>  Artificial

<220>
<223>  Antisense nucleotide

<400>  369
acagagtacc tacc                                                         14


<210>  370
<211>  16
<212>  DNA
<213>  Artificial

<220>
<223>  Antisense nucleotide

<400>  370
ccaactttcc cttaag                                                       16


<210>  371
<211>  16
<212>  DNA
<213>  Artificial

<220>
<223>  Antisense nucleotide

<400>  371
ccttatgctc aatctc                                                       16


<210>  372
<211>  14
<212>  DNA
<213>  Artificial

<220>
<223>  Antisense nucleotide

<400>  372
gtcttactca aggg                                                         14


<210>  373
<211>  16
<212>  DNA
<213>  Artificial

<220>
<223>  Antisense nucleotide

<400>  373
acagtcttac tcaagg                                                       16

<210>  374
<211>  18
<212>  DNA
<213>  Artificial

<220>
<223>  Antisense nucleotide

<400>  374
cataagacac agtcttac                                                    18


<210>  375
<211>  18
<212>  DNA
<213>  Artificial

<220>
<223>  Antisense nucleotide

<400>  375
gaaagcataa gacacagt                                                    18


<210>  376
<211>  16
<212>  DNA
<213>  Artificial

<220>
<223>  Antisense nucleotide

<400>  376
ggaaagcata agacac                                                      16


<210>  377
<211>  16
<212>  DNA
<213>  Artificial

<220>
<223>  Antisense nucleotide

<400>  377
agggataaag gaaagc                                                      16


<210>  378
<211>  14
<212>  DNA
<213>  Artificial

<220>
<223>  Antisense nucleotide

<400>  378
cctgtataca gagg                                                        14


<210>  379
<211>  16
<212>  DNA
<213>  Artificial

<220>
<223>  Antisense nucleotide

<400>  379
tgtctcctgt atacag                                                        16


<210>  380
<211>  16
<212>  DNA
<213>  Artificial

<220>
<223>  Antisense nucleotide

<400>  380
catcttctag ttggtc                                                        16


<210>  381
<211>  16
<212>  DNA
<213>  Artificial

<220>
<223>  Antisense nucleotide

<400>  381
ctcatcttct agttgg                                                        16


<210>  382
<211>  18
<212>  DNA
<213>  Artificial

<220>
<223>  Antisense nucleotide

<400>  382
cttctcatct tctagttg                                                      18


<210>  383
<211>  16
<212>  DNA
<213>  Artificial

<220>
<223>  Antisense nucleotide

<400>  383
caaagcagac ttctca                                                        16


<210>  384
<211>  14
<212>  DNA
<213>  Artificial

<220>
<223>  Antisense nucleotide

<400>  384
ctgcaaagca gact                                                          14


<210>  385
<211>  18
<212>  DNA
<213>  Artificial

<220>
<223>  Antisense nucleotide

<400>  385
ctagtttttc cttctcct                                                    18


<210>  386
<211>  18
<212>  DNA
<213>  Artificial

<220>
<223>  Antisense nucleotide

<400>  386
tctagttttt ccttctcc                                                    18


<210>  387
<211>  16
<212>  DNA
<213>  Artificial

<220>
<223>  Antisense nucleotide

<400>  387
caggatgaac tctagt                                                      16


<210>  388
<211>  14
<212>  DNA
<213>  Artificial

<220>
<223>  Antisense nucleotide

<400>  388
cgagaacatc atgg                                                        14


<210>  389
<211>  14
<212>  DNA
<213>  Artificial

<220>
<223>  Antisense nucleotide

<400>  389
gtagtaggaa aggc                                                        14


<210>  390
<211>  14
<212>  DNA
<213>  Artificial

<220>
<223>  Antisense nucleotide

<400>  390
ggtagtagga aagg                                                        14

<210>  391
<211>  14
<212>  DNA
<213>  Artificial

<220>
<223>  Antisense nucleotide

<400>  391
ggaatggtag tagg                                                         14


<210>  392
<211>  15
<212>  DNA
<213>  Artificial

<220>
<223>  Antisense nucleotide

<400>  392
ggtcattgag aagag                                                        15


<210>  393
<211>  16
<212>  DNA
<213>  Artificial

<220>
<223>  Antisense nucleotide

<400>  393
gctaatgttc ttgacc                                                       16


<210>  394
<211>  15
<212>  DNA
<213>  Artificial

<220>
<223>  Antisense nucleotide

<400>  394
gtcaggaatc ggcag                                                        15


<210>  395
<211>  16
<212>  DNA
<213>  Artificial

<220>
<223>  Antisense nucleotide

<400>  395
cttggagaag acatac                                                       16


<210>  396
<211>  14
<212>  DNA
<213>  Artificial

<220>
<223>  Antisense nucleotide

<400>  396
tgcctcccca gaag                                                          14


<210>  397
<211>  17
<212>  DNA
<213>  Artificial

<220>
<223>  Antisense nucleotide

<400>  397
cactggcagt agaaatc                                                       17


<210>  398
<211>  15
<212>  DNA
<213>  Artificial

<220>
<223>  Antisense nucleotide

<400>  398
gctcactggc agtag                                                         15


<210>  399
<211>  14
<212>  DNA
<213>  Artificial

<220>
<223>  Antisense nucleotide

<400>  399
atggtcagga atcg                                                          14


<210>  400
<211>  16
<212>  DNA
<213>  Artificial

<220>
<223>  Antisense nucleotide

<400>  400
gaatggtcag gaatcg                                                        16


<210>  401
<211>  14
<212>  DNA
<213>  Artificial

<220>
<223>  Antisense nucleotide

<400>  401
catcgtggac tgtg                                                          14


<210>  402
<211>  14
<212>  DNA
<213>  Artificial

<220>
<223>  Antisense nucleotide

<400>  402
agccatggag atag                                                          14


<210>  403
<211>  15
<212>  DNA
<213>  Artificial

<220>
<223>  Antisense nucleotide

<400>  403
cagccatgga gatag                                                         15


<210>  404
<211>  16
<212>  DNA
<213>  Artificial

<220>
<223>  Antisense nucleotide

<400>  404
acagccatgg agatag                                                        16


<210>  405
<211>  17
<212>  DNA
<213>  Artificial

<220>
<223>  Antisense nucleotide

<400>  405
cacagccatg gagatag                                                       17


<210>  406
<211>  16
<212>  DNA
<213>  Artificial

<220>
<223>  Antisense nucleotide

<400>  406
ccacagccat ggagat                                                        16


<210>  407
<211>  14
<212>  DNA
<213>  Artificial

<220>
<223>  Antisense nucleotide

<400>  407
gccatggaga tagg                                                          14

<210>  408
<211>  15
<212>  DNA
<213>  Artificial

<220>
<223>  Antisense nucleotide

<400>  408
agccatggag atagg                                                        15


<210>  409
<211>  16
<212>  DNA
<213>  Artificial

<220>
<223>  Antisense nucleotide

<400>  409
cagccatgga gatagg                                                       16


<210>  410
<211>  17
<212>  DNA
<213>  Artificial

<220>
<223>  Antisense nucleotide

<400>  410
acagccatgg agatagg                                                      17


<210>  411
<211>  14
<212>  DNA
<213>  Artificial

<220>
<223>  Antisense nucleotide

<400>  411
catggagata gggt                                                         14


<210>  412
<211>  15
<212>  DNA
<213>  Artificial

<220>
<223>  Antisense nucleotide

<400>  412
catggagata gggtg                                                        15


<210>  413
<211>  16
<212>  DNA
<213>  Artificial

<220>
<223>  Antisense nucleotide

<400>  413
catggagata gggtgg                                                    16


<210>  414
<211>  14
<212>  DNA
<213>  Artificial

<220>
<223>  Antisense nucleotide

<400>  414
atggagatag ggtg                                                      14


<210>  415
<211>  15
<212>  DNA
<213>  Artificial

<220>
<223>  Antisense nucleotide

<400>  415
atggagatag ggtgg                                                     15


<210>  416
<211>  16
<212>  DNA
<213>  Artificial

<220>
<223>  Antisense nucleotide

<400>  416
atggagatag ggtggc                                                    16


<210>  417
<211>  17
<212>  DNA
<213>  Artificial

<220>
<223>  Antisense nucleotide

<400>  417
atggagatag ggtggct                                                   17


<210>  418
<211>  14
<212>  DNA
<213>  Artificial

<220>
<223>  Antisense nucleotide

<400>  418
ggagataggg tggc                                                      14


<210>  419
<211>  15
<212>  DNA
<213>  Artificial

<220>
<223>  Antisense nucleotide

<400>  419
ggagataggg tggct                                                        15


<210>  420
<211>  14
<212>  DNA
<213>  Artificial

<220>
<223>  Antisense nucleotide

<400>  420
gaaatagccc aggc                                                         14


<210>  421
<211>  15
<212>  DNA
<213>  Artificial

<220>
<223>  Antisense nucleotide

<400>  421
gaaatagccc aggcg                                                        15


<210>  422
<211>  17
<212>  DNA
<213>  Artificial

<220>
<223>  Antisense nucleotide

<400>  422
gaaatagccc aggcgag                                                      17


<210>  423
<211>  14
<212>  DNA
<213>  Artificial

<220>
<223>  Antisense nucleotide

<400>  423
ggaaatagcc cagg                                                         14


<210>  424
<211>  15
<212>  DNA
<213>  Artificial

<220>
<223>  Antisense nucleotide

<400>  424
ggaaatagcc caggc                                                        15

<210>  425
<211>  14
<212>  DNA
<213>  Artificial

<220>
<223>  Antisense nucleotide

<400>  425
gtcttcacat cgac                                                          14


<210>  426
<211>  15
<212>  DNA
<213>  Artificial

<220>
<223>  Antisense nucleotide

<400>  426
gtcttcacat cgact                                                         15


<210>  427
<211>  16
<212>  DNA
<213>  Artificial

<220>
<223>  Antisense nucleotide

<400>  427
gtcttcacat cgactt                                                        16


<210>  428
<211>  17
<212>  DNA
<213>  Artificial

<220>
<223>  Antisense nucleotide

<400>  428
gtcttcacat cgacttt                                                       17


<210>  429
<211>  18
<212>  DNA
<213>  Artificial

<220>
<223>  Antisense nucleotide

<400>  429
gtcttcacat cgactttg                                                      18


<210>  430
<211>  18
<212>  DNA
<213>  Artificial

<220>
<223>  Antisense nucleotide

<220>
<223>  Antisense nucleotide

<400>  436
catgaactgg tccatatcga                                                    20


<210>  437
<211>  20
<212>  DNA
<213>  Artificial

<220>
<223>  Antisense nucleotide

<400>  437
gaggttctaa atcttgggac                                                    20


<210>  438
<211>  20
<212>  DNA
<213>  Artificial

<220>
<223>  Antisense nucleotide

<400>  438
gcactctggc ttttgggttc                                                    20


<210>  439
<211>  20
<212>  DNA
<213>  Artificial

<220>
<223>  Antisense nucleotide

<400>  439
tagctcaatc cgttgttcag                                                    20


<210>  440
<211>  20
<212>  DNA
<213>  Artificial

<220>
<223>  Antisense nucleotide

<400>  440
ccctagatcc ctcttgaaat                                                    20


<210>  441
<211>  20
<212>  DNA
<213>  Artificial

<220>
<223>  Antisense nucleotide

<400>  441
accaaggctc tcttatgttt                                                    20

<400>  430
gtcttcacat cgactttg                                                    18


<210>  431
<211>  18
<212>  DNA
<213>  Artificial

<220>
<223>  Antisense nucleotide

<400>  431
ccatttgtct gtcttcac                                                    18


<210>  432
<211>  20
<212>  DNA
<213>  Artificial

<220>
<223>  Antisense nucleotide

<400>  432
gtgccatcaa tacctgcaaa                                                  20


<210>  433
<211>  20
<212>  DNA
<213>  Artificial

<220>
<223>  Antisense nucleotide

<400>  433
catcagttac atcgaaggag                                                  20


<210>  434
<211>  20
<212>  DNA
<213>  Artificial

<220>
<223>  Antisense nucleotide

<400>  434
tcttgggaca cgcagcaagg                                                  20


<210>  435
<211>  20
<212>  DNA
<213>  Artificial

<220>
<223>  Antisense nucleotide

<400>  435
gaaatcaatg taaagtggac                                                  20


<210>  436
<211>  20
<212>  DNA
<213>  Artificial

<210>  442
<211>  20
<212>  DNA
<213>  Artificial

<220>
<223>  Antisense nucleotide

<400>  442
tcgagtgtgc tgcaggtaga                                                    20


<210>  443
<211>  20
<212>  DNA
<213>  Artificial

<220>
<223>  Antisense nucleotide

<400>  443
tgaacagcat cagttacatc                                                    20


<210>  444
<211>  20
<212>  DNA
<213>  Artificial

<220>
<223>  Antisense nucleotide

<400>  444
gctgggttgg agatgttaaa                                                    20


<210>  445
<211>  20
<212>  DNA
<213>  Artificial

<220>
<223>  Antisense nucleotide

<400>  445
agaggttcta aatcttggga                                                    20


<210>  446
<211>  20
<212>  DNA
<213>  Artificial

<220>
<223>  Antisense nucleotide

<400>  446
cgccggttgg tctgttgtga                                                    20


<210>  447
<211>  20
<212>  DNA
<213>  Artificial

<220>
<223>  Antisense nucleotide

<400>  447
ctgctttcac caaattggaa                                                   20


<210>  448
<211>  20
<212>  DNA
<213>  Artificial

<220>
<223>  Antisense nucleotide

<400>  448
aagtatagat caaggagagt                                                   20


<210>  449
<211>  20
<212>  DNA
<213>  Artificial

<220>
<223>  Antisense nucleotide

<400>  449
tgctcaggat ctgcccgcgg                                                   20


<210>  450
<211>  20
<212>  DNA
<213>  Artificial

<220>
<223>  Antisense nucleotide

<400>  450
gtgctgttgt agatggaaat                                                   20


<210>  451
<211>  20
<212>  DNA
<213>  Artificial

<220>
<223>  Antisense nucleotide

<400>  451
agggcggcat gtctattttg                                                   20


<210>  452
<211>  20
<212>  DNA
<213>  Artificial

<220>
<223>  Antisense nucleotide

<400>  452
taagcttatt ttaaatccca                                                   20


<210>  453
<211>  20
<212>  DNA
<213>  Artificial

<220>
<223>  Antisense nucleotide

<400>  453
tagctgcatt tgcaagactt                                                    20


<210>  454
<211>  20
<212>  DNA
<213>  Artificial

<220>
<223>  Antisense nucleotide

<400>  454
tctgttgtga ctcaagtctg                                                    20


<210>  455
<211>  20
<212>  DNA
<213>  Artificial

<220>
<223>  Antisense nucleotide

<400>  455
aagcaatagg ccgcatccaa                                                    20


<210>  456
<211>  20
<212>  DNA
<213>  Artificial

<220>
<223>  Antisense nucleotide

<400>  456
tcaatgtaaa gtggacgtag                                                    20


<210>  457
<211>  20
<212>  DNA
<213>  Artificial

<220>
<223>  Antisense nucleotide

<400>  457
attttagctg catttgcaag                                                    20


<210>  458
<211>  20
<212>  DNA
<213>  Artificial

<220>
<223>  Antisense nucleotide

<400>  458
tgtagatgga aatcacctcc                                                    20

<210>  459
<211>  20
<212>  DNA
<213>  Artificial

<220>
<223>  Antisense nucleotide

<400>  459
ttaacactga tgaaccaagg                                                    20


<210>  460
<211>  20
<212>  DNA
<213>  Artificial

<220>
<223>  Antisense nucleotide

<400>  460
attgtaccct ttgggttcgt                                                    20


<210>  461
<211>  20
<212>  DNA
<213>  Artificial

<220>
<223>  Antisense nucleotide

<400>  461
agatccctct tgaaatcaat                                                    20


<210>  462
<211>  20
<212>  DNA
<213>  Artificial

<220>
<223>  Antisense nucleotide

<400>  462
tgtaaagtgg acgtaggcag                                                    20


<210>  463
<211>  20
<212>  DNA
<213>  Artificial

<220>
<223>  Antisense nucleotide

<400>  463
ccattcgcct tctgctcttg                                                    20


<210>  464
<211>  20
<212>  DNA
<213>  Artificial

<220>
<223>  Antisense nucleotide

<400>  464
tgttaaatct ttggacttga                                                   20


<210>  465
<211>  20
<212>  DNA
<213>  Artificial

<220>
<223>  Antisense nucleotide

<400>  465
gaagggcggc atgtctattt                                                   20


<210>  466
<211>  20
<212>  DNA
<213>  Artificial

<220>
<223>  Antisense nucleotide

<400>  466
gaccctgctg tgctgagtgt                                                   20


<210>  467
<211>  20
<212>  DNA
<213>  Artificial

<220>
<223>  Antisense nucleotide

<400>  467
gaactagtac cgccttttca                                                   20


<210>  468
<211>  20
<212>  DNA
<213>  Artificial

<220>
<223>  Antisense nucleotide

<400>  468
cgatcctctt gcgcatgaac                                                   20


<210>  469
<211>  20
<212>  DNA
<213>  Artificial

<220>
<223>  Antisense nucleotide

<400>  469
ccggccaaaa gggaagagat                                                   20


<210>  470
<211>  20
<212>  DNA
<213>  Artificial

<220>
<223>  Antisense nucleotide

<400>  470
aaagagacga gtggctatta                                                    20


<210>  471
<211>  20
<212>  DNA
<213>  Artificial

<220>
<223>  Antisense nucleotide

<400>  471
aagtggaaat attaatacgg                                                    20


<210>  472
<211>  20
<212>  DNA
<213>  Artificial

<220>
<223>  Antisense nucleotide

<400>  472
agatcaagga gagttgtttg                                                    20


<210>  473
<211>  20
<212>  DNA
<213>  Artificial

<220>
<223>  Antisense nucleotide

<400>  473
agttgttttt aaaagtcaga                                                    20


<210>  474
<211>  20
<212>  DNA
<213>  Artificial

<220>
<223>  Antisense nucleotide

<400>  474
tgtaacaact gggcagacag                                                    20


<210>  475
<211>  20
<212>  DNA
<213>  Artificial

<220>
<223>  Antisense nucleotide

<400>  475
ggtgttgtaa caactgggca                                                    20

<210>  476
<211>  20
<212>  DNA
<213>  Artificial

<220>
<223>  Antisense nucleotide

<400>  476
tacccacaga gcacctggga                                                    20


<210>  477
<211>  20
<212>  DNA
<213>  Artificial

<220>
<223>  Antisense nucleotide

<400>  477
gggatggcat caaggtaccc                                                    20


<210>  478
<211>  20
<212>  DNA
<213>  Artificial

<220>
<223>  Antisense nucleotide

<400>  478
tcgtcatcat cattatcatc                                                    20


<210>  479
<211>  20
<212>  DNA
<213>  Artificial

<220>
<223>  Antisense nucleotide

<400>  479
aagggtgcct attgcatagc                                                    20


<210>  480
<211>  20
<212>  DNA
<213>  Artificial

<220>
<223>  Antisense nucleotide

<400>  480
ctcactgtta actctaagag                                                    20


<210>  481
<211>  20
<212>  DNA
<213>  Artificial

<220>
<223>  Antisense nucleotide

<400>  481
gcaaagtatt tggtctccac                                                    20


<210>  482
<211>  20
<212>  DNA
<213>  Artificial

<220>
<223>  Antisense nucleotide

<400>  482
caagttcctt aagccatcca                                                    20


<210>  483
<211>  20
<212>  DNA
<213>  Artificial

<220>
<223>  Antisense nucleotide

<400>  483
ttatcttaat gcagactttc                                                    20


<210>  484
<211>  20
<212>  DNA
<213>  Artificial

<220>
<223>  Antisense nucleotide

<400>  484
cttacaagaa gcttccttag                                                    20


<210>  485
<211>  20
<212>  DNA
<213>  Artificial

<220>
<223>  Antisense nucleotide

<400>  485
actggtgagc ttcagcttgc                                                    20


<210>  486
<211>  20
<212>  DNA
<213>  Artificial

<220>
<223>  Antisense nucleotide

<400>  486
acttgagaat ctgatatagc                                                    20


<210>  487
<211>  20
<212>  DNA
<213>  Artificial

<220>
<223>  Antisense nucleotide

<400>  487
aggttcctgt ctttatggtg                                                    20


<210>  488
<211>  20
<212>  DNA
<213>  Artificial

<220>
<223>  Antisense nucleotide

<400>  488
gtgtatccat ttccacccta                                                    20


<210>  489
<211>  20
<212>  DNA
<213>  Artificial

<220>
<223>  Antisense nucleotide

<400>  489
cagcacagaa gttggcattg                                                    20


<210>  490
<211>  20
<212>  DNA
<213>  Artificial

<220>
<223>  Antisense nucleotide

<400>  490
gcaaggagaa gcagatgctt                                                    20


<210>  491
<211>  20
<212>  DNA
<213>  Artificial

<220>
<223>  Antisense nucleotide

<400>  491
agcaaggaga agcagatgct                                                    20


<210>  492
<211>  20
<212>  DNA
<213>  Artificial

<220>
<223>  Antisense nucleotide

<400>  492
ttttccaaga attttagctg                                                    20

<210>  493
<211>  20
<212>  DNA
<213>  Artificial

<220>
<223>  Antisense nucleotide

<400>  493
ttcttgttac aagcatcatc                                                    20


<210>  494
<211>  20
<212>  DNA
<213>  Artificial

<220>
<223>  Antisense nucleotide

<400>  494
ttaaagaagg agcggttcgg                                                    20


<210>  495
<211>  20
<212>  DNA
<213>  Artificial

<220>
<223>  Antisense nucleotide

<400>  495
ctgggctgaa atttatatat                                                    20


<210>  496
<211>  20
<212>  DNA
<213>  Artificial

<220>
<223>  Antisense nucleotide

<400>  496
gggcagacag ctaggagttt                                                    20


<210>  497
<211>  20
<212>  DNA
<213>  Artificial

<220>
<223>  Antisense nucleotide

<400>  497
gtgtactcac caaggtaccc                                                    20


<210>  498
<211>  20
<212>  DNA
<213>  Artificial

<220>
<223>  Antisense nucleotide

<400>  498
cccagcactt tgggaggccg                                                    20


<210>  499
<211>  20
<212>  DNA
<213>  Artificial

<220>
<223>  Antisense nucleotide

<400>  499
ggctcacgcc tgtaatccca                                                    20


<210>  500
<211>  20
<212>  DNA
<213>  Artificial

<220>
<223>  Antisense nucleotide

<400>  500
tgaccgtgaa ctcactattt                                                    20


<210>  501
<211>  20
<212>  DNA
<213>  Artificial

<220>
<223>  Antisense nucleotide

<400>  501
atagtggtga tggctataca                                                    20


<210>  502
<211>  20
<212>  DNA
<213>  Artificial

<220>
<223>  Antisense nucleotide

<400>  502
ttttggttac ctgcaaatct                                                    20


<210>  503
<211>  20
<212>  DNA
<213>  Artificial

<220>
<223>  Antisense nucleotide

<400>  503
gaacactcac cctgctgtgc                                                    20


<210>  504
<211>  20
<212>  DNA
<213>  Artificial

<220>
<223>  Antisense nucleotide

<400>  504
gaatggctct ttaaacccta                                                    20


<210>  505
<211>  20
<212>  DNA
<213>  Artificial

<220>
<223>  Antisense nucleotide

<400>  505
gaagaaatgg agttcagtgt                                                    20


<210>  506
<211>  20
<212>  DNA
<213>  Artificial

<220>
<223>  Antisense nucleotide

<400>  506
tttctcctgg aagggagagg                                                    20


<210>  507
<211>  20
<212>  DNA
<213>  Artificial

<220>
<223>  Antisense nucleotide

<400>  507
aaatgcaacg cgttcccaac                                                    20


<210>  508
<211>  20
<212>  DNA
<213>  Artificial

<220>
<223>  Antisense nucleotide

<400>  508
aatacgaaac ttttgcaaag                                                    20


<210>  509
<211>  20
<212>  DNA
<213>  Artificial

<220>
<223>  Antisense nucleotide

<400>  509
actagtaatt ctcagagcgg                                                    20

<210>  510
<211>  20
<212>  DNA
<213>  Artificial

<220>
<223>  Antisense nucleotide

<400>  510
aagaaactag taattctcag                                                    20


<210>  511
<211>  20
<212>  DNA
<213>  Artificial

<220>
<223>  Antisense nucleotide

<400>  511
agtgcatgtt tttaaaagga                                                    20


<210>  512
<211>  20
<212>  DNA
<213>  Artificial

<220>
<223>  Antisense nucleotide

<400>  512
cagtagtgca tgtttttaaa                                                    20


<210>  513
<211>  20
<212>  DNA
<213>  Artificial

<220>
<223>  Antisense nucleotide

<400>  513
ctcagcacac agtagtgcat                                                    20


<210>  514
<211>  20
<212>  DNA
<213>  Artificial

<220>
<223>  Antisense nucleotide

<400>  514
agatgcagga gcaaaaaggt                                                    20


<210>  515
<211>  20
<212>  DNA
<213>  Artificial

<220>
<223>  Antisense nucleotide

<400>  515
caggtagaca gactgagcgc                                                    20


<210>  516
<211>  20
<212>  DNA
<213>  Artificial

<220>
<223>  Antisense nucleotide

<400>  516
gcctcgatcc tcttgcgcat                                                    20


<210>  517
<211>  20
<212>  DNA
<213>  Artificial

<220>
<223>  Antisense nucleotide

<400>  517
gcggatggcc tcgatcctct                                                    20


<210>  518
<211>  20
<212>  DNA
<213>  Artificial

<220>
<223>  Antisense nucleotide

<400>  518
ctcaggatct gcccgcggat                                                    20


<210>  519
<211>  20
<212>  DNA
<213>  Artificial

<220>
<223>  Antisense nucleotide

<400>  519
gctccggata gtcttccggg                                                    20


<210>  520
<211>  20
<212>  DNA
<213>  Artificial

<220>
<223>  Antisense nucleotide

<400>  520
agatggaaat cacctccggg                                                    20


<210>  521
<211>  20
<212>  DNA
<213>  Artificial

<220>
<223>  Antisense nucleotide

<400>  521
gttgtagatg gaaatcacct                                                    20


<210>  522
<211>  20
<212>  DNA
<213>  Artificial

<220>
<223>  Antisense nucleotide

<400>  522
ctggtactgt tgtagatgga                                                    20


<210>  523
<211>  20
<212>  DNA
<213>  Artificial

<220>
<223>  Antisense nucleotide

<400>  523
aggcggctgc cctccggctt                                                    20


<210>  524
<211>  20
<212>  DNA
<213>  Artificial

<220>
<223>  Antisense nucleotide

<400>  524
aacctccttg gcgtagtact                                                    20


<210>  525
<211>  20
<212>  DNA
<213>  Artificial

<220>
<223>  Antisense nucleotide

<400>  525
attttataaa cctccttggc                                                    20


<210>  526
<211>  20
<212>  DNA
<213>  Artificial

<220>
<223>  Antisense nucleotide

<400>  526
cggcatgtcg attttataaa                                                    20

<210>  527
<211>  20
<212>  DNA
<213>  Artificial

<220>
<223>  Antisense nucleotide

<400>  527
cgggatggca ttttcggagg                                                    20


<210>  528
<211>  20
<212>  DNA
<213>  Artificial

<220>
<223>  Antisense nucleotide

<400>  528
gtagggtctg tagaaagtgg                                                    20


<210>  529
<211>  20
<212>  DNA
<213>  Artificial

<220>
<223>  Antisense nucleotide

<400>  529
tgaagtaggg tctgtagaaa                                                    20


<210>  530
<211>  20
<212>  DNA
<213>  Artificial

<220>
<223>  Antisense nucleotide

<400>  530
attctgaagt agggtctgta                                                    20


<210>  531
<211>  20
<212>  DNA
<213>  Artificial

<220>
<223>  Antisense nucleotide

<400>  531
aagcggacga ttctgaagta                                                    20


<210>  532
<211>  20
<212>  DNA
<213>  Artificial

<220>
<223>  Antisense nucleotide

<400>  532
cccaggttcc tgtctttgtg                                                   20


<210>  533
<211>  20
<212>  DNA
<213>  Artificial

<220>
<223>  Antisense nucleotide

<400>  533
ggcagtgtaa acttatttta                                                   20


<210>  534
<211>  20
<212>  DNA
<213>  Artificial

<220>
<223>  Antisense nucleotide

<400>  534
ccatcaatac ctgcaaatct                                                   20


<210>  535
<211>  20
<212>  DNA
<213>  Artificial

<220>
<223>  Antisense nucleotide

<400>  535
aggtgccatc aatacctgca                                                   20


<210>  536
<211>  20
<212>  DNA
<213>  Artificial

<220>
<223>  Antisense nucleotide

<400>  536
agttttctga tcaccactgg                                                   20


<210>  537
<211>  20
<212>  DNA
<213>  Artificial

<220>
<223>  Antisense nucleotide

<400>  537
ttatagtttt ctgatcacca                                                   20


<210>  538
<211>  20
<212>  DNA
<213>  Artificial

<220>
<223>  Antisense nucleotide

<400>  538
cctagtggac tttatagttt                                                    20


<210>  539
<211>  20
<212>  DNA
<213>  Artificial

<220>
<223>  Antisense nucleotide

<400>  539
acattagcag gagatgtggg                                                    20


<210>  540
<211>  20
<212>  DNA
<213>  Artificial

<220>
<223>  Antisense nucleotide

<400>  540
agggcaacaa cattagcagg                                                    20


<210>  541
<211>  20
<212>  DNA
<213>  Artificial

<220>
<223>  Antisense nucleotide

<400>  541
actccagtct gtaggagggc                                                    20


<210>  542
<211>  20
<212>  DNA
<213>  Artificial

<220>
<223>  Antisense nucleotide

<400>  542
tcctgcacat ttctaaagca                                                    20


<210>  543
<211>  20
<212>  DNA
<213>  Artificial

<220>
<223>  Antisense nucleotide

<400>  543
cagcaattat cctgcacatt                                                    20

<210>  544
<211>  20
<212>  DNA
<213>  Artificial

<220>
<223>  Antisense nucleotide

<400>  544
atgtaaagag ggcgaaggca                                                    20


<210>  545
<211>  20
<212>  DNA
<213>  Artificial

<220>
<223>  Antisense nucleotide

<400>  545
ctcttaaaat caatgtaaag                                                    20


<210>  546
<211>  20
<212>  DNA
<213>  Artificial

<220>
<223>  Antisense nucleotide

<400>  546
ccaagatccc tcttaaaatc                                                    20


<210>  547
<211>  20
<212>  DNA
<213>  Artificial

<220>
<223>  Antisense nucleotide

<400>  547
cctttgggtt catggatcca                                                    20


<210>  548
<211>  20
<212>  DNA
<213>  Artificial

<220>
<223>  Antisense nucleotide

<400>  548
gcattgtacc ctttgggttc                                                    20


<210>  549
<211>  20
<212>  DNA
<213>  Artificial

<220>
<223>  Antisense nucleotide

<400>  549
gcacagaagt tagcattgta                                                   20


<210>  550
<211>  20
<212>  DNA
<213>  Artificial

<220>
<223>  Antisense nucleotide

<400>  550
ctgaggactt tggtgtgttg                                                   20


<210>  551
<211>  20
<212>  DNA
<213>  Artificial

<220>
<223>  Antisense nucleotide

<400>  551
tcctgggaca cacagcaagg                                                   20


<210>  552
<211>  20
<212>  DNA
<213>  Artificial

<220>
<223>  Antisense nucleotide

<400>  552
tttagctgca tttacaagac                                                   20


<210>  553
<211>  20
<212>  DNA
<213>  Artificial

<220>
<223>  Antisense nucleotide

<400>  553
caaggacttt agctgcattt                                                   20


<210>  554
<211>  20
<212>  DNA
<213>  Artificial

<220>
<223>  Antisense nucleotide

<400>  554
gtcattgtca ccgtgatttt                                                   20


<210>  555
<211>  20
<212>  DNA
<213>  Artificial

<220>
<223>  Antisense nucleotide

<400>  555
ccagttttaa caaacagaac                                                    20


<210>  556
<211>  20
<212>  DNA
<213>  Artificial

<220>
<223>  Antisense nucleotide

<400>  556
agatgccagt tttaacaaac                                                    20


<210>  557
<211>  20
<212>  DNA
<213>  Artificial

<220>
<223>  Antisense nucleotide

<400>  557
gttcattata tagtaacaca                                                    20


<210>  558
<211>  20
<212>  DNA
<213>  Artificial

<220>
<223>  Antisense nucleotide

<400>  558
atgaaaggtt cattatatag                                                    20


<210>  559
<211>  20
<212>  DNA
<213>  Artificial

<220>
<223>  Antisense nucleotide

<400>  559
ttccaagggt aatgaaaggt                                                    20


<210>  560
<211>  20
<212>  DNA
<213>  Artificial

<220>
<223>  Antisense nucleotide

<400>  560
cttaagccat ccatgagttt                                                    20

<210>  561
<211>  20
<212>  DNA
<213>  Artificial

<220>
<223>  Antisense nucleotide

<400>  561
cctggcttat ttgagttcaa                                                    20


<210>  562
<211>  20
<212>  DNA
<213>  Artificial

<220>
<223>  Antisense nucleotide

<400>  562
ttagtcctat aacaactcac                                                    20


<210>  563
<211>  20
<212>  DNA
<213>  Artificial

<220>
<223>  Antisense nucleotide

<400>  563
gcaaagaacc atttacaatt                                                    20


<210>  564
<211>  20
<212>  DNA
<213>  Artificial

<220>
<223>  Antisense nucleotide

<400>  564
cttgcttaaa ctggcaaaga                                                    20


<210>  565
<211>  20
<212>  DNA
<213>  Artificial

<220>
<223>  Antisense nucleotide

<400>  565
acatgtaaag tagttactgt                                                    20


<210>  566
<211>  20
<212>  DNA
<213>  Artificial

<220>
<223>  Antisense nucleotide

<400>  566
acacattaca tgtaaagtag                                                    20


<210>  567
<211>  20
<212>  DNA
<213>  Artificial

<220>
<223>  Antisense nucleotide

<400>  567
taagatctac acattacatg                                                    20


<210>  568
<211>  20
<212>  DNA
<213>  Artificial

<220>
<223>  Antisense nucleotide

<400>  568
attcaaaggt actggccagc                                                    20


<210>  569
<211>  20
<212>  DNA
<213>  Artificial

<220>
<223>  Antisense nucleotide

<400>  569
tttgtagtgc aagtcaaaat                                                    20


<210>  570
<211>  20
<212>  DNA
<213>  Artificial

<220>
<223>  Antisense nucleotide

<400>  570
catgtcatta aatggacaat                                                    20


<210>  571
<211>  20
<212>  DNA
<213>  Artificial

<220>
<223>  Antisense nucleotide

<400>  571
cctacatttg tgcgaacttc                                                    20


<210>  572
<211>  20
<212>  DNA
<213>  Artificial

<220>
<223>  Antisense nucleotide

<400>  572
   ttcccccttt gaaaaactca                                                    20


<210>  573
<211>  20
<212>  DNA
<213>  Artificial

<220>
<223>  Antisense nucleotide

<400>  573
tttttaatca gcctgcaaag                                                   20


<210>  574
<211>  20
<212>  DNA
<213>  Artificial

<220>
<223>  Antisense nucleotide

<400>  574
actgggcaga cagtttcgga                                                   20


<210>  575
<211>  20
<212>  DNA
<213>  Artificial

<220>
<223>  Antisense nucleotide

<400>  575
taacaactgg gcagacagtt                                                   20


<210>  576
<211>  20
<212>  DNA
<213>  Artificial

<220>
<223>  Antisense nucleotide

<400>  576
tgttgtaaca actgggcaga                                                   20


<210>  577
<211>  20
<212>  DNA
<213>  Artificial

<220>
<223>  Antisense nucleotide

<400>  577
cacagagcac ctgggactgt                                                   20

<210>  578
<211>  20
<212>  DNA
<213>  Artificial

<220>
<223>  Antisense nucleotide

<400>  578
gtacccacag agcacctggg                                                    20


<210>  579
<211>  20
<212>  DNA
<213>  Artificial

<220>
<223>  Antisense nucleotide

<400>  579
tcaaggtacc cacagagcac                                                    20


<210>  580
<211>  20
<212>  DNA
<213>  Artificial

<220>
<223>  Antisense nucleotide

<400>  580
tggcatcaag gtacccacag                                                    20


<210>  581
<211>  20
<212>  DNA
<213>  Artificial

<220>
<223>  Antisense nucleotide

<400>  581
ggcgggatgg catcaaggta                                                    20


<210>  582
<211>  20
<212>  DNA
<213>  Artificial

<220>
<223>  Antisense nucleotide

<400>  582
tttgcaggta ttgatggcac                                                    20


<210>  583
<211>  20
<212>  DNA
<213>  Artificial

<220>
<223>  Antisense nucleotide

<400>  583
ctccttcgat gtaactgatg                                                    20


<210>  584
<211>  20
<212>  DNA
<213>  Artificial

<220>
<223>  Antisense nucleotide

<400>  584
ccttgctgcg tgtcccaaga                                                    20


<210>  585
<211>  20
<212>  DNA
<213>  Artificial

<220>
<223>  Antisense nucleotide

<400>  585
tcgatatgga ccagttcatg                                                    20


<210>  586
<211>  20
<212>  DNA
<213>  Artificial

<220>
<223>  Antisense nucleotide

<400>  586
gtcccaagat ttagaacctc                                                    20


<210>  587
<211>  20
<212>  DNA
<213>  Artificial

<220>
<223>  Antisense nucleotide

<400>  587
gaacccaaaa gccagagtgc                                                    20


<210>  588
<211>  20
<212>  DNA
<213>  Artificial

<220>
<223>  Antisense nucleotide

<400>  588
atttcaagag ggatctaggg                                                    20


<210>  589
<211>  20
<212>  DNA
<213>  Artificial

<220>
<223>  Antisense nucleotide

<400>  589
aaacataaga gagccttggt                                                    20


<210>  590
<211>  20
<212>  DNA
<213>  Artificial

<220>
<223>  Antisense nucleotide

<400>  590
tctacctgca gcacactcga                                                    20


<210>  591
<211>  20
<212>  DNA
<213>  Artificial

<220>
<223>  Antisense nucleotide

<400>  591
tcacaacaga ccaaccggcg                                                    20


<210>  592
<211>  20
<212>  DNA
<213>  Artificial

<220>
<223>  Antisense nucleotide

<400>  592
ttccaatttg gtgaaagcag                                                    20


<210>  593
<211>  20
<212>  DNA
<213>  Artificial

<220>
<223>  Antisense nucleotide

<400>  593
actctccttg atctatactt                                                    20


<210>  594
<211>  20
<212>  DNA
<213>  Artificial

<220>
<223>  Antisense nucleotide

<400>  594
ccgcgggcag atcctgagca                                                    20

<210>  595
<211>  20
<212>  DNA
<213>  Artificial

<220>
<223>  Antisense nucleotide

<400>  595
caaaatagac atgccgccct                                                    20


<210>  596
<211>  20
<212>  DNA
<213>  Artificial

<220>
<223>  Antisense nucleotide

<400>  596
tgggatttaa aataagctta                                                    20


<210>  597
<211>  20
<212>  DNA
<213>  Artificial

<220>
<223>  Antisense nucleotide

<400>  597
cagacttgag tcacaacaga                                                    20


<210>  598
<211>  20
<212>  DNA
<213>  Artificial

<220>
<223>  Antisense nucleotide

<400>  598
ttggatgcgg cctattgctt                                                    20


<210>  599
<211>  20
<212>  DNA
<213>  Artificial

<220>
<223>  Antisense nucleotide

<400>  599
ctacgtccac tttacattga                                                    20


<210>  600
<211>  20
<212>  DNA
<213>  Artificial

<220>
<223>  Antisense nucleotide

<400>  600
ggaggtgatt tccatctaca                                                   20


<210>  601
<211>  20
<212>  DNA
<213>  Artificial

<220>
<223>  Antisense nucleotide

<400>  601
ccttggttca tcagtgttaa                                                   20


<210>  602
<211>  20
<212>  DNA
<213>  Artificial

<220>
<223>  Antisense nucleotide

<400>  602
acgaacccaa agggtacaat                                                   20


<210>  603
<211>  20
<212>  DNA
<213>  Artificial

<220>
<223>  Antisense nucleotide

<400>  603
attgatttca agagggatct                                                   20


<210>  604
<211>  20
<212>  DNA
<213>  Artificial

<220>
<223>  Antisense nucleotide

<400>  604
ctgcctacgt ccactttaca                                                   20


<210>  605
<211>  20
<212>  DNA
<213>  Artificial

<220>
<223>  Antisense nucleotide

<400>  605
caagagcaga aggcgaatgg                                                   20


<210>  606
<211>  20
<212>  DNA
<213>  Artificial

<220>
<223>  Antisense nucleotide

<400>  606
tcaagtccaa agatttaaca                                                    20


<210>  607
<211>  20
<212>  DNA
<213>  Artificial

<220>
<223>  Antisense nucleotide

<400>  607
aaatagacat gccgcccttc                                                    20


<210>  608
<211>  20
<212>  DNA
<213>  Artificial

<220>
<223>  Antisense nucleotide

<400>  608
acactcagca cagcagggtc                                                    20


<210>  609
<211>  20
<212>  DNA
<213>  Artificial

<220>
<223>  Antisense nucleotide

<400>  609
tgaaaaggcg gtactagttc                                                    20


<210>  610
<211>  20
<212>  DNA
<213>  Artificial

<220>
<223>  Antisense nucleotide

<400>  610
gttcatgcgc aagaggatcg                                                    20


<210>  611
<211>  20
<212>  DNA
<213>  Artificial

<220>
<223>  Antisense nucleotide

<400>  611
atctcttccc ttttggccgg                                                    20

<210>  612
<211>  20
<212>  DNA
<213>  Artificial

<220>
<223>  Antisense nucleotide

<400>  612
taatagccac tcgtctcttt                                                    20


<210>  613
<211>  20
<212>  DNA
<213>  Artificial

<220>
<223>  Antisense nucleotide

<400>  613
ccgtattaat atttccactt                                                    20


<210>  614
<211>  20
<212>  DNA
<213>  Artificial

<220>
<223>  Antisense nucleotide

<400>  614
caaacaactc tccttgatct                                                    20


<210>  615
<211>  20
<212>  DNA
<213>  Artificial

<220>
<223>  Antisense nucleotide

<400>  615
ctgtctgccc agttgttaca                                                    20


<210>  616
<211>  20
<212>  DNA
<213>  Artificial

<220>
<223>  Antisense nucleotide

<400>  616
tgcccagttg ttacaacacc                                                    20


<210>  617
<211>  20
<212>  DNA
<213>  Artificial

<220>
<223>  Antisense nucleotide

<400>  617
tcccaggtgc tctgtgggta                                                    20


<210>  618
<211>  20
<212>  DNA
<213>  Artificial

<220>
<223>  Antisense nucleotide

<400>  618
gatgataatg atgatgacga                                                    20


<210>  619
<211>  20
<212>  DNA
<213>  Artificial

<220>
<223>  Antisense nucleotide

<400>  619
gctatgcaat aggcaccctt                                                    20


<210>  620
<211>  20
<212>  DNA
<213>  Artificial

<220>
<223>  Antisense nucleotide

<400>  620
ctcttagagt taacagtgag                                                    20


<210>  621
<211>  20
<212>  DNA
<213>  Artificial

<220>
<223>  Antisense nucleotide

<400>  621
gtggagacca aatactttgc                                                    20


<210>  622
<211>  20
<212>  DNA
<213>  Artificial

<220>
<223>  Antisense nucleotide

<400>  622
gaaagtctgc attaagataa                                                    20


<210>  623
<211>  20
<212>  DNA
<213>  Artificial

<220>
<223>  Antisense nucleotide

<400>  623
ctaaggaagc ttcttgtaag                                                    20


<210>  624
<211>  20
<212>  DNA
<213>  Artificial

<220>
<223>  Antisense nucleotide

<400>  624
gcaagctgaa gctcaccagt                                                    20


<210>  625
<211>  20
<212>  DNA
<213>  Artificial

<220>
<223>  Antisense nucleotide

<400>  625
tagggtggaa atggatacac                                                    20


<210>  626
<211>  20
<212>  DNA
<213>  Artificial

<220>
<223>  Antisense nucleotide

<400>  626
caatgccaac ttctgtgctg                                                    20


<210>  627
<211>  20
<212>  DNA
<213>  Artificial

<220>
<223>  Antisense nucleotide

<400>  627
aagcatctgc ttctccttgc                                                    20


<210>  628
<211>  20
<212>  DNA
<213>  Artificial

<220>
<223>  Antisense nucleotide

<400>  628
cagctaaaat tcttggaaaa                                                    20

<210> 629
<211> 20
<212> DNA
<213> Artificial

<220>
<223> Antisense nucleotide

<400> 629
gatgatgctt gtaacaagaa 20


<210> 630
<211> 20
<212> DNA
<213> Artificial

<220>
<223> Antisense nucleotide

<400> 630
aaactcctag ctgtctgccc 20


<210> 631
<211> 20
<212> DNA
<213> Artificial

<220>
<223> Antisense nucleotide

<400> 631
gggtaccttg gtgagtacac 20


<210> 632
<211> 20
<212> DNA
<213> Artificial

<220>
<223> Antisense nucleotide

<400> 632
cggcctccca aagtgctggg 20


<210> 633
<211> 20
<212> DNA
<213> Artificial

<220>
<223> Antisense nucleotide

<400> 633
tgggattaca ggcgtgagcc 20


<210> 634
<211> 20
<212> DNA
<213> Artificial

<220>
<223> Antisense nucleotide

<400>  634
tgtatagcca tcaccactat                                                    20


<210>  635
<211>  20
<212>  DNA
<213>  Artificial

<220>
<223>  Antisense nucleotide

<400>  635
acactgaact ccatttcttc                                                    20


<210>  636
<211>  20
<212>  DNA
<213>  Artificial

<220>
<223>  Antisense nucleotide

<400>  636
cctctccctt ccaggagaaa                                                    20


<210>  637
<211>  20
<212>  DNA
<213>  Artificial

<220>
<223>  Antisense nucleotide

<400>  637
gttgggaacg cgttgcattt                                                    20


<210>  638
<211>  20
<212>  DNA
<213>  Artificial

<220>
<223>  Antisense nucleotide

<400>  638
ccgctctgag aattactagt                                                    20


<210>  639
<211>  20
<212>  DNA
<213>  Artificial

<220>
<223>  Antisense nucleotide

<400>  639
ctgagaatta ctagtttctt                                                    20


<210>  640
<211>  20
<212>  DNA
<213>  Artificial

<220>
<223>  Antisense nucleotide

<400>  640
tttaaaaaca tgcactactg                                                    20


<210>  641
<211>  20
<212>  DNA
<213>  Artificial

<220>
<223>  Antisense nucleotide

<400>  641
atgcactact gtgtgctgag                                                    20


<210>  642
<211>  20
<212>  DNA
<213>  Artificial

<220>
<223>  Antisense nucleotide

<400>  642
gcgctcagtc tgtctacctg                                                    20


<210>  643
<211>  20
<212>  DNA
<213>  Artificial

<220>
<223>  Antisense nucleotide

<400>  643
atgcgcaaga ggatcgaggc                                                    20


<210>  644
<211>  20
<212>  DNA
<213>  Artificial

<220>
<223>  Antisense nucleotide

<400>  644
agaggatcga ggccatccgc                                                    20


<210>  645
<211>  20
<212>  DNA
<213>  Artificial

<220>
<223>  Antisense nucleotide

<400>  645
atccgcgggc agatcctgag                                                    20

<210>  646
<211>  20
<212>  DNA
<213>  Artificial

<220>
<223>  Antisense nucleotide

<400>  646
cccggaagac tatccggagc                                                    20


<210>  647
<211>  20
<212>  DNA
<213>  Artificial

<220>
<223>  Antisense nucleotide

<400>  647
cccggaggtg atttccatct                                                    20


<210>  648
<211>  20
<212>  DNA
<213>  Artificial

<220>
<223>  Antisense nucleotide

<400>  648
aggtgatttc catctacaac                                                    20


<210>  649
<211>  20
<212>  DNA
<213>  Artificial

<220>
<223>  Antisense nucleotide

<400>  649
tccatctaca acagtaccag                                                    20


<210>  650
<211>  20
<212>  DNA
<213>  Artificial

<220>
<223>  Antisense nucleotide

<400>  650
aagccggagg gcagccgcct                                                    20


<210>  651
<211>  20
<212>  DNA
<213>  Artificial

<220>
<223>  Antisense nucleotide

<400>  651
agtactacgc caaggaggtt                                                   20


<210>  652
<211>  20
<212>  DNA
<213>  Artificial

<220>
<223>  Antisense nucleotide

<400>  652
gccaaggagg tttataaaat                                                   20


<210>  653
<211>  20
<212>  DNA
<213>  Artificial

<220>
<223>  Antisense nucleotide

<400>  653
tttataaaat cgacatgccg                                                   20


<210>  654
<211>  20
<212>  DNA
<213>  Artificial

<220>
<223>  Antisense nucleotide

<400>  654
cctccgaaaa tgccatcccg                                                   20


<210>  655
<211>  20
<212>  DNA
<213>  Artificial

<220>
<223>  Antisense nucleotide

<400>  655
ccactttcta cagaccctac                                                   20


<210>  656
<211>  20
<212>  DNA
<213>  Artificial

<220>
<223>  Antisense nucleotide

<400>  656
tttctacaga ccctacttca                                                   20


<210>  657
<211>  20
<212>  DNA
<213>  Artificial

<220>
<223>  Antisense nucleotide

<400>  657
tacagaccct acttcagaat                                                    20


<210>  658
<211>  20
<212>  DNA
<213>  Artificial

<220>
<223>  Antisense nucleotide

<400>  658
tacttcagaa tcgtccgctt                                                    20


<210>  659
<211>  20
<212>  DNA
<213>  Artificial

<220>
<223>  Antisense nucleotide

<400>  659
cacaaagaca ggaacctggg                                                    20


<210>  660
<211>  20
<212>  DNA
<213>  Artificial

<220>
<223>  Antisense nucleotide

<400>  660
taaaataagt ttacactgcc                                                    20


<210>  661
<211>  20
<212>  DNA
<213>  Artificial

<220>
<223>  Antisense nucleotide

<400>  661
tgcaggtatt gatggcacct                                                    20


<210>  662
<211>  20
<212>  DNA
<213>  Artificial

<220>
<223>  Antisense nucleotide

<400>  662
ccagtggtga tcagaaaact                                                    20

<210>  663
<211>  20
<212>  DNA
<213>  Artificial

<220>
<223>  Antisense nucleotide

<400>  663
tggtgatcag aaaactataa                                                    20


<210>  664
<211>  20
<212>  DNA
<213>  Artificial

<220>
<223>  Antisense nucleotide

<400>  664
cctgctaatg ttgttgccct                                                    20


<210>  665
<211>  20
<212>  DNA
<213>  Artificial

<220>
<223>  Antisense nucleotide

<400>  665
gccctcctac agactggagt                                                    20


<210>  666
<211>  20
<212>  DNA
<213>  Artificial

<220>
<223>  Antisense nucleotide

<400>  666
tgctttagaa atgtgcagga                                                    20


<210>  667
<211>  20
<212>  DNA
<213>  Artificial

<220>
<223>  Antisense nucleotide

<400>  667
tgccttcgcc ctctttacat                                                    20


<210>  668
<211>  20
<212>  DNA
<213>  Artificial

<220>
<223>  Antisense nucleotide

<400>  668
gattttaaga gggatcttgg                                                    20


<210>  669
<211>  20
<212>  DNA
<213>  Artificial

<220>
<223>  Antisense nucleotide

<400>  669
tggatccatg aacccaaagg                                                    20


<210>  670
<211>  20
<212>  DNA
<213>  Artificial

<220>
<223>  Antisense nucleotide

<400>  670
gaacccaaag ggtacaatgc                                                    20


<210>  671
<211>  20
<212>  DNA
<213>  Artificial

<220>
<223>  Antisense nucleotide

<400>  671
tacaatgcta acttctgtgc                                                    20


<210>  672
<211>  20
<212>  DNA
<213>  Artificial

<220>
<223>  Antisense nucleotide

<400>  672
ccttgctgtg tgtcccagga                                                    20


<210>  673
<211>  20
<212>  DNA
<213>  Artificial

<220>
<223>  Antisense nucleotide

<400>  673
gtcttgtaaa tgcagctaaa                                                    20


<210>  674
<211>  20
<212>  DNA
<213>  Artificial

<220>
<223>  Antisense nucleotide

<400>  674
aaatgcagct aaagtccttg                                                    20


<210>  675
<211>  20
<212>  DNA
<213>  Artificial

<220>
<223>  Antisense nucleotide

<400>  675
aaaatcacgg tgacaatgac                                                    20


<210>  676
<211>  20
<212>  DNA
<213>  Artificial

<220>
<223>  Antisense nucleotide

<400>  676
gttctgtttg ttaaaactgg                                                    20


<210>  677
<211>  20
<212>  DNA
<213>  Artificial

<220>
<223>  Antisense nucleotide

<400>  677
gtttgttaaa actggcatct                                                    20


<210>  678
<211>  20
<212>  DNA
<213>  Artificial

<220>
<223>  Antisense nucleotide

<400>  678
tgtgttacta tataatgaac                                                    20


<210>  679
<211>  20
<212>  DNA
<213>  Artificial

<220>
<223>  Antisense nucleotide

<400>  679
acctttcatt acccttggaa                                                    20

<210>  680
<211>  20
<212>  DNA
<213>  Artificial

<220>
<223>  Antisense nucleotide

<400>  680
aaactcatgg atggcttaag                                                    20


<210>  681
<211>  20
<212>  DNA
<213>  Artificial

<220>
<223>  Antisense nucleotide

<400>  681
aattgtaaat ggttctttgc                                                    20


<210>  682
<211>  20
<212>  DNA
<213>  Artificial

<220>
<223>  Antisense nucleotide

<400>  682
tctttgccag tttaagcaag                                                    20


<210>  683
<211>  20
<212>  DNA
<213>  Artificial

<220>
<223>  Antisense nucleotide

<400>  683
acagtaacta ctttacatgt                                                    20


<210>  684
<211>  20
<212>  DNA
<213>  Artificial

<220>
<223>  Antisense nucleotide

<400>  684
ctactttaca tgtaatgtgt                                                    20


<210>  685
<211>  20
<212>  DNA
<213>  Artificial

<220>
<223>  Antisense nucleotide

<400>  685
catgtaatgt gtagatctta                                                    20


<210>  686
<211>  20
<212>  DNA
<213>  Artificial

<220>
<223>  Antisense nucleotide

<400>  686
gctggccagt acctttgaat                                                    20


<210>  687
<211>  20
<212>  DNA
<213>  Artificial

<220>
<223>  Antisense nucleotide

<400>  687
ctttgcaggc tgattaaaaa                                                    20


<210>  688
<211>  20
<212>  DNA
<213>  Artificial

<220>
<223>  Antisense nucleotide

<400>  688
aactgtctgc ccagttgtta                                                    20


<210>  689
<211>  20
<212>  DNA
<213>  Artificial

<220>
<223>  Antisense nucleotide

<400>  689
tctgcccagt tgttacaaca                                                    20


<210>  690
<211>  20
<212>  DNA
<213>  Artificial

<220>
<223>  Antisense nucleotide

<400>  690
acagtcccag gtgctctgtg                                                    20


<210>  691
<211>  20
<212>  DNA
<213>  Artificial

<220>
<223>  Antisense nucleotide

<400>  691
cccaggtgct ctgtgggtac                                                    20


<210>  692
<211>  20
<212>  DNA
<213>  Artificial

<220>
<223>  Antisense nucleotide

<400>  692
gtgctctgtg ggtaccttga                                                    20


<210>  693
<211>  20
<212>  DNA
<213>  Artificial

<220>
<223>  Antisense nucleotide

<400>  693
ctgtgggtac cttgatgcca                                                    20


<210>  694
<211>  20
<212>  DNA
<213>  Artificial

<220>
<223>  Antisense nucleotide

<400>  694
taccttgatg ccatcccgcc                                                    20


<210>  695
<211>  20
<212>  DNA
<213>  Artificial

<220>
<223>  Antisense nucleotide

<400>  695
ttccaccatt agcacgcggg                                                    20


<210>  696
<211>  20
<212>  DNA
<213>  Artificial

<220>
<223>  Antisense nucleotide

<400>  696
ccgtgaccag atgcaggatc                                                    20

<210>  697
<211>  21
<212>  DNA
<213>  Artificial

<220>
<223>  Antisense nucleotide

<400>  697
cccggagggc ggcatggggg a                                                21


<210>  698
<211>  18
<212>  DNA
<213>  Artificial

<220>
<223>  Antisense nucleotide

<400>  698
cctcagggag aagggcgc                                                    18


<210>  699
<211>  18
<212>  DNA
<213>  Artificial

<220>
<223>  Antisense nucleotide

<400>  699
gtaggagggc ctcgaggg                                                    18


<210>  700
<211>  18
<212>  DNA
<213>  Artificial

<220>
<223>  Antisense nucleotide

<400>  700
ctgcaggggc tgggggtc                                                    18


<210>  701
<211>  18
<212>  DNA
<213>  Artificial

<220>
<223>  Antisense nucleotide

<400>  701
agggctggtg tggtgggg                                                    18


<210>  702
<211>  18
<212>  DNA
<213>  Artificial

<220>
<223>  Antisense nucleotide

<400>  702
ggcatggggg aggcggcg                                                    18


<210>  703
<211>  18
<212>  DNA
<213>  Artificial

<220>
<223>  Antisense nucleotide

<400>  703
ccggagggcg gcatgggg                                                    18


<210>  704
<211>  18
<212>  DNA
<213>  Artificial

<220>
<223>  Antisense nucleotide

<400>  704
ggggggctgg cgagccgc                                                    18


<210>  705
<211>  23
<212>  DNA
<213>  Artificial

<220>
<223>  Antisense nucleotide

<400>  705
ggacaggatc tggccgcgga tgg                                              23


<210>  706
<211>  18
<212>  DNA
<213>  Artificial

<220>
<223>  Antisense nucleotide

<400>  706
ccccctggct cggggggc                                                    18


<210>  707
<211>  18
<212>  DNA
<213>  Artificial

<220>
<223>  Antisense nucleotide

<400>  707
gggccgggcg gcacctcc                                                    18


<210>  708
<211>  18
<212>  DNA
<213>  Artificial

<220>
<223>  Antisense nucleotide

<400>  708
gggcagcggg ccgggcgg                                                    18


<210>  709
<211>  18
<212>  DNA
<213>  Artificial

<220>
<223>  Antisense nucleotide

<400>  709
acggcctcgg gcagcggg                                                    18


<210>  710
<211>  18
<212>  DNA
<213>  Artificial

<220>
<223>  Antisense nucleotide

<400>  710
gggtgctgtt gtacaggg                                                    18


<210>  711
<211>  24
<212>  DNA
<213>  Artificial

<220>
<223>  Antisense nucleotide

<400>  711
gggtttccac cattagcacg cggg                                             24


<210>  712
<211>  14
<212>  DNA
<213>  Artificial

<220>
<223>  Antisense nucleotide

<400>  712
tcatagattt cgtt                                                        14


<210>  713
<211>  13
<212>  DNA
<213>  Artificial

<220>
<223>  Antisense nucleotide

<400>  713
ttgtcataga ttt                                                         13

<210>  714
<211>  15
<212>  DNA
<213>  Artificial

<220>
<223>  Antisense nucleotide

<400>  714
aagaacatat atatg                                                        15


<210>  715
<211>  14
<212>  DNA
<213>  Artificial

<220>
<223>  Antisense nucleotide

<400>  715
aagaacatat atat                                                         14


<210>  716
<211>  16
<212>  DNA
<213>  Artificial

<220>
<223>  Antisense nucleotide

<400>  716
ttgaagaaca tatata                                                       16


<210>  717
<211>  18
<212>  DNA
<213>  Artificial

<220>
<223>  Antisense nucleotide

<400>  717
ccgggagagc aacacggg                                                     18


<210>  718
<211>  13
<212>  DNA
<213>  Artificial

<220>
<223>  Antisense nucleotide

<400>  718
acttttaact tga                                                          13


<210>  719
<211>  15
<212>  DNA
<213>  Artificial

<220>
<223>  Antisense nucleotide

<400>  719
attgttgctg tattt                                                        15


<210>  720
<211>  14
<212>  DNA
<213>  Artificial

<220>
<223>  Antisense nucleotide

<400>  720
attgttgctg tatt                                                         14


<210>  721
<211>  15
<212>  DNA
<213>  Artificial

<220>
<223>  Antisense nucleotide

<400>  721
aattgttgct gtatt                                                        15


<210>  722
<211>  14
<212>  DNA
<213>  Artificial

<220>
<223>  Antisense nucleotide

<400>  722
aattgttgct gtat                                                         14


<210>  723
<211>  27
<212>  DNA
<213>  Artificial

<220>
<223>  Antisense nucleotide

<400>  723
ggcgagtcgc tgggtgccag cagccgg                                           27


<210>  724
<211>  14
<212>  DNA
<213>  Artificial

<220>
<223>  Antisense nucleotide

<400>  724
ggcgagtcgc tggg                                                         14


<210>  725
<211>  14
<212>  DNA
<213>  Artificial

<220>
<223>  Antisense nucleotide

<400>  725
acatcaaaag ataa                                                          14


<210>  726
<211>  14
<212>  DNA
<213>  Artificial

<220>
<223>  Antisense nucleotide

<400>  726
tgacatcaaa agat                                                          14


<210>  727
<211>  18
<212>  DNA
<213>  Artificial

<220>
<223>  Antisense nucleotide

<400>  727
gggccctctc cagcgggg                                                      18


<210>  728
<211>  18
<212>  DNA
<213>  Artificial

<220>
<223>  Antisense nucleotide

<400>  728
gggctcggcg gtgccggg                                                      18


<210>  729
<211>  18
<212>  DNA
<213>  Artificial

<220>
<223>  Antisense nucleotide

<400>  729
ggggcagggc ccgaggca                                                      18


<210>  730
<211>  18
<212>  DNA
<213>  Artificial

<220>
<223>  Antisense nucleotide

<400>  730
ggctccaaat gtaggggc                                                      18

<210>  731
<211>  24
<212>  DNA
<213>  Artificial

<220>
<223>  Antisense nucleotide

<400>  731
cgggttatgc tggttgtaca gggc                                              24


<210>  732
<211>  22
<212>  DNA
<213>  Artificial

<220>
<223>  Antisense nucleotide

<400>  732
cggcgccgcc gaggcgcccg gg                                                22


<210>  733
<211>  16
<212>  DNA
<213>  Artificial

<220>
<223>  Antisense nucleotide

<400>  733
ggggcggggc gggacc                                                       16


<210>  734
<211>  18
<212>  DNA
<213>  Artificial

<220>
<223>  Antisense nucleotide

<400>  734
gggcggggcg gggcgggg                                                     18


<210>  735
<211>  18
<212>  DNA
<213>  Artificial

<220>
<223>  Antisense nucleotide

<400>  735
gggcggggtg gggccggg                                                     18


<210>  736
<211>  22
<212>  DNA
<213>  Artificial

<220>
<223>  Antisense nucleotide

<400>  736
gggcaaggca gcgggggcgg gg                                              22


<210>  737
<211>  14
<212>  DNA
<213>  Artificial

<220>
<223>  Antisense nucleotide

<400>  737
cggtagcagc agcg                                                       14


<210>  738
<211>  14
<212>  DNA
<213>  Artificial

<220>
<223>  Antisense nucleotide

<400>  738
ccagtagcca cagc                                                       14


<210>  739
<211>  15
<212>  DNA
<213>  Artificial

<220>
<223>  Antisense nucleotide

<400>  739
gcaggtggat agtcc                                                      15


<210>  740
<211>  15
<212>  DNA
<213>  Artificial

<220>
<223>  Antisense nucleotide

<400>  740
cttgcaggtg gatag                                                      15


<210>  741
<211>  15
<212>  DNA
<213>  Artificial

<220>
<223>  Antisense nucleotide

<400>  741
cgatagtctt gcagg                                                      15


<210>  742
<211>  18
<212>  DNA
<213>  Artificial

<220>
<223>  Antisense nucleotide

<400>  742
ccatgtcgat agtcttgc                                                    18


<210>  743
<211>  15
<212>  DNA
<213>  Artificial

<220>
<223>  Antisense nucleotide

<400>  743
ctcgatgcgc ttccg                                                       15


<210>  744
<211>  15
<212>  DNA
<213>  Artificial

<220>
<223>  Antisense nucleotide

<400>  744
cctcgatgcg cttcc                                                       15


<210>  745
<211>  15
<212>  DNA
<213>  Artificial

<220>
<223>  Antisense nucleotide

<400>  745
ggatggcctc gatgc                                                       15


<210>  746
<211>  15
<212>  DNA
<213>  Artificial

<220>
<223>  Antisense nucleotide

<400>  746
ggacaggatc tggcc                                                       15


<210>  747
<211>  15
<212>  DNA
<213>  Artificial

<220>
<223>  Antisense nucleotide

<400>  747
cgcagcttgg acagg                                                       15

<210>  748
<211>  14
<212>  DNA
<213>  Artificial

<220>
<223>  Antisense nucleotide

<400>  748
gagccgcagc ttgg                                                         14


<210>  749
<211>  14
<212>  DNA
<213>  Artificial

<220>
<223>  Antisense nucleotide

<400>  749
cgagccgcag cttg                                                         14


<210>  750
<211>  14
<212>  DNA
<213>  Artificial

<220>
<223>  Antisense nucleotide

<400>  750
acctccccct ggct                                                         14


<210>  751
<211>  14
<212>  DNA
<213>  Artificial

<220>
<223>  Antisense nucleotide

<400>  751
ccaccattag cacg                                                         14


<210>  752
<211>  18
<212>  DNA
<213>  Artificial

<220>
<223>  Antisense nucleotide

<400>  752
gaacttgtca tagatttc                                                     18


<210>  753
<211>  16
<212>  DNA
<213>  Artificial

<220>
<223>  Antisense nucleotide

<400>  753
gctgtgtgta ctctgc                                                        16


<210>  754
<211>  14
<212>  DNA
<213>  Artificial

<220>
<223>  Antisense nucleotide

<400>  754
gctccacgtg ctgc                                                          14


<210>  755
<211>  18
<212>  DNA
<213>  Artificial

<220>
<223>  Antisense nucleotide

<400>  755
gaattgttgc tgtatttc                                                      18


<210>  756
<211>  15
<212>  DNA
<213>  Artificial

<220>
<223>  Antisense nucleotide

<400>  756
gccaggaatt gttgc                                                         15


<210>  757
<211>  18
<212>  DNA
<213>  Artificial

<220>
<223>  Antisense nucleotide

<400>  757
gtgacatcaa aagataac                                                      18


<210>  758
<211>  15
<212>  DNA
<213>  Artificial

<220>
<223>  Antisense nucleotide

<400>  758
ggctcaacca ctgcc                                                         15


<210>  759
<211>  14
<212>  DNA
<213>  Artificial

<220>
<223>  Antisense nucleotide

<400>  759
gctgtcacag gagc                                                          14


<210>  760
<211>  14
<212>  DNA
<213>  Artificial

<220>
<223>  Antisense nucleotide

<400>  760
cctgctgtca cagg                                                          14


<210>  761
<211>  18
<212>  DNA
<213>  Artificial

<220>
<223>  Antisense nucleotide

<400>  761
gcagtgtgtt atccctgc                                                      18


<210>  762
<211>  15
<212>  DNA
<213>  Artificial

<220>
<223>  Antisense nucleotide

<400>  762
gcagtgtgtt atccc                                                         15


<210>  763
<211>  14
<212>  DNA
<213>  Artificial

<220>
<223>  Antisense nucleotide

<400>  763
ccaggtcacc tcgg                                                          14


<210>  764
<211>  15
<212>  DNA
<213>  Artificial

<220>
<223>  Antisense nucleotide

<400>  764
gccatgaatg gtggc                                                         15

<210>  765
<211>  14
<212>  DNA
<213>  Artificial

<220>
<223>  Antisense nucleotide

<400>  765
gccatgaatg gtgg                                                          14


<210>  766
<211>  14
<212>  DNA
<213>  Artificial

<220>
<223>  Antisense nucleotide

<400>  766
ccatgagaag cagg                                                          14


<210>  767
<211>  17
<212>  DNA
<213>  Artificial

<220>
<223>  Antisense nucleotide

<400>  767
ggaagtcaat gtacagc                                                       17


<210>  768
<211>  18
<212>  DNA
<213>  Artificial

<220>
<223>  Antisense nucleotide

<400>  768
ccacgtagta cacgatgg                                                      18


<210>  769
<211>  14
<212>  DNA
<213>  Artificial

<220>
<223>  Antisense nucleotide

<400>  769
gcacttgcag gagc                                                          14


<210>  770
<211>  14
<212>  DNA
<213>  Artificial

<220>
<223>  Antisense nucleotide

<400>  770
ccatggcagt gacc                                                          14


<210>  771
<211>  14
<212>  DNA
<213>  Artificial

<220>
<223>  Antisense nucleotide

<400>  771
ggctcctcca tggc                                                          14


<210>  772
<211>  16
<212>  DNA
<213>  Artificial

<220>
<223>  Antisense nucleotide

<400>  772
gctaggatct gactgc                                                        16


<210>  773
<211>  14
<212>  DNA
<213>  Artificial

<220>
<223>  Antisense nucleotide

<400>  773
cctgactcag aggg                                                          14


<210>  774
<211>  17
<212>  DNA
<213>  Artificial

<220>
<223>  Antisense nucleotide

<400>  774
ggtctgaaaa tgtttcc                                                       17


<210>  775
<211>  16
<212>  DNA
<213>  Artificial

<220>
<223>  Antisense nucleotide

<400>  775
ccattgcttg ggacgg                                                        16


<210>  776
<211>  14
<212>  DNA
<213>  Artificial

<220>
<223>  Antisense nucleotide

<400>  776
gcatcaaatc atcc                                                          14


<210>  777
<211>  16
<212>  DNA
<213>  Artificial

<220>
<223>  Antisense nucleotide

<400>  777
ccattgttca atatcg                                                        16


<210>  778
<211>  15
<212>  DNA
<213>  Artificial

<220>
<223>  Antisense nucleotide

<400>  778
ggtcttcagt gaacc                                                         15


<210>  779
<211>  17
<212>  DNA
<213>  Artificial

<220>
<223>  Antisense nucleotide

<400>  779
ggagcttcat ctggacc                                                       17


<210>  780
<211>  15
<212>  DNA
<213>  Artificial

<220>
<223>  Antisense nucleotide

<400>  780
cctctggcat tctgg                                                         15


<210>  781
<211>  14
<212>  DNA
<213>  Artificial

<220>
<223>  Antisense nucleotide

<400>  781
agggacagaa gatg                                                          14

<210>  782
<211>  14
<212>  DNA
<213>  Artificial

<220>
<223>  Antisense nucleotide

<400>  782
gttttctggg aagg                                                         14


<210>  783
<211>  14
<212>  DNA
<213>  Artificial

<220>
<223>  Antisense nucleotide

<400>  783
ggttttctgg gaag                                                         14


<210>  784
<211>  15
<212>  DNA
<213>  Artificial

<220>
<223>  Antisense nucleotide

<400>  784
aggttttctg ggaag                                                        15


<210>  785
<211>  14
<212>  DNA
<213>  Artificial

<220>
<223>  Antisense nucleotide

<400>  785
gtaggttttc tggg                                                         14


<210>  786
<211>  14
<212>  DNA
<213>  Artificial

<220>
<223>  Antisense nucleotide

<400>  786
ggtaggtttt ctgg                                                         14


<210>  787
<211>  17
<212>  DNA
<213>  Artificial

<220>
<223>  Antisense nucleotide

<400>  787
ccagaatgca agaagcc                                                    17


<210>  788
<211>  15
<212>  DNA
<213>  Artificial

<220>
<223>  Antisense nucleotide

<400>  788
gctgtcccag aatgc                                                      15


<210>  789
<211>  18
<212>  DNA
<213>  Artificial

<220>
<223>  Antisense nucleotide

<400>  789
gcaagtcaca gacttggc                                                   18


<210>  790
<211>  15
<212>  DNA
<213>  Artificial

<220>
<223>  Antisense nucleotide

<400>  790
ccacagctgc acagg                                                      15


<210>  791
<211>  15
<212>  DNA
<213>  Artificial

<220>
<223>  Antisense nucleotide

<400>  791
ggtgtggaat caacc                                                      15


<210>  792
<211>  14
<212>  DNA
<213>  Artificial

<220>
<223>  Antisense nucleotide

<400>  792
gtcatgtgct gtga                                                       14


<210>  793
<211>  16
<212>  DNA
<213>  Artificial

<220>
<223>  Antisense nucleotide

<400>  793
cgctatctga gcagcg                                                    16


<210>  794
<211>  15
<212>  DNA
<213>  Artificial

<220>
<223>  Antisense nucleotide

<400>  794
ccagtgtgat gatgg                                                     15


<210>  795
<211>  18
<212>  DNA
<213>  Artificial

<220>
<223>  Antisense nucleotide

<400>  795
ccagtagatt accactgg                                                  18


<210>  796
<211>  16
<212>  DNA
<213>  Artificial

<220>
<223>  Antisense nucleotide

<400>  796
ggcacaaaca cgcacc                                                    16


<210>  797
<211>  15
<212>  DNA
<213>  Artificial

<220>
<223>  Antisense nucleotide

<400>  797
ccacggatct gaagg                                                     15


<210>  798
<211>  17
<212>  DNA
<213>  Artificial

<220>
<223>  Antisense nucleotide

<400>  798
cggaacatct cgaagcg                                                   17

<210>  799
<211>  17
<212>  DNA
<213>  Artificial

<220>
<223>  Antisense nucleotide

<400>  799
cctcattcag ctctcgg                                                    17


<210>  800
<211>  15
<212>  DNA
<213>  Artificial

<220>
<223>  Antisense nucleotide

<400>  800
ccttgagttc caagg                                                      15


<210>  801
<211>  17
<212>  DNA
<213>  Artificial

<220>
<223>  Antisense nucleotide

<400>  801
   cctttttgga cttcagg                                                   17


<210>  802
<211>  16
<212>  DNA
<213>  Artificial

<220>
<223>  Antisense nucleotide

<400>  802
ggaggtagac tgaccc                                                     16


<210>  803
<211>  12
<212>  DNA
<213>  Artificial

<220>
<223>  Antisense nucleotide

<400>  803
aaaatgtttc ct                                                         12


<210>  804
<211>  12
<212>  DNA
<213>  Artificial

<220>
<223>  Antisense nucleotide

<400>  804
tgaaaatgtt tc                                                          12


<210>  805
<211>  12
<212>  DNA
<213>  Artificial

<220>
<223>  Antisense nucleotide

<400>  805
ctgaaaatgt tt                                                          12


<210>  806
<211>  13
<212>  DNA
<213>  Artificial

<220>
<223>  Antisense nucleotide

<400>  806
tctgaaaatg ttt                                                         13


<210>  807
<211>  12
<212>  DNA
<213>  Artificial

<220>
<223>  Antisense nucleotide

<400>  807
tctgaaaatg tt                                                          12


<210>  808
<211>  12
<212>  DNA
<213>  Artificial

<220>
<223>  Antisense nucleotide

<400>  808
aaatcatcca tt                                                          12


<210>  809
<211>  12
<212>  DNA
<213>  Artificial

<220>
<223>  Antisense nucleotide

<400>  809
ttgttcaata tc                                                          12


<210>  810
<211>  13
<212>  DNA
<213>  Artificial

<220>
<223>  Antisense nucleotide

<400>  810
attgttcaat atc                                                          13


<210>  811
<211>  12
<212>  DNA
<213>  Artificial

<220>
<223>  Antisense nucleotide

<400>  811
attgttcaat at                                                           12


<210>  812
<211>  13
<212>  DNA
<213>  Artificial

<220>
<223>  Antisense nucleotide

<400>  812
cattgttcaa tat                                                          13


<210>  813
<211>  12
<212>  DNA
<213>  Artificial

<220>
<223>  Antisense nucleotide

<400>  813
cattgttcaa ta                                                           12


<210>  814
<211>  12
<212>  DNA
<213>  Artificial

<220>
<223>  Antisense nucleotide

<400>  814
aaaagtgttt ct                                                           12


<210>  815
<211>  15
<212>  DNA
<213>  Artificial

<220>
<223>  Antisense nucleotide

<400>  815
acatgagttt tttat                                                        15

<210>  816
<211>  16
<212>  DNA
<213>  Artificial

<220>
<223>  Antisense nucleotide

<400>  816
aacatgagtt ttttat                                                        16


<210>  817
<211>  14
<212>  DNA
<213>  Artificial

<220>
<223>  Antisense nucleotide

<400>  817
acatgagttt ttta                                                          14


<210>  818
<211>  15
<212>  DNA
<213>  Artificial

<220>
<223>  Antisense nucleotide

<400>  818
aacatgagtt tttta                                                         15


<210>  819
<211>  14
<212>  DNA
<213>  Artificial

<220>
<223>  Antisense nucleotide

<400>  819
aacatgagtt tttt                                                          14


<210>  820
<211>  13
<212>  DNA
<213>  Artificial

<220>
<223>  Antisense nucleotide

<400>  820
aaaacatctt gtt                                                           13


<210>  821
<211>  17
<212>  DNA
<213>  Artificial

<220>
<223>  Antisense nucleotide

<400>  821
cagagggggc tcgacgc                                                    17


<210>  822
<211>  18
<212>  DNA
<213>  Artificial

<220>
<223>  Antisense nucleotide

<400>  822
ctgactcaga gggggctc                                                   18


<210>  823
<211>  14
<212>  DNA
<213>  Artificial

<220>
<223>  Antisense nucleotide

<400>  823
agggggacag aacg                                                       14


<210>  824
<211>  23
<212>  DNA
<213>  Artificial

<220>
<223>  Antisense nucleotide

<400>  824
ttgggacggc aagggggaca gaa                                             23


<210>  825
<211>  17
<212>  DNA
<213>  Artificial

<220>
<223>  Antisense nucleotide

<400>  825
tgggacggca aggggga                                                    17


<210>  826
<211>  15
<212>  DNA
<213>  Artificial

<220>
<223>  Antisense nucleotide

<400>  826
gccacggggg gagca                                                      15


<210>  827
<211>  19
<212>  DNA
<213>  Artificial

<220>
<223>  Antisense nucleotide

<400>  827
gcaggggcca cgggggggag                                                    19


<210>  828
<211>  14
<212>  DNA
<213>  Artificial

<220>
<223>  Antisense nucleotide

<400>  828
aggggccacg gggg                                                          14


<210>  829
<211>  14
<212>  DNA
<213>  Artificial

<220>
<223>  Antisense nucleotide

<400>  829
caggggccac gggg                                                          14


<210>  830
<211>  15
<212>  DNA
<213>  Artificial

<220>
<223>  Antisense nucleotide

<400>  830
ggtgcagggg ccacg                                                         15


<210>  831
<211>  18
<212>  DNA
<213>  Artificial

<220>
<223>  Antisense nucleotide

<400>  831
tggtgcaggg gccgccgg                                                      18


<210>  832
<211>  18
<212>  DNA
<213>  Artificial

<220>
<223>  Antisense nucleotide

<400>  832
ggggctggtg caggggcc                                                      18

<210>  833
<211>  18
<212>  DNA
<213>  Artificial

<220>
<223>  Antisense nucleotide

<400>  833
agggggctgg tgcagggg                                                    18


<210>  834
<211>  14
<212>  DNA
<213>  Artificial

<220>
<223>  Antisense nucleotide

<400>  834
gggctggtgc aggg                                                        14


<210>  835
<211>  16
<212>  DNA
<213>  Artificial

<220>
<223>  Antisense nucleotide

<400>  835
gagggggctg gtgcag                                                      16


<210>  836
<211>  15
<212>  DNA
<213>  Artificial

<220>
<223>  Antisense nucleotide

<400>  836
aggagggggc tggtg                                                       15


<210>  837
<211>  18
<212>  DNA
<213>  Artificial

<220>
<223>  Antisense nucleotide

<400>  837
gggccaggag ggggctgg                                                    18


<210>  838
<211>  18
<212>  DNA
<213>  Artificial

<220>
<223>  Antisense nucleotide

<400>  838
aggggccagg agggggct                                                    18


<210>  839
<211>  14
<212>  DNA
<213>  Artificial

<220>
<223>  Antisense nucleotide

<400>  839
ggggccagga gggg                                                        14


<210>  840
<211>  15
<212>  DNA
<213>  Artificial

<220>
<223>  Antisense nucleotide

<400>  840
caggggccag gaggg                                                       15


<210>  841
<211>  16
<212>  DNA
<213>  Artificial

<220>
<223>  Antisense nucleotide

<400>  841
tctgggaagg gacaga                                                      16


<210>  842
<211>  16
<212>  DNA
<213>  Artificial

<220>
<223>  Antisense nucleotide

<400>  842
tgagggcagg ggagta                                                      16


<210>  843
<211>  15
<212>  DNA
<213>  Artificial

<220>
<223>  Antisense nucleotide

<400>  843
ttgagggcag gggag                                                       15


<210>  844
<211>  19
<212>  DNA
<213>  Artificial

<220>
<223>  Antisense nucleotide

<400>  844
cgggtgccgg gcgggggtg                                                    19


<210>  845
<211>  24
<212>  DNA
<213>  Artificial

<220>
<223>  Antisense nucleotide

<400>  845
cggacgcggg tgccgggcgg gggt                                              24


<210>  846
<211>  15
<212>  DNA
<213>  Artificial

<220>
<223>  Antisense nucleotide

<400>  846
cgggtgccgg gcggg                                                        15


<210>  847
<211>  18
<212>  DNA
<213>  Artificial

<220>
<223>  Antisense nucleotide

<400>  847
ggacgcgggt gccgggcg                                                     18


<210>  848
<211>  18
<212>  DNA
<213>  Artificial

<220>
<223>  Antisense nucleotide

<400>  848
tgggggcagc gcctcaca                                                     18


<210>  849
<211>  16
<212>  DNA
<213>  Artificial

<220>
<223>  Antisense nucleotide

<400>  849
ggtgggggca gcgcct                                                       16

<210>  850
<211>  20
<212>  DNA
<213>  Artificial

<220>
<223>  Antisense nucleotide

<400>  850
ccattttagt gcacatccgg                                                    20


<210>  851
<211>  18
<212>  DNA
<213>  Artificial

<220>
<223>  Antisense nucleotide

<400>  851
ccattttagt gcacatcc                                                      18


<210>  852
<211>  18
<212>  DNA
<213>  Artificial

<220>
<223>  Antisense nucleotide

<400>  852
gctgttccat tttagtgc                                                      18


<210>  853
<211>  14
<212>  DNA
<213>  Artificial

<220>
<223>  Antisense nucleotide

<400>  853
gtagtcgtgt agag                                                          14


<210>  854
<211>  16
<212>  DNA
<213>  Artificial

<220>
<223>  Antisense nucleotide

<400>  854
gtttgtagtc gtgtag                                                        16


<210>  855
<211>  17
<212>  DNA
<213>  Artificial

<220>
<223>  Antisense nucleotide

<400>  855
gtttcaggag tttgtag                                                    17


<210>  856
<211>  15
<212>  DNA
<213>  Artificial

<220>
<223>  Antisense nucleotide

<400>  856
ccagctccga agagg                                                      15


<210>  857
<211>  16
<212>  DNA
<213>  Artificial

<220>
<223>  Antisense nucleotide

<400>  857
cgtcgtcgtg atcacg                                                     16


<210>  858
<211>  16
<212>  DNA
<213>  Artificial

<220>
<223>  Antisense nucleotide

<400>  858
ggtaaaagta ctgtcc                                                     16


<210>  859
<211>  15
<212>  DNA
<213>  Artificial

<220>
<223>  Antisense nucleotide

<400>  859
ggctttgaca aagcc                                                      15


<210>  860
<211>  18
<212>  DNA
<213>  Artificial

<220>
<223>  Antisense nucleotide

<400>  860
cttgtgcaga tcgtccag                                                   18


<210>  861
<211>  17
<212>  DNA
<213>  Artificial

<220>
<223>  Antisense nucleotide

<400>  861
cgtggttcat cttgtgc                                                    17


<210>  862
<211>  18
<212>  DNA
<213>  Artificial

<220>
<223>  Antisense nucleotide

<400>  862
cacgtggttc atcttgtg                                                   18


<210>  863
<211>  15
<212>  DNA
<213>  Artificial

<220>
<223>  Antisense nucleotide

<400>  863
cctccttgaa ggtgg                                                      15


<210>  864
<211>  17
<212>  DNA
<213>  Artificial

<220>
<223>  Antisense nucleotide

<400>  864
cgctccactt tgatgcg                                                    17


<210>  865
<211>  14
<212>  DNA
<213>  Artificial

<220>
<223>  Antisense nucleotide

<400>  865
ccttgtcctc cagg                                                       14


<210>  866
<211>  14
<212>  DNA
<213>  Artificial

<220>
<223>  Antisense nucleotide

<400>  866
ggtactcgac agcc                                                       14

<210>  867
<211>  15
<212>  DNA
<213>  Artificial

<220>
<223>  Antisense nucleotide

<400>  867
ctgacgtggg tcatg                                                        15


<210>  868
<211>  15
<212>  DNA
<213>  Artificial

<220>
<223>  Antisense nucleotide

<400>  868
ccgttgctga cgtgg                                                        15


<210>  869
<211>  14
<212>  DNA
<213>  Artificial

<220>
<223>  Antisense nucleotide

<400>  869
catcctccgc ctcc                                                         14


<210>  870
<211>  14
<212>  DNA
<213>  Artificial

<220>
<223>  Antisense nucleotide

<400>  870
gtttccatcc tccg                                                         14


<210>  871
<211>  16
<212>  DNA
<213>  Artificial

<220>
<223>  Antisense nucleotide

<400>  871
ggtgtttcca tcctcc                                                       16


<210>  872
<211>  15
<212>  DNA
<213>  Artificial

<220>
<223>  Antisense nucleotide

<400>  872
ggtgtttcca tcctc                                                        15


<210>  873
<211>  15
<212>  DNA
<213>  Artificial

<220>
<223>  Antisense nucleotide

<400>  873
gctcagcgcc tcatc                                                        15


<210>  874
<211>  18
<212>  DNA
<213>  Artificial

<220>
<223>  Antisense nucleotide

<400>  874
ccttcttcat catgctgc                                                     18


<210>  875
<211>  17
<212>  DNA
<213>  Artificial

<220>
<223>  Antisense nucleotide

<400>  875
ccttcttcat catgctg                                                      17


<210>  876
<211>  15
<212>  DNA
<213>  Artificial

<220>
<223>  Antisense nucleotide

<400>  876
ccttcttcat catgc                                                        15


<210>  877
<211>  19
<212>  DNA
<213>  Artificial

<220>
<223>  Antisense nucleotide

<400>  877
gcgtccttct tcatcatgc                                                    19


<210>  878
<211>  14
<212>  DNA
<213>  Artificial

<220>
<223>  Antisense nucleotide

<400>  878
cctgctcact cagg                                                          14


<210>  879
<211>  14
<212>  DNA
<213>  Artificial

<220>
<223>  Antisense nucleotide

<400>  879
cgcaggcttg agcg                                                          14


<210>  880
<211>  15
<212>  DNA
<213>  Artificial

<220>
<223>  Antisense nucleotide

<400>  880
gccagcttca gcagc                                                         15


<210>  881
<211>  14
<212>  DNA
<213>  Artificial

<220>
<223>  Antisense nucleotide

<400>  881
ggtggtgacc agcc                                                          14


<210>  882
<211>  14
<212>  DNA
<213>  Artificial

<220>
<223>  Antisense nucleotide

<400>  882
cctcggcgaa ctcc                                                          14


<210>  883
<211>  14
<212>  DNA
<213>  Artificial

<220>
<223>  Antisense nucleotide

<400>  883
gcttgtgtaa atcc                                                          14

<210> 884
<211> 20
<212> DNA
<213> Artificial

<220>
<223> Antisense nucleotide

<400> 884
ggttctgctt gtgtaaatcc 20


<210> 885
<211> 15
<212> DNA
<213> Artificial

<220>
<223> Antisense nucleotide

<400> 885
gctgctcagg ttcgc 15


<210> 886
<211> 14
<212> DNA
<213> Artificial

<220>
<223> Antisense nucleotide

<400> 886
gaaggcgacc gtcg 14


<210> 887
<211> 14
<212> DNA
<213> Artificial

<220>
<223> Antisense nucleotide

<400> 887
cgaaggcgac cgtc 14


<210> 888
<211> 14
<212> DNA
<213> Artificial

<220>
<223> Antisense nucleotide

<400> 888
gcaccgtctg tggc 14


<210> 889
<211> 17
<212> DNA
<213> Artificial

<220>
<223> Antisense nucleotide

<400>  889
cgtgtccatg tcgatgg                                                    17


<210>  890
<211>  16
<212>  DNA
<213>  Artificial

<220>
<223>  Antisense nucleotide

<400>  890
cgtgtccatg tcgatg                                                     16


<210>  891
<211>  14
<212>  DNA
<213>  Artificial

<220>
<223>  Antisense nucleotide

<400>  891
gcgtgtccat gtcg                                                       14


<210>  892
<211>  15
<212>  DNA
<213>  Artificial

<220>
<223>  Antisense nucleotide

<400>  892
ccagcttgcg cttgc                                                      15


<210>  893
<211>  14
<212>  DNA
<213>  Artificial

<220>
<223>  Antisense nucleotide

<400>  893
cgctccagct tgcg                                                       14


<210>  894
<211>  18
<212>  DNA
<213>  Artificial

<220>
<223>  Antisense nucleotide

<400>  894
cgtgttctga ctcttgag                                                   18


<210>  895
<211>  16
<212>  DNA
<213>  Artificial

<220>
<223>  Antisense nucleotide

<400>  895
cgtgttctga ctcttg                                                    16


<210>  896
<211>  14
<212>  DNA
<213>  Artificial

<220>
<223>  Antisense nucleotide

<400>  896
gctgttgacg tggc                                                      14


<210>  897
<211>  14
<212>  DNA
<213>  Artificial

<220>
<223>  Antisense nucleotide

<400>  897
cgactcagta cgcc                                                      14


<210>  898
<211>  14
<212>  DNA
<213>  Artificial

<220>
<223>  Antisense nucleotide

<400>  898
gccatgcccg actc                                                      14


<210>  899
<211>  14
<212>  DNA
<213>  Artificial

<220>
<223>  Antisense nucleotide

<400>  899
cccttggagg tggc                                                      14


<210>  900
<211>  13
<212>  DNA
<213>  Artificial

<220>
<223>  Antisense nucleotide

<400>  900
ttttagtgca cat                                                       13

<210>  901
<211>  14
<212>  DNA
<213>  Artificial

<220>
<223>  Antisense nucleotide

<400>  901
tgttccattt tagt                                                          14


<210>  902
<211>  14
<212>  DNA
<213>  Artificial

<220>
<223>  Antisense nucleotide

<400>  902
aaaaaaagtg gaag                                                          14


<210>  903
<211>  14
<212>  DNA
<213>  Artificial

<220>
<223>  Antisense nucleotide

<400>  903
tacaaaaaaa agtg                                                          14


<210>  904
<211>  14
<212>  DNA
<213>  Artificial

<220>
<223>  Antisense nucleotide

<400>  904
atacaaaaaa aagt                                                          14


<210>  905
<211>  15
<212>  DNA
<213>  Artificial

<220>
<223>  Antisense nucleotide

<400>  905
catacaaaaa aaagt                                                         15


<210>  906
<211>  14
<212>  DNA
<213>  Artificial

<220>
<223>  Antisense nucleotide

<400>  906
catacaaaaa aaag                                                          14


<210>  907
<211>  14
<212>  DNA
<213>  Artificial

<220>
<223>  Antisense nucleotide

<400>  907
gaaaaaaaac atac                                                          14


<210>  908
<211>  16
<212>  DNA
<213>  Artificial

<220>
<223>  Antisense nucleotide

<400>  908
cagaaaaaaa acatac                                                        16


<210>  909
<211>  14
<212>  DNA
<213>  Artificial

<220>
<223>  Antisense nucleotide

<400>  909
cagaaaaaaa acat                                                          14


<210>  910
<211>  14
<212>  DNA
<213>  Artificial

<220>
<223>  Antisense nucleotide

<400>  910
ttcaatatga atcg                                                          14


<210>  911
<211>  16
<212>  DNA
<213>  Artificial

<220>
<223>  Antisense nucleotide

<400>  911
tattcaatat gaatcg                                                        16


<210>  912
<211>  15
<212>  DNA
<213>  Artificial

<220>
<223>  Antisense nucleotide

<400>  912
tattcaatat gaatc                                                        15


<210>  913
<211>  14
<212>  DNA
<213>  Artificial

<220>
<223>  Antisense nucleotide

<400>  913
tattcaatat gaat                                                         14


<210>  914
<211>  15
<212>  DNA
<213>  Artificial

<220>
<223>  Antisense nucleotide

<400>  914
tatattcaat atgaa                                                        15


<210>  915
<211>  15
<212>  DNA
<213>  Artificial

<220>
<223>  Antisense nucleotide

<400>  915
ttatattcaa tatga                                                        15


<210>  916
<211>  17
<212>  DNA
<213>  Artificial

<220>
<223>  Antisense nucleotide

<400>  916
tattatattc aatatga                                                      17


<210>  917
<211>  14
<212>  DNA
<213>  Artificial

<220>
<223>  Antisense nucleotide

<400>  917
ttatattcaa tatg                                                         14

<210>  918
<211>  16
<212>  DNA
<213>  Artificial

<220>
<223>  Antisense nucleotide

<400>  918
tattatattc aatatg                                                        16


<210>  919
<211>  14
<212>  DNA
<213>  Artificial

<220>
<223>  Antisense nucleotide

<400>  919
attatattca atat                                                          14


<210>  920
<211>  15
<212>  DNA
<213>  Artificial

<220>
<223>  Antisense nucleotide

<400>  920
tattatattc aatat                                                         15


<210>  921
<211>  18
<212>  DNA
<213>  Artificial

<220>
<223>  Antisense nucleotide

<400>  921
atatattata ttcaatat                                                      18


<210>  922
<211>  20
<212>  DNA
<213>  Artificial

<220>
<223>  Antisense nucleotide

<400>  922
aaatatatta tattcaatat                                                    20


<210>  923
<211>  14
<212>  DNA
<213>  Artificial

<220>
<223>  Antisense nucleotide

<400>  923
tattatattc aata                                                         14


<210>  924
<211>  17
<212>  DNA
<213>  Artificial

<220>
<223>  Antisense nucleotide

<400>  924
atatattata ttcaata                                                      17


<210>  925
<211>  20
<212>  DNA
<213>  Artificial

<220>
<223>  Antisense nucleotide

<400>  925
caaatatatt atattcaata                                                   20


<210>  926
<211>  15
<212>  DNA
<213>  Artificial

<220>
<223>  Antisense nucleotide

<400>  926
tatattatat tcaat                                                        15


<210>  927
<211>  17
<212>  DNA
<213>  Artificial

<220>
<223>  Antisense nucleotide

<400>  927
aatatattat attcaat                                                      17


<210>  928
<211>  14
<212>  DNA
<213>  Artificial

<220>
<223>  Antisense nucleotide

<400>  928
tatattatat tcaa                                                         14


<210>  929
<211>  18
<212>  DNA
<213>  Artificial

<220>
<223>  Antisense nucleotide

<400>  929
caaatatatt atattcaa                                                    18


<210>  930
<211>  17
<212>  DNA
<213>  Artificial

<220>
<223>  Antisense nucleotide

<400>  930
caaatatatt atattca                                                     17


<210>  931
<211>  16
<212>  DNA
<213>  Artificial

<220>
<223>  Antisense nucleotide

<400>  931
caaatatatt atattc                                                      16


<210>  932
<211>  18
<212>  DNA
<213>  Artificial

<220>
<223>  Antisense nucleotide

<400>  932
cacaaatata ttatattc                                                    18


<210>  933
<211>  14
<212>  DNA
<213>  Artificial

<220>
<223>  Antisense nucleotide

<400>  933
aaatatatta tatt                                                        14


<210>  934
<211>  15
<212>  DNA
<213>  Artificial

<220>
<223>  Antisense nucleotide

<400>  934
caaatatatt atatt                                                       15

<210>  935
<211>  14
<212>  DNA
<213>  Artificial

<220>
<223>  Antisense nucleotide

<400>  935
caaatatatt atat                                                         14


<210>  936
<211>  16
<212>  DNA
<213>  Artificial

<220>
<223>  Antisense nucleotide

<400>  936
cacaaatata ttatat                                                       16


<210>  937
<211>  14
<212>  DNA
<213>  Artificial

<220>
<223>  Antisense nucleotide

<400>  937
cacaaatata ttat                                                         14


<210>  938
<211>  16
<212>  DNA
<213>  Artificial

<220>
<223>  Antisense nucleotide

<400>  938
tacacaaata tattat                                                       16


<210>  939
<211>  15
<212>  DNA
<213>  Artificial

<220>
<223>  Antisense nucleotide

<400>  939
tacacaaata tatta                                                        15


<210>  940
<211>  18
<212>  DNA
<213>  Artificial

<220>
<223>  Antisense nucleotide

<400>  940
taaatacaca aatatatt                                                    18


<210>  941
<211>  14
<212>  DNA
<213>  Artificial

<220>
<223>  Antisense nucleotide

<400>  941
aatacacaaa tata                                                        14


<210>  942
<211>  16
<212>  DNA
<213>  Artificial

<220>
<223>  Antisense nucleotide

<400>  942
gttaaataca caaata                                                      16


<210>  943
<211>  14
<212>  DNA
<213>  Artificial

<220>
<223>  Antisense nucleotide

<400>  943
tgttaaatac acaa                                                        14


<210>  944
<211>  14
<212>  DNA
<213>  Artificial

<220>
<223>  Antisense nucleotide

<400>  944
tttagagact aagt                                                        14


<210>  945
<211>  14
<212>  DNA
<213>  Artificial

<220>
<223>  Antisense nucleotide

<400>  945
ataaactctt taga                                                        14


<210>  946
<211>  17
<212>  DNA
<213>  Artificial

<220>
<223>  Antisense nucleotide

<400>  946
taaaataaac tctttag                                                    17


<210>  947
<211>  16
<212>  DNA
<213>  Artificial

<220>
<223>  Antisense nucleotide

<400>  947
taaaataaac tcttta                                                     16


<210>  948
<211>  16
<212>  DNA
<213>  Artificial

<220>
<223>  Antisense nucleotide

<400>  948
ttaaaataaa ctcttt                                                     16


<210>  949
<211>  14
<212>  DNA
<213>  Artificial

<220>
<223>  Antisense nucleotide

<400>  949
cttaaaataa actc                                                       14


<210>  950
<211>  15
<212>  DNA
<213>  Artificial

<220>
<223>  Antisense nucleotide

<400>  950
taaaaagaac aaaca                                                      15


<210>  951
<211>  14
<212>  DNA
<213>  Artificial

<220>
<223>  Antisense nucleotide

<400>  951
taaaaagaac aaac                                                       14

<210>  952
<211>  15
<212>  DNA
<213>  Artificial

<220>
<223>  Antisense nucleotide

<400>  952
caataaaaag aacaa                                                        15


<210>  953
<211>  16
<212>  DNA
<213>  Artificial

<220>
<223>  Antisense nucleotide

<400>  953
tcaataaaaa gaacaa                                                       16


<210>  954
<211>  14
<212>  DNA
<213>  Artificial

<220>
<223>  Antisense nucleotide

<400>  954
tcaataaaaa gaac                                                         14


<210>  955
<211>  14
<212>  DNA
<213>  Artificial

<220>
<223>  Antisense nucleotide

<400>  955
ttcaataaaa agaa                                                         14


<210>  956
<211>  17
<212>  DNA
<213>  Artificial

<220>
<223>  Antisense nucleotide

<400>  956
tagattcaat aaaaaga                                                      17


<210>  957
<211>  17
<212>  DNA
<213>  Artificial

<220>
<223>  Antisense nucleotide

<400>  957
tggcgcgggc gggtagc                                                    17


<210>  958
<211>  20
<212>  DNA
<213>  Artificial

<220>
<223>  Antisense nucleotide

<400>  958
gggctggcgc gggcgggtag                                                 20


<210>  959
<211>  21
<212>  DNA
<213>  Artificial

<220>
<223>  Antisense nucleotide

<400>  959
tcgggggctg gcgcgggcgg g                                               21


<210>  960
<211>  26
<212>  DNA
<213>  Artificial

<220>
<223>  Antisense nucleotide

<400>  960
tgggtgcctg gtcgcgcgtt ctcggg                                          26


<210>  961
<211>  18
<212>  DNA
<213>  Artificial

<220>
<223>  Antisense nucleotide

<400>  961
agggtccctg cggggccg                                                   18


<210>  962
<211>  18
<212>  DNA
<213>  Artificial

<220>
<223>  Antisense nucleotide

<400>  962
gggagggtcc ctgcgggg                                                   18


<210>  963
<211>  16
<212>  DNA
<213>  Artificial

<220>
<223>  Antisense nucleotide

<400>  963
gggagggtcc ctgcgg                                                        16


<210>  964
<211>  14
<212>  DNA
<213>  Artificial

<220>
<223>  Antisense nucleotide

<400>  964
tgggccgggt ccgc                                                          14


<210>  965
<211>  14
<212>  DNA
<213>  Artificial

<220>
<223>  Antisense nucleotide

<400>  965
tcccgggggt gtag                                                          14


<210>  966
<211>  19
<212>  DNA
<213>  Artificial

<220>
<223>  Antisense nucleotide

<400>  966
agtactgtcc cgggggtgt                                                     19


<210>  967
<211>  18
<212>  DNA
<213>  Artificial

<220>
<223>  Antisense nucleotide

<400>  967
gggacacgtt gggggtg                                                       18


<210>  968
<211>  20
<212>  DNA
<213>  Artificial

<220>
<223>  Antisense nucleotide

<400>  968
gccgggggcc ccccggtagc                                                    20

<210>  969
<211>  17
<212>  DNA
<213>  Artificial

<220>
<223>  Antisense nucleotide

<400>  969
cgggcccagc cgggggc                                                    17


<210>  970
<211>  14
<212>  DNA
<213>  Artificial

<220>
<223>  Antisense nucleotide

<400>  970
cgggcccagc cggg                                                       14


<210>  971
<211>  20
<212>  DNA
<213>  Artificial

<220>
<223>  Antisense nucleotide

<400>  971
gggaggtggc tccgggccgg                                                 20


<210>  972
<211>  18
<212>  DNA
<213>  Artificial

<220>
<223>  Antisense nucleotide

<400>  972
agggcggcgc gtgtggga                                                   18


<210>  973
<211>  20
<212>  DNA
<213>  Artificial

<220>
<223>  Antisense nucleotide

<400>  973
gggtggccac cggcgaaggg                                                 20


<210>  974
<211>  15
<212>  DNA
<213>  Artificial

<220>
<223>  Antisense nucleotide

<400>  974
aggggcaggg gacgt                                                        15


<210>  975
<211>  18
<212>  DNA
<213>  Artificial

<220>
<223>  Antisense nucleotide

<400>  975
taaaggggca ggggacgt                                                     18


<210>  976
<211>  20
<212>  DNA
<213>  Artificial

<220>
<223>  Antisense nucleotide

<400>  976
agggggtgtc cgtaaagggg                                                   20


<210>  977
<211>  21
<212>  DNA
<213>  Artificial

<220>
<223>  Antisense nucleotide

<400>  977
ggggacgcga acgtgccgcc g                                                 21


<210>  978
<211>  21
<212>  DNA
<213>  Artificial

<220>
<223>  Antisense nucleotide

<400>  978
cggggaacaa gcggcccggg g                                                 21


<210>  979
<211>  14
<212>  DNA
<213>  Artificial

<220>
<223>  Antisense nucleotide

<400>  979
ggccgtcggg ggcg                                                         14


<210>  980
<211>  15
<212>  DNA
<213>  Artificial

<220>
<223>  Antisense nucleotide

<400>  980
gcggccgtcg ggggc                                                        15


<210>  981
<211>  18
<212>  DNA
<213>  Artificial

<220>
<223>  Antisense nucleotide

<400>  981
   aggggggtag gaggcggg                                                      18


<210>  982
<211>  14
<212>  DNA
<213>  Artificial

<220>
<223>  Antisense nucleotide

<400>  982
gcgctggggg cgcc                                                         14


<210>  983
<211>  14
<212>  DNA
<213>  Artificial

<220>
<223>  Antisense nucleotide

<400>  983
ggccgtcggg gggt                                                         14


<210>  984
<211>  15
<212>  DNA
<213>  Artificial

<220>
<223>  Antisense nucleotide

<400>  984
ggggaggcca gcttc                                                        15


<210>  985
<211>  16
<212>  DNA
<213>  Artificial

<220>
<223>  Antisense nucleotide

<400>  985
ggccgccacc ttgggg                                                       16

<210>  986
<211>  16
<212>  DNA
<213>  Artificial

<220>
<223>  Antisense nucleotide

<400>  986
gcggccgccg ccgggg                                                    16


<210>  987
<211>  20
<212>  DNA
<213>  Artificial

<220>
<223>  Antisense nucleotide

<400>  987
gggcgcggcc gccgccgggg                                                20


<210>  988
<211>  16
<212>  DNA
<213>  Artificial

<220>
<223>  Antisense nucleotide

<400>  988
ggggtggcgg cggcgg                                                    16


<210>  989
<211>  15
<212>  DNA
<213>  Artificial

<220>
<223>  Antisense nucleotide

<400>  989
gggggtggcg gcggc                                                     15


<210>  990
<211>  18
<212>  DNA
<213>  Artificial

<220>
<223>  Antisense nucleotide

<400>  990
tggggcagca gctggcag                                                  18


<210>  991
<211>  18
<212>  DNA
<213>  Artificial

<220>
<223>  Antisense nucleotide

<400>  991
cggggcgccc acgacacc                                                    18


<210>  992
<211>  17
<212>  DNA
<213>  Artificial

<220>
<223>  Antisense nucleotide

<400>  992
cggggcgccc acgacac                                                     17


<210>  993
<211>  26
<212>  DNA
<213>  Artificial

<220>
<223>  Antisense nucleotide

<400>  993
gggccgcacc ctctccaagt ccgggg                                           26


<210>  994
<211>  14
<212>  DNA
<213>  Artificial

<220>
<223>  Antisense nucleotide

<400>  994
gcagcagtca gtgg                                                        14


<210>  995
<211>  16
<212>  DNA
<213>  Artificial

<220>
<223>  Antisense nucleotide

<400>  995
ccattgtcta gcacgg                                                      16


<210>  996
<211>  17
<212>  DNA
<213>  Artificial

<220>
<223>  Antisense nucleotide

<400>  996
ggtctccatt gtctagc                                                     17


<210>  997
<211>  16
<212>  DNA
<213>  Artificial

<220>
<223>  Antisense nucleotide

<400>  997
ggtggtattg ttcagc                                                        16


<210>  998
<211>  15
<212>  DNA
<213>  Artificial

<220>
<223>  Antisense nucleotide

<400>  998
gctggatcaa gaccc                                                         15


<210>  999
<211>  16
<212>  DNA
<213>  Artificial

<220>
<223>  Antisense nucleotide

<400>  999
ccacaaaatc gtgtcc                                                        16


<210>  1000
<211>  20
<212>  DNA
<213>  Artificial

<220>
<223>  Antisense nucleotide

<400>  1000
ccttccacaa aatcgtgtcc                                                    20


<210>  1001
<211>  14
<212>  DNA
<213>  Artificial

<220>
<223>  Antisense nucleotide

<400>  1001
ggttgttctt gtgg                                                          14


<210>  1002
<211>  14
<212>  DNA
<213>  Artificial

<220>
<223>  Antisense nucleotide

<400>  1002
cctcttggtt gtgc                                                          14

<210>  1003
<211>  20
<212>  DNA
<213>  Artificial

<220>
<223>  Antisense nucleotide

<400>  1003
ccagagtctc aaacacttgg                                                    20


<210>  1004
<211>  20
<212>  DNA
<213>  Artificial

<220>
<223>  Antisense nucleotide

<400>  1004
ggtaacctgt gatctcttcc                                                    20


<210>  1005
<211>  14
<212>  DNA
<213>  Artificial

<220>
<223>  Antisense nucleotide

<400>  1005
cctgcagtac tcgg                                                          14


<210>  1006
<211>  16
<212>  DNA
<213>  Artificial

<220>
<223>  Antisense nucleotide

<400>  1006
ggcattcaca tactcc                                                        16


<210>  1007
<211>  16
<212>  DNA
<213>  Artificial

<220>
<223>  Antisense nucleotide

<400>  1007
gcaaacagtg cctggc                                                        16


<210>  1008
<211>  17
<212>  DNA
<213>  Artificial

<220>
<223>  Antisense nucleotide

<400>  1008
cgcatcgtgt acttccg                                                    17


<210>  1009
<211>  14
<212>  DNA
<213>  Artificial

<220>
<223>  Antisense nucleotide

<400>  1009
gcacgttccg agcg                                                       14


<210>  1010
<211>  15
<212>  DNA
<213>  Artificial

<220>
<223>  Antisense nucleotide

<400>  1010
ggtaccagat actcc                                                      15


<210>  1011
<211>  15
<212>  DNA
<213>  Artificial

<220>
<223>  Antisense nucleotide

<400>  1011
ccagtggaga cctgg                                                      15


<210>  1012
<211>  17
<212>  DNA
<213>  Artificial

<220>
<223>  Antisense nucleotide

<400>  1012
cctgaggaca catcagg                                                    17


<210>  1013
<211>  18
<212>  DNA
<213>  Artificial

<220>
<223>  Antisense nucleotide

<400>  1013
cctcacttgg ttgtgagc                                                   18


<210>  1014
<211>  15
<212>  DNA
<213>  Artificial

<220>
<223>  Antisense nucleotide

<400>  1014
ggaagatgtc cttcc                                                        15


<210>  1015
<211>  16
<212>  DNA
<213>  Artificial

<220>
<223>  Antisense nucleotide

<400>  1015
gcacactgct catggc                                                       16


<210>  1016
<211>  15
<212>  DNA
<213>  Artificial

<220>
<223>  Antisense nucleotide

<400>  1016
gctgtcacct cttgg                                                        15


<210>  1017
<211>  14
<212>  DNA
<213>  Artificial

<220>
<223>  Antisense nucleotide

<400>  1017
cctctgctgt cacc                                                         14


<210>  1018
<211>  16
<212>  DNA
<213>  Artificial

<220>
<223>  Antisense nucleotide

<400>  1018
ccacacatca ctctgg                                                       16


<210>  1019
<211>  15
<212>  DNA
<213>  Artificial

<220>
<223>  Antisense nucleotide

<400>  1019
cctcctcttc agagg                                                        15

<210>  1020
<211>  18
<212>  DNA
<213>  Artificial

<220>
<223>  Antisense nucleotide

<400>  1020
ccttctggtt cacactgg                                                    18


<210>  1021
<211>  16
<212>  DNA
<213>  Artificial

<220>
<223>  Antisense nucleotide

<400>  1021
catggtgctc actgcg                                                      16


<210>  1022
<211>  14
<212>  DNA
<213>  Artificial

<220>
<223>  Antisense nucleotide

<400>  1022
cttggttgtg agcg                                                        14


<210>  1023
<211>  14
<212>  DNA
<213>  Artificial

<220>
<223>  Antisense nucleotide

<400>  1023
ggacaggcag tcac                                                        14


<210>  1024
<211>  18
<212>  DNA
<213>  Artificial

<220>
<223>  Antisense nucleotide

<400>  1024
gtcacctctt ggttgtgc                                                    18


<210>  1025
<211>  16
<212>  DNA
<213>  Artificial

<220>
<223>  Antisense nucleotide

<400>  1025
ccagagtctc aaacac                                                        16


<210>  1026
<211>  14
<212>  DNA
<213>  Artificial

<220>
<223>  Antisense nucleotide

<400>  1026
cacatactcc ctgg                                                          14


<210>  1027
<211>  15
<212>  DNA
<213>  Artificial

<220>
<223>  Antisense nucleotide

<400>  1027
gaccagcacg ttccg                                                         15


<210>  1028
<211>  17
<212>  DNA
<213>  Artificial

<220>
<223>  Antisense nucleotide

<400>  1028
gttggtgtct atcagtg                                                       17


<210>  1029
<211>  14
<212>  DNA
<213>  Artificial

<220>
<223>  Antisense nucleotide

<400>  1029
ccctggtaga ggtg                                                          14


<210>  1030
<211>  16
<212>  DNA
<213>  Artificial

<220>
<223>  Antisense nucleotide

<400>  1030
ctcaaacact tggagc                                                        16


<210>  1031
<211>  18
<212>  DNA
<213>  Artificial

<220>
<223>  Antisense nucleotide

<400>  1031
cacacatcac tctggtgg                                                    18


<210>  1032
<211>  15
<212>  DNA
<213>  Artificial

<220>
<223>  Antisense nucleotide

<400>  1032
gcacagacag tgcgc                                                       15


<210>  1033
<211>  15
<212>  DNA
<213>  Artificial

<220>
<223>  Antisense nucleotide

<400>  1033
catggcagca gtcag                                                       15


<210>  1034
<211>  16
<212>  DNA
<213>  Artificial

<220>
<223>  Antisense nucleotide

<400>  1034
ctgctcatgg cagcag                                                      16


<210>  1035
<211>  20
<212>  DNA
<213>  Artificial

<220>
<223>  Antisense nucleotide

<400>  1035
catctggaaa cttccagatg                                                  20


<210>  1036
<211>  14
<212>  DNA
<213>  Artificial

<220>
<223>  Antisense nucleotide

<400>  1036
ctggaaactt ccag                                                        14

<210>  1037
<211>  20
<212>  DNA
<213>  Artificial

<220>
<223>  Antisense nucleotide

<400>  1037
cataactcca cacatcactc                                                    20


<210>  1038
<211>  19
<212>  DNA
<213>  Artificial

<220>
<223>  Antisense nucleotide

<400>  1038
caccataact ccacacatc                                                     19


<210>  1039
<211>  14
<212>  DNA
<213>  Artificial

<220>
<223>  Antisense nucleotide

<400>  1039
ctggtgggtg aacc                                                          14


<210>  1040
<211>  15
<212>  DNA
<213>  Artificial

<220>
<223>  Antisense nucleotide

<400>  1040
cggattactt gcagg                                                         15


<210>  1041
<211>  15
<212>  DNA
<213>  Artificial

<220>
<223>  Antisense nucleotide

<400>  1041
cgctaggtgt cagcg                                                         15


<210>  1042
<211>  14
<212>  DNA
<213>  Artificial

<220>
<223>  Antisense nucleotide

<400>  1042
gccatcacgt atgc                                                          14


<210>  1043
<211>  17
<212>  DNA
<213>  Artificial

<220>
<223>  Antisense nucleotide

<400>  1043
gcatacacca gttcagc                                                       17


<210>  1044
<211>  16
<212>  DNA
<213>  Artificial

<220>
<223>  Antisense nucleotide

<400>  1044
ccatcaaata catcgg                                                        16


<210>  1045
<211>  15
<212>  DNA
<213>  Artificial

<220>
<223>  Antisense nucleotide

<400>  1045
ccagcagaag tcagg                                                         15


<210>  1046
<211>  15
<212>  DNA
<213>  Artificial

<220>
<223>  Antisense nucleotide

<400>  1046
gcttcatgtc tgtgc                                                         15


<210>  1047
<211>  18
<212>  DNA
<213>  Artificial

<220>
<223>  Antisense nucleotide

<400>  1047
ggtgagttcc aggtttcc                                                      18


<210>  1048
<211>  19
<212>  DNA
<213>  Artificial

<220>
<223>  Antisense nucleotide

<400>  1048
ccacaaaatc gtgtcctgg                                                    19


<210>  1049
<211>  14
<212>  DNA
<213>  Artificial

<220>
<223>  Antisense nucleotide

<400>  1049
cccttacaca tcgg                                                         14


<210>  1050
<211>  15
<212>  DNA
<213>  Artificial

<220>
<223>  Antisense nucleotide

<400>  1050
gcagctcaca gatgc                                                        15


<210>  1051
<211>  14
<212>  DNA
<213>  Artificial

<220>
<223>  Antisense nucleotide

<400>  1051
gcactggtaa ctgc                                                         14


<210>  1052
<211>  18
<212>  DNA
<213>  Artificial

<220>
<223>  Antisense nucleotide

<400>  1052
cctggatatt ggcactgg                                                     18


<210>  1053
<211>  15
<212>  DNA
<213>  Artificial

<220>
<223>  Antisense nucleotide

<400>  1053
ccagcaaact cctgg                                                        15

<210>  1054
<211>  15
<212>  DNA
<213>  Artificial

<220>
<223>  Antisense nucleotide

<400>  1054
gcagaaatgc caggc                                                        15


<210>  1055
<211>  17
<212>  DNA
<213>  Artificial

<220>
<223>  Antisense nucleotide

<400>  1055
ccattgtgca gaattcg                                                      17


<210>  1056
<211>  15
<212>  DNA
<213>  Artificial

<220>
<223>  Antisense nucleotide

<400>  1056
ccctgcagta ctcgg                                                        15


<210>  1057
<211>  17
<212>  DNA
<213>  Artificial

<220>
<223>  Antisense nucleotide

<400>  1057
ggcattcaca tactccc                                                      17


<210>  1058
<211>  16
<212>  DNA
<213>  Artificial

<220>
<223>  Antisense nucleotide

<400>  1058
ggtcaggttt cacacc                                                       16


<210>  1059
<211>  15
<212>  DNA
<213>  Artificial

<220>
<223>  Antisense nucleotide

<400>  1059
ccaggtccac acagg                                                        15


<210>  1060
<211>  14
<212>  DNA
<213>  Artificial

<220>
<223>  Antisense nucleotide

<400>  1060
ccttgtcatc cagg                                                         14


<210>  1061
<211>  14
<212>  DNA
<213>  Artificial

<220>
<223>  Antisense nucleotide

<400>  1061
ggatcccaaa gacc                                                         14


<210>  1062
<211>  17
<212>  DNA
<213>  Artificial

<220>
<223>  Antisense nucleotide

<400>  1062
cctcaacact ttgatgg                                                      17


<210>  1063
<211>  14
<212>  DNA
<213>  Artificial

<220>
<223>  Antisense nucleotide

<400>  1063
gctgtgtcac cagc                                                         14


<210>  1064
<211>  16
<212>  DNA
<213>  Artificial

<220>
<223>  Antisense nucleotide

<400>  1064
ggtctaagag gcagcc                                                       16


<210>  1065
<211>  17
<212>  DNA
<213>  Artificial

<220>
<223>  Antisense nucleotide

<400>  1065
ggcaatctgc atacacc                                                    17


<210>  1066
<211>  14
<212>  DNA
<213>  Artificial

<220>
<223>  Antisense nucleotide

<400>  1066
cctgtgtacg agcc                                                       14


<210>  1067
<211>  15
<212>  DNA
<213>  Artificial

<220>
<223>  Antisense nucleotide

<400>  1067
ccatccactt gatgg                                                      15


<210>  1068
<211>  16
<212>  DNA
<213>  Artificial

<220>
<223>  Antisense nucleotide

<400>  1068
cccacacagt cacacc                                                     16


<210>  1069
<211>  16
<212>  DNA
<213>  Artificial

<220>
<223>  Antisense nucleotide

<400>  1069
ccatcgtaag gtttgg                                                     16


<210>  1070
<211>  14
<212>  DNA
<213>  Artificial

<220>
<223>  Antisense nucleotide

<400>  1070
   ccttttccag cagg                                                      14

<210> 1071
<211> 16
<212> DNA
<213> Artificial

<220>
<223> Antisense nucleotide

<400> 1071
ggagaattca gacacc 16


<210> 1072
<211> 17
<212> DNA
<213> Artificial

<220>
<223> Antisense nucleotide

<400> 1072
ccaagtcctc attctgg 17


<210> 1073
<211> 16
<212> DNA
<213> Artificial

<220>
<223> Antisense nucleotide

<400> 1073
ccatcagtct cagagg 16


<210> 1074
<211> 16
<212> DNA
<213> Artificial

<220>
<223> Antisense nucleotide

<400> 1074
cctttgaagg tgctgg 16


<210> 1075
<211> 15
<212> DNA
<213> Artificial

<220>
<223> Antisense nucleotide

<400> 1075
ggcatggcag gttcc 15


<210> 1076
<211> 14
<212> DNA
<213> Artificial

<220>
<223> Antisense nucleotide

<400>  1076
cctggcatgg cagg                                                         14


<210>  1077
<211>  14
<212>  DNA
<213>  Artificial

<220>
<223>  Antisense nucleotide

<400>  1077
agatgtatag gtaa                                                         14


<210>  1078
<211>  14
<212>  DNA
<213>  Artificial

<220>
<223>  Antisense nucleotide

<400>  1078
attttcacat tctc                                                         14


<210>  1079
<211>  15
<212>  DNA
<213>  Artificial

<220>
<223>  Antisense nucleotide

<400>  1079
aattttcaca ttctc                                                        15


<210>  1080
<211>  14
<212>  DNA
<213>  Artificial

<220>
<223>  Antisense nucleotide

<400>  1080
aattttcaca ttct                                                         14


<210>  1081
<211>  14
<212>  DNA
<213>  Artificial

<220>
<223>  Antisense nucleotide

<400>  1081
gaattttcac attc                                                         14


<210>  1082
<211>  14
<212>  DNA
<213>  Artificial

<220>
<223>  Antisense nucleotide

<400>  1082
ggaattttca catt                                                          14


<210>  1083
<211>  14
<212>  DNA
<213>  Artificial

<220>
<223>  Antisense nucleotide

<400>  1083
agatttcttt gttg                                                          14


<210>  1084
<211>  15
<212>  DNA
<213>  Artificial

<220>
<223>  Antisense nucleotide

<400>  1084
aagatttctt tgttg                                                         15


<210>  1085
<211>  14
<212>  DNA
<213>  Artificial

<220>
<223>  Antisense nucleotide

<400>  1085
aagatttctt tgtt                                                          14


<210>  1086
<211>  15
<212>  DNA
<213>  Artificial

<220>
<223>  Antisense nucleotide

<400>  1086
taagatttct ttgtt                                                         15


<210>  1087
<211>  16
<212>  DNA
<213>  Artificial

<220>
<223>  Antisense nucleotide

<400>  1087
ctaagatttc tttgtt                                                        16

<210> 1088
<211> 14
<212> DNA
<213> Artificial

<220>
<223> Antisense nucleotide

<400> 1088
taagatttct ttgt 14

<210> 1089
<211> 15
<212> DNA
<213> Artificial

<220>
<223> Antisense nucleotide

<400> 1089
ctaagatttc tttgt 15

<210> 1090
<211> 14
<212> DNA
<213> Artificial

<220>
<223> Antisense nucleotide

<400> 1090
ctaagatttc tttg 14

<210> 1091
<211> 14
<212> DNA
<213> Artificial

<220>
<223> Antisense nucleotide

<400> 1091
tctaagattt cttt 14

<210> 1092
<211> 15
<212> DNA
<213> Artificial

<220>
<223> Antisense nucleotide

<400> 1092
gtctaagatt tcttt 15

<210> 1093
<211> 14
<212> DNA
<213> Artificial

<220>
<223> Antisense nucleotide

<400>  1093
gtctaagatt tctt                                                         14


<210>  1094
<211>  14
<212>  DNA
<213>  Artificial

<220>
<223>  Antisense nucleotide

<400>  1094
ttcgtctaag attt                                                         14


<210>  1095
<211>  14
<212>  DNA
<213>  Artificial

<220>
<223>  Antisense nucleotide

<400>  1095
attttgacat ggtt                                                         14


<210>  1096
<211>  15
<212>  DNA
<213>  Artificial

<220>
<223>  Antisense nucleotide

<400>  1096
aattttgaca tggtt                                                        15


<210>  1097
<211>  14
<212>  DNA
<213>  Artificial

<220>
<223>  Antisense nucleotide

<400>  1097
aattttgaca tggt                                                         14


<210>  1098
<211>  15
<212>  DNA
<213>  Artificial

<220>
<223>  Antisense nucleotide

<400>  1098
taattttgac atggt                                                        15


<210>  1099
<211>  14
<212>  DNA
<213>  Artificial

<220>
<223>  Antisense nucleotide

<400>  1099
taattttgac atgg                                                         14


<210>  1100
<211>  14
<212>  DNA
<213>  Artificial

<220>
<223>  Antisense nucleotide

<400>  1100
gtaattttga catg                                                         14


<210>  1101
<211>  15
<212>  DNA
<213>  Artificial

<220>
<223>  Antisense nucleotide

<400>  1101
tgtaattttg acatg                                                        15


<210>  1102
<211>  14
<212>  DNA
<213>  Artificial

<220>
<223>  Antisense nucleotide

<400>  1102
tgtaattttg acat                                                         14


<210>  1103
<211>  16
<212>  DNA
<213>  Artificial

<220>
<223>  Antisense nucleotide

<400>  1103
tctgtaattt tgacat                                                       16


<210>  1104
<211>  14
<212>  DNA
<213>  Artificial

<220>
<223>  Antisense nucleotide

<400>  1104
ctgtaatttt gaca                                                         14

<210> 1105
<211> 15
<212> DNA
<213> Artificial

<220>
<223> Antisense nucleotide

<400> 1105
tctgtaattt tgaca 15


<210> 1106
<211> 14
<212> DNA
<213> Artificial

<220>
<223> Antisense nucleotide

<400> 1106
tctgtaattt tgac 14


<210> 1107
<211> 14
<212> DNA
<213> Artificial

<220>
<223> Antisense nucleotide

<400> 1107
gtctgtaatt ttga 14


<210> 1108
<211> 16
<212> DNA
<213> Artificial

<220>
<223> Antisense nucleotide

<400> 1108
aagtctgtaa ttttga 16


<210> 1109
<211> 14
<212> DNA
<213> Artificial

<220>
<223> Antisense nucleotide

<400> 1109
agtctgtaat tttg 14


<210> 1110
<211> 15
<212> DNA
<213> Artificial

<220>
<223> Antisense nucleotide

<400>  1110
aagtctgtaa ttttg                                                        15


<210>  1111
<211>  14
<212>  DNA
<213>  Artificial

<220>
<223>  Antisense nucleotide

<400>  1111
aagtctgtaa tttt                                                         14


<210>  1112
<211>  15
<212>  DNA
<213>  Artificial

<220>
<223>  Antisense nucleotide

<400>  1112
gaagtctgta atttt                                                        15


<210>  1113
<211>  14
<212>  DNA
<213>  Artificial

<220>
<223>  Antisense nucleotide

<400>  1113
gaagtctgta attt                                                         14


<210>  1114
<211>  14
<212>  DNA
<213>  Artificial

<220>
<223>  Antisense nucleotide

<400>  1114
atgtagacat caat                                                         14


<210>  1115
<211>  14
<212>  DNA
<213>  Artificial

<220>
<223>  Antisense nucleotide

<400>  1115
atcatccaac attt                                                         14


<210>  1116
<211>  15
<212>  DNA
<213>  Artificial

<220>
<223>  Antisense nucleotide

<400>  1116
aatcatccaa cattt                                                        15


<210>  1117
<211>  14
<212>  DNA
<213>  Artificial

<220>
<223>  Antisense nucleotide

<400>  1117
aatcatccaa catt                                                         14


<210>  1118
<211>  14
<212>  DNA
<213>  Artificial

<220>
<223>  Antisense nucleotide

<400>  1118
accatcaaat acat                                                         14


<210>  1119
<211>  14
<212>  DNA
<213>  Artificial

<220>
<223>  Antisense nucleotide

<400>  1119
aaaaacgtct ttga                                                         14


<210>  1120
<211>  15
<212>  DNA
<213>  Artificial

<220>
<223>  Antisense nucleotide

<400>  1120
ttttgttctt agaca                                                        15


<210>  1121
<211>  14
<212>  DNA
<213>  Artificial

<220>
<223>  Antisense nucleotide

<400>  1121
ttttgttctt agac                                                         14

<210>  1122
<211>  14
<212>  DNA
<213>  Artificial

<220>
<223>  Antisense nucleotide

<400>  1122
taaacagaaa agca                                                          14


<210>  1123
<211>  14
<212>  DNA
<213>  Artificial

<220>
<223>  Antisense nucleotide

<400>  1123
actaaacaga aaag                                                          14


<210>  1124
<211>  16
<212>  DNA
<213>  Artificial

<220>
<223>  Antisense nucleotide

<400>  1124
aaactaaaca gaaaag                                                        16


<210>  1125
<211>  14
<212>  DNA
<213>  Artificial

<220>
<223>  Antisense nucleotide

<400>  1125
aactaaacag aaaa                                                          14


<210>  1126
<211>  15
<212>  DNA
<213>  Artificial

<220>
<223>  Antisense nucleotide

<400>  1126
aaactaaaca gaaaa                                                         15


<210>  1127
<211>  14
<212>  DNA
<213>  Artificial

<220>
<223>  Antisense nucleotide

<400>  1127
aaactaaaca gaaa                                                          14


<210>  1128
<211>  17
<212>  DNA
<213>  Artificial

<220>
<223>  Antisense nucleotide

<400>  1128
taaaaactaa acagaaa                                                       17


<210>  1129
<211>  14
<212>  DNA
<213>  Artificial

<220>
<223>  Antisense nucleotide

<400>  1129
aaaactaaac agaa                                                          14


<210>  1130
<211>  17
<212>  DNA
<213>  Artificial

<220>
<223>  Antisense nucleotide

<400>  1130
gtaaaaacta aacagaa                                                       17


<210>  1131
<211>  14
<212>  DNA
<213>  Artificial

<220>
<223>  Antisense nucleotide

<400>  1131
aaaaactaaa caga                                                          14


<210>  1132
<211>  15
<212>  DNA
<213>  Artificial

<220>
<223>  Antisense nucleotide

<400>  1132
taaaaactaa acaga                                                         15


<210>  1133
<211>  14
<212>  DNA
<213>  Artificial

<220>
<223>  Antisense nucleotide

<400>  1133
taaaaactaa acag                                                          14


<210>  1134
<211>  14
<212>  DNA
<213>  Artificial

<220>
<223>  Antisense nucleotide

<400>  1134
gtaaaaacta aaca                                                          14


<210>  1135
<211>  19
<212>  DNA
<213>  Artificial

<220>
<223>  Antisense nucleotide

<400>  1135
aaaaagtaaa aactaaaca                                                     19


<210>  1136
<211>  14
<212>  DNA
<213>  Artificial

<220>
<223>  Antisense nucleotide

<400>  1136
agtaaaaact aaac                                                          14


<210>  1137
<211>  20
<212>  DNA
<213>  Artificial

<220>
<223>  Antisense nucleotide

<400>  1137
aaaaaaagta aaaactaaac                                                    20


<210>  1138
<211>  14
<212>  DNA
<213>  Artificial

<220>
<223>  Antisense nucleotide

<400>  1138
aagtaaaaac taaa                                                          14

<210>  1139
<211>  19
<212>  DNA
<213>  Artificial

<220>
<223>  Antisense nucleotide

<400>  1139
aaaaaaagta aaaactaaa                                                    19


<210>  1140
<211>  14
<212>  DNA
<213>  Artificial

<220>
<223>  Antisense nucleotide

<400>  1140
aaagtaaaaa ctaa                                                         14


<210>  1141
<211>  14
<212>  DNA
<213>  Artificial

<220>
<223>  Antisense nucleotide

<400>  1141
aaaagtaaaa acta                                                         14


<210>  1142
<211>  17
<212>  DNA
<213>  Artificial

<220>
<223>  Antisense nucleotide

<400>  1142
aaaaaaagta aaaacta                                                      17


<210>  1143
<211>  14
<212>  DNA
<213>  Artificial

<220>
<223>  Antisense nucleotide

<400>  1143
aaaaagtaaa aact                                                         14


<210>  1144
<211>  16
<212>  DNA
<213>  Artificial

<220>
<223>  Antisense nucleotide

<400>  1144
aaaaaaagta aaaact                                                        16


<210>  1145
<211>  15
<212>  DNA
<213>  Artificial

<220>
<223>  Antisense nucleotide

<400>  1145
aaaaaaagta aaaac                                                         15


<210>  1146
<211>  16
<212>  DNA
<213>  Artificial

<220>
<223>  Antisense nucleotide

<400>  1146
caaaaaaagt aaaaac                                                        16


<210>  1147
<211>  14
<212>  DNA
<213>  Artificial

<220>
<223>  Antisense nucleotide

<400>  1147
aaaaaaagta aaaa                                                          14


<210>  1148
<211>  14
<212>  DNA
<213>  Artificial

<220>
<223>  Antisense nucleotide

<400>  1148
caaaaaaagt aaaa                                                          14


<210>  1149
<211>  20
<212>  DNA
<213>  Artificial

<220>
<223>  Antisense nucleotide

<400>  1149
aacaaaacaa aaaaagtaaa                                                    20


<210>  1150
<211>  14
<212>  DNA
<213>  Artificial

<220>
<223>  Antisense nucleotide

<400>  1150
aaacaaaaaa agta                                                          14


<210>  1151
<211>  16
<212>  DNA
<213>  Artificial

<220>
<223>  Antisense nucleotide

<400>  1151
caaaacaaaa aaagta                                                        16


<210>  1152
<211>  15
<212>  DNA
<213>  Artificial

<220>
<223>  Antisense nucleotide

<400>  1152
caaaacaaaa aaagt                                                         15


<210>  1153
<211>  14
<212>  DNA
<213>  Artificial

<220>
<223>  Antisense nucleotide

<400>  1153
caaaacaaaa aaag                                                          14


<210>  1154
<211>  17
<212>  DNA
<213>  Artificial

<220>
<223>  Antisense nucleotide

<400>  1154
ctttaaaaaa acaaaac                                                       17


<210>  1155
<211>  16
<212>  DNA
<213>  Artificial

<220>
<223>  Antisense nucleotide

<400>  1155
tctttaaaaa aacaaa                                                        16

<210>  1156
<211>  17
<212>  DNA
<213>  Artificial

<220>
<223>  Antisense nucleotide

<400>  1156
gtctttaaaa aaacaaa                                                    17


<210>  1157
<211>  15
<212>  DNA
<213>  Artificial

<220>
<223>  Antisense nucleotide

<400>  1157
gtctttaaaa aaaca                                                      15


<210>  1158
<211>  14
<212>  DNA
<213>  Artificial

<220>
<223>  Antisense nucleotide

<400>  1158
gtctttaaaa aaac                                                       14


<210>  1159
<211>  14
<212>  DNA
<213>  Artificial

<220>
<223>  Antisense nucleotide

<400>  1159
tttatttcgt cttt                                                       14


<210>  1160
<211>  14
<212>  DNA
<213>  Artificial

<220>
<223>  Antisense nucleotide

<400>  1160
tctttatttc gtct                                                       14


<210>  1161
<211>  14
<212>  DNA
<213>  Artificial

<220>
<223>  Antisense nucleotide

<400>  1161
tatttgcaaa tgga                                                          14


<210>  1162
<211>  15
<212>  DNA
<213>  Artificial

<220>
<223>  Antisense nucleotide

<400>  1162
tatatttgca aatgg                                                         15


<210>  1163
<211>  1854
<212>  DNA
<213>  Artificial

<220>
<223>  Antisense nucleotide


<220>
<221>  misc_feature
<222>  (17)..(17)
<223>  n is a, c, g, or t

<220>
<221>  misc_feature
<222>  (45)..(45)
<223>  n is a, c, g, or t

<220>
<221>  misc_feature
<222>  (72)..(72)
<223>  n is a, c, g, or t

<220>
<221>  misc_feature
<222>  (96)..(96)
<223>  n is a, c, g, or t

<220>
<221>  misc_feature
<222>  (119)..(119)
<223>  n is a, c, g, or t

<220>
<221>  misc_feature
<222>  (144)..(144)
<223>  n is a, c, g, or t

<220>
<221>  misc_feature
<222>  (170)..(170)
<223>  n is a, c, g, or t

<220>
<221>  misc_feature
<222>  (196)..(196)
<223>  n is a, c, g, or t

<220>
<221>  misc_feature
<222>  (219)..(219)

<223>  n is a, c, g, or t

<220>
<221>  misc_feature
<222>  (242)..(242)
<223>  n is a, c, g, or t

<220>
<221>  misc_feature
<222>  (269)..(269)
<223>  n is a, c, g, or t

<220>
<221>  misc_feature
<222>  (295)..(295)
<223>  n is a, c, g, or t

<220>
<221>  misc_feature
<222>  (319)..(319)
<223>  n is a, c, g, or t

<220>
<221>  misc_feature
<222>  (346)..(346)
<223>  n is a, c, g, or t

<220>
<221>  misc_feature
<222>  (370)..(370)
<223>  n is a, c, g, or t

<220>
<221>  misc_feature
<222>  (393)..(393)
<223>  n is a, c, g, or t

<220>
<221>  misc_feature
<222>  (416)..(416)
<223>  n is a, c, g, or t

<220>
<221>  misc_feature
<222>  (439)..(439)
<223>  n is a, c, g, or t

<220>
<221>  misc_feature
<222>  (462)..(462)
<223>  n is a, c, g, or t

<220>
<221>  misc_feature
<222>  (485)..(485)
<223>  n is a, c, g, or t

<220>
<221>  misc_feature
<222>  (510)..(510)
<223>  n is a, c, g, or t

<220>
<221>  misc_feature
<222>  (533)..(533)
<223>  n is a, c, g, or t

<220>

<221>  misc_feature
<222>  (556)..(556)
<223>  n is a, c, g, or t

<220>
<221>  misc_feature
<222>  (579)..(579)
<223>  n is a, c, g, or t

<220>
<221>  misc_feature
<222>  (606)..(606)
<223>  n is a, c, g, or t

<220>
<221>  misc_feature
<222>  (630)..(630)
<223>  n is a, c, g, or t

<220>
<221>  misc_feature
<222>  (654)..(654)
<223>  n is a, c, g, or t

<220>
<221>  misc_feature
<222>  (677)..(677)
<223>  n is a, c, g, or t

<220>
<221>  misc_feature
<222>  (703)..(703)
<223>  n is a, c, g, or t

<220>
<221>  misc_feature
<222>  (730)..(730)
<223>  n is a, c, g, or t

<220>
<221>  misc_feature
<222>  (754)..(754)
<223>  n is a, c, g, or t

<220>
<221>  misc_feature
<222>  (777)..(777)
<223>  n is a, c, g, or t

<220>
<221>  misc_feature
<222>  (801)..(801)
<223>  n is a, c, g, or t

<220>
<221>  misc_feature
<222>  (825)..(825)
<223>  n is a, c, g, or t

<220>
<221>  misc_feature
<222>  (849)..(849)
<223>  n is a, c, g, or t

<220>
<221>  misc_feature
<222>  (873)..(873)
<223>  n is a, c, g, or t

<220>
<221>  misc_feature
<222>  (896)..(896)
<223>  n is a, c, g, or t

<220>
<221>  misc_feature
<222>  (920)..(920)
<223>  n is a, c, g, or t

<220>
<221>  misc_feature
<222>  (944)..(944)
<223>  n is a, c, g, or t

<220>
<221>  misc_feature
<222>  (970)..(970)
<223>  n is a, c, g, or t

<220>
<221>  misc_feature
<222>  (993)..(993)
<223>  n is a, c, g, or t

<220>
<221>  misc_feature
<222>  (1016)..(1016)
<223>  n is a, c, g, or t

<220>
<221>  misc_feature
<222>  (1039)..(1039)
<223>  n is a, c, g, or t

<220>
<221>  misc_feature
<222>  (1062)..(1062)
<223>  n is a, c, g, or t

<220>
<221>  misc_feature
<222>  (1085)..(1085)
<223>  n is a, c, g, or t

<220>
<221>  misc_feature
<222>  (1108)..(1108)
<223>  n is a, c, g, or t

<220>
<221>  misc_feature
<222>  (1131)..(1131)
<223>  n is a, c, g, or t

<220>
<221>  misc_feature
<222>  (1155)..(1155)
<223>  n is a, c, g, or t

<220>
<221>  misc_feature
<222>  (1180)..(1180)
<223>  n is a, c, g, or t

<220>
<221>  misc_feature

<222>  (1203)..(1203)
<223>  n is a, c, g, or t

<220>
<221>  misc_feature
<222>  (1227)..(1227)
<223>  n is a, c, g, or t

<220>
<221>  misc_feature
<222>  (1250)..(1250)
<223>  n is a, c, g, or t

<220>
<221>  misc_feature
<222>  (1273)..(1273)
<223>  n is a, c, g, or t

<220>
<221>  misc_feature
<222>  (1298)..(1298)
<223>  n is a, c, g, or t

<220>
<221>  misc_feature
<222>  (1321)..(1321)
<223>  n is a, c, g, or t

<220>
<221>  misc_feature
<222>  (1347)..(1347)
<223>  n is a, c, g, or t

<220>
<221>  misc_feature
<222>  (1371)..(1371)
<223>  n is a, c, g, or t

<220>
<221>  misc_feature
<222>  (1394)..(1394)
<223>  n is a, c, g, or t

<220>
<221>  misc_feature
<222>  (1420)..(1420)
<223>  n is a, c, g, or t

<220>
<221>  misc_feature
<222>  (1443)..(1443)
<223>  n is a, c, g, or t

<220>
<221>  misc_feature
<222>  (1466)..(1466)
<223>  n is a, c, g, or t

<220>
<221>  misc_feature
<222>  (1494)..(1494)
<223>  n is a, c, g, or t

<220>
<221>  misc_feature
<222>  (1523)..(1523)
<223>  n is a, c, g, or t

<220>
<221> misc_feature
<222> (1547)..(1547)
<223> n is a, c, g, or t

<220>
<221> misc_feature
<222> (1570)..(1570)
<223> n is a, c, g, or t

<220>
<221> misc_feature
<222> (1599)..(1599)
<223> n is a, c, g, or t

<220>
<221> misc_feature
<222> (1626)..(1626)
<223> n is a, c, g, or t

<220>
<221> misc_feature
<222> (1654)..(1654)
<223> n is a, c, g, or t

<220>
<221> misc_feature
<222> (1677)..(1677)
<223> n is a, c, g, or t

<220>
<221> misc_feature
<222> (1706)..(1706)
<223> n is a, c, g, or t

<220>
<221> misc_feature
<222> (1729)..(1729)
<223> n is a, c, g, or t

<220>
<221> misc_feature
<222> (1757)..(1757)
<223> n is a, c, g, or t

<220>
<221> misc_feature
<222> (1781)..(1781)
<223> n is a, c, g, or t

<220>
<221> misc_feature
<222> (1805)..(1805)
<223> n is a, c, g, or t

<220>
<221> misc_feature
<222> (1830)..(1830)
<223> n is a, c, g, or t

<400> 1163
tatatttgca aatgsdnaar tcacaaaata tatttgcaaa tgsdnaartc acaaaatata 60

tttgcaaats dnaartcaca aaatatattt gcasdnaart cacaaaatat atttgcsdna 120

artcattcca aaatatattt gsdnaartca ttttccaaaa tatatttsdn aartcagttt 180

tccaaaatat attsdnaart cagttttcca aaatatsdna artcaggtta ggcaaagccs 240

dnaartcacc gagaacatca tcgtggsdna artcaccgag aacatcatcg tgsdnaartc 300
accgagaaca tcatcgsdna artcacgtag tctgcgttga agcsdnaart caccatgctg 360
gagaaggsdn aartcaccgt gcagaagtcc sdnaartcag gaatgaagtt ggcsdnaart 420
catgaccgtg ggaatgsdna artcatggca gtgaccgtgs dnaartcaag atggcagtga 480
ccsdnaartc acgagatggc agtgaccsdn aartcaccag ccactgcagg sdnaartcag 540
caccagccac tgcsdnaart caccctggag taagccsdna artcaggaga taactgttcc 600
accsdnaart caggagataa ctgttccsdn aartcacttc tagttggtct gsdnaartca 660
catcttctag ttggsdnaar tcatctcatc ttctagttgg sdnaartcac tgcaaagcag 720
acttctcsdn aartcacctt cagcaggttg gsdnaartca cccaggtcat caggsdnaar 780
tcaccagtca gatcaaggsd naartcaggt gaaggcctcc tcsdnaartc acagggtgaa 840
ggcctcsdna artcacctgg atgatgctgg sdnaartcac cactgtgcag aggsdnaart 900
caggagtaca ggtgaccsdn aartcagctc attgctgctg csdnaartca ggaaggctca 960
ttgctgcsdn aartcatttt ctcttcttct sdnaartcaa tcttattcct ttcsdnaart 1020
cacatcttat tcctttsdna artcatagtt tttccttcts dnaartcatc tagtttttcc 1080
ttsdnaartc aaactctagt ttttcsdnaa rtcagaactc tagtttttsd naartcatga 1140
actctagttt ttsdnaartc aatgaactct agtttttsdn aartcatgaa ctctagtttt 1200
sdnaartcaa tgaactctag ttttsdnaar tcaatgaact ctagtttsdn aartcagcac 1260
acagtagtgc sdnaartcag caggatcaga aaagcsdnaa rtcagcaggt agacaggcsd 1320
naartcagct tgctcaggat ctgcsdnaar tcagcaagtc cctggtgcsd naartcacct 1380
ggagcaagtc csdnaartca cgtagtactc ttcgtcgsdn aartcacgta gtactcttcg 1440
sdnaartcag taaacctcct tggsdnaart cagtctattt tgtaaacctc csdnaartca 1500
gcatgtctat tttgtaaacc sdnaartcag gcatcaaggt acccsdnaar tcaggcatca 1560
aggtaccsdn aartcagctt tcaccaaatt ggaagcsdna artcagagaa tctgatatag 1620
ctcsdnaart caggagatgt taaatctttg gsdnaartca gctgtcgatg tagcsdnaar 1680
tcaccaggtt cctgtcttta tggsdnaart cacagcaggg acagtgsdna artcacttgc 1740
ttctagttct tcacsdnaar tcagccatca atacctgcsd naartcaggt gccatcaata 1800
ccsdnaartc accactggta tatgtggsdn aartcaggac tttatagttt tctg 1854

<210> 1164
<211> 16
<212> DNA
<213> Artificial

<220>
<223> Antisense nucleotide

<400> 1164
ctcaagtctg taggag 16

<210>  1165
<211>  15
<212>  DNA
<213>  Artificial

<220>
<223>  Antisense nucleotide

<400>  1165
ggtctgttgt gactc                                                        15


<210>  1166
<211>  19
<212>  DNA
<213>  Artificial

<220>
<223>  Antisense nucleotide

<400>  1166
caattatcct gcacatttc                                                    19


<210>  1167
<211>  16
<212>  DNA
<213>  Artificial

<220>
<223>  Antisense nucleotide

<400>  1167
gcagcaatta tcctgc                                                       16


<210>  1168
<211>  14
<212>  DNA
<213>  Artificial

<220>
<223>  Antisense nucleotide

<400>  1168
ggcagcaatt atcc                                                         14


<210>  1169
<211>  19
<212>  DNA
<213>  Artificial

<220>
<223>  Antisense nucleotide

<400>  1169
ggttcgtgta tccatttcc                                                    19


<210>  1170
<211>  14
<212>  DNA
<213>  Artificial

<220>
<223>  Antisense nucleotide

<400>  1170
gcacagaagt tggc                                                         14


<210>  1171
<211>  16
<212>  DNA
<213>  Artificial

<220>
<223>  Antisense nucleotide

<400>  1171
ccagcacaga agttgg                                                       16


<210>  1172
<211>  14
<212>  DNA
<213>  Artificial

<220>
<223>  Antisense nucleotide

<400>  1172
gtgctgagtg tctg                                                         14


<210>  1173
<211>  16
<212>  DNA
<213>  Artificial

<220>
<223>  Antisense nucleotide

<400>  1173
cctgctgtgc tgagtg                                                       16


<210>  1174
<211>  14
<212>  DNA
<213>  Artificial

<220>
<223>  Antisense nucleotide

<400>  1174
gctcaggacc ctgc                                                         14


<210>  1175
<211>  15
<212>  DNA
<213>  Artificial

<220>
<223>  Antisense nucleotide

<400>  1175
gcagcaagga gaagc                                                        15


<210>  1176
<211>  19
<212>  DNA
<213>  Artificial

<220>
<223>  Antisense nucleotide

<400>  1176
ccaatgtagt agagaatgg                                                    19


<210>  1177
<211>  14
<212>  DNA
<213>  Artificial

<220>
<223>  Antisense nucleotide

<400>  1177
gctgcatttg caag                                                         14


<210>  1178
<211>  14
<212>  DNA
<213>  Artificial

<220>
<223>  Antisense nucleotide

<400>  1178
aaaaaagaaa tcaa                                                         14


<210>  1179
<211>  15
<212>  DNA
<213>  Artificial

<220>
<223>  Antisense nucleotide

<400>  1179
aaaaaaagaa atcaa                                                        15


<210>  1180
<211>  16
<212>  DNA
<213>  Artificial

<220>
<223>  Antisense nucleotide

<400>  1180
aaaaaaaaga aatcaa                                                       16


<210>  1181
<211>  17
<212>  DNA
<213>  Artificial

<220>
<223>  Antisense nucleotide

<400>  1181
taaaaaaaag aaatcaa                                                      17

<210>  1182
<211>  18
<212>  DNA
<213>  Artificial

<220>
<223>  Antisense nucleotide

<400>  1182
ataaaaaaaa gaaatcaa                                                    18


<210>  1183
<211>  19
<212>  DNA
<213>  Artificial

<220>
<223>  Antisense nucleotide

<400>  1183
aataaaaaaa agaaatcaa                                                   19


<210>  1184
<211>  17
<212>  DNA
<213>  Artificial

<220>
<223>  Antisense nucleotide

<400>  1184
gaataaaaaa aagaaat                                                     17


<210>  1185
<211>  18
<212>  DNA
<213>  Artificial

<220>
<223>  Antisense nucleotide

<400>  1185
agaataaaaa aaagaaat                                                    18


<210>  1186
<211>  14
<212>  DNA
<213>  Artificial

<220>
<223>  Antisense nucleotide

<400>  1186
cagaataaaa aaaa                                                        14


<210>  1187
<211>  14
<212>  DNA
<213>  Artificial

<220>
<223>  Antisense nucleotide

<400>  1187
tcagaataaa aaaa                                                         14


<210>  1188
<211>  14
<212>  DNA
<213>  Artificial

<220>
<223>  Antisense nucleotide

<400>  1188
ttgtttttaa aagt                                                         14


<210>  1189
<211>  14
<212>  DNA
<213>  Artificial

<220>
<223>  Antisense nucleotide

<400>  1189
agttgttttt aaaa                                                         14


<210>  1190
<211>  15
<212>  DNA
<213>  Artificial

<220>
<223>  Antisense nucleotide

<400>  1190
aagttgtttt taaaa                                                        15


<210>  1191
<211>  16
<212>  DNA
<213>  Artificial

<220>
<223>  Antisense nucleotide

<400>  1191
aaagttgttt ttaaaa                                                       16


<210>  1192
<211>  17
<212>  DNA
<213>  Artificial

<220>
<223>  Antisense nucleotide

<400>  1192
aaaagttgtt tttaaaa                                                      17


<210>  1193
<211>  18
<212>  DNA
<213>  Artificial

<220>
<223>  Antisense nucleotide

<400>  1193
aaaaagttgt ttttaaaa                                                    18


<210>  1194
<211>  19
<212>  DNA
<213>  Artificial

<220>
<223>  Antisense nucleotide

<400>  1194
aaaaaagttg tttttaaaa                                                   19


<210>  1195
<211>  20
<212>  DNA
<213>  Artificial

<220>
<223>  Antisense nucleotide

<400>  1195
aaaaaaagtt gtttttaaaa                                                  20


<210>  1196
<211>  20
<212>  DNA
<213>  Artificial

<220>
<223>  Antisense nucleotide

<400>  1196
aaaaaaaagt tgtttttaaa                                                  20


<210>  1197
<211>  14
<212>  DNA
<213>  Artificial

<220>
<223>  Antisense nucleotide

<400>  1197
tttttaaaaa agtg                                                        14


<210>  1198
<211>  15
<212>  DNA
<213>  Artificial

<220>
<223>  Antisense nucleotide

<400>  1198
ttttttaaaa aagtg                                                       15

<210>  1199
<211>  16
<212>  DNA
<213>  Artificial

<220>
<223>  Antisense nucleotide

<400>  1199
atttttaaa aaagtg                                                        16


<210>  1200
<211>  16
<212>  DNA
<213>  Artificial

<220>
<223>  Antisense nucleotide

<400>  1200
catttttaa aaaagt                                                        16


<210>  1201
<211>  15
<212>  DNA
<213>  Artificial

<220>
<223>  Antisense nucleotide

<400>  1201
   gcatttttta aaaaa                                                         15


<210>  1202
<211>  16
<212>  DNA
<213>  Artificial

<220>
<223>  Antisense nucleotide

<400>  1202
   tgcatttttt aaaaaa                                                        16


<210>  1203
<211>  14
<212>  DNA
<213>  Artificial

<220>
<223>  Antisense nucleotide

<400>  1203
agcttatttt aaat                                                         14


<210>  1204
<211>  15
<212>  DNA
<213>  Artificial

<220>
<223>  Antisense nucleotide

<400> 1204
aagcttattt taaat 15

<210> 1205
<211> 16
<212> DNA
<213> Artificial

<220>
<223> Antisense nucleotide

<400> 1205
taagcttatt ttaaat 16

<210> 1206
<211> 14
<212> DNA
<213> Artificial

<220>
<223> Antisense nucleotide

<400> 1206
tgtaattatt agat 14

<210> 1207
<211> 15
<212> DNA
<213> Artificial

<220>
<223> Antisense nucleotide

<400> 1207
atgtaattat tagat 15

<210> 1208
<211> 14
<212> DNA
<213> Artificial

<220>
<223> Antisense nucleotide

<400> 1208
tgatgtaatt atta 14

<210> 1209
<211> 15
<212> DNA
<213> Artificial

<220>
<223> Antisense nucleotide

<400> 1209
atgatgtaat tatta 15

<210> 1210
<211> 14
<212> DNA
<213> Artificial

<220>
<223>  Antisense nucleotide

<400>  1210
atggtattat ataa                                                          14


<210>  1211
<211>  15
<212>  DNA
<213>  Artificial

<220>
<223>  Antisense nucleotide

<400>  1211
tatggtatta tataa                                                         15


<210>  1212
<211>  16
<212>  DNA
<213>  Artificial

<220>
<223>  Antisense nucleotide

<400>  1212
ttatggtatt atataa                                                        16


<210>  1213
<211>  17
<212>  DNA
<213>  Artificial

<220>
<223>  Antisense nucleotide

<400>  1213
tttatggtat tatataa                                                       17


<210>  1214
<211>  18
<212>  DNA
<213>  Artificial

<220>
<223>  Antisense nucleotide

<400>  1214
atttatggta ttatataa                                                      18


<210>  1215
<211>  14
<212>  DNA
<213>  Artificial

<220>
<223>  Antisense nucleotide

<400>  1215
aatcatatta gaaa                                                          14

<210>  1216
<211>  14
<212>  DNA
<213>  Artificial

<220>
<223>  Antisense nucleotide

<400>  1216
ttacaatcat atta                                                          14


<210>  1217
<211>  15
<212>  DNA
<213>  Artificial

<220>
<223>  Antisense nucleotide

<400>  1217
tttacaatca tatta                                                         15


<210>  1218
<211>  16
<212>  DNA
<213>  Artificial

<220>
<223>  Antisense nucleotide

<400>  1218
ggcatgacgc ctttcc                                                        16


<210>  1219
<211>  14
<212>  DNA
<213>  Artificial

<220>
<223>  Antisense nucleotide

<400>  1219
gcatgacgcc tttc                                                          14


<210>  1220
<211>  14
<212>  DNA
<213>  Artificial

<220>
<223>  Antisense nucleotide

<400>  1220
gcctgacgag aggc                                                          14


<210>  1221
<211>  15
<212>  DNA
<213>  Artificial

<220>
<223>  Antisense nucleotide

<400>  1221
ctcaagcctg acgag                                                        15


<210>  1222
<211>  16
<212>  DNA
<213>  Artificial

<220>
<223>  Antisense nucleotide

<400>  1222
ccacagttcc tttttc                                                       16


<210>  1223
<211>  18
<212>  DNA
<213>  Artificial

<220>
<223>  Antisense nucleotide

<400>  1223
gctgcaataa agatacag                                                     18


<210>  1224
<211>  16
<212>  DNA
<213>  Artificial

<220>
<223>  Antisense nucleotide

<400>  1224
gctgcaataa agatac                                                       16


<210>  1225
<211>  17
<212>  DNA
<213>  Artificial

<220>
<223>  Antisense nucleotide

<400>  1225
ggacactgat ttctatg                                                      17


<210>  1226
<211>  16
<212>  DNA
<213>  Artificial

<220>
<223>  Antisense nucleotide

<400>  1226
gcattatcaa ctttgg                                                       16


<210>  1227
<211>  16
<212>  DNA
<213>  Artificial

<220>
<223>  Antisense nucleotide

<400>  1227
acttttagca ccaatg                                                    16


<210>  1228
<211>  19
<212>  DNA
<213>  Artificial

<220>
<223>  Antisense nucleotide

<400>  1228
ccaagaaact tttagcacc                                                 19


<210>  1229
<211>  14
<212>  DNA
<213>  Artificial

<220>
<223>  Antisense nucleotide

<400>  1229
ccagatcatc ttcc                                                      14


<210>  1230
<211>  16
<212>  DNA
<213>  Artificial

<220>
<223>  Antisense nucleotide

<400>  1230
agtcaaggac acatag                                                    16


<210>  1231
<211>  16
<212>  DNA
<213>  Artificial

<220>
<223>  Antisense nucleotide

<400>  1231
tctttgagca acatgg                                                    16


<210>  1232
<211>  14
<212>  DNA
<213>  Artificial

<220>
<223>  Antisense nucleotide

<400>  1232
gggtataaca gctg                                                      14

<210>  1233
<211>  18
<212>  DNA
<213>  Artificial

<220>
<223>  Antisense nucleotide

<400>  1233
gaggtgaacc attaatgg                                                    18


<210>  1234
<211>  16
<212>  DNA
<213>  Artificial

<220>
<223>  Antisense nucleotide

<400>  1234
tcttcgtatc gtttag                                                      16


<210>  1235
<211>  15
<212>  DNA
<213>  Artificial

<220>
<223>  Antisense nucleotide

<400>  1235
tgttggatag tgttc                                                       15


<210>  1236
<211>  15
<212>  DNA
<213>  Artificial

<220>
<223>  Antisense nucleotide

<400>  1236
gttgatcact tgctg                                                       15


<210>  1237
<211>  15
<212>  DNA
<213>  Artificial

<220>
<223>  Antisense nucleotide

<400>  1237
ggattccatt actcg                                                       15


<210>  1238
<211>  20
<212>  DNA
<213>  Artificial

<220>
<223>  Antisense nucleotide

<400>  1238
gacatatgaa aaatgttgtc                                                    20


<210>  1239
<211>  17
<212>  DNA
<213>  Artificial

<220>
<223>  Antisense nucleotide

<400>  1239
gccaataaag acatatg                                                       17


<210>  1240
<211>  17
<212>  DNA
<213>  Artificial

<220>
<223>  Antisense nucleotide

<400>  1240
ccagaatcaa gattctg                                                       17


<210>  1241
<211>  16
<212>  DNA
<213>  Artificial

<220>
<223>  Antisense nucleotide

<400>  1241
ctgttccaga atcaag                                                        16


<210>  1242
<211>  20
<212>  DNA
<213>  Artificial

<220>
<223>  Antisense nucleotide

<400>  1242
gacaaatctg ttccagaatc                                                    20


<210>  1243
<211>  19
<212>  DNA
<213>  Artificial

<220>
<223>  Antisense nucleotide

<400>  1243
ggaaagacaa atctgttcc                                                     19


<210>  1244
<211>  16
<212>  DNA
<213>  Artificial

<220>
<223>  Antisense nucleotide

<400>  1244
gattaagagg acaagc                                                    16


<210>  1245
<211>  14
<212>  DNA
<213>  Artificial

<220>
<223>  Antisense nucleotide

<400>  1245
ggaagattaa gagg                                                      14


<210>  1246
<211>  18
<212>  DNA
<213>  Artificial

<220>
<223>  Antisense nucleotide

<400>  1246
gcagtgtgat tattctgg                                                  18


<210>  1247
<211>  19
<212>  DNA
<213>  Artificial

<220>
<223>  Antisense nucleotide

<400>  1247
ggagaaagat acatatctg                                                 19


<210>  1248
<211>  14
<212>  DNA
<213>  Artificial

<220>
<223>  Antisense nucleotide

<400>  1248
ggagatctta cagg                                                      14


<210>  1249
<211>  20
<212>  DNA
<213>  Artificial

<220>
<223>  Antisense nucleotide

<400>  1249
gcatttgcag tagaatttac                                                20

<210>  1250
<211>  14
<212>  DNA
<213>  Artificial

<220>
<223>  Antisense nucleotide

<400>  1250
cagtgaaaga gagg                                                          14


<210>  1251
<211>  14
<212>  DNA
<213>  Artificial

<220>
<223>  Antisense nucleotide

<400>  1251
gctagccgat acac                                                          14


<210>  1252
<211>  17
<212>  DNA
<213>  Artificial

<220>
<223>  Antisense nucleotide

<400>  1252
ggaagatcct tgtatgc                                                       17


<210>  1253
<211>  15
<212>  DNA
<213>  Artificial

<220>
<223>  Antisense nucleotide

<400>  1253
gcatgaggaa gatcc                                                         15


<210>  1254
<211>  16
<212>  DNA
<213>  Artificial

<220>
<223>  Antisense nucleotide

<400>  1254
ggagtcattt ttgttg                                                        16


<210>  1255
<211>  19
<212>  DNA
<213>  Artificial

<220>
<223>  Antisense nucleotide

<400>  1255
ccaattgata ctaagattc                                                    19


<210>  1256
<211>  15
<212>  DNA
<213>  Artificial

<220>
<223>  Antisense nucleotide

<400>  1256
tcttttgagc acacg                                                        15


<210>  1257
<211>  18
<212>  DNA
<213>  Artificial

<220>
<223>  Antisense nucleotide

<400>  1257
ccttcagcac ttcttttg                                                     18


<210>  1258
<211>  16
<212>  DNA
<213>  Artificial

<220>
<223>  Antisense nucleotide

<400>  1258
ggttgcttcc ttcagc                                                       16


<210>  1259
<211>  14
<212>  DNA
<213>  Artificial

<220>
<223>  Antisense nucleotide

<400>  1259
cagtggttta ggag                                                         14


<210>  1260
<211>  17
<212>  DNA
<213>  Artificial

<220>
<223>  Antisense nucleotide

<400>  1260
cctgagatcc tcatttc                                                      17


<210>  1261
<211>  17
<212>  DNA
<213>  Artificial

<220>
<223>  Antisense nucleotide

<400>  1261
ccaaggtcct gagatcc                                                    17


<210>  1262
<211>  16
<212>  DNA
<213>  Artificial

<220>
<223>  Antisense nucleotide

<400>  1262
ggtgtacaca gtgtcc                                                     16


<210>  1263
<211>  14
<212>  DNA
<213>  Artificial

<220>
<223>  Antisense nucleotide

<400>  1263
tatctttaat ttct                                                       14


<210>  1264
<211>  14
<212>  DNA
<213>  Artificial

<220>
<223>  Antisense nucleotide

<400>  1264
tcttttgaat ataa                                                       14


<210>  1265
<211>  15
<212>  DNA
<213>  Artificial

<220>
<223>  Antisense nucleotide

<400>  1265
ttcttttgaa tataa                                                      15


<210>  1266
<211>  16
<212>  DNA
<213>  Artificial

<220>
<223>  Antisense nucleotide

<400>  1266
tttcttttga atataa                                                     16

<210>  1267
<211>  17
<212>  DNA
<213>  Artificial

<220>
<223>  Antisense nucleotide

<400>  1267
ttttcttttg aatataa                                                    17


<210>  1268
<211>  18
<212>  DNA
<213>  Artificial

<220>
<223>  Antisense nucleotide

<400>  1268
tttttctttt gaatataa                                                   18


<210>  1269
<211>  14
<212>  DNA
<213>  Artificial

<220>
<223>  Antisense nucleotide

<400>  1269
atttctatgt tttt                                                       14


<210>  1270
<211>  14
<212>  DNA
<213>  Artificial

<220>
<223>  Antisense nucleotide

<400>  1270
ttaaagaatt tatg                                                       14


<210>  1271
<211>  14
<212>  DNA
<213>  Artificial

<220>
<223>  Antisense nucleotide

<400>  1271
gttaaagaat ttat                                                       14


<210>  1272
<211>  15
<212>  DNA
<213>  Artificial

<220>
<223>  Antisense nucleotide

<400>  1272
agttaaagaa tttat                                                        15


<210>  1273
<211>  16
<212>  DNA
<213>  Artificial

<220>
<223>  Antisense nucleotide

<400>  1273
aagttaaaga atttat                                                       16


<210>  1274
<211>  17
<212>  DNA
<213>  Artificial

<220>
<223>  Antisense nucleotide

<400>  1274
taagttaaag aatttat                                                      17


<210>  1275
<211>  14
<212>  DNA
<213>  Artificial

<220>
<223>  Antisense nucleotide

<400>  1275
tttagtaagt taaa                                                         14


<210>  1276
<211>  15
<212>  DNA
<213>  Artificial

<220>
<223>  Antisense nucleotide

<400>  1276
ttttagtaag ttaaa                                                        15


<210>  1277
<211>  14
<212>  DNA
<213>  Artificial

<220>
<223>  Antisense nucleotide

<400>  1277
atttctttta gtaa                                                         14


<210>  1278
<211>  15
<212>  DNA
<213>  Artificial

<220>
<223>  Antisense nucleotide

<400>  1278
aatttctttt agtaa                                                        15


<210>  1279
<211>  14
<212>  DNA
<213>  Artificial

<220>
<223>  Antisense nucleotide

<400>  1279
atcaatttct ttta                                                         14


<210>  1280
<211>  15
<212>  DNA
<213>  Artificial

<220>
<223>  Antisense nucleotide

<400>  1280
tatcaatttc tttta                                                        15


<210>  1281
<211>  14
<212>  DNA
<213>  Artificial

<220>
<223>  Antisense nucleotide

<400>  1281
aatatataag ttca                                                         14


<210>  1282
<211>  15
<212>  DNA
<213>  Artificial

<220>
<223>  Antisense nucleotide

<400>  1282
aaatatataa gttca                                                        15


<210>  1283
<211>  14
<212>  DNA
<213>  Artificial

<220>
<223>  Antisense nucleotide

<400>  1283
caaatatata agtt                                                         14

<210>  1284
<211>  15
<212>  DNA
<213>  Artificial

<220>
<223>  Antisense nucleotide

<400>  1284
tcaaatatat aagtt                                                        15


<210>  1285
<211>  14
<212>  DNA
<213>  Artificial

<220>
<223>  Antisense nucleotide

<400>  1285
tgtcaaatat ataa                                                         14


<210>  1286
<211>  14
<212>  DNA
<213>  Artificial

<220>
<223>  Antisense nucleotide

<400>  1286
aatttatttc agta                                                         14


<210>  1287
<211>  14
<212>  DNA
<213>  Artificial

<220>
<223>  Antisense nucleotide

<400>  1287
aataaaaatg tgat                                                         14


<210>  1288
<211>  15
<212>  DNA
<213>  Artificial

<220>
<223>  Antisense nucleotide

<400>  1288
taataaaaat gtgat                                                        15


<210>  1289
<211>  14
<212>  DNA
<213>  Artificial

<220>
<223>  Antisense nucleotide

<400>  1289
tagctaataa aaat                                                         14


<210>  1290
<211>  15
<212>  DNA
<213>  Artificial

<220>
<223>  Antisense nucleotide

<400>  1290
ttagctaata aaaat                                                        15


<210>  1291
<211>  16
<212>  DNA
<213>  Artificial

<220>
<223>  Antisense nucleotide

<400>  1291
tttagctaat aaaaat                                                       16


<210>  1292
<211>  14
<212>  DNA
<213>  Artificial

<220>
<223>  Antisense nucleotide

<400>  1292
aataaaatag tcaa                                                         14


<210>  1293
<211>  15
<212>  DNA
<213>  Artificial

<220>
<223>  Antisense nucleotide

<400>  1293
taataaaata gtcaa                                                        15


<210>  1294
<211>  16
<212>  DNA
<213>  Artificial

<220>
<223>  Antisense nucleotide

<400>  1294
ttaataaaat agtcaa                                                       16


<210>  1295
<211>  17
<212>  DNA
<213>  Artificial

<220>
<223>  Antisense nucleotide

<400>  1295
tttaataaaa tagtcaa                                                    17


<210>  1296
<211>  15
<212>  DNA
<213>  Artificial

<220>
<223>  Antisense nucleotide

<400>  1296
gtttaataaa atagt                                                      15


<210>  1297
<211>  16
<212>  DNA
<213>  Artificial

<220>
<223>  Antisense nucleotide

<400>  1297
agtttaataa aatagt                                                     16


<210>  1298
<211>  15
<212>  DNA
<213>  Artificial

<220>
<223>  Antisense nucleotide

<400>  1298
gagtttaata aaata                                                      15


<210>  1299
<211>  16
<212>  DNA
<213>  Artificial

<220>
<223>  Antisense nucleotide

<400>  1299
agagtttaat aaaata                                                     16


<210>  1300
<211>  14
<212>  DNA
<213>  Artificial

<220>
<223>  Antisense nucleotide

<400>  1300
aataattctt gtat                                                       14

<210>  1301
<211>  14
<212>  DNA
<213>  Artificial

<220>
<223>  Antisense nucleotide

<400>  1301
tatattacat tcat                                                          14


<210>  1302
<211>  14
<212>  DNA
<213>  Artificial

<220>
<223>  Antisense nucleotide

<400>  1302
atctatatta catt                                                          14


<210>  1303
<211>  14
<212>  DNA
<213>  Artificial

<220>
<223>  Antisense nucleotide

<400>  1303
ataaacattt ttca                                                          14


<210>  1304
<211>  15
<212>  DNA
<213>  Artificial

<220>
<223>  Antisense nucleotide

<400>  1304
aataaacatt tttca                                                         15


<210>  1305
<211>  16
<212>  DNA
<213>  Artificial

<220>
<223>  Antisense nucleotide

<400>  1305
aaataaacat ttttca                                                        16


<210>  1306
<211>  15
<212>  DNA
<213>  Artificial

<220>
<223>  Antisense nucleotide

<400>  1306
gaaataaaca ttttt                                                        15


<210>  1307
<211>  16
<212>  DNA
<213>  Artificial

<220>
<223>  Antisense nucleotide

<400>  1307
tgaaataaac attttt                                                       16


<210>  1308
<211>  17
<212>  DNA
<213>  Artificial

<220>
<223>  Antisense nucleotide

<400>  1308
   ttgaaataaa cattttt                                                       17


<210>  1309
<211>  18
<212>  DNA
<213>  Artificial

<220>
<223>  Antisense nucleotide

<400>  1309
tttgaaataa acattttt                                                     18


<210>  1310
<211>  19
<212>  DNA
<213>  Artificial

<220>
<223>  Antisense nucleotide

<400>  1310
ttttgaaata aacattttt                                                    19


<210>  1311
<211>  20
<212>  DNA
<213>  Artificial

<220>
<223>  Antisense nucleotide

<400>  1311
tttttgaaat aaacattttt                                                   20


<210>  1312
<211>  20
<212>  DNA
<213>  Artificial

<220>
<223>  Antisense nucleotide

<400>  1312
atttttgaaa taaacatttt                                                    20


<210>  1313
<211>  19
<212>  DNA
<213>  Artificial

<220>
<223>  Antisense nucleotide

<400>  1313
aatttttgaa ataaacatt                                                     19


<210>  1314
<211>  20
<212>  DNA
<213>  Artificial

<220>
<223>  Antisense nucleotide

<400>  1314
aaatttttga aataaacatt                                                    20


<210>  1315
<211>  20
<212>  DNA
<213>  Artificial

<220>
<223>  Antisense nucleotide

<400>  1315
aaaatttttg aaataaacat                                                    20


<210>  1316
<211>  20
<212>  DNA
<213>  Artificial

<220>
<223>  Antisense nucleotide

<400>  1316
taaaattttt gaaataaaca                                                    20


<210>  1317
<211>  20
<212>  DNA
<213>  Artificial

<220>
<223>  Antisense nucleotide

<400>  1317
ataaaatttt tgaaataaac                                                    20

<210>  1318
<211>  20
<212>  DNA
<213>  Artificial

<220>
<223>  Antisense nucleotide

<400>  1318
tataaaattt ttgaaataaa                                                    20


<210>  1319
<211>  18
<212>  DNA
<213>  Artificial

<220>
<223>  Antisense nucleotide

<400>  1319
gtataaaatt tttgaaat                                                      18


<210>  1320
<211>  14
<212>  DNA
<213>  Artificial

<220>
<223>  Antisense nucleotide

<400>  1320
ggtataaaat tttt                                                          14


<210>  1321
<211>  15
<212>  DNA
<213>  Artificial

<220>
<223>  Antisense nucleotide

<400>  1321
aggtataaaa ttttt                                                         15


<210>  1322
<211>  16
<212>  DNA
<213>  Artificial

<220>
<223>  Antisense nucleotide

<400>  1322
aaggtataaa attttt                                                        16


<210>  1323
<211>  17
<212>  DNA
<213>  Artificial

<220>
<223>  Antisense nucleotide

<400>  1323
aaaggtataa aattttt                                                    17


<210>  1324
<211>  18
<212>  DNA
<213>  Artificial

<220>
<223>  Antisense nucleotide

<400>  1324
aaaaggtata aaattttt                                                   18


<210>  1325
<211>  19
<212>  DNA
<213>  Artificial

<220>
<223>  Antisense nucleotide

<400>  1325
taaaaggtat aaaattttt                                                  19


<210>  1326
<211>  20
<212>  DNA
<213>  Artificial

<220>
<223>  Antisense nucleotide

<400>  1326
ataaaaggta taaaattttt                                                 20


<210>  1327
<211>  14
<212>  DNA
<213>  Artificial

<220>
<223>  Antisense nucleotide

<400>  1327
tttagaaaga tttt                                                       14


<210>  1328
<211>  14
<212>  DNA
<213>  Artificial

<220>
<223>  Antisense nucleotide

<400>  1328
aagataaatt tctt                                                       14


<210>  1329
<211>  15
<212>  DNA
<213>  Artificial

<220>
<223>  Antisense nucleotide

<400>  1329
taagataaat ttctt                                                        15


<210>  1330
<211>  16
<212>  DNA
<213>  Artificial

<220>
<223>  Antisense nucleotide

<400>  1330
ttaagataaa tttctt                                                       16


<210>  1331
<211>  17
<212>  DNA
<213>  Artificial

<220>
<223>  Antisense nucleotide

<400>  1331
tttaagataa atttctt                                                      17


<210>  1332
<211>  18
<212>  DNA
<213>  Artificial

<220>
<223>  Antisense nucleotide

<400>  1332
ttttaagata aatttctt                                                     18


<210>  1333
<211>  19
<212>  DNA
<213>  Artificial

<220>
<223>  Antisense nucleotide

<400>  1333
tttttaagat aaatttctt                                                    19


<210>  1334
<211>  20
<212>  DNA
<213>  Artificial

<220>
<223>  Antisense nucleotide

<400>  1334
atttttaaga taaatttctt                                                   20

<210>  1335
<211>  20
<212>  DNA
<213>  Artificial

<220>
<223>  Antisense nucleotide

<400>  1335
tatttttaag ataaatttct                                                    20


<210>  1336
<211>  18
<212>  DNA
<213>  Artificial

<220>
<223>  Antisense nucleotide

<400>  1336
ttatttttaa gataaatt                                                      18


<210>  1337
<211>  19
<212>  DNA
<213>  Artificial

<220>
<223>  Antisense nucleotide

<400>  1337
tttattttta agataaatt                                                     19


<210>  1338
<211>  19
<212>  DNA
<213>  Artificial

<220>
<223>  Antisense nucleotide

<400>  1338
ctttattttt aagataaat                                                     19


<210>  1339
<211>  20
<212>  DNA
<213>  Artificial

<220>
<223>  Antisense nucleotide

<400>  1339
tctttatttt taagataaat                                                    20


<210>  1340
<211>  20
<212>  DNA
<213>  Artificial

<220>
<223>  Antisense nucleotide

<400>  1340
atctttattt ttaagataaa                                                    20


<210>  1341
<211>  14
<212>  DNA
<213>  Artificial

<220>
<223>  Antisense nucleotide

<400>  1341
atctttattt ttaa                                                          14


<210>  1342
<211>  15
<212>  DNA
<213>  Artificial

<220>
<223>  Antisense nucleotide

<400>  1342
gatctttatt tttaa                                                         15


<210>  1343
<211>  16
<212>  DNA
<213>  Artificial

<220>
<223>  Antisense nucleotide

<400>  1343
agatctttat ttttaa                                                        16


<210>  1344
<211>  17
<212>  DNA
<213>  Artificial

<220>
<223>  Antisense nucleotide

<400>  1344
tagatcttta tttttaa                                                       17


<210>  1345
<211>  14
<212>  DNA
<213>  Artificial

<220>
<223>  Antisense nucleotide

<400>  1345
aatcatcatt aatt                                                          14


<210>  1346
<211>  15
<212>  DNA
<213>  Artificial

<220>
<223>  Antisense nucleotide

<400>  1346
aaatcatcat taatt                                                        15


<210>  1347
<211>  16
<212>  DNA
<213>  Artificial

<220>
<223>  Antisense nucleotide

<400>  1347
aaaatcatca ttaatt                                                       16


<210>  1348
<211>  17
<212>  DNA
<213>  Artificial

<220>
<223>  Antisense nucleotide

<400>  1348
taaaatcatc attaatt                                                      17


<210>  1349
<211>  18
<212>  DNA
<213>  Artificial

<220>
<223>  Antisense nucleotide

<400>  1349
ttaaaatcat cattaatt                                                     18


<210>  1350
<211>  19
<212>  DNA
<213>  Artificial

<220>
<223>  Antisense nucleotide

<400>  1350
tttaaaatca tcattaatt                                                    19


<210>  1351
<211>  20
<212>  DNA
<213>  Artificial

<220>
<223>  Antisense nucleotide

<400>  1351
atttaaaatc atcattaatt                                                   20

<210>  1352
<211>  19
<212>  DNA
<213>  Artificial

<220>
<223>  Antisense nucleotide

<400>  1352
aatttaaaat catcattaa                                                    19


<210>  1353
<211>  14
<212>  DNA
<213>  Artificial

<220>
<223>  Antisense nucleotide

<400>  1353
gaatttaaaa tcat                                                         14


<210>  1354
<211>  15
<212>  DNA
<213>  Artificial

<220>
<223>  Antisense nucleotide

<400>  1354
tgaatttaaa atcat                                                        15


<210>  1355
<211>  14
<212>  DNA
<213>  Artificial

<220>
<223>  Antisense nucleotide

<400>  1355
ttaaaatagg aaat                                                         14


<210>  1356
<211>  14
<212>  DNA
<213>  Artificial

<220>
<223>  Antisense nucleotide

<400>  1356
aatttctctt taaa                                                         14


<210>  1357
<211>  15
<212>  DNA
<213>  Artificial

<220>
<223>  Antisense nucleotide

<400>  1357
aaatttctct ttaaa                                                        15


<210>  1358
<211>  14
<212>  DNA
<213>  Artificial

<220>
<223>  Antisense nucleotide

<400>  1358
taaaattttg aatg                                                         14


<210>  1359
<211>  14
<212>  DNA
<213>  Artificial

<220>
<223>  Antisense nucleotide

<400>  1359
ctaaaatttt gaat                                                         14


<210>  1360
<211>  14
<212>  DNA
<213>  Artificial

<220>
<223>  Antisense nucleotide

<400>  1360
tttgctaaaa tttt                                                         14


<210>  1361
<211>  14
<212>  DNA
<213>  Artificial

<220>
<223>  Antisense nucleotide

<400>  1361
atatgaaaaa tgtt                                                         14


<210>  1362
<211>  14
<212>  DNA
<213>  Artificial

<220>
<223>  Antisense nucleotide

<400>  1362
ttttaaatta agca                                                         14


<210>  1363
<211>  14
<212>  DNA
<213>  Artificial

<220>
<223>  Antisense nucleotide

<400>  1363
ttgtaaaaat caaa                                                          14


<210>  1364
<211>  15
<212>  DNA
<213>  Artificial

<220>
<223>  Antisense nucleotide

<400>  1364
tttgtaaaaa tcaaa                                                         15


<210>  1365
<211>  14
<212>  DNA
<213>  Artificial

<220>
<223>  Antisense nucleotide

<400>  1365
tttgataaaa cttt                                                          14


<210>  1366
<211>  14
<212>  DNA
<213>  Artificial

<220>
<223>  Antisense nucleotide

<400>  1366
atgttttatc attt                                                          14


<210>  1367
<211>  15
<212>  DNA
<213>  Artificial

<220>
<223>  Antisense nucleotide

<400>  1367
aatgttttat cattt                                                         15


<210>  1368
<211>  16
<212>  DNA
<213>  Artificial

<220>
<223>  Antisense nucleotide

<400>  1368
aaatgtttta tcattt                                                        16

<210>  1369
<211>  17
<212>  DNA
<213>  Artificial

<220>
<223>  Antisense nucleotide

<400>  1369
taaatgtttt atcattt                                                    17


<210>  1370
<211>  14
<212>  DNA
<213>  Artificial

<220>
<223>  Antisense nucleotide

<400>  1370
tctaaatgtt ttat                                                       14


<210>  1371
<211>  15
<212>  DNA
<213>  Artificial

<220>
<223>  Antisense nucleotide

<400>  1371
ttctaaatgt tttat                                                      15


<210>  1372
<211>  14
<212>  DNA
<213>  Artificial

<220>
<223>  Antisense nucleotide

<400>  1372
taagatcaaa taaa                                                       14


<210>  1373
<211>  15
<212>  DNA
<213>  Artificial

<220>
<223>  Antisense nucleotide

<400>  1373
ataagatcaa ataaa                                                      15


<210>  1374
<211>  16
<212>  DNA
<213>  Artificial

<220>
<223>  Antisense nucleotide

<400>  1374
aataagatca aataaa                                                        16


<210>  1375
<211>  17
<212>  DNA
<213>  Artificial

<220>
<223>  Antisense nucleotide

<400>  1375
taataagatc aaataaa                                                       17


<210>  1376
<211>  18
<212>  DNA
<213>  Artificial

<220>
<223>  Antisense nucleotide

<400>  1376
ttaataagat caaataaa                                                      18


<210>  1377
<211>  19
<212>  DNA
<213>  Artificial

<220>
<223>  Antisense nucleotide

<400>  1377
tttaataaga tcaaataaa                                                     19


<210>  1378
<211>  14
<212>  DNA
<213>  Artificial

<220>
<223>  Antisense nucleotide

<400>  1378
ttgtttaata agat                                                          14


<210>  1379
<211>  15
<212>  DNA
<213>  Artificial

<220>
<223>  Antisense nucleotide

<400>  1379
attgtttaat aagat                                                         15


<210>  1380
<211>  14
<212>  DNA
<213>  Artificial

<220>
<223>  Antisense nucleotide

<400>  1380
tgattgttta ataa                                                          14


<210>  1381
<211>  15
<212>  DNA
<213>  Artificial

<220>
<223>  Antisense nucleotide

<400>  1381
ttgattgttt aataa                                                         15


<210>  1382
<211>  16
<212>  DNA
<213>  Artificial

<220>
<223>  Antisense nucleotide

<400>  1382
tttgattgtt taataa                                                        16


<210>  1383
<211>  14
<212>  DNA
<213>  Artificial

<220>
<223>  Antisense nucleotide

<400>  1383
ttttataaaa cagt                                                          14


<210>  1384
<211>  15
<212>  DNA
<213>  Artificial

<220>
<223>  Antisense nucleotide

<400>  1384
tttttataaa acagt                                                         15


<210>  1385
<211>  16
<212>  DNA
<213>  Artificial

<220>
<223>  Antisense nucleotide

<400>  1385
ttttttataa aacagt                                                        16

<210>  1386
<211>  15
<212>  DNA
<213>  Artificial

<220>
<223>  Antisense nucleotide

<400>  1386
   cttttttata aaaca                                                    15


<210>  1387
<211>  16
<212>  DNA
<213>  Artificial

<220>
<223>  Antisense nucleotide

<400>  1387
   acttttttat aaaaca                                                   16


<210>  1388
<211>  15
<212>  DNA
<213>  Artificial

<220>
<223>  Antisense nucleotide

<400>  1388
   cactttttta taaaa                                                    15


<210>  1389
<211>  16
<212>  DNA
<213>  Artificial

<220>
<223>  Antisense nucleotide

<400>  1389
acactttttt ataaaa                                                  16


<210>  1390
<211>  17
<212>  DNA
<213>  Artificial

<220>
<223>  Antisense nucleotide

<400>  1390
tacacttttt tataaaa                                                 17


<210>  1391
<211>  18
<212>  DNA
<213>  Artificial

<220>
<223>  Antisense nucleotide

<400>  1391
atacactttt ttataaaa                                                    18


<210>  1392
<211>  14
<212>  DNA
<213>  Artificial

<220>
<223>  Antisense nucleotide

<400>  1392
attttgaatt taag                                                        14


<210>  1393
<211>  14
<212>  DNA
<213>  Artificial

<220>
<223>  Antisense nucleotide

<400>  1393
gattttgaat ttaa                                                        14


<210>  1394
<211>  15
<212>  DNA
<213>  Artificial

<220>
<223>  Antisense nucleotide

<400>  1394
tgattttgaa tttaa                                                       15


<210>  1395
<211>  16
<212>  DNA
<213>  Artificial

<220>
<223>  Antisense nucleotide

<400>  1395
atgattttga atttaa                                                      16


<210>  1396
<211>  17
<212>  DNA
<213>  Artificial

<220>
<223>  Antisense nucleotide

<400>  1396
aatgattttg aatttaa                                                     17


<210>  1397
<211>  14
<212>  DNA
<213>  Artificial

<220>
<223>  Antisense nucleotide

<400>  1397
ataatagaat cata                                                          14


<210>  1398
<211>  15
<212>  DNA
<213>  Artificial

<220>
<223>  Antisense nucleotide

<400>  1398
tataatagaa tcata                                                         15


<210>  1399
<211>  14
<212>  DNA
<213>  Artificial

<220>
<223>  Antisense nucleotide

<400>  1399
tataatagaa tcat                                                          14


<210>  1400
<211>  14
<212>  DNA
<213>  Artificial

<220>
<223>  Antisense nucleotide

<400>  1400
tactataata gaat                                                          14


<210>  1401
<211>  15
<212>  DNA
<213>  Artificial

<220>
<223>  Antisense nucleotide

<400>  1401
atactataat agaat                                                         15


<210>  1402
<211>  16
<212>  DNA
<213>  Artificial

<220>
<223>  Antisense nucleotide

<400>  1402
aatactataa tagaat                                                        16

<210>  1403
<211>  14
<212>  DNA
<213>  Artificial

<220>
<223>  Antisense nucleotide

<400>  1403
agaatactat aata                                                          14


<210>  1404
<211>  15
<212>  DNA
<213>  Artificial

<220>
<223>  Antisense nucleotide

<400>  1404
tagaatacta taata                                                         15


<210>  1405
<211>  16
<212>  DNA
<213>  Artificial

<220>
<223>  Antisense nucleotide

<400>  1405
atagaatact ataata                                                        16


<210>  1406
<211>  17
<212>  DNA
<213>  Artificial

<220>
<223>  Antisense nucleotide

<400>  1406
tatagaatac tataata                                                       17


<210>  1407
<211>  18
<212>  DNA
<213>  Artificial

<220>
<223>  Antisense nucleotide

<400>  1407
ttatagaata ctataata                                                      18


<210>  1408
<211>  14
<212>  DNA
<213>  Artificial

<220>
<223>  Antisense nucleotide

<400>  1408
aatatttgtt ttca                                                          14


<210>  1409
<211>  15
<212>  DNA
<213>  Artificial

<220>
<223>  Antisense nucleotide

<400>  1409
aaatatttgt tttca                                                         15


<210>  1410
<211>  16
<212>  DNA
<213>  Artificial

<220>
<223>  Antisense nucleotide

<400>  1410
aaaatatttg ttttca                                                        16


<210>  1411
<211>  15
<212>  DNA
<213>  Artificial

<220>
<223>  Antisense nucleotide

<400>  1411
caaaatattt gtttt                                                         15


<210>  1412
<211>  14
<212>  DNA
<213>  Artificial

<220>
<223>  Antisense nucleotide

<400>  1412
aaattttata tgga                                                          14


<210>  1413
<211>  14
<212>  DNA
<213>  Artificial

<220>
<223>  Antisense nucleotide

<400>  1413
tgaaatttta tatg                                                          14


<210>  1414
<211>  14
<212>  DNA
<213>  Artificial

<220>
<223>  Antisense nucleotide

<400>  1414
ctgaaatttt atat                                                          14


<210>  1415
<211>  15
<212>  DNA
<213>  Artificial

<220>
<223>  Antisense nucleotide

<400>  1415
tctgaaattt tatat                                                         15


<210>  1416
<211>  16
<212>  DNA
<213>  Artificial

<220>
<223>  Antisense nucleotide

<400>  1416
ttctgaaatt ttatat                                                        16


<210>  1417
<211>  14
<212>  DNA
<213>  Artificial

<220>
<223>  Antisense nucleotide

<400>  1417
atctgattta tttt                                                          14


<210>  1418
<211>  14
<212>  DNA
<213>  Artificial

<220>
<223>  Antisense nucleotide

<400>  1418
aagatattaa atgt                                                          14


<210>  1419
<211>  14
<212>  DNA
<213>  Artificial

<220>
<223>  Antisense nucleotide

<400>  1419
tgaagatatt aaat                                                          14

<210>  1420
<211>  14
<212>  DNA
<213>  Artificial

<220>
<223>  Antisense nucleotide

<400>  1420
ataaataaca atga                                                          14


<210>  1421
<211>  15
<212>  DNA
<213>  Artificial

<220>
<223>  Antisense nucleotide

<400>  1421
tataaataac aatga                                                         15


<210>  1422
<211>  14
<212>  DNA
<213>  Artificial

<220>
<223>  Antisense nucleotide

<400>  1422
gtataaataa caat                                                          14


<210>  1423
<211>  15
<212>  DNA
<213>  Artificial

<220>
<223>  Antisense nucleotide

<400>  1423
tgtataaata acaat                                                         15


<210>  1424
<211>  16
<212>  DNA
<213>  Artificial

<220>
<223>  Antisense nucleotide

<400>  1424
ttgtataaat aacaat                                                        16


<210>  1425
<211>  14
<212>  DNA
<213>  Artificial

<220>
<223>  Antisense nucleotide

<400>  1425
tcttgtataa ataa                                                          14


<210>  1426
<211>  15
<212>  DNA
<213>  Artificial

<220>
<223>  Antisense nucleotide

<400>  1426
atcttgtata aataa                                                         15


<210>  1427
<211>  16
<212>  DNA
<213>  Artificial

<220>
<223>  Antisense nucleotide

<400>  1427
aatcttgtat aaataa                                                        16


<210>  1428
<211>  14
<212>  DNA
<213>  Artificial

<220>
<223>  Antisense nucleotide

<400>  1428
acaacttttt aaat                                                          14


<210>  1429
<211>  15
<212>  DNA
<213>  Artificial

<220>
<223>  Antisense nucleotide

<400>  1429
tacaactttt taaat                                                         15


<210>  1430
<211>  14
<212>  DNA
<213>  Artificial

<220>
<223>  Antisense nucleotide

<400>  1430
tacaactttt taaa                                                          14


<210>  1431
<211>  21
<212>  DNA
<213>  Artificial

<220>
<223>  Antisense nucleotide

<400>  1431
cgggggtttt tgggcggcat g                                                21


<210>  1432
<211>  23
<212>  DNA
<213>  Artificial

<220>
<223>  Antisense nucleotide

<400>  1432
ttttcggggg gttttgggcg gca                                              23


<210>  1433
<211>  19
<212>  DNA
<213>  Artificial

<220>
<223>  Antisense nucleotide

<400>  1433
   tcggggggtt ttgggcggc                                                    19


<210>  1434
<211>  24
<212>  DNA
<213>  Artificial

<220>
<223>  Antisense nucleotide

<400>  1434
ggtggcggcc gtttttcggg gggt                                             24


<210>  1435
<211>  23
<212>  DNA
<213>  Artificial

<220>
<223>  Antisense nucleotide

<400>  1435
ccgggggttc cgcggcggca gcg                                              23


<210>  1436
<211>  17
<212>  DNA
<213>  Artificial

<220>
<223>  Antisense nucleotide

<400>  1436
cgggggttcc gcggcgg                                                     17

<210>  1437
<211>  23
<212>  DNA
<213>  Artificial

<220>
<223>  Antisense nucleotide

<400>  1437
ggcggcggtg ccgggggttc cgc                                              23


<210>  1438
<211>  22
<212>  DNA
<213>  Artificial

<220>
<223>  Antisense nucleotide

<400>  1438
ggagggggcg gcggcggcgg tg                                               22


<210>  1439
<211>  17
<212>  DNA
<213>  Artificial

<220>
<223>  Antisense nucleotide

<400>  1439
gggggcggcg gcggcgg                                                     17


<210>  1440
<211>  15
<212>  DNA
<213>  Artificial

<220>
<223>  Antisense nucleotide

<400>  1440
ggggcggcgg cggcg                                                       15


<210>  1441
<211>  17
<212>  DNA
<213>  Artificial

<220>
<223>  Antisense nucleotide

<400>  1441
agggggcctg gtggaag                                                     17


<210>  1442
<211>  14
<212>  DNA
<213>  Artificial

<220>
<223>  Antisense nucleotide

<400>  1442
tagggggcct ggtg                                                          14


<210>  1443
<211>  14
<212>  DNA
<213>  Artificial

<220>
<223>  Antisense nucleotide

<400>  1443
gtagggggcc tggt                                                          14


<210>  1444
<211>  26
<212>  DNA
<213>  Artificial

<220>
<223>  Antisense nucleotide

<400>  1444
gaggtattgg tgacaaggta gggggc                                             26


<210>  1445
<211>  26
<212>  DNA
<213>  Artificial

<220>
<223>  Antisense nucleotide

<400>  1445
tcttcagggg tgaaatatag atgttc                                             26


<210>  1446
<211>  16
<212>  DNA
<213>  Artificial

<220>
<223>  Antisense nucleotide

<400>  1446
ggactcttca ggggtg                                                        16


<210>  1447
<211>  14
<212>  DNA
<213>  Artificial

<220>
<223>  Antisense nucleotide

<400>  1447
tcggactata ctgc                                                          14


<210>  1448
<211>  16
<212>  DNA
<213>  Artificial

<220>
<223>  Antisense nucleotide

<400>  1448
cagttcggac tatact                                                        16


<210>  1449
<211>  14
<212>  DNA
<213>  Artificial

<220>
<223>  Antisense nucleotide

<400>  1449
aagcctaaga cgca                                                          14


<210>  1450
<211>  14
<212>  DNA
<213>  Artificial

<220>
<223>  Antisense nucleotide

<400>  1450
gcccaagttc aaca                                                          14


<210>  1451
<211>  14
<212>  DNA
<213>  Artificial

<220>
<223>  Antisense nucleotide

<400>  1451
tgaaaagtcg cggt                                                          14


<210>  1452
<211>  18
<212>  DNA
<213>  Artificial

<220>
<223>  Antisense nucleotide

<400>  1452
ggttaattaa gatgcctc                                                      18


<210>  1453
<211>  14
<212>  DNA
<213>  Artificial

<220>
<223>  Antisense nucleotide

<400>  1453
tctctaagag cgca                                                          14

<210>  1454
<211>  16
<212>  DNA
<213>  Artificial

<220>
<223>  Antisense nucleotide

<400>  1454
acgtgaggtt agtttg                                                        16


<210>  1455
<211>  14
<212>  DNA
<213>  Artificial

<220>
<223>  Antisense nucleotide

<400>  1455
cacgtgaggt tagt                                                          14


<210>  1456
<211>  14
<212>  DNA
<213>  Artificial

<220>
<223>  Antisense nucleotide

<400>  1456
catagaacag tccg                                                          14


<210>  1457
<211>  16
<212>  DNA
<213>  Artificial

<220>
<223>  Antisense nucleotide

<400>  1457
cagtcataga acagtc                                                        16


<210>  1458
<211>  18
<212>  DNA
<213>  Artificial

<220>
<223>  Antisense nucleotide

<400>  1458
ctttgcagtc atagaaca                                                      18


<210>  1459
<211>  14
<212>  DNA
<213>  Artificial

<220>
<223>  Antisense nucleotide

<400>  1459
tgcagtcata gaac                                                          14


<210>  1460
<211>  14
<212>  DNA
<213>  Artificial

<220>
<223>  Antisense nucleotide

<400>  1460
ggtcgtttcc atct                                                          14


<210>  1461
<211>  16
<212>  DNA
<213>  Artificial

<220>
<223>  Antisense nucleotide

<400>  1461
catagaaggt cgtttc                                                        16


<210>  1462
<211>  14
<212>  DNA
<213>  Artificial

<220>
<223>  Antisense nucleotide

<400>  1462
cgtcatagaa ggtc                                                          14


<210>  1463
<211>  15
<212>  DNA
<213>  Artificial

<220>
<223>  Antisense nucleotide

<400>  1463
catcgtcata gaagg                                                         15


<210>  1464
<211>  24
<212>  DNA
<213>  Artificial

<220>
<223>  Antisense nucleotide

<400>  1464
ggacgggagg aacgaggcgt tgag                                               24


<210>  1465
<211>  14
<212>  DNA
<213>  Artificial

<220>
<223>  Antisense nucleotide

<400>  1465
tagccataag gtcc                                                          14


<210>  1466
<211>  14
<212>  DNA
<213>  Artificial

<220>
<223>  Antisense nucleotide

<400>  1466
ggttactgta gcca                                                          14


<210>  1467
<211>  14
<212>  DNA
<213>  Artificial

<220>
<223>  Antisense nucleotide

<400>  1467
ggttactgta gcca                                                          14


<210>  1468
<211>  18
<212>  DNA
<213>  Artificial

<220>
<223>  Antisense nucleotide

<400>  1468
agttcttggc gcggaggt                                                      18


<210>  1469
<211>  18
<212>  DNA
<213>  Artificial

<220>
<223>  Antisense nucleotide

<400>  1469
aggtgaggag gtccgagt                                                      18


<210>  1470
<211>  16
<212>  DNA
<213>  Artificial

<220>
<223>  Antisense nucleotide

<400>  1470
tggactggat tatcag                                                        16

<210>  1471
<211>  18
<212>  DNA
<213>  Artificial

<220>
<223>  Antisense nucleotide

<400>  1471
gtggtggtga tgtgcccg                                                    18


<210>  1472
<211>  14
<212>  DNA
<213>  Artificial

<220>
<223>  Antisense nucleotide

<400>  1472
tgtcacgttc ttgg                                                        14


<210>  1473
<211>  14
<212>  DNA
<213>  Artificial

<220>
<223>  Antisense nucleotide

<400>  1473
ctcatctgtc acgt                                                        14


<210>  1474
<211>  18
<212>  DNA
<213>  Artificial

<220>
<223>  Antisense nucleotide

<400>  1474
cgaagccctc ggcgaacc                                                    18


<210>  1475
<211>  24
<212>  DNA
<213>  Artificial

<220>
<223>  Antisense nucleotide

<400>  1475
gcgtgttctg gctgtgcagt tcgg                                             24


<210>  1476
<211>  14
<212>  DNA
<213>  Artificial

<220>
<223>  Antisense nucleotide

<400>  1476
ctgccccgtt gacc                                                         14


<210>  1477
<211>  14
<212>  DNA
<213>  Artificial

<220>
<223>  Antisense nucleotide

<400>  1477
aggtttgcgt agac                                                         14


<210>  1478
<211>  14
<212>  DNA
<213>  Artificial

<220>
<223>  Antisense nucleotide

<400>  1478
ggttgaagtt gctg                                                         14


<210>  1479
<211>  14
<212>  DNA
<213>  Artificial

<220>
<223>  Antisense nucleotide

<400>  1479
ctgggttgaa gttg                                                         14


<210>  1480
<211>  20
<212>  DNA
<213>  Artificial

<220>
<223>  Antisense nucleotide

<400>  1480
tgctgcacgg gcatctgctg                                                   20


<210>  1481
<211>  27
<212>  DNA
<213>  Artificial

<220>
<223>  Antisense nucleotide

<400>  1481
ggcactgtct gaggctcctc cttcagg                                           27


<210>  1482
<211>  14
<212>  DNA
<213>  Artificial

<220>
<223>  Antisense nucleotide

<400>  1482
actccatgtc gatg                                                          14


<210>  1483
<211>  16
<212>  DNA
<213>  Artificial

<220>
<223>  Antisense nucleotide

<400>  1483
ctctccgcct tgatcc                                                        16


<210>  1484
<211>  16
<212>  DNA
<213>  Artificial

<220>
<223>  Antisense nucleotide

<400>  1484
gttcctcatg cgcttc                                                        16


<210>  1485
<211>  14
<212>  DNA
<213>  Artificial

<220>
<223>  Antisense nucleotide

<400>  1485
ctgagctttc aagg                                                          14


<210>  1486
<211>  26
<212>  DNA
<213>  Artificial

<220>
<223>  Antisense nucleotide

<400>  1486
gcgattctct ccagcttcct ttttcg                                             26


<210>  1487
<211>  30
<212>  DNA
<213>  Artificial

<220>
<223>  Antisense nucleotide

<400>  1487
   ctgagctttc aaggttttca ctttttcctc                                          30

<210>  1488
<211>  14
<212>  DNA
<213>  Artificial

<220>
<223>  Antisense nucleotide

<400>  1488
tccctgagca tgtt                                                          14


<210>  1489
<211>  16
<212>  DNA
<213>  Artificial

<220>
<223>  Antisense nucleotide

<400>  1489
tctgtttaag ctgtgc                                                        16


<210>  1490
<211>  18
<212>  DNA
<213>  Artificial

<220>
<223>  Antisense nucleotide

<400>  1490
ctttctgttt aagctgtg                                                      18


<210>  1491
<211>  16
<212>  DNA
<213>  Artificial

<220>
<223>  Antisense nucleotide

<400>  1491
ggttcatgac tttctg                                                        16


<210>  1492
<211>  14
<212>  DNA
<213>  Artificial

<220>
<223>  Antisense nucleotide

<400>  1492
cgtggttcat gact                                                          14


<210>  1493
<211>  16
<212>  DNA
<213>  Artificial

<220>
<223>  Antisense nucleotide

<400>  1493
actgttaacg tggttc                                                        16


<210>  1494
<211>  14
<212>  DNA
<213>  Artificial

<220>
<223>  Antisense nucleotide

<400>  1494
ccactgttaa cgtg                                                          14


<210>  1495
<211>  14
<212>  DNA
<213>  Artificial

<220>
<223>  Antisense nucleotide

<400>  1495
cccactgtta acgt                                                          14


<210>  1496
<211>  14
<212>  DNA
<213>  Artificial

<220>
<223>  Antisense nucleotide

<400>  1496
agcatgagtt ggca                                                          14


<210>  1497
<211>  14
<212>  DNA
<213>  Artificial

<220>
<223>  Antisense nucleotide

<400>  1497
gcgttagcat gagt                                                          14


<210>  1498
<211>  14
<212>  DNA
<213>  Artificial

<220>
<223>  Antisense nucleotide

<400>  1498
gtttgcaact gctg                                                          14


<210>  1499
<211>  18
<212>  DNA
<213>  Artificial

<220>
<223>  Antisense nucleotide

<400>  1499
caaaatgttt gcaactgc                                                    18


<210>  1500
<211>  18
<212>  DNA
<213>  Artificial

<220>
<223>  Antisense nucleotide

<400>  1500
tccattttag tgcacatc                                                    18


<210>  1501
<211>  18
<212>  DNA
<213>  Artificial

<220>
<223>  Antisense nucleotide

<400>  1501
ctgttccatt ttagtgca                                                    18


<210>  1502
<211>  14
<212>  DNA
<213>  Artificial

<220>
<223>  Antisense nucleotide

<400>  1502
gtgtatgagt cgtc                                                        14


<210>  1503
<211>  14
<212>  DNA
<213>  Artificial

<220>
<223>  Antisense nucleotide

<400>  1503
ctgtgtatga gtcg                                                        14


<210>  1504
<211>  14
<212>  DNA
<213>  Artificial

<220>
<223>  Antisense nucleotide

<400>  1504
cgtagctgtg tatg                                                        14

<210>  1505
<211>  14
<212>  DNA
<213>  Artificial

<220>
<223>  Antisense nucleotide

<400>  1505
tcgtgtagag agag                                                          14


<210>  1506
<211>  18
<212>  DNA
<213>  Artificial

<220>
<223>  Antisense nucleotide

<400>  1506
agtttgtagt cgtgtaga                                                      18


<210>  1507
<211>  16
<212>  DNA
<213>  Artificial

<220>
<223>  Antisense nucleotide

<400>  1507
gtttgtagtc gtgtag                                                        16


<210>  1508
<211>  14
<212>  DNA
<213>  Artificial

<220>
<223>  Antisense nucleotide

<400>  1508
agtttgtagt cgtg                                                          14


<210>  1509
<211>  14
<212>  DNA
<213>  Artificial

<220>
<223>  Antisense nucleotide

<400>  1509
ggagtttgta gtcg                                                          14


<210>  1510
<211>  16
<212>  DNA
<213>  Artificial

<220>
<223>  Antisense nucleotide

<400>  1510
tcaggagttt gtagtc                                                        16


<210>  1511
<211>  18
<212>  DNA
<213>  Artificial

<220>
<223>  Antisense nucleotide

<400>  1511
gtttcaggag tttgtagt                                                      18


<210>  1512
<211>  14
<212>  DNA
<213>  Artificial

<220>
<223>  Antisense nucleotide

<400>  1512
tcggtttcag gagt                                                          14


<210>  1513
<211>  14
<212>  DNA
<213>  Artificial

<220>
<223>  Antisense nucleotide

<400>  1513
ttgagactcc ggta                                                          14


<210>  1514
<211>  16
<212>  DNA
<213>  Artificial

<220>
<223>  Antisense nucleotide

<400>  1514
accagaaaag tagctg                                                        16


<210>  1515
<211>  14
<212>  DNA
<213>  Artificial

<220>
<223>  Antisense nucleotide

<400>  1515
cctgaccaga aaag                                                          14


<210>  1516
<211>  14
<212>  DNA
<213>  Artificial

<220>
<223>  Antisense nucleotide

<400>  1516
attcaggcgt tcca                                                         14


<210>  1517
<211>  16
<212>  DNA
<213>  Artificial

<220>
<223>  Antisense nucleotide

<400>  1517
ggtaaaagta ctgtcc                                                       16


<210>  1518
<211>  16
<212>  DNA
<213>  Artificial

<220>
<223>  Antisense nucleotide

<400>  1518
gggtaaaagt actgtc                                                       16


<210>  1519
<211>  18
<212>  DNA
<213>  Artificial

<220>
<223>  Antisense nucleotide

<400>  1519
gcacctccac cgctgcca                                                     18


<210>  1520
<211>  18
<212>  DNA
<213>  Artificial

<220>
<223>  Antisense nucleotide

<400>  1520
ctcctgctcc tcggtgac                                                     18


<210>  1521
<211>  14
<212>  DNA
<213>  Artificial

<220>
<223>  Antisense nucleotide

<400>  1521
gctttgacaa agcc                                                         14

<210>  1522
<211>  14
<212>  DNA
<213>  Artificial

<220>
<223>  Antisense nucleotide

<400>  1522
cttgtgcaga tcgt                                                         14


<210>  1523
<211>  16
<212>  DNA
<213>  Artificial

<220>
<223>  Antisense nucleotide

<400>  1523
tcatcttgtg cagatc                                                       16


<210>  1524
<211>  16
<212>  DNA
<213>  Artificial

<220>
<223>  Antisense nucleotide

<400>  1524
gttcatcttg tgcaga                                                       16


<210>  1525
<211>  14
<212>  DNA
<213>  Artificial

<220>
<223>  Antisense nucleotide

<400>  1525
cgtggttcat cttg                                                         14


<210>  1526
<211>  14
<212>  DNA
<213>  Artificial

<220>
<223>  Antisense nucleotide

<400>  1526
tcacgtggtt catc                                                         14


<210>  1527
<211>  14
<212>  DNA
<213>  Artificial

<220>
<223>  Antisense nucleotide

<400>  1527
ggttggtgta aacg                                                         14


<210>  1528
<211>  20
<212>  DNA
<213>  Artificial

<220>
<223>  Antisense nucleotide

<400>  1528
tacgagctcc cggtcccgac                                                   20


<210>  1529
<211>  14
<212>  DNA
<213>  Artificial

<220>
<223>  Antisense nucleotide

<400>  1529
tagctgatgg tggt                                                         14


<210>  1530
<211>  14
<212>  DNA
<213>  Artificial

<220>
<223>  Antisense nucleotide

<400>  1530
tccttgaagg tgga                                                         14


<210>  1531
<211>  16
<212>  DNA
<213>  Artificial

<220>
<223>  Antisense nucleotide

<400>  1531
tcttccatgt tgatgg                                                       16


<210>  1532
<211>  14
<212>  DNA
<213>  Artificial

<220>
<223>  Antisense nucleotide

<400>  1532
ctttgatgcg ctct                                                         14


<210>  1533
<211>  14
<212>  DNA
<213>  Artificial

<220>
<223>  Antisense nucleotide

<400>  1533
ctccactttg atgc                                                          14


<210>  1534
<211>  30
<212>  DNA
<213>  Artificial

<220>
<223>  Antisense nucleotide

<400>  1534
gctccagctt ccgcttccgg cacttggtgg                                         30


<210>  1535
<211>  30
<212>  DNA
<213>  Artificial

<220>
<223>  Antisense nucleotide

<400>  1535
ggccttgagc gtcttcacct tgtcctccag                                         30


<210>  1536
<211>  16
<212>  DNA
<213>  Artificial

<220>
<223>  Antisense nucleotide

<400>  1536
tgaccttctg tttgag                                                        16


<210>  1537
<211>  16
<212>  DNA
<213>  Artificial

<220>
<223>  Antisense nucleotide

<400>  1537
catgaccttc tgtttg                                                        16


<210>  1538
<211>  14
<212>  DNA
<213>  Artificial

<220>
<223>  Antisense nucleotide

<400>  1538
gtcatgacct tctg                                                          14

<210>  1539
<211>  14
<212>  DNA
<213>  Artificial

<220>
<223>  Antisense nucleotide

<400>  1539
cgagaacatc atcg                                                          14


<210>  1540
<211>  14
<212>  DNA
<213>  Artificial

<220>
<223>  Antisense nucleotide

<400>  1540
gtagtctgcg ttga                                                          14


<210>  1541
<211>  20
<212>  DNA
<213>  Artificial

<220>
<223>  Antisense nucleotide

<400>  1541
gctgcagcgg gaggatgacg                                                    20


<210>  1542
<211>  16
<212>  DNA
<213>  Artificial

<220>
<223>  Antisense nucleotide

<400>  1542
agtaagagag gctatc                                                        16


<210>  1543
<211>  14
<212>  DNA
<213>  Artificial

<220>
<223>  Antisense nucleotide

<400>  1543
gtagtaagag aggc                                                          14


<210>  1544
<211>  14
<212>  DNA
<213>  Artificial

<220>
<223>  Antisense nucleotide

<400>  1544
ggtagtaaga gagg                                                         14


<210>  1545
<211>  16
<212>  DNA
<213>  Artificial

<220>
<223>  Antisense nucleotide

<400>  1545
gtgagtggta gtaaga                                                       16


<210>  1546
<211>  18
<212>  DNA
<213>  Artificial

<220>
<223>  Antisense nucleotide

<400>  1546
gtccgtgcag aagtcctg                                                     18


<210>  1547
<211>  16
<212>  DNA
<213>  Artificial

<220>
<223>  Antisense nucleotide

<400>  1547
gaatgaagtt ggcact                                                       16


<210>  1548
<211>  14
<212>  DNA
<213>  Artificial

<220>
<223>  Antisense nucleotide

<400>  1548
ggaatgaagt tggc                                                         14


<210>  1549
<211>  14
<212>  DNA
<213>  Artificial

<220>
<223>  Antisense nucleotide

<400>  1549
gggaatgaag ttgg                                                         14


<210>  1550
<211>  30
<212>  DNA
<213>  Artificial

<220>
<223>  Antisense nucleotide

<400>  1550
gctgcaccag ccactgcagg tccggactgg                                        30


<210>  1551
<211>  16
<212>  DNA
<213>  Artificial

<220>
<223>  Antisense nucleotide

<400>  1551
tcatggtctt cacaac                                                       16


<210>  1552
<211>  30
<212>  DNA
<213>  Artificial

<220>
<223>  Antisense nucleotide

<400>  1552
caatgctctg cgctcggcct cctgtcatgg                                        30


<210>  1553
<211>  14
<212>  DNA
<213>  Artificial

<220>
<223>  Antisense nucleotide

<400>  1553
ctagagttcc tcac                                                         14


<210>  1554
<211>  14
<212>  DNA
<213>  Artificial

<220>
<223>  Antisense nucleotide

<400>  1554
gagtacgcta gagt                                                         14


<210>  1555
<211>  14
<212>  DNA
<213>  Artificial

<220>
<223>  Antisense nucleotide

<400>  1555
gaagagtacg ctag                                                         14

<210>  1556
<211>  28
<212>  DNA
<213>  Artificial

<220>
<223>  Antisense nucleotide

<400>  1556
ctgcttccca cccagccccc acattccc                                          28


<210>  1557
<211>  20
<212>  DNA
<213>  Artificial

<220>
<223>  Antisense nucleotide

<400>  1557
ttcatcctct gtactgggct                                                   20


<210>  1558
<211>  14
<212>  DNA
<213>  Artificial

<220>
<223>  Antisense nucleotide

<400>  1558
gttacggatg tgca                                                         14


<210>  1559
<211>  14
<212>  DNA
<213>  Artificial

<220>
<223>  Antisense nucleotide

<400>  1559
cagttacgga tgtg                                                         14


<210>  1560
<211>  14
<212>  DNA
<213>  Artificial

<220>
<223>  Antisense nucleotide

<400>  1560
ccagttacgg atgt                                                         14


<210>  1561
<211>  14
<212>  DNA
<213>  Artificial

<220>
<223>  Antisense nucleotide

<400>  1561
agagtctgag ttgg                                                         14


<210>  1562
<211>  14
<212>  DNA
<213>  Artificial

<220>
<223>  Antisense nucleotide

<400>  1562
gtgagactca gagt                                                         14


<210>  1563
<211>  14
<212>  DNA
<213>  Artificial

<220>
<223>  Antisense nucleotide

<400>  1563
tcttagggtg agac                                                         14


<210>  1564
<211>  16
<212>  DNA
<213>  Artificial

<220>
<223>  Antisense nucleotide

<400>  1564
gagagtactt cttagg                                                       16


<210>  1565
<211>  18
<212>  DNA
<213>  Artificial

<220>
<223>  Antisense nucleotide

<400>  1565
ggaagaaact atgagagt                                                     18


<210>  1566
<211>  18
<212>  DNA
<213>  Artificial

<220>
<223>  Antisense nucleotide

<400>  1566
cttagggaag aaactatg                                                     18


<210>  1567
<211>  16
<212>  DNA
<213>  Artificial

<220>
<223>  Antisense nucleotide

<400>  1567
cggtaagaaa cttagg                                                        16


<210>  1568
<211>  14
<212>  DNA
<213>  Artificial

<220>
<223>  Antisense nucleotide

<400>  1568
agcatgcggt aaga                                                          14


<210>  1569
<211>  14
<212>  DNA
<213>  Artificial

<220>
<223>  Antisense nucleotide

<400>  1569
gtctgaaagc atgc                                                          14


<210>  1570
<211>  16
<212>  DNA
<213>  Artificial

<220>
<223>  Antisense nucleotide

<400>  1570
agaacaaaga agagcc                                                        16


<210>  1571
<211>  18
<212>  DNA
<213>  Artificial

<220>
<223>  Antisense nucleotide

<400>  1571
caagagaaca aagaagag                                                      18


<210>  1572
<211>  16
<212>  DNA
<213>  Artificial

<220>
<223>  Antisense nucleotide

<400>  1572
cagcaagaga acaaag                                                        16

<210>  1573
<211>  14
<212>  DNA
<213>  Artificial

<220>
<223>  Antisense nucleotide

<400>  1573
tcctcagcaa gaga                                                          14


<210>  1574
<211>  14
<212>  DNA
<213>  Artificial

<220>
<223>  Antisense nucleotide

<400>  1574
aggtgtgact tgca                                                          14


<210>  1575
<211>  16
<212>  DNA
<213>  Artificial

<220>
<223>  Antisense nucleotide

<400>  1575
gaataggtgt gacttg                                                        16


<210>  1576
<211>  16
<212>  DNA
<213>  Artificial

<220>
<223>  Antisense nucleotide

<400>  1576
cagaataggt gtgact                                                        16


<210>  1577
<211>  14
<212>  DNA
<213>  Artificial

<220>
<223>  Antisense nucleotide

<400>  1577
gcagaatagg tgtg                                                          14


<210>  1578
<211>  16
<212>  DNA
<213>  Artificial

<220>
<223>  Antisense nucleotide

<400>  1578
cagttgcaga ataggt                                                    16


<210>  1579
<211>  16
<212>  DNA
<213>  Artificial

<220>
<223>  Antisense nucleotide

<400>  1579
gaaaccattt ctgacc                                                    16


<210>  1580
<211>  18
<212>  DNA
<213>  Artificial

<220>
<223>  Antisense nucleotide

<400>  1580
tgtgaaacca tttctgac                                                  18


<210>  1581
<211>  18
<212>  DNA
<213>  Artificial

<220>
<223>  Antisense nucleotide

<400>  1581
cactgtgaaa ccatttct                                                  18


<210>  1582
<211>  14
<212>  DNA
<213>  Artificial

<220>
<223>  Antisense nucleotide

<400>  1582
ccactgtgaa acca                                                      14


<210>  1583
<211>  30
<212>  DNA
<213>  Artificial

<220>
<223>  Antisense nucleotide

<400>  1583
agaactggct cctgcagctt ccctgcttcc                                     30


<210>  1584
<211>  15
<212>  DNA
<213>  Artificial

<220>
<223>  Antisense nucleotide

<400>  1584
cacctccatt caccc                                                        15


<210>  1585
<211>  16
<212>  DNA
<213>  Artificial

<220>
<223>  Antisense nucleotide

<400>  1585
cagtaaaagt gtctgc                                                       16


<210>  1586
<211>  18
<212>  DNA
<213>  Artificial

<220>
<223>  Antisense nucleotide

<400>  1586
cgacattcag taaaagtg                                                     18


<210>  1587
<211>  14
<212>  DNA
<213>  Artificial

<220>
<223>  Antisense nucleotide

<400>  1587
gaccgacatt cagt                                                         14


<210>  1588
<211>  18
<212>  DNA
<213>  Artificial

<220>
<223>  Antisense nucleotide

<400>  1588
cttctggaga taactaga                                                     18


<210>  1589
<211>  18
<212>  DNA
<213>  Artificial

<220>
<223>  Antisense nucleotide

<400>  1589
catcttattc ctttccct                                                     18

<210>  1590
<211>  16
<212>  DNA
<213>  Artificial

<220>
<223>  Antisense nucleotide

<400>  1590
cagccatctt attcct                                                    16


<210>  1591
<211>  16
<212>  DNA
<213>  Artificial

<220>
<223>  Antisense nucleotide

<400>  1591
tgcagccatc ttattc                                                    16


<210>  1592
<211>  15
<212>  DNA
<213>  Artificial

<220>
<223>  Antisense nucleotide

<400>  1592
gagtgtatca gtcag                                                     15


<210>  1593
<211>  14
<212>  DNA
<213>  Artificial

<220>
<223>  Antisense nucleotide

<400>  1593
ggagtgtatc agtc                                                      14


<210>  1594
<211>  16
<212>  DNA
<213>  Artificial

<220>
<223>  Antisense nucleotide

<400>  1594
cttggagtgt atcagt                                                    16


<210>  1595
<211>  14
<212>  DNA
<213>  Artificial

<220>
<223>  Antisense nucleotide

<400>  1595
acagagtacc tacc                                                         14


<210>  1596
<211>  16
<212>  DNA
<213>  Artificial

<220>
<223>  Antisense nucleotide

<400>  1596
ccaactttcc cttaag                                                       16


<210>  1597
<211>  16
<212>  DNA
<213>  Artificial

<220>
<223>  Antisense nucleotide

<400>  1597
ccttatgctc aatctc                                                       16


<210>  1598
<211>  14
<212>  DNA
<213>  Artificial

<220>
<223>  Antisense nucleotide

<400>  1598
gtcttactca aggg                                                         14


<210>  1599
<211>  16
<212>  DNA
<213>  Artificial

<220>
<223>  Antisense nucleotide

<400>  1599
acagtcttac tcaagg                                                       16


<210>  1600
<211>  18
<212>  DNA
<213>  Artificial

<220>
<223>  Antisense nucleotide

<400>  1600
cataagacac agtcttac                                                     18


<210>  1601
<211>  18
<212>  DNA
<213>  Artificial

<220>
<223>  Antisense nucleotide

<400>  1601
gaaagcataa gacacagt                                                    18


<210>  1602
<211>  16
<212>  DNA
<213>  Artificial

<220>
<223>  Antisense nucleotide

<400>  1602
ggaaagcata agacac                                                      16


<210>  1603
<211>  16
<212>  DNA
<213>  Artificial

<220>
<223>  Antisense nucleotide

<400>  1603
agggataaag gaaagc                                                      16


<210>  1604
<211>  14
<212>  DNA
<213>  Artificial

<220>
<223>  Antisense nucleotide

<400>  1604
cctgtataca gagg                                                        14


<210>  1605
<211>  16
<212>  DNA
<213>  Artificial

<220>
<223>  Antisense nucleotide

<400>  1605
tgtctcctgt atacag                                                      16


<210>  1606
<211>  16
<212>  DNA
<213>  Artificial

<220>
<223>  Antisense nucleotide

<400>  1606
catcttctag ttggtc                                                      16

<210>  1607
<211>  16
<212>  DNA
<213>  Artificial

<220>
<223>  Antisense nucleotide

<400>  1607
ctcatcttct agttgg                                                        16


<210>  1608
<211>  18
<212>  DNA
<213>  Artificial

<220>
<223>  Antisense nucleotide

<400>  1608
cttctcatct tctagttg                                                      18


<210>  1609
<211>  16
<212>  DNA
<213>  Artificial

<220>
<223>  Antisense nucleotide

<400>  1609
caaagcagac ttctca                                                        16


<210>  1610
<211>  14
<212>  DNA
<213>  Artificial

<220>
<223>  Antisense nucleotide

<400>  1610
ctgcaaagca gact                                                          14


<210>  1611
<211>  18
<212>  DNA
<213>  Artificial

<220>
<223>  Antisense nucleotide

<400>  1611
ctagtttttc cttctcct                                                      18


<210>  1612
<211>  18
<212>  DNA
<213>  Artificial

<220>
<223>  Antisense nucleotide

<400>  1612
tctagttttt ccttctcc                                                    18


<210>  1613
<211>  16
<212>  DNA
<213>  Artificial

<220>
<223>  Antisense nucleotide

<400>  1613
caggatgaac tctagt                                                      16


<210>  1614
<211>  14
<212>  DNA
<213>  Artificial

<220>
<223>  Antisense nucleotide

<400>  1614
tcgtagaagg tcgt                                                        14


<210>  1615
<211>  14
<212>  DNA
<213>  Artificial

<220>
<223>  Antisense nucleotide

<400>  1615
agggttactg tagc                                                        14


<210>  1616
<211>  14
<212>  DNA
<213>  Artificial

<220>
<223>  Antisense nucleotide

<400>  1616
gtagtggtga tgtg                                                        14


<210>  1617
<211>  14
<212>  DNA
<213>  Artificial

<220>
<223>  Antisense nucleotide

<400>  1617
cgtcgtagaa ggtc                                                        14


<210>  1618
<211>  14
<212>  DNA
<213>  Artificial

<220>
<223>  Antisense nucleotide

<400>  1618
tttcgtgcac atcc                                                          14

<210>  1619
<211>  18
<212>  DNA
<213>  Artificial

<220>
<223>  Antisense nucleotide

<400>  1619
agtttgtagt cgtgaaga                                                      18

<210>  1620
<211>  14
<212>  DNA
<213>  Artificial

<220>
<223>  Antisense nucleotide

<400>  1620
cgagaacatc atgg                                                          14

<210>  1621
<211>  14
<212>  DNA
<213>  Artificial

<220>
<223>  Antisense nucleotide

<400>  1621
gtagtaggaa aggc                                                          14

<210>  1622
<211>  14
<212>  DNA
<213>  Artificial

<220>
<223>  Antisense nucleotide

<400>  1622
ggtagtagga aagg                                                          14

<210>  1623
<211>  14
<212>  DNA
<213>  Artificial

<220>
<223>  Antisense nucleotide

<400>  1623
ggaatggtag tagg                                                          14

<210>  1624
<211>  15
<212>  DNA
<213>  Artificial

<220>
<223>  Antisense nucleotide

<400>  1624
ggtcattgag aagag                                                        15


<210>  1625
<211>  16
<212>  DNA
<213>  Artificial

<220>
<223>  Antisense nucleotide

<400>  1625
gctaatgttc ttgacc                                                       16


<210>  1626
<211>  14
<212>  DNA
<213>  Artificial

<220>
<223>  Antisense nucleotide

<400>  1626
gccaaggtcc tcat                                                         14


<210>  1627
<211>  14
<212>  DNA
<213>  Artificial

<220>
<223>  Antisense nucleotide

<400>  1627
ggagtctatc tcca                                                         14


<210>  1628
<211>  16
<212>  DNA
<213>  Artificial

<220>
<223>  Antisense nucleotide

<400>  1628
ccaaagaatc ctgact                                                       16


<210>  1629
<211>  14
<212>  DNA
<213>  Artificial

<220>
<223>  Antisense nucleotide

<400>  1629
cacatgctta gtgg                                                          14


<210>  1630
<211>  14
<212>  DNA
<213>  Artificial

<220>
<223>  Antisense nucleotide

<400>  1630
ctcgtaaatg accg                                                          14


<210>  1631
<211>  18
<212>  DNA
<213>  Artificial

<220>
<223>  Antisense nucleotide

<400>  1631
aggaatctcg taaatgac                                                      18


<210>  1632
<211>  14
<212>  DNA
<213>  Artificial

<220>
<223>  Antisense nucleotide

<400>  1632
cagcagcgat tcat                                                          14


<210>  1633
<211>  16
<212>  DNA
<213>  Artificial

<220>
<223>  Antisense nucleotide

<400>  1633
ggagatcatc aaagga                                                        16


<210>  1634
<211>  14
<212>  DNA
<213>  Artificial

<220>
<223>  Antisense nucleotide

<400>  1634
ctcagcaatg gtca                                                          14


<210>  1635
<211>  14
<212>  DNA
<213>  Artificial

<220>
<223>  Antisense nucleotide

<400>  1635
gatctcgaac acct                                                          14


<210>  1636
<211>  18
<212>  DNA
<213>  Artificial

<220>
<223>  Antisense nucleotide

<400>  1636
cacaatctcg atctttct                                                      18


<210>  1637
<211>  18
<212>  DNA
<213>  Artificial

<220>
<223>  Antisense nucleotide

<400>  1637
ccttcttaaa gattggct                                                      18


<210>  1638
<211>  14
<212>  DNA
<213>  Artificial

<220>
<223>  Antisense nucleotide

<400>  1638
cacataccaa ctgg                                                          14


<210>  1639
<211>  14
<212>  DNA
<213>  Artificial

<220>
<223>  Antisense nucleotide

<400>  1639
agcttgatgt gagg                                                          14


<210>  1640
<211>  18
<212>  DNA
<213>  Artificial

<220>
<223>  Antisense nucleotide

<400>  1640
gaagttgtag cttgatgt                                                      18

<210>  1641
<211>  16
<212>  DNA
<213>  Artificial

<220>
<223>  Antisense nucleotide

<400>  1641
gcttgaagtt gtagct                                                        16


<210>  1642
<211>  16
<212>  DNA
<213>  Artificial

<220>
<223>  Antisense nucleotide

<400>  1642
ctgcttgaag ttgtag                                                        16


<210>  1643
<211>  14
<212>  DNA
<213>  Artificial

<220>
<223>  Antisense nucleotide

<400>  1643
gacacaactc ctct                                                          14


<210>  1644
<211>  18
<212>  DNA
<213>  Artificial

<220>
<223>  Antisense nucleotide

<400>  1644
tcctttgata gacacaac                                                      18


<210>  1645
<211>  16
<212>  DNA
<213>  Artificial

<220>
<223>  Antisense nucleotide

<400>  1645
ctcgtttgat agacac                                                        16


<210>  1646
<211>  14
<212>  DNA
<213>  Artificial

<220>
<223>  Antisense nucleotide

<400>  1646
ggttagcaca cact                                                          14


<210>  1647
<211>  14
<212>  DNA
<213>  Artificial

<220>
<223>  Antisense nucleotide

<400>  1647
ggtaacggtt agca                                                          14


<210>  1648
<211>  18
<212>  DNA
<213>  Artificial

<220>
<223>  Antisense nucleotide

<400>  1648
cgtaacacat ttagaagc                                                      18


<210>  1649
<211>  14
<212>  DNA
<213>  Artificial

<220>
<223>  Antisense nucleotide

<400>  1649
ctcatccgta acac                                                          14


<210>  1650
<211>  18
<212>  DNA
<213>  Artificial

<220>
<223>  Antisense nucleotide

<400>  1650
ccggtaagta ttgtagtt                                                      18


<210>  1651
<211>  16
<212>  DNA
<213>  Artificial

<220>
<223>  Antisense nucleotide

<400>  1651
ggtgtatttc cttgac                                                        16


<210>  1652
<211>  16
<212>  DNA
<213>  Artificial

<220>
<223>  Antisense nucleotide

<400>  1652
acataccaac tggtgt                                                       16


<210>  1653
<211>  14
<212>  DNA
<213>  Artificial

<220>
<223>  Antisense nucleotide

<400>  1653
gtccctatac gaac                                                         14


<210>  1654
<211>  14
<212>  DNA
<213>  Artificial

<220>
<223>  Antisense nucleotide

<400>  1654
ttcatgtctg tgcc                                                         14


<210>  1655
<211>  14
<212>  DNA
<213>  Artificial

<220>
<223>  Antisense nucleotide

<400>  1655
gtaggtgagt tcca                                                         14


<210>  1656
<211>  14
<212>  DNA
<213>  Artificial

<220>
<223>  Antisense nucleotide

<400>  1656
gttgtgagcg atga                                                         14


<210>  1657
<211>  18
<212>  DNA
<213>  Artificial

<220>
<223>  Antisense nucleotide

<400>  1657
catagttgtc ctcaaaga                                                     18

<210>  1658
<211>  14
<212>  DNA
<213>  Artificial

<220>
<223>  Antisense nucleotide

<400>  1658
ggcatagttg tcct                                                          14


<210>  1659
<211>  14
<212>  DNA
<213>  Artificial

<220>
<223>  Antisense nucleotide

<400>  1659
cattgtctag cacg                                                          14


<210>  1660
<211>  14
<212>  DNA
<213>  Artificial

<220>
<223>  Antisense nucleotide

<400>  1660
ctccattgtc tagc                                                          14


<210>  1661
<211>  14
<212>  DNA
<213>  Artificial

<220>
<223>  Antisense nucleotide

<400>  1661
gtattgttca gcgg                                                          14


<210>  1662
<211>  16
<212>  DNA
<213>  Artificial

<220>
<223>  Antisense nucleotide

<400>  1662
tcaagatctc tgtgag                                                        16


<210>  1663
<211>  16
<212>  DNA
<213>  Artificial

<220>
<223>  Antisense nucleotide

<400>  1663
cacaaaatcg tgtcct                                                        16


<210>  1664
<211>  16
<212>  DNA
<213>  Artificial

<220>
<223>  Antisense nucleotide

<400>  1664
tccttccaca aaatcg                                                        16


<210>  1665
<211>  14
<212>  DNA
<213>  Artificial

<220>
<223>  Antisense nucleotide

<400>  1665
gtggaagatg tcct                                                          14


<210>  1666
<211>  16
<212>  DNA
<213>  Artificial

<220>
<223>  Antisense nucleotide

<400>  1666
tcttgtggaa gatgtc                                                        16


<210>  1667
<211>  16
<212>  DNA
<213>  Artificial

<220>
<223>  Antisense nucleotide

<400>  1667
tctatcagtg tgagag                                                        16


<210>  1668
<211>  14
<212>  DNA
<213>  Artificial

<220>
<223>  Antisense nucleotide

<400>  1668
ggttggtgtc tatc                                                          14


<210>  1669
<211>  14
<212>  DNA
<213>  Artificial

<220>
<223>  Antisense nucleotide

<400>  1669
acatcggaga acag                                                          14


<210>  1670
<211>  14
<212>  DNA
<213>  Artificial

<220>
<223>  Antisense nucleotide

<400>  1670
ccttacacat cgga                                                          14


<210>  1671
<211>  16
<212>  DNA
<213>  Artificial

<220>
<223>  Antisense nucleotide

<400>  1671
acaatcctca gaactc                                                        16


<210>  1672
<211>  14
<212>  DNA
<213>  Artificial

<220>
<223>  Antisense nucleotide

<400>  1672
gctctgacaa tcct                                                          14


<210>  1673
<211>  14
<212>  DNA
<213>  Artificial

<220>
<223>  Antisense nucleotide

<400>  1673
tggttgaagt ggag                                                          14


<210>  1674
<211>  14
<212>  DNA
<213>  Artificial

<220>
<223>  Antisense nucleotide

<400>  1674
ctgtggttga agtg                                                          14

<210>  1675
<211>  14
<212>  DNA
<213>  Artificial

<220>
<223>  Antisense nucleotide

<400>  1675
gttgtaggtg acca                                                          14


<210>  1676
<211>  14
<212>  DNA
<213>  Artificial

<220>
<223>  Antisense nucleotide

<400>  1676
ctgtgttgta ggtg                                                          14


<210>  1677
<211>  14
<212>  DNA
<213>  Artificial

<220>
<223>  Antisense nucleotide

<400>  1677
gactcaaacg tgtc                                                          14


<210>  1678
<211>  14
<212>  DNA
<213>  Artificial

<220>
<223>  Antisense nucleotide

<400>  1678
catggactca aacg                                                          14


<210>  1679
<211>  14
<212>  DNA
<213>  Artificial

<220>
<223>  Antisense nucleotide

<400>  1679
cgaatgtata ccgg                                                          14


<210>  1680
<211>  14
<212>  DNA
<213>  Artificial

<220>
<223>  Antisense nucleotide

<400>  1680
ccgaatgtat accg                                                          14


<210>  1681
<211>  14
<212>  DNA
<213>  Artificial

<220>
<223>  Antisense nucleotide

<400>  1681
gccgaatgta tacc                                                          14


<210>  1682
<211>  14
<212>  DNA
<213>  Artificial

<220>
<223>  Antisense nucleotide

<400>  1682
gtagttgtag ggac                                                          14


<210>  1683
<211>  18
<212>  DNA
<213>  Artificial

<220>
<223>  Antisense nucleotide

<400>  1683
tagaaaggta gttgtagg                                                      18


<210>  1684
<211>  18
<212>  DNA
<213>  Artificial

<220>
<223>  Antisense nucleotide

<400>  1684
gtagaaaggt agttgtag                                                      18


<210>  1685
<211>  16
<212>  DNA
<213>  Artificial

<220>
<223>  Antisense nucleotide

<400>  1685
cgtagaaagg tagttg                                                        16


<210>  1686
<211>  14
<212>  DNA
<213>  Artificial

<220>
<223>  Antisense nucleotide

<400>  1686
ccgtagaaag gtag                                                          14


<210>  1687
<211>  14
<212>  DNA
<213>  Artificial

<220>
<223>  Antisense nucleotide

<400>  1687
gaccatagca cact                                                          14


<210>  1688
<211>  14
<212>  DNA
<213>  Artificial

<220>
<223>  Antisense nucleotide

<400>  1688
ggatattggc actg                                                          14


<210>  1689
<211>  14
<212>  DNA
<213>  Artificial

<220>
<223>  Antisense nucleotide

<400>  1689
cctggatatt ggca                                                          14


<210>  1690
<211>  14
<212>  DNA
<213>  Artificial

<220>
<223>  Antisense nucleotide

<400>  1690
gctcccaaag atct                                                          14


<210>  1691
<211>  14
<212>  DNA
<213>  Artificial

<220>
<223>  Antisense nucleotide

<400>  1691
cccatcaaag ctct                                                          14

<210>  1692
<211>  14
<212>  DNA
<213>  Artificial

<220>
<223>  Antisense nucleotide

<400>  1692
caaacacttg gagc                                                          14


<210>  1693
<211>  16
<212>  DNA
<213>  Artificial

<220>
<223>  Antisense nucleotide

<400>  1693
gtctcaaaca cttgga                                                        16


<210>  1694
<211>  16
<212>  DNA
<213>  Artificial

<220>
<223>  Antisense nucleotide

<400>  1694
gagtctcaaa cacttg                                                        16


<210>  1695
<211>  16
<212>  DNA
<213>  Artificial

<220>
<223>  Antisense nucleotide

<400>  1695
gtaacctgtg atctct                                                        16


<210>  1696
<211>  14
<212>  DNA
<213>  Artificial

<220>
<223>  Antisense nucleotide

<400>  1696
ggtaacctgt gatc                                                          14


<210>  1697
<211>  16
<212>  DNA
<213>  Artificial

<220>
<223>  Antisense nucleotide

<400>  1697
gtataggtaa cctgtg                                                        16


<210>  1698
<211>  18
<212>  DNA
<213>  Artificial

<220>
<223>  Antisense nucleotide

<400>  1698
tgagatgtat aggtaacc                                                      18


<210>  1699
<211>  16
<212>  DNA
<213>  Artificial

<220>
<223>  Antisense nucleotide

<400>  1699
tgctgagatg tatagg                                                        16


<210>  1700
<211>  14
<212>  DNA
<213>  Artificial

<220>
<223>  Antisense nucleotide

<400>  1700
ccatgctgag atgt                                                          14


<210>  1701
<211>  14
<212>  DNA
<213>  Artificial

<220>
<223>  Antisense nucleotide

<400>  1701
ggattacttg cagg                                                          14


<210>  1702
<211>  16
<212>  DNA
<213>  Artificial

<220>
<223>  Antisense nucleotide

<400>  1702
tgttatggtg gatgag                                                        16


<210>  1703
<211>  14
<212>  DNA
<213>  Artificial

<220>
<223>  Antisense nucleotide

<400>  1703
ggtgttatgg tgga                                                          14


<210>  1704
<211>  14
<212>  DNA
<213>  Artificial

<220>
<223>  Antisense nucleotide

<400>  1704
gcagttgaca cact                                                          14


<210>  1705
<211>  14
<212>  DNA
<213>  Artificial

<220>
<223>  Antisense nucleotide

<400>  1705
agtactcggc attc                                                          14


<210>  1706
<211>  16
<212>  DNA
<213>  Artificial

<220>
<223>  Antisense nucleotide

<400>  1706
cattcacata ctccct                                                        16


<210>  1707
<211>  16
<212>  DNA
<213>  Artificial

<220>
<223>  Antisense nucleotide

<400>  1707
tccaaaacag gtcact                                                        16


<210>  1708
<211>  14
<212>  DNA
<213>  Artificial

<220>
<223>  Antisense nucleotide

<400>  1708
ggtccttata gtgg                                                          14

<210>  1709
<211>  14
<212>  DNA
<213>  Artificial

<220>
<223>  Antisense nucleotide

<400>  1709
cagaatgcca acca                                                          14


<210>  1710
<211>  14
<212>  DNA
<213>  Artificial

<220>
<223>  Antisense nucleotide

<400>  1710
acgagaatgc caac                                                          14


<210>  1711
<211>  14
<212>  DNA
<213>  Artificial

<220>
<223>  Antisense nucleotide

<400>  1711
gatcccaaag acca                                                          14


<210>  1712
<211>  14
<212>  DNA
<213>  Artificial

<220>
<223>  Antisense nucleotide

<400>  1712
tcgcttgatg agga                                                          14


<210>  1713
<211>  14
<212>  DNA
<213>  Artificial

<220>
<223>  Antisense nucleotide

<400>  1713
catcgtgtac ttcc                                                          14


<210>  1714
<211>  14
<212>  DNA
<213>  Artificial

<220>
<223>  Antisense nucleotide

<400>  1714
gcatcgtgta cttc                                                          14


<210>  1715
<211>  14
<212>  DNA
<213>  Artificial

<220>
<223>  Antisense nucleotide

<400>  1715
actgtgccaa aagc                                                          14


<210>  1716
<211>  14
<212>  DNA
<213>  Artificial

<220>
<223>  Antisense nucleotide

<400>  1716
cttgtagact gtgc                                                          14


<210>  1717
<211>  14
<212>  DNA
<213>  Artificial

<220>
<223>  Antisense nucleotide

<400>  1717
cccttgtaga ctgt                                                          14


<210>  1718
<211>  16
<212>  DNA
<213>  Artificial

<220>
<223>  Antisense nucleotide

<400>  1718
tcaacacttt gatggc                                                        16


<210>  1719
<211>  14
<212>  DNA
<213>  Artificial

<220>
<223>  Antisense nucleotide

<400>  1719
ccctcaacac tttg                                                          14


<210>  1720
<211>  16
<212>  DNA
<213>  Artificial

<220>
<223>  Antisense nucleotide

<400>  1720
gtgttttccc tcaaca                                                        16


<210>  1721
<211>  16
<212>  DNA
<213>  Artificial

<220>
<223>  Antisense nucleotide

<400>  1721
gtatgcttcg tctaag                                                        16


<210>  1722
<211>  14
<212>  DNA
<213>  Artificial

<220>
<223>  Antisense nucleotide

<400>  1722
cgtatgcttc gtct                                                          14


<210>  1723
<211>  14
<212>  DNA
<213>  Artificial

<220>
<223>  Antisense nucleotide

<400>  1723
ccatcacgta tgct                                                          14


<210>  1724
<211>  14
<212>  DNA
<213>  Artificial

<220>
<223>  Antisense nucleotide

<400>  1724
gcataagctg tgtc                                                          14


<210>  1725
<211>  14
<212>  DNA
<213>  Artificial

<220>
<223>  Antisense nucleotide

<400>  1725
catggtctaa gagg                                                          14

<210>  1726
<211>  16
<212>  DNA
<213>  Artificial

<220>
<223>  Antisense nucleotide

<400>  1726
caatctgcat acacca                                                    16


<210>  1727
<211>  14
<212>  DNA
<213>  Artificial

<220>
<223>  Antisense nucleotide

<400>  1727
ggcaatctgc atac                                                      14


<210>  1728
<211>  14
<212>  DNA
<213>  Artificial

<220>
<223>  Antisense nucleotide

<400>  1728
ctgtctcgtc aatg                                                      14


<210>  1729
<211>  16
<212>  DNA
<213>  Artificial

<220>
<223>  Antisense nucleotide

<400>  1729
cataactcca cacatc                                                    16


<210>  1730
<211>  16
<212>  DNA
<213>  Artificial

<220>
<223>  Antisense nucleotide

<400>  1730
agtcacacca taactc                                                    16


<210>  1731
<211>  16
<212>  DNA
<213>  Artificial

<220>
<223>  Antisense nucleotide

<400>  1731
acagtcacac cataac                                                    16

<210>  1732
<211>  14
<212>  DNA
<213>  Artificial

<220>
<223>  Antisense nucleotide

<400>  1732
ccccaaaagt catc                                                      14

<210>  1733
<211>  14
<212>  DNA
<213>  Artificial

<220>
<223>  Antisense nucleotide

<400>  1733
tcgtaaggtt tggc                                                      14

<210>  1734
<211>  14
<212>  DNA
<213>  Artificial

<220>
<223>  Antisense nucleotide

<400>  1734
gatcccatcg taag                                                      14

<210>  1735
<211>  14
<212>  DNA
<213>  Artificial

<220>
<223>  Antisense nucleotide

<400>  1735
caatggtgca gatg                                                      14

<210>  1736
<211>  14
<212>  DNA
<213>  Artificial

<220>
<223>  Antisense nucleotide

<400>  1736
gacatcaatg gtgc                                                      14

<210>  1737
<211>  16
<212>  DNA
<213>  Artificial

<220>
<223>  Antisense nucleotide

<400>  1737
gtagacatca atggtg                                                    16


<210>  1738
<211>  18
<212>  DNA
<213>  Artificial

<220>
<223>  Antisense nucleotide

<400>  1738
catgatcatg tagacatc                                                  18


<210>  1739
<211>  16
<212>  DNA
<213>  Artificial

<220>
<223>  Antisense nucleotide

<400>  1739
ccatgatcat gtagac                                                    16


<210>  1740
<211>  18
<212>  DNA
<213>  Artificial

<220>
<223>  Antisense nucleotide

<400>  1740
catttgacca tgatcatg                                                  18


<210>  1741
<211>  16
<212>  DNA
<213>  Artificial

<220>
<223>  Antisense nucleotide

<400>  1741
ccaacatttg accatg                                                    16


<210>  1742
<211>  18
<212>  DNA
<213>  Artificial

<220>
<223>  Antisense nucleotide

<400>  1742
tcatccaaca tttgacca                                                  18

<210>  1743
<211>  18
<212>  DNA
<213>  Artificial

<220>
<223>  Antisense nucleotide

<400>  1743
gagtcaatca tccaacat                                                    18


<210>  1744
<211>  16
<212>  DNA
<213>  Artificial

<220>
<223>  Antisense nucleotide

<400>  1744
cagagtcaat catcca                                                      16


<210>  1745
<211>  14
<212>  DNA
<213>  Artificial

<220>
<223>  Antisense nucleotide

<400>  1745
ccgacattca gagt                                                        14


<210>  1746
<211>  16
<212>  DNA
<213>  Artificial

<220>
<223>  Antisense nucleotide

<400>  1746
gaattcagac accaac                                                      16


<210>  1747
<211>  14
<212>  DNA
<213>  Artificial

<220>
<223>  Antisense nucleotide

<400>  1747
gatgaccaca aagc                                                        14


<210>  1748
<211>  16
<212>  DNA
<213>  Artificial

<220>
<223>  Antisense nucleotide

<400>  1748
ccatcaaata catcgg                                                        16


<210>  1749
<211>  18
<212>  DNA
<213>  Artificial

<220>
<223>  Antisense nucleotide

<400>  1749
tcaccatcaa atacatcg                                                      18


<210>  1750
<211>  14
<212>  DNA
<213>  Artificial

<220>
<223>  Antisense nucleotide

<400>  1750
caacgtagcc atca                                                          14


<210>  1751
<211>  14
<212>  DNA
<213>  Artificial

<220>
<223>  Antisense nucleotide

<400>  1751
acgtctttga cgac                                                          14


<210>  1752
<211>  18
<212>  DNA
<213>  Artificial

<220>
<223>  Antisense nucleotide

<400>  1752
caaaaacgtc tttgacga                                                      18


<210>  1753
<211>  16
<212>  DNA
<213>  Artificial

<220>
<223>  Antisense nucleotide

<400>  1753
ggcaaaaacg tctttg                                                        16


<210>  1754
<211>  16
<212>  DNA
<213>  Artificial

<220>
<223>  Antisense nucleotide

<400>  1754
caaaggcaaa aacgtc                                                        16


<210>  1755
<211>  14
<212>  DNA
<213>  Artificial

<220>
<223>  Antisense nucleotide

<400>  1755
gtgtcaagta ctcg                                                          14


<210>  1756
<211>  16
<212>  DNA
<213>  Artificial

<220>
<223>  Antisense nucleotide

<400>  1756
gtaatagagg ttgtcg                                                        16


<210>  1757
<211>  14
<212>  DNA
<213>  Artificial

<220>
<223>  Antisense nucleotide

<400>  1757
cccagtaata gagg                                                          14


<210>  1758
<211>  14
<212>  DNA
<213>  Artificial

<220>
<223>  Antisense nucleotide

<400>  1758
catggtgctc actg                                                          14


<210>  1759
<211>  14
<212>  DNA
<213>  Artificial

<220>
<223>  Antisense nucleotide

<400>  1759
gtgcctgtac gtac                                                          14

<210>  1760
<211>  14
<212>  DNA
<213>  Artificial

<220>
<223>  Antisense nucleotide

<400>  1760
tgcaggtgga tagt                                                          14


<210>  1761
<211>  18
<212>  DNA
<213>  Artificial

<220>
<223>  Antisense nucleotide

<400>  1761
catgtcgata gtcttgca                                                      18


<210>  1762
<211>  14
<212>  DNA
<213>  Artificial

<220>
<223>  Antisense nucleotide

<400>  1762
gtcgatagtc ttgc                                                          14


<210>  1763
<211>  14
<212>  DNA
<213>  Artificial

<220>
<223>  Antisense nucleotide

<400>  1763
ccatgtcgat agtc                                                          14


<210>  1764
<211>  14
<212>  DNA
<213>  Artificial

<220>
<223>  Antisense nucleotide

<400>  1764
ctccatgtcg atag                                                          14


<210>  1765
<211>  14
<212>  DNA
<213>  Artificial

<220>
<223>  Antisense nucleotide

<400>  1765
cttggacagg atct                                                          14


<210>  1766
<211>  14
<212>  DNA
<213>  Artificial

<220>
<223>  Antisense nucleotide

<400>  1766
tgctgttgta cagg                                                          14


<210>  1767
<211>  14
<212>  DNA
<213>  Artificial

<220>
<223>  Antisense nucleotide

<400>  1767
gtgctgttgt acag                                                          14


<210>  1768
<211>  14
<212>  DNA
<213>  Artificial

<220>
<223>  Antisense nucleotide

<400>  1768
ttggcgtagt agtc                                                          14


<210>  1769
<211>  14
<212>  DNA
<213>  Artificial

<220>
<223>  Antisense nucleotide

<400>  1769
tccaccatta gcac                                                          14


<210>  1770
<211>  14
<212>  DNA
<213>  Artificial

<220>
<223>  Antisense nucleotide

<400>  1770
gatttcgttg tggg                                                          14


<210>  1771
<211>  18
<212>  DNA
<213>  Artificial

<220>
<223>  Antisense nucleotide

<400>  1771
gtcatagatt tcgttgtg                                                    18


<210>  1772
<211>  16
<212>  DNA
<213>  Artificial

<220>
<223>  Antisense nucleotide

<400>  1772
tgtactctgc ttgaac                                                      16


<210>  1773
<211>  14
<212>  DNA
<213>  Artificial

<220>
<223>  Antisense nucleotide

<400>  1773
gtgtactctg cttg                                                        14


<210>  1774
<211>  14
<212>  DNA
<213>  Artificial

<220>
<223>  Antisense nucleotide

<400>  1774
tgctgtgtgt actc                                                        14


<210>  1775
<211>  18
<212>  DNA
<213>  Artificial

<220>
<223>  Antisense nucleotide

<400>  1775
ctgatgtgtt gaagaaca                                                    18


<210>  1776
<211>  16
<212>  DNA
<213>  Artificial

<220>
<223>  Antisense nucleotide

<400>  1776
ctctgatgtg ttgaag                                                      16

<210>  1777
<211>  14
<212>  DNA
<213>  Artificial

<220>
<223>  Antisense nucleotide

<400>  1777
gctctgatgt gttg                                                         14


<210>  1778
<211>  14
<212>  DNA
<213>  Artificial

<220>
<223>  Antisense nucleotide

<400>  1778
gagctctgat gtgt                                                         14


<210>  1779
<211>  18
<212>  DNA
<213>  Artificial

<220>
<223>  Antisense nucleotide

<400>  1779
cacttttaac ttgagcct                                                     18


<210>  1780
<211>  18
<212>  DNA
<213>  Artificial

<220>
<223>  Antisense nucleotide

<400>  1780
ctccactttt aacttgag                                                     18


<210>  1781
<211>  18
<212>  DNA
<213>  Artificial

<220>
<223>  Antisense nucleotide

<400>  1781
tgctgtattt ctggtaca                                                     18


<210>  1782
<211>  14
<212>  DNA
<213>  Artificial

<220>
<223>  Antisense nucleotide

<400>  1782
ccaggaattg ttgc                                                          14


<210>  1783
<211>  14
<212>  DNA
<213>  Artificial

<220>
<223>  Antisense nucleotide

<400>  1783
ttgctgaggt atcg                                                          14


<210>  1784
<211>  14
<212>  DNA
<213>  Artificial

<220>
<223>  Antisense nucleotide

<400>  1784
gataaccact ctgg                                                          14


<210>  1785
<211>  18
<212>  DNA
<213>  Artificial

<220>
<223>  Antisense nucleotide

<400>  1785
caaaagataa ccactctg                                                      18


<210>  1786
<211>  15
<212>  DNA
<213>  Artificial

<220>
<223>  Antisense nucleotide

<400>  1786
cggtgacatc aaaag                                                         15


<210>  1787
<211>  14
<212>  DNA
<213>  Artificial

<220>
<223>  Antisense nucleotide

<400>  1787
cctcaatttc ccct                                                          14


<210>  1788
<211>  14
<212>  DNA
<213>  Artificial

<220>
<223>  Antisense nucleotide

<400>  1788
gttatccctg ctgt                                                          14


<210>  1789
<211>  14
<212>  DNA
<213>  Artificial

<220>
<223>  Antisense nucleotide

<400>  1789
gcagtgtgtt atcc                                                          14


<210>  1790
<211>  14
<212>  DNA
<213>  Artificial

<220>
<223>  Antisense nucleotide

<400>  1790
gatgtccact tgca                                                          14


<210>  1791
<211>  14
<212>  DNA
<213>  Artificial

<220>
<223>  Antisense nucleotide

<400>  1791
tagtgaaccc gttg                                                          14


<210>  1792
<211>  14
<212>  DNA
<213>  Artificial

<220>
<223>  Antisense nucleotide

<400>  1792
tgccatgaat ggtg                                                          14


<210>  1793
<211>  16
<212>  DNA
<213>  Artificial

<220>
<223>  Antisense nucleotide

<400>  1793
gttcatgcca tgaatg                                                        16

<210>  1794
<211>  14
<212>  DNA
<213>  Artificial

<220>
<223>  Antisense nucleotide

<400>  1794
catgagaagc agga                                                          14


<210>  1795
<211>  14
<212>  DNA
<213>  Artificial

<220>
<223>  Antisense nucleotide

<400>  1795
gctttgcaga tgct                                                          14


<210>  1796
<211>  14
<212>  DNA
<213>  Artificial

<220>
<223>  Antisense nucleotide

<400>  1796
gagctttgca gatg                                                          14


<210>  1797
<211>  14
<212>  DNA
<213>  Artificial

<220>
<223>  Antisense nucleotide

<400>  1797
tagttggtgt ccag                                                          14


<210>  1798
<211>  16
<212>  DNA
<213>  Artificial

<220>
<223>  Antisense nucleotide

<400>  1798
ctgaagcaat agttgg                                                        16


<210>  1799
<211>  18
<212>  DNA
<213>  Artificial

<220>
<223>  Antisense nucleotide

<400>  1799
agctgaagca atagttgg                                                    18


<210>  1800
<211>  14
<212>  DNA
<213>  Artificial

<220>
<223>  Antisense nucleotide

<400>  1800
ggagctgaag caat                                                        14


<210>  1801
<211>  14
<212>  DNA
<213>  Artificial

<220>
<223>  Antisense nucleotide

<400>  1801
caatgtacag ctgc                                                        14


<210>  1802
<211>  16
<212>  DNA
<213>  Artificial

<220>
<223>  Antisense nucleotide

<400>  1802
ggaagtcaat gtacag                                                      16


<210>  1803
<211>  15
<212>  DNA
<213>  Artificial

<220>
<223>  Antisense nucleotide

<400>  1803
cggaagtcaa tgtac                                                       15


<210>  1804
<211>  14
<212>  DNA
<213>  Artificial

<220>
<223>  Antisense nucleotide

<400>  1804
gcggaagtca atgt                                                        14


<210>  1805
<211>  14
<212>  DNA
<213>  Artificial

<220>
<223>  Antisense nucleotide

<400>  1805
agttggcatg gtag                                                          14


<210>  1806
<211>  14
<212>  DNA
<213>  Artificial

<220>
<223>  Antisense nucleotide

<400>  1806
gcagaagttg gcat                                                          14


<210>  1807
<211>  14
<212>  DNA
<213>  Artificial

<220>
<223>  Antisense nucleotide

<400>  1807
ctccaaatgt aggg                                                          14


<210>  1808
<211>  14
<212>  DNA
<213>  Artificial

<220>
<223>  Antisense nucleotide

<400>  1808
accttgctgt actg                                                          14


<210>  1809
<211>  14
<212>  DNA
<213>  Artificial

<220>
<223>  Antisense nucleotide

<400>  1809
tgctggttgt acag                                                          14


<210>  1810
<211>  14
<212>  DNA
<213>  Artificial

<220>
<223>  Antisense nucleotide

<400>  1810
ggttatgctg gttg                                                          14

<210>  1811
<211>  14
<212>  DNA
<213>  Artificial

<220>
<223>  Antisense nucleotide

<400>  1811
gtagtacacg atgg                                                          14


<210>  1812
<211>  14
<212>  DNA
<213>  Artificial

<220>
<223>  Antisense nucleotide

<400>  1812
cgtagtacac gatg                                                          14


<210>  1813
<211>  14
<212>  DNA
<213>  Artificial

<220>
<223>  Antisense nucleotide

<400>  1813
cacgtagtac acga                                                          14


<210>  1814
<211>  14
<212>  DNA
<213>  Artificial

<220>
<223>  Antisense nucleotide

<400>  1814
catgttggac agct                                                          14


<210>  1815
<211>  14
<212>  DNA
<213>  Artificial

<220>
<223>  Antisense nucleotide

<400>  1815
gcacgatcat gttg                                                          14


<210>  1816
<211>  14
<212>  DNA
<213>  Artificial

<220>
<223>  Antisense nucleotide

<400>  1816
cacacagtag tgca                                                          14


<210>  1817
<211>  14
<212>  DNA
<213>  Artificial

<220>
<223>  Antisense nucleotide

<400>  1817
gatcagaaaa gcgc                                                          14


<210>  1818
<211>  14
<212>  DNA
<213>  Artificial

<220>
<223>  Antisense nucleotide

<400>  1818
accgtgacca gatg                                                          14


<210>  1819
<211>  14
<212>  DNA
<213>  Artificial

<220>
<223>  Antisense nucleotide

<400>  1819
gtagacaggc tgag                                                          14


<210>  1820
<211>  14
<212>  DNA
<213>  Artificial

<220>
<223>  Antisense nucleotide

<400>  1820
tatcgagtgt gctg                                                          14


<210>  1821
<211>  14
<212>  DNA
<213>  Artificial

<220>
<223>  Antisense nucleotide

<400>  1821
ttgcgcatga actg                                                          14


<210>  1822
<211>  14
<212>  DNA
<213>  Artificial

<220>
<223>  Antisense nucleotide

<400>  1822
ttgctcagga tctg                                                          14


<210>  1823
<211>  14
<212>  DNA
<213>  Artificial

<220>
<223>  Antisense nucleotide

<400>  1823
actggtgagc ttca                                                          14


<210>  1824
<211>  14
<212>  DNA
<213>  Artificial

<220>
<223>  Antisense nucleotide

<400>  1824
gctcaggata gtct                                                          14


<210>  1825
<211>  18
<212>  DNA
<213>  Artificial

<220>
<223>  Antisense nucleotide

<400>  1825
tgtagatgga aatcacct                                                      18


<210>  1826
<211>  14
<212>  DNA
<213>  Artificial

<220>
<223>  Antisense nucleotide

<400>  1826
tggtgctgtt gtag                                                          14


<210>  1827
<211>  14
<212>  DNA
<213>  Artificial

<220>
<223>  Antisense nucleotide

<400>  1827
ttctcctgga gcaa                                                          14

<210>  1828
<211>  14
<212>  DNA
<213>  Artificial

<220>
<223>  Antisense nucleotide

<400>  1828
tactcttcgt cgct                                                          14


<210>  1829
<211>  14
<212>  DNA
<213>  Artificial

<220>
<223>  Antisense nucleotide

<400>  1829
cttggcgtag tact                                                          14


<210>  1830
<211>  18
<212>  DNA
<213>  Artificial

<220>
<223>  Antisense nucleotide

<400>  1830
cggcatgtct attttgta                                                      18


<210>  1831
<211>  14
<212>  DNA
<213>  Artificial

<220>
<223>  Antisense nucleotide

<400>  1831
cgggatggca tttt                                                          14


<210>  1832
<211>  14
<212>  DNA
<213>  Artificial

<220>
<223>  Antisense nucleotide

<400>  1832
ctgtagaaag tggg                                                          14


<210>  1833
<211>  18
<212>  DNA
<213>  Artificial

<220>
<223>  Antisense nucleotide

<400>  1833
acaattctga agtagggt                                                    18


<210>  1834
<211>  18
<212>  DNA
<213>  Artificial

<220>
<223>  Antisense nucleotide

<400>  1834
attgctgaga cgtcaaat                                                    18


<210>  1835
<211>  14
<212>  DNA
<213>  Artificial

<220>
<223>  Antisense nucleotide

<400>  1835
tctccattgc tgag                                                        14


<210>  1836
<211>  18
<212>  DNA
<213>  Artificial

<220>
<223>  Antisense nucleotide

<400>  1836
tcaccaaatt ggaagcat                                                    18


<210>  1837
<211>  14
<212>  DNA
<213>  Artificial

<220>
<223>  Antisense nucleotide

<400>  1837
ctctgaactc tgct                                                        14


<210>  1838
<211>  18
<212>  DNA
<213>  Artificial

<220>
<223>  Antisense nucleotide

<400>  1838
aacgaaagac tctgaact                                                    18


<210>  1839
<211>  14
<212>  DNA
<213>  Artificial

<220>
<223>  Antisense nucleotide

<400>  1839
tgggttctgc aaac                                                          14


<210>  1840
<211>  14
<212>  DNA
<213>  Artificial

<220>
<223>  Antisense nucleotide

<400>  1840
ctggcttttg ggtt                                                          14


<210>  1841
<211>  14
<212>  DNA
<213>  Artificial

<220>
<223>  Antisense nucleotide

<400>  1841
gttgttcagg cact                                                          14


<210>  1842
<211>  18
<212>  DNA
<213>  Artificial

<220>
<223>  Antisense nucleotide

<400>  1842
tctgatatag ctcaatcc                                                      18


<210>  1843
<211>  18
<212>  DNA
<213>  Artificial

<220>
<223>  Antisense nucleotide

<400>  1843
tctttggact tgagaatc                                                      18


<210>  1844
<211>  14
<212>  DNA
<213>  Artificial

<220>
<223>  Antisense nucleotide

<400>  1844
tgggttggag atgt                                                          14

<210>  1845
<211>  14
<212>  DNA
<213>  Artificial

<220>
<223>  Antisense nucleotide

<400>  1845
tgctgtcgat gtag                                                          14


<210>  1846
<211>  16
<212>  DNA
<213>  Artificial

<220>
<223>  Antisense nucleotide

<400>  1846
acaactttgc tgtcga                                                        16


<210>  1847
<211>  14
<212>  DNA
<213>  Artificial

<220>
<223>  Antisense nucleotide

<400>  1847
attcgccttc tgct                                                          14


<210>  1848
<211>  14
<212>  DNA
<213>  Artificial

<220>
<223>  Antisense nucleotide

<400>  1848
gaaggagagc catt                                                          14


<210>  1849
<211>  16
<212>  DNA
<213>  Artificial

<220>
<223>  Antisense nucleotide

<400>  1849
tcagttacat cgaagg                                                        16


<210>  1850
<211>  18
<212>  DNA
<213>  Artificial

<220>
<223>  Antisense nucleotide

<400>  1850
tgaagccatt catgaaca                                                    18


<210>  1851
<211>  16
<212>  DNA
<213>  Artificial

<220>
<223>  Antisense nucleotide

<400>  1851
tcctgtcttt atggtg                                                      16


<210>  1852
<211>  14
<212>  DNA
<213>  Artificial

<220>
<223>  Antisense nucleotide

<400>  1852
aaatcccagg ttcc                                                        14


<210>  1853
<211>  18
<212>  DNA
<213>  Artificial

<220>
<223>  Antisense nucleotide

<400>  1853
ggacagtgta agcttatt                                                    18


<210>  1854
<211>  16
<212>  DNA
<213>  Artificial

<220>
<223>  Antisense nucleotide

<400>  1854
gtacaaaagt gcagca                                                      16


<210>  1855
<211>  18
<212>  DNA
<213>  Artificial

<220>
<223>  Antisense nucleotide

<400>  1855
tagatggtac aaaagtgc                                                    18


<210>  1856
<211>  20
<212>  DNA
<213>  Artificial

<220>
<223>  Antisense nucleotide

<400>  1856
cacttttatt tgggatgatg                                                    20


<210>  1857
<211>  18
<212>  DNA
<213>  Artificial

<220>
<223>  Antisense nucleotide

<400>  1857
gcaaatcttg cttctagt                                                      18


<210>  1858
<211>  14
<212>  DNA
<213>  Artificial

<220>
<223>  Antisense nucleotide

<400>  1858
gtgccatcaa tacc                                                          14


<210>  1859
<211>  14
<212>  DNA
<213>  Artificial

<220>
<223>  Antisense nucleotide

<400>  1859
ggtatatgtg gagg                                                          14


<210>  1860
<211>  14
<212>  DNA
<213>  Artificial

<220>
<223>  Antisense nucleotide

<400>  1860
tctgatcacc actg                                                          14


<210>  1861
<211>  18
<212>  DNA
<213>  Artificial

<220>
<223>  Antisense nucleotide

<400>  1861
tcctagtgga ctttatag                                                      18

<210>  1862
<211>  16
<212>  DNA
<213>  Artificial

<220>
<223>  Antisense nucleotide

<400>  1862
tttttcctag tggact                                                        16


<210>  1863
<211>  16
<212>  DNA
<213>  Artificial

<220>
<223>  Antisense nucleotide

<400>  1863
caataacatt agcagg                                                        16


<210>  1864
<211>  14
<212>  DNA
<213>  Artificial

<220>
<223>  Antisense nucleotide

<400>  1864
aagtctgtag gagg                                                          14


<210>  1865
<211>  16
<212>  DNA
<213>  Artificial

<220>
<223>  Antisense nucleotide

<400>  1865
tctgttgtga ctcaag                                                        16


<210>  1866
<211>  14
<212>  DNA
<213>  Artificial

<220>
<223>  Antisense nucleotide

<400>  1866
gttggtctgt tgtg                                                          14


<210>  1867
<211>  14
<212>  DNA
<213>  Artificial

<220>
<223>  Antisense nucleotide

<400>  1867
caaagcacgc ttct                                                          14


<210>  1868
<211>  18
<212>  DNA
<213>  Artificial

<220>
<223>  Antisense nucleotide

<400>  1868
tttctaaagc aataggcc                                                      18


<210>  1869
<211>  16
<212>  DNA
<213>  Artificial

<220>
<223>  Antisense nucleotide

<400>  1869
gcaattatcc tgcaca                                                        16


<210>  1870
<211>  14
<212>  DNA
<213>  Artificial

<220>
<223>  Antisense nucleotide

<400>  1870
acgtaggcag caat                                                          14


<210>  1871
<211>  18
<212>  DNA
<213>  Artificial

<220>
<223>  Antisense nucleotide

<400>  1871
atcaatgtaa agtggacg                                                      18


<210>  1872
<211>  14
<212>  DNA
<213>  Artificial

<220>
<223>  Antisense nucleotide

<400>  1872
ctagatccct cttg                                                          14


<210>  1873
<211>  14
<212>  DNA
<213>  Artificial

<220>
<223>  Antisense nucleotide

<400>  1873
ccatttccac ccta                                                          14


<210>  1874
<211>  14
<212>  DNA
<213>  Artificial

<220>
<223>  Antisense nucleotide

<400>  1874
tgggttcgtg tatc                                                          14


<210>  1875
<211>  14
<212>  DNA
<213>  Artificial

<220>
<223>  Antisense nucleotide

<400>  1875
tggcattgta ccct                                                          14


<210>  1876
<211>  14
<212>  DNA
<213>  Artificial

<220>
<223>  Antisense nucleotide

<400>  1876
tccagcacag aagt                                                          14


<210>  1877
<211>  16
<212>  DNA
<213>  Artificial

<220>
<223>  Antisense nucleotide

<400>  1877
ataaatacgg gcatgc                                                        16


<210>  1878
<211>  14
<212>  DNA
<213>  Artificial

<220>
<223>  Antisense nucleotide

<400>  1878
agtgtctgaa ctcc                                                          14

<210>  1879
<211>  14
<212>  DNA
<213>  Artificial

<220>
<223>  Antisense nucleotide

<400>  1879
tgtgctgagt gtct                                                          14


<210>  1880
<211>  14
<212>  DNA
<213>  Artificial

<220>
<223>  Antisense nucleotide

<400>  1880
ataagctcag gacc                                                          14


<210>  1881
<211>  14
<212>  DNA
<213>  Artificial

<220>
<223>  Antisense nucleotide

<400>  1881
aggagaagca gatg                                                          14


<210>  1882
<211>  14
<212>  DNA
<213>  Artificial

<220>
<223>  Antisense nucleotide

<400>  1882
agcaaggaga agca                                                          14


<210>  1883
<211>  14
<212>  DNA
<213>  Artificial

<220>
<223>  Antisense nucleotide

<400>  1883
aatcttggga cacg                                                          14


<210>  1884
<211>  18
<212>  DNA
<213>  Artificial

<220>
<223>  Antisense nucleotide

<400>  1884
tagagaatgg ttagaggt                                                    18


<210>  1885
<211>  18
<212>  DNA
<213>  Artificial

<220>
<223>  Antisense nucleotide

<400>  1885
gttttgccaa tgtagtag                                                    18


<210>  1886
<211>  14
<212>  DNA
<213>  Artificial

<220>
<223>  Antisense nucleotide

<400>  1886
cttgggtgtt ttgc                                                        14


<210>  1887
<211>  16
<212>  DNA
<213>  Artificial

<220>
<223>  Antisense nucleotide

<400>  1887
gcaagacttt acaatc                                                      16


<210>  1888
<211>  18
<212>  DNA
<213>  Artificial

<220>
<223>  Antisense nucleotide

<400>  1888
gcatttgcaa gactttac                                                    18


<210>  1889
<211>  18
<212>  DNA
<213>  Artificial

<220>
<223>  Antisense nucleotide

<400>  1889
tttagctgca tttgcaag                                                    18


<210>  1890
<211>  14
<212>  DNA
<213>  Artificial

<220>
<223>  Antisense nucleotide

<400>  1890
gccacttttc caag                                                          14


<210>  1891
<211>  14
<212>  DNA
<213>  Artificial

<220>
<223>  Antisense nucleotide

<400>  1891
ttggtcttgc cact                                                          14


<210>  1892
<211>  14
<212>  DNA
<213>  Artificial

<220>
<223>  Antisense nucleotide

<400>  1892
cagcacacag tagt                                                          14


<210>  1893
<211>  14
<212>  DNA
<213>  Artificial

<220>
<223>  Antisense nucleotide

<400>  1893
cgatagtctt gcag                                                          14


<210>  1894
<211>  18
<212>  DNA
<213>  Artificial

<220>
<223>  Antisense nucleotide

<400>  1894
ctttcaccaa attggaag                                                      18


<210>  1895
<211>  15
<212>  DNA
<213>  Artificial

<220>
<223>  Antisense nucleotide

<400>  1895
caccaaattg gaagc                                                         15

<210>  1896
<211>  16
<212>  DNA
<213>  Artificial

<220>
<223>  Antisense nucleotide

<400>  1896
tcaccaaatt ggaagc                                                        16


<210>  1897
<211>  14
<212>  DNA
<213>  Artificial

<220>
<223>  Antisense nucleotide

<400>  1897
ctctggcttt tggg                                                          14


<210>  1898
<211>  16
<212>  DNA
<213>  Artificial

<220>
<223>  Antisense nucleotide

<400>  1898
cggcatgtct attttg                                                        16


<210>  1899
<211>  14
<212>  DNA
<213>  Artificial

<220>
<223>  Antisense nucleotide

<400>  1899
cactacagac gagc                                                          14


<210>  1900
<211>  15
<212>  DNA
<213>  Artificial

<220>
<223>  Antisense nucleotide

<400>  1900
cgtgcactac agacg                                                         15


<210>  1901
<211>  14
<212>  DNA
<213>  Artificial

<220>
<223>  Antisense nucleotide

<400>  1901
ggaacagttc gtcc                                                         14


<210>  1902
<211>  16
<212>  DNA
<213>  Artificial

<220>
<223>  Antisense nucleotide

<400>  1902
gaacagttcg tccatg                                                       16


<210>  1903
<211>  15
<212>  DNA
<213>  Artificial

<220>
<223>  Antisense nucleotide

<400>  1903
ccagagtttc ggttc                                                        15


<210>  1904
<211>  15
<212>  DNA
<213>  Artificial

<220>
<223>  Antisense nucleotide

<400>  1904
ctaggactgg gacag                                                        15


<210>  1905
<211>  13
<212>  DNA
<213>  Artificial

<220>
<223>  Antisense nucleotide

<400>  1905
cgcacttgta gcg                                                          13


<210>  1906
<211>  14
<212>  DNA
<213>  Artificial

<220>
<223>  Antisense nucleotide

<400>  1906
ctcgcacttg tagc                                                         14


<210>  1907
<211>  11
<212>  DNA
<213>  Artificial

<220>
<223>  Antisense nucleotide

<400>  1907
gcacttgtag c                                                            11


<210>  1908
<211>  14
<212>  DNA
<213>  Artificial

<220>
<223>  Antisense nucleotide

<400>  1908
gcgcactgtc cctg                                                         14


<210>  1909
<211>  14
<212>  DNA
<213>  Artificial

<220>
<223>  Antisense nucleotide

<400>  1909
ccagggagat gcgc                                                         14


<210>  1910
<211>  13
<212>  DNA
<213>  Artificial

<220>
<223>  Antisense nucleotide

<400>  1910
gccggtgagg agg                                                          13


<210>  1911
<211>  13
<212>  DNA
<213>  Artificial

<220>
<223>  Antisense nucleotide

<400>  1911
ccggtgagga ggg                                                          13


<210>  1912
<211>  13
<212>  DNA
<213>  Artificial

<220>
<223>  Antisense nucleotide

<400>  1912
cggttcactc ggc                                                          13

<210>  1913
<211>  16
<212>  DNA
<213>  Artificial

<220>
<223>  Antisense nucleotide

<400>  1913
gagtttcggt tcactc                                                        16


<210>  1914
<211>  16
<212>  DNA
<213>  Artificial

<220>
<223>  Antisense nucleotide

<400>  1914
ggcacgattg tcaaag                                                        16


<210>  1915
<211>  14
<212>  DNA
<213>  Artificial

<220>
<223>  Antisense nucleotide

<400>  1915
caggcgtcac cccc                                                          14


<210>  1916
<211>  13
<212>  DNA
<213>  Artificial

<220>
<223>  Antisense nucleotide

<400>  1916
gcaggcgtca ccc                                                           13


<210>  1917
<211>  13
<212>  DNA
<213>  Artificial

<220>
<223>  Antisense nucleotide

<400>  1917
ctccctccta agc                                                           13


<210>  1918
<211>  13
<212>  DNA
<213>  Artificial

<220>
<223>  Antisense nucleotide

<400>  1918
ccctcctaag cgg                                                          13


<210>  1919
<211>  14
<212>  DNA
<213>  Artificial

<220>
<223>  Antisense nucleotide

<400>  1919
cgagtccgcg ttcg                                                         14


<210>  1920
<211>  15
<212>  DNA
<213>  Artificial

<220>
<223>  Antisense nucleotide

<400>  1920
catcttctgc cattc                                                        15


<210>  1921
<211>  15
<212>  DNA
<213>  Artificial

<220>
<223>  Antisense nucleotide

<400>  1921
gtgttttccc accag                                                        15


<210>  1922
<211>  16
<212>  DNA
<213>  Artificial

<220>
<223>  Antisense nucleotide

<400>  1922
ggttttggtt cactag                                                       16


<210>  1923
<211>  16
<212>  DNA
<213>  Artificial

<220>
<223>  Antisense nucleotide

<400>  1923
gcatcttcac gtctcc                                                       16


<210>  1924
<211>  16
<212>  DNA
<213>  Artificial

<220>
<223>  Antisense nucleotide

<400>  1924
cttcacgtct cctgtc                                                        16


<210>  1925
<211>  14
<212>  DNA
<213>  Artificial

<220>
<223>  Antisense nucleotide

<400>  1925
gtcaccgcgt agtc                                                          14


<210>  1926
<211>  15
<212>  DNA
<213>  Artificial

<220>
<223>  Antisense nucleotide

<400>  1926
caaataggca aggtc                                                         15


<210>  1927
<211>  16
<212>  DNA
<213>  Artificial

<220>
<223>  Antisense nucleotide

<400>  1927
cttgcaaata ggcaag                                                        16


<210>  1928
<211>  14
<212>  DNA
<213>  Artificial

<220>
<223>  Antisense nucleotide

<400>  1928
tgcttgcaaa tagg                                                          14


<210>  1929
<211>  15
<212>  DNA
<213>  Artificial

<220>
<223>  Antisense nucleotide

<400>  1929
ctgcttgcaa atagg                                                         15

<210>  1930
<211>  14
<212>  DNA
<213>  Artificial

<220>
<223>  Antisense nucleotide

<400>  1930
gcaggtggat attt                                                          14


<210>  1931
<211>  13
<212>  DNA
<213>  Artificial

<220>
<223>  Antisense nucleotide

<400>  1931
ctgctgttgg cag                                                           13


<210>  1932
<211>  15
<212>  DNA
<213>  Artificial

<220>
<223>  Antisense nucleotide

<400>  1932
cactagtttc caagt                                                         15


<210>  1933
<211>  15
<212>  DNA
<213>  Artificial

<220>
<223>  Antisense nucleotide

<400>  1933
gttttggttc actag                                                         15


<210>  1934
<211>  17
<212>  DNA
<213>  Artificial

<220>
<223>  Antisense nucleotide

<400>  1934
ctttgatttc aggatag                                                       17


<210>  1935
<211>  17
<212>  DNA
<213>  Artificial

<220>
<223>  Antisense nucleotide

<400>  1935
gcacttcttc tttatct                                                    17

<210>  1936
<211>  15
<212>  DNA
<213>  Artificial

<220>
<223>  Antisense nucleotide

<400>  1936
ccaagtcaga tttcc                                                      15

<210>  1937
<211>  18
<212>  DNA
<213>  Artificial

<220>
<223>  Antisense nucleotide

<400>  1937
gtttccaagt cagatttc                                                   18

<210>  1938
<211>  15
<212>  DNA
<213>  Artificial

<220>
<223>  Antisense nucleotide

<400>  1938
ggttcactag tttcc                                                      15

<210>  1939
<211>  16
<212>  DNA
<213>  Artificial

<220>
<223>  Antisense nucleotide

<400>  1939
ggttttggtt cactag                                                     16

<210>  1940
<211>  15
<212>  DNA
<213>  Artificial

<220>
<223>  Antisense nucleotide

<400>  1940
ccgaaaaatt gggca                                                      15

<210>  1941
<211>  13
<212>  DNA
<213>  Artificial

<220>
<223>  Antisense nucleotide

<400>  1941
ccgaaaaatt ggg                                                          13


<210>  1942
<211>  16
<212>  DNA
<213>  Artificial

<220>
<223>  Antisense nucleotide

<400>  1942
ctatccgaaa aattgg                                                       16


<210>  1943
<211>  17
<212>  DNA
<213>  Artificial

<220>
<223>  Antisense nucleotide

<400>  1943
gttgataatg tcatcag                                                      17


<210>  1944
<211>  17
<212>  DNA
<213>  Artificial

<220>
<223>  Antisense nucleotide

<400>  1944
ctcatgttga taatgtc                                                      17


<210>  1945
<211>  14
<212>  DNA
<213>  Artificial

<220>
<223>  Antisense nucleotide

<400>  1945
ctgtcaccgc gtag                                                         14


<210>  1946
<211>  15
<212>  DNA
<213>  Artificial

<220>
<223>  Antisense nucleotide

<400>  1946
cgtctcctgt caccg                                                        15

<210>  1947
<211>  14
<212>  DNA
<213>  Artificial

<220>
<223>  Antisense nucleotide

<400>  1947
cttcacgtct cctg                                                          14


<210>  1948
<211>  17
<212>  DNA
<213>  Artificial

<220>
<223>  Antisense nucleotide

<400>  1948
gagaacttta tcatgtc                                                       17


<210>  1949
<211>  13
<212>  DNA
<213>  Artificial

<220>
<223>  Antisense nucleotide

<400>  1949
gctatatgca ggg                                                           13


<210>  1950
<211>  18
<212>  DNA
<213>  Artificial

<220>
<223>  Antisense nucleotide

<400>  1950
ccagctgcta tatgcagg                                                      18


<210>  1951
<211>  16
<212>  DNA
<213>  Artificial

<220>
<223>  Antisense nucleotide

<400>  1951
aggctaaatt ttgcct                                                        16


<210>  1952
<211>  14
<212>  DNA
<213>  Artificial

<220>
<223>  Antisense nucleotide

<400>  1952
ggctaaattt tgcc                                                         14


<210>  1953
<211>  17
<212>  DNA
<213>  Artificial

<220>
<223>  Antisense nucleotide

<400>  1953
ggctaaattt tgccttc                                                      17


<210>  1954
<211>  17
<212>  DNA
<213>  Artificial

<220>
<223>  Antisense nucleotide

<400>  1954
gcaggctaaa ttttgcc                                                      17


<210>  1955
<211>  14
<212>  DNA
<213>  Artificial

<220>
<223>  Antisense nucleotide

<400>  1955
gagttaccca agcg                                                         14


<210>  1956
<211>  16
<212>  DNA
<213>  Artificial

<220>
<223>  Antisense nucleotide

<400>  1956
cagagttacc caagcg                                                       16


<210>  1957
<211>  14
<212>  DNA
<213>  Artificial

<220>
<223>  Antisense nucleotide

<400>  1957
cagagttacc caag                                                         14


<210>  1958
<211>  15
<212>  DNA
<213>  Artificial

<220>
<223>  Antisense nucleotide

<400>  1958
acagagttac ccaag                                                        15


<210>  1959
<211>  14
<212>  DNA
<213>  Artificial

<220>
<223>  Antisense nucleotide

<400>  1959
ggtgcaaaac agag                                                         14


<210>  1960
<211>  15
<212>  DNA
<213>  Artificial

<220>
<223>  Antisense nucleotide

<400>  1960
ctaggtgcaa aacag                                                        15


<210>  1961
<211>  18
<212>  DNA
<213>  Artificial

<220>
<223>  Antisense nucleotide

<400>  1961
gagaacttta tcatgtcc                                                     18


<210>  1962
<211>  14
<212>  DNA
<213>  Artificial

<220>
<223>  Antisense nucleotide

<400>  1962
gctagatgaa tggc                                                         14


<210>  1963
<211>  15
<212>  DNA
<213>  Artificial

<220>
<223>  Antisense nucleotide

<400>  1963
gcaaacatgg caggc                                                        15

<210>  1964
<211>  14
<212>  DNA
<213>  Artificial

<220>
<223>  Antisense nucleotide

<400>  1964
cagcaaacat ggca                                                          14


<210>  1965
<211>  14
<212>  DNA
<213>  Artificial

<220>
<223>  Antisense nucleotide

<400>  1965
gcagcaaaca tggc                                                          14


<210>  1966
<211>  14
<212>  DNA
<213>  Artificial

<220>
<223>  Antisense nucleotide

<400>  1966
agcagcaaac atgg                                                          14


<210>  1967
<211>  14
<212>  DNA
<213>  Artificial

<220>
<223>  Antisense nucleotide

<400>  1967
cagcagcaaa catg                                                          14


<210>  1968
<211>  14
<212>  DNA
<213>  Artificial

<220>
<223>  Antisense nucleotide

<400>  1968
agcagcagca aaca                                                          14


<210>  1969
<211>  15
<212>  DNA
<213>  Artificial

<220>
<223>  Antisense nucleotide

<400>  1969
cagcagcagc aaaca                                                        15


<210>  1970
<211>  14
<212>  DNA
<213>  Artificial

<220>
<223>  Antisense nucleotide

<400>  1970
cagcagcagc aaac                                                         14


<210>  1971
<211>  14
<212>  DNA
<213>  Artificial

<220>
<223>  Antisense nucleotide

<400>  1971
caccagcagc agca                                                         14


<210>  1972
<211>  14
<212>  DNA
<213>  Artificial

<220>
<223>  Antisense nucleotide

<400>  1972
gcattgacgt cagc                                                         14


<210>  1973
<211>  15
<212>  DNA
<213>  Artificial

<220>
<223>  Antisense nucleotide

<400>  1973
gatgttgtcg tgctc                                                        15


<210>  1974
<211>  17
<212>  DNA
<213>  Artificial

<220>
<223>  Antisense nucleotide

<400>  1974
tgagatgttg tcgtgct                                                      17


<210>  1975
<211>  15
<212>  DNA
<213>  Artificial

<220>
<223>  Antisense nucleotide

<400>  1975
tgagatgttg tcgtg                                                        15


<210>  1976
<211>  15
<212>  DNA
<213>  Artificial

<220>
<223>  Antisense nucleotide

<400>  1976
gccaatgaga tgttg                                                        15


<210>  1977
<211>  14
<212>  DNA
<213>  Artificial

<220>
<223>  Antisense nucleotide

<400>  1977
ctgccaatga gatg                                                         14


<210>  1978
<211>  14
<212>  DNA
<213>  Artificial

<220>
<223>  Antisense nucleotide

<400>  1978
cacatgggca tcac                                                         14


<210>  1979
<211>  14
<212>  DNA
<213>  Artificial

<220>
<223>  Antisense nucleotide

<400>  1979
tgtccacatg ggca                                                         14


<210>  1980
<211>  14
<212>  DNA
<213>  Artificial

<220>
<223>  Antisense nucleotide

<400>  1980
gtactgtcca catg                                                         14

<210>  1981
<211>  14
<212>  DNA
<213>  Artificial

<220>
<223>  Antisense nucleotide

<400>  1981
cagctgctat atgc                                                          14


<210>  1982
<211>  14
<212>  DNA
<213>  Artificial

<220>
<223>  Antisense nucleotide

<400>  1982
gttctccacc aggg                                                          14


<210>  1983
<211>  14
<212>  DNA
<213>  Artificial

<220>
<223>  Antisense nucleotide

<400>  1983
agttctccac cagg                                                          14


<210>  1984
<211>  16
<212>  DNA
<213>  Artificial

<220>
<223>  Antisense nucleotide

<400>  1984
caaagttctc caccag                                                        16


<210>  1985
<211>  16
<212>  DNA
<213>  Artificial

<220>
<223>  Antisense nucleotide

<400>  1985
ccaagagtca tccagg                                                        16


<210>  1986
<211>  14
<212>  DNA
<213>  Artificial

<220>
<223>  Antisense nucleotide

<400>  1986
cccaagagtc atcc                                                          14


<210>  1987
<211>  16
<212>  DNA
<213>  Artificial

<220>
<223>  Antisense nucleotide

<400>  1987
cctgcatttt cccaag                                                        16


<210>  1988
<211>  14
<212>  DNA
<213>  Artificial

<220>
<223>  Antisense nucleotide

<400>  1988
tcctgcattt tccc                                                          14


<210>  1989
<211>  15
<212>  DNA
<213>  Artificial

<220>
<223>  Antisense nucleotide

<400>  1989
gccatatcta gaggc                                                         15


<210>  1990
<211>  14
<212>  DNA
<213>  Artificial

<220>
<223>  Antisense nucleotide

<400>  1990
tcacatcttc agcc                                                          14


<210>  1991
<211>  16
<212>  DNA
<213>  Artificial

<220>
<223>  Antisense nucleotide

<400>  1991
gcttcacatc ttcagc                                                        16


<210>  1992
<211>  15
<212>  DNA
<213>  Artificial

<220>
<223>  Antisense nucleotide

<400>  1992
cagcttcaca tcttc                                                        15


<210>  1993
<211>  17
<212>  DNA
<213>  Artificial

<220>
<223>  Antisense nucleotide

<400>  1993
gtaacttata cagctgc                                                      17


<210>  1994
<211>  15
<212>  DNA
<213>  Artificial

<220>
<223>  Antisense nucleotide

<400>  1994
ccagtttttg tctgg                                                        15


<210>  1995
<211>  14
<212>  DNA
<213>  Artificial

<220>
<223>  Antisense nucleotide

<400>  1995
ccatttgtct cagg                                                         14


<210>  1996
<211>  14
<212>  DNA
<213>  Artificial

<220>
<223>  Antisense nucleotide

<400>  1996
gtgtagccca tttg                                                         14


<210>  1997
<211>  14
<212>  DNA
<213>  Artificial

<220>
<223>  Antisense nucleotide

<400>  1997
gcttcggtgt agcc                                                         14

<210>  1998
<211>  18
<212>  DNA
<213>  Artificial

<220>
<223>  Antisense nucleotide

<400>  1998
gatcacttca attgcttc                                                    18


<210>  1999
<211>  14
<212>  DNA
<213>  Artificial

<220>
<223>  Antisense nucleotide

<400>  1999
cttgtggagg cagg                                                        14


<210>  2000
<211>  14
<212>  DNA
<213>  Artificial

<220>
<223>  Antisense nucleotide

<400>  2000
gctgccttgt ggag                                                        14


<210>  2001
<211>  18
<212>  DNA
<213>  Artificial

<220>
<223>  Antisense nucleotide

<400>  2001
ctatttgctg ccttgtgg                                                    18


<210>  2002
<211>  14
<212>  DNA
<213>  Artificial

<220>
<223>  Antisense nucleotide

<400>  2002
ggatgtctcc acgc                                                        14


<210>  2003
<211>  14
<212>  DNA
<213>  Artificial

<220>
<223>  Antisense nucleotide

<400>  2003
ggaaggatgt ctcc                                                          14


<210>  2004
<211>  14
<212>  DNA
<213>  Artificial

<220>
<223>  Antisense nucleotide

<400>  2004
tgcggaagga tgtc                                                          14


<210>  2005
<211>  17
<212>  DNA
<213>  Artificial

<220>
<223>  Antisense nucleotide

<400>  2005
gtttgcggaa ggatgtc                                                       17


<210>  2006
<211>  14
<212>  DNA
<213>  Artificial

<220>
<223>  Antisense nucleotide

<400>  2006
gctgagtttg cgga                                                          14


<210>  2007
<211>  16
<212>  DNA
<213>  Artificial

<220>
<223>  Antisense nucleotide

<400>  2007
ggtaaagctg agtttg                                                        16


<210>  2008
<211>  14
<212>  DNA
<213>  Artificial

<220>
<223>  Antisense nucleotide

<400>  2008
tcggtaaagc tgag                                                          14


<210>  2009
<211>  15
<212>  DNA
<213>  Artificial

<220>
<223>  Antisense nucleotide

<400>  2009
gactcggtaa agctg                                                        15


<210>  2010
<211>  16
<212>  DNA
<213>  Artificial

<220>
<223>  Antisense nucleotide

<400>  2010
agagactcgg taaagc                                                       16


<210>  2011
<211>  16
<212>  DNA
<213>  Artificial

<220>
<223>  Antisense nucleotide

<400>  2011
gaaattgtca gcaggc                                                       16


<210>  2012
<211>  15
<212>  DNA
<213>  Artificial

<220>
<223>  Antisense nucleotide

<400>  2012
gaaattgtca gcagg                                                        15


<210>  2013
<211>  16
<212>  DNA
<213>  Artificial

<220>
<223>  Antisense nucleotide

<400>  2013
ggaaattgtc agcagg                                                       16


<210>  2014
<211>  15
<212>  DNA
<213>  Artificial

<220>
<223>  Antisense nucleotide

<400>  2014
ggaaattgtc agcag                                                        15

<210>  2015
<211>  14
<212>  DNA
<213>  Artificial

<220>
<223>  Antisense nucleotide

<400>  2015
gggaaattgt cagc                                                          14


<210>  2016
<211>  15
<212>  DNA
<213>  Artificial

<220>
<223>  Antisense nucleotide

<400>  2016
gtgtgggaaa ttgtc                                                         15


<210>  2017
<211>  15
<212>  DNA
<213>  Artificial

<220>
<223>  Antisense nucleotide

<400>  2017
ggtttacacg gtgtg                                                         15


<210>  2018
<211>  15
<212>  DNA
<213>  Artificial

<220>
<223>  Antisense nucleotide

<400>  2018
gctttggttt acacg                                                         15


<210>  2019
<211>  15
<212>  DNA
<213>  Artificial

<220>
<223>  Antisense nucleotide

<400>  2019
gcacctttgg gatgc                                                         15


<210>  2020
<211>  15
<212>  DNA
<213>  Artificial

<220>
<223>  Antisense nucleotide

<400>  2020
ccaggttctg cttcc                                                        15


<210>  2021
<211>  15
<212>  DNA
<213>  Artificial

<220>
<223>  Antisense nucleotide

<400>  2021
gctctgtcta gtggc                                                        15


<210>  2022
<211>  14
<212>  DNA
<213>  Artificial

<220>
<223>  Antisense nucleotide

<400>  2022
actctccatg tctc                                                         14


<210>  2023
<211>  16
<212>  DNA
<213>  Artificial

<220>
<223>  Antisense nucleotide

<400>  2023
caactctcca tgtctc                                                       16


<210>  2024
<211>  14
<212>  DNA
<213>  Artificial

<220>
<223>  Antisense nucleotide

<400>  2024
caactctcca tgtc                                                         14


<210>  2025
<211>  14
<212>  DNA
<213>  Artificial

<220>
<223>  Antisense nucleotide

<400>  2025
agcaactctc catg                                                         14


<210>  2026
<211>  16
<212>  DNA
<213>  Artificial

<220>
<223>  Antisense nucleotide

<400>  2026
gtagcaactc tccatg                                                    16


<210>  2027
<211>  14
<212>  DNA
<213>  Artificial

<220>
<223>  Antisense nucleotide

<400>  2027
gtagcaactc tcca                                                      14


<210>  2028
<211>  17
<212>  DNA
<213>  Artificial

<220>
<223>  Antisense nucleotide

<400>  2028
ggttgtagca actctcc                                                   17


<210>  2029
<211>  14
<212>  DNA
<213>  Artificial

<220>
<223>  Antisense nucleotide

<400>  2029
cgggcagtcc tcca                                                      14


<210>  2030
<211>  13
<212>  DNA
<213>  Artificial

<220>
<223>  Antisense nucleotide

<400>  2030
gcaccgggca gtc                                                       13


<210>  2031
<211>  13
<212>  DNA
<213>  Artificial

<220>
<223>  Antisense nucleotide

<400>  2031
aggcaccggg cag                                                       13

<210>  2032
<211>  15
<212>  DNA
<213>  Artificial

<220>
<223>  Antisense nucleotide

<400>  2032
gtgtgttacc aggtc                                                        15


<210>  2033
<211>  15
<212>  DNA
<213>  Artificial

<220>
<223>  Antisense nucleotide

<400>  2033
tgtgtgttac caggt                                                        15


<210>  2034
<211>  14
<212>  DNA
<213>  Artificial

<220>
<223>  Antisense nucleotide

<400>  2034
tgggtcactg tgtg                                                         14


<210>  2035
<211>  15
<212>  DNA
<213>  Artificial

<220>
<223>  Antisense nucleotide

<400>  2035
cagactgtgg gcatg                                                        15


<210>  2036
<211>  16
<212>  DNA
<213>  Artificial

<220>
<223>  Antisense nucleotide

<400>  2036
cccaccagac tgtggg                                                       16


<210>  2037
<211>  14
<212>  DNA
<213>  Artificial

<220>
<223>  Antisense nucleotide

<400>  2037
ccaccagact gtgg                                                         14


<210>  2038
<211>  14
<212>  DNA
<213>  Artificial

<220>
<223>  Antisense nucleotide

<400>  2038
tgcccaccag actg                                                         14


<210>  2039
<211>  13
<212>  DNA
<213>  Artificial

<220>
<223>  Antisense nucleotide

<400>  2039
cggcttcctc ccc                                                          13


<210>  2040
<211>  14
<212>  DNA
<213>  Artificial

<220>
<223>  Antisense nucleotide

<400>  2040
ccttgtcttc cacc                                                         14


<210>  2041
<211>  14
<212>  DNA
<213>  Artificial

<220>
<223>  Antisense nucleotide

<400>  2041
accgaggctg ccac                                                         14


<210>  2042
<211>  14
<212>  DNA
<213>  Artificial

<220>
<223>  Antisense nucleotide

<400>  2042
ggaagaaacc gagg                                                         14


<210>  2043
<211>  14
<212>  DNA
<213>  Artificial

<220>
<223>  Antisense nucleotide

<400>  2043
gggaagaaac cgag                                                          14


<210>  2044
<211>  13
<212>  DNA
<213>  Artificial

<220>
<223>  Antisense nucleotide

<400>  2044
ggccatctgc gcc                                                           13


<210>  2045
<211>  13
<212>  DNA
<213>  Artificial

<220>
<223>  Antisense nucleotide

<400>  2045
gcggccatct gcg                                                           13


<210>  2046
<211>  14
<212>  DNA
<213>  Artificial

<220>
<223>  Antisense nucleotide

<400>  2046
gtggcggcca tctg                                                          14


<210>  2047
<211>  14
<212>  DNA
<213>  Artificial

<220>
<223>  Antisense nucleotide

<400>  2047
accgtggcgg ccat                                                          14


<210>  2048
<211>  17
<212>  DNA
<213>  Artificial

<220>
<223>  Antisense nucleotide

<400>  2048
gccgctcaat cttcatc                                                       17

<210>  2049
<211>  17
<212>  DNA
<213>  Artificial

<220>
<223>  Antisense nucleotide

<400>  2049
cttcatcttg tgatagg                                                    17


<210>  2050
<211>  17
<212>  DNA
<213>  Artificial

<220>
<223>  Antisense nucleotide

<400>  2050
gctcaatctt catcttg                                                    17


<210>  2051
<211>  17
<212>  DNA
<213>  Artificial

<220>
<223>  Antisense nucleotide

<400>  2051
cagaaacact gttacag                                                    17


<210>  2052
<211>  17
<212>  DNA
<213>  Artificial

<220>
<223>  Antisense nucleotide

<400>  2052
cagttgcaga aacactg                                                    17


<210>  2053
<211>  17
<212>  DNA
<213>  Artificial

<220>
<223>  Antisense nucleotide

<400>  2053
gtttcagttg cagaaac                                                    17


<210>  2054
<211>  14
<212>  DNA
<213>  Artificial

<220>
<223>  Antisense nucleotide

<400>  2054
cttccaccag aggg                                                         14


<210>  2055
<211>  14
<212>  DNA
<213>  Artificial

<220>
<223>  Antisense nucleotide

<400>  2055
gtcttccacc agag                                                         14


<210>  2056
<211>  17
<212>  DNA
<213>  Artificial

<220>
<223>  Antisense nucleotide

<400>  2056
cttgtcttcc accagag                                                      17


<210>  2057
<211>  14
<212>  DNA
<213>  Artificial

<220>
<223>  Antisense nucleotide

<400>  2057
tccttgtctt ccac                                                         14


<210>  2058
<211>  16
<212>  DNA
<213>  Artificial

<220>
<223>  Antisense nucleotide

<400>  2058
cttccttgtc ttccac                                                       16


<210>  2059
<211>  15
<212>  DNA
<213>  Artificial

<220>
<223>  Antisense nucleotide

<400>  2059
catcttgtga taggg                                                        15


<210>  2060
<211>  15
<212>  DNA
<213>  Artificial

<220>
<223>  Antisense nucleotide

<400>  2060
gctaggtgca gtggt                                                        15


<210>  2061
<211>  14
<212>  DNA
<213>  Artificial

<220>
<223>  Antisense nucleotide

<400>  2061
gatggctagg tgca                                                         14


<210>  2062
<211>  15
<212>  DNA
<213>  Artificial

<220>
<223>  Antisense nucleotide

<400>  2062
gtggatgatg gctag                                                        15


<210>  2063
<211>  14
<212>  DNA
<213>  Artificial

<220>
<223>  Antisense nucleotide

<400>  2063
cccgtggatg atgg                                                         14


<210>  2064
<211>  14
<212>  DNA
<213>  Artificial

<220>
<223>  Antisense nucleotide

<400>  2064
ctgcccgtgg atga                                                         14


<210>  2065
<211>  14
<212>  DNA
<213>  Artificial

<220>
<223>  Antisense nucleotide

<400>  2065
agagcctcca ccca                                                         14

<210>  2066
<211>  15
<212>  DNA
<213>  Artificial

<220>
<223>  Antisense nucleotide

<400>  2066
gttgtactct cgagc                                                        15


<210>  2067
<211>  13
<212>  DNA
<213>  Artificial

<220>
<223>  Antisense nucleotide

<400>  2067
cgttgtactc tcg                                                          13


<210>  2068
<211>  14
<212>  DNA
<213>  Artificial

<220>
<223>  Antisense nucleotide

<400>  2068
cgcgttgtac tctc                                                         14


<210>  2069
<211>  14
<212>  DNA
<213>  Artificial

<220>
<223>  Antisense nucleotide

<400>  2069
gagtctccat gccg                                                         14


<210>  2070
<211>  14
<212>  DNA
<213>  Artificial

<220>
<223>  Antisense nucleotide

<400>  2070
ctgagtctcc atgc                                                         14


<210>  2071
<211>  14
<212>  DNA
<213>  Artificial

<220>
<223>  Antisense nucleotide

<400>  2071
catggctgag tctc                                                         14


<210>  2072
<211>  14
<212>  DNA
<213>  Artificial

<220>
<223>  Antisense nucleotide

<400>  2072
tgcatggctg agtc                                                         14


<210>  2073
<211>  14
<212>  DNA
<213>  Artificial

<220>
<223>  Antisense nucleotide

<400>  2073
gcgttcacgt tggc                                                         14


<210>  2074
<211>  14
<212>  DNA
<213>  Artificial

<220>
<223>  Antisense nucleotide

<400>  2074
gtgcgagcgt tcac                                                         14


<210>  2075
<211>  14
<212>  DNA
<213>  Artificial

<220>
<223>  Antisense nucleotide

<400>  2075
aggtgcgagc gttc                                                         14


<210>  2076
<211>  14
<212>  DNA
<213>  Artificial

<220>
<223>  Antisense nucleotide

<400>  2076
gcaaaggtgc gagc                                                         14


<210>  2077
<211>  14
<212>  DNA
<213>  Artificial

<220>
<223>  Antisense nucleotide

<400>  2077
cctggtggct cagg                                                          14


<210>  2078
<211>  14
<212>  DNA
<213>  Artificial

<220>
<223>  Antisense nucleotide

<400>  2078
gtcagtcacc tgag                                                          14


<210>  2079
<211>  15
<212>  DNA
<213>  Artificial

<220>
<223>  Antisense nucleotide

<400>  2079
caggtcagtc acctg                                                         15


<210>  2080
<211>  15
<212>  DNA
<213>  Artificial

<220>
<223>  Antisense nucleotide

<400>  2080
cagcaggtca gtcac                                                         15


<210>  2081
<211>  14
<212>  DNA
<213>  Artificial

<220>
<223>  Antisense nucleotide

<400>  2081
gcagcaggtc agtc                                                          14


<210>  2082
<211>  17
<212>  DNA
<213>  Artificial

<220>
<223>  Antisense nucleotide

<400>  2082
catttagcag caaggtc                                                       17

<210>  2083
<211>  15
<212>  DNA
<213>  Artificial

<220>
<223>  Antisense nucleotide

<400>  2083
gcagcattta gcagc                                                        15


<210>  2084
<211>  15
<212>  DNA
<213>  Artificial

<220>
<223>  Antisense nucleotide

<400>  2084
ctgagcagca tttag                                                        15


<210>  2085
<211>  14
<212>  DNA
<213>  Artificial

<220>
<223>  Antisense nucleotide

<400>  2085
cccatgagaa tcct                                                         14


<210>  2086
<211>  17
<212>  DNA
<213>  Artificial

<220>
<223>  Antisense nucleotide

<400>  2086
ccttcccatg agaatcc                                                      17


<210>  2087
<211>  14
<212>  DNA
<213>  Artificial

<220>
<223>  Antisense nucleotide

<400>  2087
tcctcccctt ccca                                                         14


<210>  2088
<211>  14
<212>  DNA
<213>  Artificial

<220>
<223>  Antisense nucleotide

<400>  2088
gcctccagta gacc                                                         14


<210>  2089
<211>  14
<212>  DNA
<213>  Artificial

<220>
<223>  Antisense nucleotide

<400>  2089
gtcagacagg gcct                                                         14


<210>  2090
<211>  14
<212>  DNA
<213>  Artificial

<220>
<223>  Antisense nucleotide

<400>  2090
ccatgtcaga cagg                                                         14


<210>  2091
<211>  14
<212>  DNA
<213>  Artificial

<220>
<223>  Antisense nucleotide

<400>  2091
ggcccatgtc agac                                                         14


<210>  2092
<211>  17
<212>  DNA
<213>  Artificial

<220>
<223>  Antisense nucleotide

<400>  2092
gctattcctg aaatcac                                                      17


<210>  2093
<211>  17
<212>  DNA
<213>  Artificial

<220>
<223>  Antisense nucleotide

<400>  2093
cctcttgtct tcttacc                                                      17


<210>  2094
<211>  17
<212>  DNA
<213>  Artificial

<220>
<223>  Antisense nucleotide

<400>  2094
ggagaagaaa cctcttg                                                    17


<210>  2095
<211>  17
<212>  DNA
<213>  Artificial

<220>
<223>  Antisense nucleotide

<400>  2095
ccttgctgaa gtttctt                                                    17


<210>  2096
<211>  14
<212>  DNA
<213>  Artificial

<220>
<223>  Antisense nucleotide

<400>  2096
ccaagactcc ttgc                                                       14


<210>  2097
<211>  14
<212>  DNA
<213>  Artificial

<220>
<223>  Antisense nucleotide

<400>  2097
ccctttcatg gagc                                                       14


<210>  2098
<211>  14
<212>  DNA
<213>  Artificial

<220>
<223>  Antisense nucleotide

<400>  2098
cctcttggtg tgac                                                       14


<210>  2099
<211>  14
<212>  DNA
<213>  Artificial

<220>
<223>  Antisense nucleotide

<400>  2099
gactaaggat gccg                                                       14

<210>  2100
<211>  15
<212>  DNA
<213>  Artificial

<220>
<223>  Antisense nucleotide

<400>  2100
gtggcaggac taagg                                                          15


<210>  2101
<211>  14
<212>  DNA
<213>  Artificial

<220>
<223>  Antisense nucleotide

<400>  2101
agacgtggca ggac                                                           14


<210>  2102
<211>  14
<212>  DNA
<213>  Artificial

<220>
<223>  Antisense nucleotide

<400>  2102
cttccagcag gcag                                                           14


<210>  2103
<211>  14
<212>  DNA
<213>  Artificial

<220>
<223>  Antisense nucleotide

<400>  2103
gttcctctgc ctgg                                                           14


<210>  2104
<211>  16
<212>  DNA
<213>  Artificial

<220>
<223>  Antisense nucleotide

<400>  2104
gatgttcctc tgcctg                                                         16


<210>  2105
<211>  16
<212>  DNA
<213>  Artificial

<220>
<223>  Antisense nucleotide

<400>  2105
gagatgttcc tctgcc                                                        16


<210>  2106
<211>  16
<212>  DNA
<213>  Artificial

<220>
<223>  Antisense nucleotide

<400>  2106
gtgagatgtt cctctg                                                        16


<210>  2107
<211>  18
<212>  DNA
<213>  Artificial

<220>
<223>  Antisense nucleotide

<400>  2107
cagagagtga gatgttcc                                                      18


<210>  2108
<211>  18
<212>  DNA
<213>  Artificial

<220>
<223>  Antisense nucleotide

<400>  2108
ccagagagtg agatgttc                                                      18


<210>  2109
<211>  15
<212>  DNA
<213>  Artificial

<220>
<223>  Antisense nucleotide

<400>  2109
ggtccagaga gtgag                                                         15


<210>  2110
<211>  15
<212>  DNA
<213>  Artificial

<220>
<223>  Antisense nucleotide

<400>  2110
gaggtccaga gagtg                                                         15


<210>  2111
<211>  14
<212>  DNA
<213>  Artificial

<220>
<223>  Antisense nucleotide

<400>  2111
ggtcctgtag tgcc                                                          14


<210>  2112
<211>  17
<212>  DNA
<213>  Artificial

<220>
<223>  Antisense nucleotide

<400>  2112
gattttatga tgcaggc                                                       17


<210>  2113
<211>  18
<212>  DNA
<213>  Artificial

<220>
<223>  Antisense nucleotide

<400>  2113
gacctgcatc ccttattg                                                      18


<210>  2114
<211>  18
<212>  DNA
<213>  Artificial

<220>
<223>  Antisense nucleotide

<400>  2114
tagttgattt tccagcag                                                      18


<210>  2115
<211>  16
<212>  DNA
<213>  Artificial

<220>
<223>  Antisense nucleotide

<400>  2115
gaatctcacg ttttgc                                                        16


<210>  2116
<211>  18
<212>  DNA
<213>  Artificial

<220>
<223>  Antisense nucleotide

<400>  2116
cagagaaaga atctcacg                                                      18

<210>  2117
<211>  18
<212>  DNA
<213>  Artificial

<220>
<223>  Antisense nucleotide

<400>  2117
tttcaccatc agagaaag                                                    18


<210>  2118
<211>  17
<212>  DNA
<213>  Artificial

<220>
<223>  Antisense nucleotide

<400>  2118
catttggaca tttcacc                                                     17


<210>  2119
<211>  18
<212>  DNA
<213>  Artificial

<220>
<223>  Antisense nucleotide

<400>  2119
ccttcatttg gacatttc                                                    18


<210>  2120
<211>  18
<212>  DNA
<213>  Artificial

<220>
<223>  Antisense nucleotide

<400>  2120
caatgtgctt gatgatcc                                                    18


<210>  2121
<211>  15
<212>  DNA
<213>  Artificial

<220>
<223>  Antisense nucleotide

<400>  2121
cgcatcggat ttctc                                                       15


<210>  2122
<211>  18
<212>  DNA
<213>  Artificial

<220>
<223>  Antisense nucleotide

<400>  2122
caaaccgcat cggatttc                                                    18


<210>  2123
<211>  14
<212>  DNA
<213>  Artificial

<220>
<223>  Antisense nucleotide

<400>  2123
gaactgcaaa ccgc                                                        14


<210>  2124
<211>  15
<212>  DNA
<213>  Artificial

<220>
<223>  Antisense nucleotide

<400>  2124
gcagagaaga actgc                                                       15


<210>  2125
<211>  15
<212>  DNA
<213>  Artificial

<220>
<223>  Antisense nucleotide

<400>  2125
gcaagtaaac atggg                                                       15


<210>  2126
<211>  14
<212>  DNA
<213>  Artificial

<220>
<223>  Antisense nucleotide

<400>  2126
ggtccacgtt ttgg                                                        14


<210>  2127
<211>  16
<212>  DNA
<213>  Artificial

<220>
<223>  Antisense nucleotide

<400>  2127
gcaagggtcc acgttt                                                      16


<210>  2128
<211>  17
<212>  DNA
<213>  Artificial

<220>
<223>  Antisense nucleotide

<400>  2128
tggcttcttc ttcaggg                                                    17


<210>  2129
<211>  16
<212>  DNA
<213>  Artificial

<220>
<223>  Antisense nucleotide

<400>  2129
tcctgctggc ttcttc                                                     16


<210>  2130
<211>  14
<212>  DNA
<213>  Artificial

<220>
<223>  Antisense nucleotide

<400>  2130
gtcctgctgg cttc                                                       14


<210>  2131
<211>  17
<212>  DNA
<213>  Artificial

<220>
<223>  Antisense nucleotide

<400>  2131
ggtagtctag gaattgg                                                    17


<210>  2132
<211>  17
<212>  DNA
<213>  Artificial

<220>
<223>  Antisense nucleotide

<400>  2132
cttgcaggta gtctagg                                                    17


<210>  2133
<211>  17
<212>  DNA
<213>  Artificial

<220>
<223>  Antisense nucleotide

<400>  2133
gaaactcttg caggtag                                                    17

<210>  2134
<211>  17
<212>  DNA
<213>  Artificial

<220>
<223>  Antisense nucleotide

<400>  2134
caccaagaaa ctcttgc                                                    17


<210>  2135
<211>  18
<212>  DNA
<213>  Artificial

<220>
<223>  Antisense nucleotide

<400>  2135
cattacacca agaaactc                                                   18


<210>  2136
<211>  18
<212>  DNA
<213>  Artificial

<220>
<223>  Antisense nucleotide

<400>  2136
ctcggtgttc attacacc                                                   18


<210>  2137
<211>  18
<212>  DNA
<213>  Artificial

<220>
<223>  Antisense nucleotide

<400>  2137
ctttctatta tccactcg                                                   18


<210>  2138
<211>  18
<212>  DNA
<213>  Artificial

<220>
<223>  Antisense nucleotide

<400>  2138
ccagtttagt ctcaactt                                                   18


<210>  2139
<211>  18
<212>  DNA
<213>  Artificial

<220>
<223>  Antisense nucleotide

<400>  2139
aaccagttta gtctcaac                                                    18


<210>  2140
<211>  18
<212>  DNA
<213>  Artificial

<220>
<223>  Antisense nucleotide

<400>  2140
acaaaccagt ttagtctc                                                    18


<210>  2141
<211>  18
<212>  DNA
<213>  Artificial

<220>
<223>  Antisense nucleotide

<400>  2141
ctcgcgaaaa agtttctt                                                    18


<210>  2142
<211>  18
<212>  DNA
<213>  Artificial

<220>
<223>  Antisense nucleotide

<400>  2142
ccctcgcgaa aaagtttc                                                    18


<210>  2143
<211>  16
<212>  DNA
<213>  Artificial

<220>
<223>  Antisense nucleotide

<400>  2143
gtccctcgcg aaaaag                                                      16


<210>  2144
<211>  15
<212>  DNA
<213>  Artificial

<220>
<223>  Antisense nucleotide

<400>  2144
cagttgaacc gtccc                                                       15


<210>  2145
<211>  18
<212>  DNA
<213>  Artificial

<220>
<223> Antisense nucleotide

<400> 2145
gctttcgaag tttcagtt 18

<210> 2146
<211> 17
<212> DNA
<213> Artificial

<220>
<223> Antisense nucleotide

<400> 2146
gatgctttcg aagtttc 17

<210> 2147
<211> 18
<212> DNA
<213> Artificial

<220>
<223> Antisense nucleotide

<400> 2147
ctgtctctgc aaataatg 18

<210> 2148
<211> 18
<212> DNA
<213> Artificial

<220>
<223> Antisense nucleotide

<400> 2148
cacttattac attcaccc 18

<210> 2149
<211> 17
<212> DNA
<213> Artificial

<220>
<223> Antisense nucleotide

<400> 2149
ttttcctcca gttcctc 17

<210> 2150
<211> 20
<212> DNA
<213> Artificial

<220>
<223> Antisense nucleotide

<400> 2150
ggacaatatg tacaaaactc 20

<210>  2151
<211>  18
<212>  DNA
<213>  Artificial

<220>
<223>  Antisense nucleotide

<400>  2151
gttgatgaac atttggac                                                    18


<210>  2152
<211>  18
<212>  DNA
<213>  Artificial

<220>
<223>  Antisense nucleotide

<400>  2152
gtgttgatga acatttgg                                                    18


<210>  2153
<211>  16
<212>  DNA
<213>  Artificial

<220>
<223>  Antisense nucleotide

<400>  2153
caaaatttgg ccaggg                                                      16


<210>  2154
<211>  15
<212>  DNA
<213>  Artificial

<220>
<223>  Antisense nucleotide

<400>  2154
gcccaaaatt tggcc                                                       15


<210>  2155
<211>  17
<212>  DNA
<213>  Artificial

<220>
<223>  Antisense nucleotide

<400>  2155
cccagcccaa aatttgg                                                     17


<210>  2156
<211>  17
<212>  DNA
<213>  Artificial

<220>
<223>  Antisense nucleotide

<400>  2156
gtccccagcc caaaatt                                                    17


<210>  2157
<211>  16
<212>  DNA
<213>  Artificial

<220>
<223>  Antisense nucleotide

<400>  2157
aaatcgccag aggctg                                                     16


<210>  2158
<211>  16
<212>  DNA
<213>  Artificial

<220>
<223>  Antisense nucleotide

<400>  2158
accaaatcgc cagagg                                                     16


<210>  2159
<211>  17
<212>  DNA
<213>  Artificial

<220>
<223>  Antisense nucleotide

<400>  2159
catcaccaaa tcgccag                                                    17


<210>  2160
<211>  16
<212>  DNA
<213>  Artificial

<220>
<223>  Antisense nucleotide

<400>  2160
taggagtggt tgaggc                                                     16


<210>  2161
<211>  17
<212>  DNA
<213>  Artificial

<220>
<223>  Antisense nucleotide

<400>  2161
gtgtaggagt ggttgag                                                    17


<210>  2162
<211>  14
<212>  DNA
<213>  Artificial

<220>
<223>  Antisense nucleotide

<400>  2162
ctgtgtagga gtgg                                                         14


<210>  2163
<211>  14
<212>  DNA
<213>  Artificial

<220>
<223>  Antisense nucleotide

<400>  2163
cccacatgcc tgtg                                                         14


<210>  2164
<211>  14
<212>  DNA
<213>  Artificial

<220>
<223>  Antisense nucleotide

<400>  2164
cgatgaacaa cgag                                                         14


<210>  2165
<211>  16
<212>  DNA
<213>  Artificial

<220>
<223>  Antisense nucleotide

<400>  2165
ctggcgatga acaacg                                                       16


<210>  2166
<211>  14
<212>  DNA
<213>  Artificial

<220>
<223>  Antisense nucleotide

<400>  2166
cgctggcgat gaac                                                         14


<210>  2167
<211>  15
<212>  DNA
<213>  Artificial

<220>
<223>  Antisense nucleotide

<400>  2167
gagctagtcc cgttg                                                        15

<210>  2168
<211>  15
<212>  DNA
<213>  Artificial

<220>
<223>  Antisense nucleotide

<400>  2168
gcgaagagct agtcc                                                        15


<210>  2169
<211>  17
<212>  DNA
<213>  Artificial

<220>
<223>  Antisense nucleotide

<400>  2169
ccagttatgc gaagagc                                                      17


<210>  2170
<211>  16
<212>  DNA
<213>  Artificial

<220>
<223>  Antisense nucleotide

<400>  2170
ccccagttat gcgaag                                                       16


<210>  2171
<211>  14
<212>  DNA
<213>  Artificial

<220>
<223>  Antisense nucleotide

<400>  2171
cacatgcttg gcgc                                                         14


<210>  2172
<211>  16
<212>  DNA
<213>  Artificial

<220>
<223>  Antisense nucleotide

<400>  2172
gatcacatgc ttggcg                                                       16


<210>  2173
<211>  18
<212>  DNA
<213>  Artificial

<220>
<223>  Antisense nucleotide

<400>  2173
gacaaagagc atgatcac                                                    18


<210>  2174
<211>  17
<212>  DNA
<213>  Artificial

<220>
<223>  Antisense nucleotide

<400>  2174
gagtcacagg gacaaag                                                     17


<210>  2175
<211>  15
<212>  DNA
<213>  Artificial

<220>
<223>  Antisense nucleotide

<400>  2175
gagagtcaca gggac                                                       15


<210>  2176
<211>  15
<212>  DNA
<213>  Artificial

<220>
<223>  Antisense nucleotide

<400>  2176
gcagagagtc acagg                                                       15


<210>  2177
<211>  14
<212>  DNA
<213>  Artificial

<220>
<223>  Antisense nucleotide

<400>  2177
ccatgcagag agtc                                                        14


<210>  2178
<211>  15
<212>  DNA
<213>  Artificial

<220>
<223>  Antisense nucleotide

<400>  2178
ccaccatgca gagag                                                       15


<210>  2179
<211>  14
<212>  DNA
<213>  Artificial

<220>
<223>  Antisense nucleotide

<400>  2179
tagccacgac cacc                                                          14


<210>  2180
<211>  18
<212>  DNA
<213>  Artificial

<220>
<223>  Antisense nucleotide

<400>  2180
gattagctgc ccatcctt                                                      18


<210>  2181
<211>  17
<212>  DNA
<213>  Artificial

<220>
<223>  Antisense nucleotide

<400>  2181
ggtatagatt agctgcc                                                       17


<210>  2182
<211>  18
<212>  DNA
<213>  Artificial

<220>
<223>  Antisense nucleotide

<400>  2182
gtatcttctg tgaatggg                                                      18


<210>  2183
<211>  14
<212>  DNA
<213>  Artificial

<220>
<223>  Antisense nucleotide

<400>  2183
ctggcccaca gtct                                                          14


<210>  2184
<211>  14
<212>  DNA
<213>  Artificial

<220>
<223>  Antisense nucleotide

<400>  2184
ctctggccca cagt                                                          14

<210> 2185
<211> 14
<212> DNA
<213> Artificial

<220>
<223> Antisense nucleotide

<400> 2185
tgcagggctc tctg 14


<210> 2186
<211> 14
<212> DNA
<213> Artificial

<220>
<223> Antisense nucleotide

<400> 2186
agtgcagggc tctc 14


<210> 2187
<211> 16
<212> DNA
<213> Artificial

<220>
<223> Antisense nucleotide

<400> 2187
cactgatcat gatggc 16


<210> 2188
<211> 18
<212> DNA
<213> Artificial

<220>
<223> Antisense nucleotide

<400> 2188
gacactgatc atgatggc 18


<210> 2189
<211> 18
<212> DNA
<213> Artificial

<220>
<223> Antisense nucleotide

<400> 2189
acaatgacac tgatcatg 18


<210> 2190
<211> 16
<212> DNA
<213> Artificial

<220>
<223> Antisense nucleotide

<400>  2190
gaaccaccag gaggat                                                    16


<210>  2191
<211>  17
<212>  DNA
<213>  Artificial

<220>
<223>  Antisense nucleotide

<400>  2191
gacacaaaac agccact                                                   17


<210>  2192
<211>  15
<212>  DNA
<213>  Artificial

<220>
<223>  Antisense nucleotide

<400>  2192
gtggaccttt cggac                                                     15


<210>  2193
<211>  17
<212>  DNA
<213>  Artificial

<220>
<223>  Antisense nucleotide

<400>  2193
caaccagcat acgaagt                                                   17


<210>  2194
<211>  14
<212>  DNA
<213>  Artificial

<220>
<223>  Antisense nucleotide

<400>  2194
tccctctggg cttc                                                      14


<210>  2195
<211>  14
<212>  DNA
<213>  Artificial

<220>
<223>  Antisense nucleotide

<400>  2195
actgtccctc tggg                                                      14


<210>  2196
<211>  14
<212>  DNA
<213>  Artificial

<220>
<223>  Antisense nucleotide

<400>  2196
gactgtccct ctgg                                                          14


<210>  2197
<211>  16
<212>  DNA
<213>  Artificial

<220>
<223>  Antisense nucleotide

<400>  2197
cctagatgac tgtccc                                                        16


<210>  2198
<211>  15
<212>  DNA
<213>  Artificial

<220>
<223>  Antisense nucleotide

<400>  2198
cagcgaggat actgc                                                         15


<210>  2199
<211>  16
<212>  DNA
<213>  Artificial

<220>
<223>  Antisense nucleotide

<400>  2199
cttcaccagc gaggat                                                        16


<210>  2200
<211>  18
<212>  DNA
<213>  Artificial

<220>
<223>  Antisense nucleotide

<400>  2200
tttcctctgg gtcttcac                                                      18


<210>  2201
<211>  17
<212>  DNA
<213>  Artificial

<220>
<223>  Antisense nucleotide

<400>  2201
ctttcctctg ggtcttc                                                       17

<210>  2202
<211>  17
<212>  DNA
<213>  Artificial

<220>
<223>  Antisense nucleotide

<400>  2202
ctcccaatcc aagtttt                                                    17


<210>  2203
<211>  14
<212>  DNA
<213>  Artificial

<220>
<223>  Antisense nucleotide

<400>  2203
ttcatcccgg agcc                                                       14


<210>  2204
<211>  16
<212>  DNA
<213>  Artificial

<220>
<223>  Antisense nucleotide

<400>  2204
ttcttcatcc cggagc                                                     16


<210>  2205
<211>  16
<212>  DNA
<213>  Artificial

<220>
<223>  Antisense nucleotide

<400>  2205
gctcagccag ttcttc                                                     16


<210>  2206
<211>  14
<212>  DNA
<213>  Artificial

<220>
<223>  Antisense nucleotide

<400>  2206
gacagagagg gcac                                                       14


<210>  2207
<211>  15
<212>  DNA
<213>  Artificial

<220>
<223>  Antisense nucleotide

<400>  2207
cttcacctcc gacag                                                        15


<210>  2208
<211>  16
<212>  DNA
<213>  Artificial

<220>
<223>  Antisense nucleotide

<400>  2208
gaaaagtctg ggcagg                                                       16


<210>  2209
<211>  18
<212>  DNA
<213>  Artificial

<220>
<223>  Antisense nucleotide

<400>  2209
gaccctggaa cagaaaag                                                     18


<210>  2210
<211>  15
<212>  DNA
<213>  Artificial

<220>
<223>  Antisense nucleotide

<400>  2210
ctgaccctgg aacag                                                        15


<210>  2211
<211>  16
<212>  DNA
<213>  Artificial

<220>
<223>  Antisense nucleotide

<400>  2211
actacaggct gaccct                                                       16


<210>  2212
<211>  18
<212>  DNA
<213>  Artificial

<220>
<223>  Antisense nucleotide

<400>  2212
attcactaca ggctgacc                                                     18


<210>  2213
<211>  14
<212>  DNA
<213>  Artificial

<220>
<223>  Antisense nucleotide

<400>  2213
cgattcacta cagg                                                          14


<210>  2214
<211>  14
<212>  DNA
<213>  Artificial

<220>
<223>  Antisense nucleotide

<400>  2214
ggccgattca ctac                                                          14


<210>  2215
<211>  16
<212>  DNA
<213>  Artificial

<220>
<223>  Antisense nucleotide

<400>  2215
cgaacgtctg ttggtc                                                        16


<210>  2216
<211>  15
<212>  DNA
<213>  Artificial

<220>
<223>  Antisense nucleotide

<400>  2216
cgcgaacgtc tgttg                                                         15


<210>  2217
<211>  18
<212>  DNA
<213>  Artificial

<220>
<223>  Antisense nucleotide

<400>  2217
cttctgtttg tcgaggat                                                      18


<210>  2218
<211>  18
<212>  DNA
<213>  Artificial

<220>
<223>  Antisense nucleotide

<400>  2218
ttcaccacct tctgtttg                                                      18

<210>  2219
<211>  18
<212>  DNA
<213>  Artificial

<220>
<223>  Antisense nucleotide

<400>  2219
aggatgcgct tttcattc                                                    18


<210>  2220
<211>  15
<212>  DNA
<213>  Artificial

<220>
<223>  Antisense nucleotide

<400>  2220
agcttgcagg atgcg                                                       15


<210>  2221
<211>  18
<212>  DNA
<213>  Artificial

<220>
<223>  Antisense nucleotide

<400>  2221
gttgacagct tgcaggat                                                    18


<210>  2222
<211>  18
<212>  DNA
<213>  Artificial

<220>
<223>  Antisense nucleotide

<400>  2222
ggaacggaaa gttgacag                                                    18


<210>  2223
<211>  18
<212>  DNA
<213>  Artificial

<220>
<223>  Antisense nucleotide

<400>  2223
aactcgagtt tgacgagg                                                    18


<210>  2224
<211>  17
<212>  DNA
<213>  Artificial

<220>
<223>  Antisense nucleotide

<400>  2224
tgtccttgaa ggagaac                                                    17


<210>  2225
<211>  18
<212>  DNA
<213>  Artificial

<220>
<223>  Antisense nucleotide

<400>  2225
cgtactccat gaccatgt                                                   18


<210>  2226
<211>  16
<212>  DNA
<213>  Artificial

<220>
<223>  Antisense nucleotide

<400>  2226
gcacgtactc catgac                                                     16


<210>  2227
<211>  16
<212>  DNA
<213>  Artificial

<220>
<223>  Antisense nucleotide

<400>  2227
gattctccgg cttcag                                                     16


<210>  2228
<211>  18
<212>  DNA
<213>  Artificial

<220>
<223>  Antisense nucleotide

<400>  2228
tcaatgagca gattctcc                                                   18


<210>  2229
<211>  17
<212>  DNA
<213>  Artificial

<220>
<223>  Antisense nucleotide

<400>  2229
ggtcaatgag cagattc                                                    17


<210>  2230
<211>  15
<212>  DNA
<213>  Artificial

<220>
<223>  Antisense nucleotide

<400>  2230
ccctgctggt caatg                                                        15


<210>  2231
<211>  14
<212>  DNA
<213>  Artificial

<220>
<223>  Antisense nucleotide

<400>  2231
tagccctgct ggtc                                                         14


<210>  2232
<211>  14
<212>  DNA
<213>  Artificial

<220>
<223>  Antisense nucleotide

<400>  2232
cgcttggcga aacc                                                         14


<210>  2233
<211>  14
<212>  DNA
<213>  Artificial

<220>
<223>  Antisense nucleotide

<400>  2233
ccttcacgcg cttg                                                         14


<210>  2234
<211>  14
<212>  DNA
<213>  Artificial

<220>
<223>  Antisense nucleotide

<400>  2234
aaggtccaag tgcg                                                         14


<210>  2235
<211>  14
<212>  DNA
<213>  Artificial

<220>
<223>  Antisense nucleotide

<400>  2235
tgccgcacaa ggtc                                                         14

<210>  2236
<211>  17
<212>  DNA
<213>  Artificial

<220>
<223>  Antisense nucleotide

<400>  2236
ggtgaggacc accattt                                                    17


<210>  2237
<211>  14
<212>  DNA
<213>  Artificial

<220>
<223>  Antisense nucleotide

<400>  2237
gggtgtcaca ggtg                                                       14


<210>  2238
<211>  18
<212>  DNA
<213>  Artificial

<220>
<223>  Antisense nucleotide

<400>  2238
ataccatctt cttcaggg                                                   18


<210>  2239
<211>  18
<212>  DNA
<213>  Artificial

<220>
<223>  Antisense nucleotide

<400>  2239
ggtgatacca tcttcttc                                                   18


<210>  2240
<211>  18
<212>  DNA
<213>  Artificial

<220>
<223>  Antisense nucleotide

<400>  2240
ccaggtgata ccatcttc                                                   18


<210>  2241
<211>  15
<212>  DNA
<213>  Artificial

<220>
<223>  Antisense nucleotide

<400>  2241
cctcactgct ctggt                                                        15


<210>  2242
<211>  14
<212>  DNA
<213>  Artificial

<220>
<223>  Antisense nucleotide

<400>  2242
taagacctca ctgc                                                         14


<210>  2243
<211>  16
<212>  DNA
<213>  Artificial

<220>
<223>  Antisense nucleotide

<400>  2243
cagagcctaa gacctc                                                       16


<210>  2244
<211>  15
<212>  DNA
<213>  Artificial

<220>
<223>  Antisense nucleotide

<400>  2244
ccagagccta agacc                                                        15


<210>  2245
<211>  18
<212>  DNA
<213>  Artificial

<220>
<223>  Antisense nucleotide

<400>  2245
tcttcctttt tgtgaagc                                                     18


<210>  2246
<211>  18
<212>  DNA
<213>  Artificial

<220>
<223>  Antisense nucleotide

<400>  2246
gaccaaattc catcttcc                                                     18


<210>  2247
<211>  18
<212>  DNA
<213>  Artificial

<220>
<223>  Antisense nucleotide

<400>  2247
atcagtggac caaattcc                                                    18


<210>  2248
<211>  18
<212>  DNA
<213>  Artificial

<220>
<223>  Antisense nucleotide

<400>  2248
ggttctttct ggtccttt                                                    18


<210>  2249
<211>  18
<212>  DNA
<213>  Artificial

<220>
<223>  Antisense nucleotide

<400>  2249
tttttgggtt ctttctgg                                                    18


<210>  2250
<211>  18
<212>  DNA
<213>  Artificial

<220>
<223>  Antisense nucleotide

<400>  2250
ggtcttattt ttgggttc                                                    18


<210>  2251
<211>  17
<212>  DNA
<213>  Artificial

<220>
<223>  Antisense nucleotide

<400>  2251
aatgggcaga ctctcct                                                     17


<210>  2252
<211>  18
<212>  DNA
<213>  Artificial

<220>
<223>  Antisense nucleotide

<400>  2252
tccaccatga cctcaatg                                                    18

<210>  2253
<211>  16
<212>  DNA
<213>  Artificial

<220>
<223>  Antisense nucleotide

<400>  2253
aacggcatcc accatg                                                        16


<210>  2254
<211>  15
<212>  DNA
<213>  Artificial

<220>
<223>  Antisense nucleotide

<400>  2254
gtgaacggca tccac                                                         15


<210>  2255
<211>  18
<212>  DNA
<213>  Artificial

<220>
<223>  Antisense nucleotide

<400>  2255
acttgagctt gtgaacgg                                                      18


<210>  2256
<211>  18
<212>  DNA
<213>  Artificial

<220>
<223>  Antisense nucleotide

<400>  2256
ttcatacttg agcttgtg                                                      18


<210>  2257
<211>  18
<212>  DNA
<213>  Artificial

<220>
<223>  Antisense nucleotide

<400>  2257
ctggtgtagt tttcatac                                                      18


<210>  2258
<211>  18
<212>  DNA
<213>  Artificial

<220>
<223>  Antisense nucleotide

<400>  2258
agctgctggt gtagtttt                                                    18


<210>  2259
<211>  14
<212>  DNA
<213>  Artificial

<220>
<223>  Antisense nucleotide

<400>  2259
aggaggacca gggt                                                        14


<210>  2260
<211>  14
<212>  DNA
<213>  Artificial

<220>
<223>  Antisense nucleotide

<400>  2260
aggtggtcca ggag                                                        14


<210>  2261
<211>  18
<212>  DNA
<213>  Artificial

<220>
<223>  Antisense nucleotide

<400>  2261
tttctggcca aactgagg                                                    18


<210>  2262
<211>  14
<212>  DNA
<213>  Artificial

<220>
<223>  Antisense nucleotide

<400>  2262
ggaggtttct ggcc                                                        14


<210>  2263
<211>  14
<212>  DNA
<213>  Artificial

<220>
<223>  Antisense nucleotide

<400>  2263
tctggagtgg ccac                                                        14


<210>  2264
<211>  18
<212>  DNA
<213>  Artificial

<220>
<223>  Antisense nucleotide

<400>  2264
cttctggagc atgttgct                                                    18


<210>  2265
<211>  15
<212>  DNA
<213>  Artificial

<220>
<223>  Antisense nucleotide

<400>  2265
gccttctgga gcatg                                                       15


<210>  2266
<211>  17
<212>  DNA
<213>  Artificial

<220>
<223>  Antisense nucleotide

<400>  2266
gtttgtctgg ccttctg                                                     17


<210>  2267
<211>  18
<212>  DNA
<213>  Artificial

<220>
<223>  Antisense nucleotide

<400>  2267
gagtttgtct ggccttct                                                    18


<210>  2268
<211>  18
<212>  DNA
<213>  Artificial

<220>
<223>  Antisense nucleotide

<400>  2268
ctagagtttg tctggcct                                                    18


<210>  2269
<211>  18
<212>  DNA
<213>  Artificial

<220>
<223>  Antisense nucleotide

<400>  2269
gcaagggtaa aattctag                                                    18

<210>  2270
<211>  18
<212>  DNA
<213>  Artificial

<220>
<223>  Antisense nucleotide

<400>  2270
agtgcaaggg taaaattc                                                    18


<210>  2271
<211>  15
<212>  DNA
<213>  Artificial

<220>
<223>  Antisense nucleotide

<400>  2271
aaacaggcct ccact                                                       15


<210>  2272
<211>  18
<212>  DNA
<213>  Artificial

<220>
<223>  Antisense nucleotide

<400>  2272
cttggttaat tccaatgg                                                    18


<210>  2273
<211>  18
<212>  DNA
<213>  Artificial

<220>
<223>  Antisense nucleotide

<400>  2273
aggcaactcc cattagtt                                                    18


<210>  2274
<211>  18
<212>  DNA
<213>  Artificial

<220>
<223>  Antisense nucleotide

<400>  2274
tactactaag gcacaggg                                                    18


<210>  2275
<211>  18
<212>  DNA
<213>  Artificial

<220>
<223>  Antisense nucleotide

<400>  2275
aatactacta aggcacag                                                     18


<210>  2276
<211>  18
<212>  DNA
<213>  Artificial

<220>
<223>  Antisense nucleotide

<400>  2276
gtacatcttc aagtcttc                                                     18


<210>  2277
<211>  14
<212>  DNA
<213>  Artificial

<220>
<223>  Antisense nucleotide

<400>  2277
ggagtggaca tgat                                                         14


<210>  2278
<211>  18
<212>  DNA
<213>  Artificial

<220>
<223>  Antisense nucleotide

<400>  2278
aagaagatga agcctttg                                                     18


<210>  2279
<211>  18
<212>  DNA
<213>  Artificial

<220>
<223>  Antisense nucleotide

<400>  2279
ccgtcttact cttcttgg                                                     18


<210>  2280
<211>  17
<212>  DNA
<213>  Artificial

<220>
<223>  Antisense nucleotide

<400>  2280
ccgatacaat tccaagg                                                      17


<210>  2281
<211>  15
<212>  DNA
<213>  Artificial

<220>
<223>  Antisense nucleotide

<400>  2281
ccttttcctt ctgag                                                        15


<210>  2282
<211>  14
<212>  DNA
<213>  Artificial

<220>
<223>  Antisense nucleotide

<400>  2282
ctgttgcaag tacg                                                         14


<210>  2283
<211>  14
<212>  DNA
<213>  Artificial

<220>
<223>  Antisense nucleotide

<400>  2283
cagaagcaga gggc                                                         14


<210>  2284
<211>  15
<212>  DNA
<213>  Artificial

<220>
<223>  Antisense nucleotide

<400>  2284
cctcagaagc agagg                                                        15


<210>  2285
<211>  14
<212>  DNA
<213>  Artificial

<220>
<223>  Antisense nucleotide

<400>  2285
ctcctcagaa gcag                                                         14


<210>  2286
<211>  14
<212>  DNA
<213>  Artificial

<220>
<223>  Antisense nucleotide

<400>  2286
acaggctggt ggca                                                         14

<210>  2287
<211>  16
<212>  DNA
<213>  Artificial

<220>
<223>  Antisense nucleotide

<400>  2287
ccactctcaa acaggc                                                    16


<210>  2288
<211>  14
<212>  DNA
<213>  Artificial

<220>
<223>  Antisense nucleotide

<400>  2288
acggtagccg aagc                                                      14


<210>  2289
<211>  15
<212>  DNA
<213>  Artificial

<220>
<223>  Antisense nucleotide

<400>  2289
gacggtagcc gaagc                                                     15


<210>  2290
<211>  14
<212>  DNA
<213>  Artificial

<220>
<223>  Antisense nucleotide

<400>  2290
ggccagacgg tagc                                                      14


<210>  2291
<211>  18
<212>  DNA
<213>  Artificial

<220>
<223>  Antisense nucleotide

<400>  2291
gtgtagggcc agacggta                                                  18


<210>  2292
<211>  18
<212>  DNA
<213>  Artificial

<220>
<223>  Antisense nucleotide

<400>  2292
ccgaagccat ttttcagg                                                    18


<210>  2293
<211>  17
<212>  DNA
<213>  Artificial

<220>
<223>  Antisense nucleotide

<400>  2293
ccccgaagcc atttttc                                                     17


<210>  2294
<211>  14
<212>  DNA
<213>  Artificial

<220>
<223>  Antisense nucleotide

<400>  2294
ggttgatgtc gtcc                                                        14


<210>  2295
<211>  15
<212>  DNA
<213>  Artificial

<220>
<223>  Antisense nucleotide

<400>  2295
gcttgagaca ctcgc                                                       15


<210>  2296
<211>  14
<212>  DNA
<213>  Artificial

<220>
<223>  Antisense nucleotide

<400>  2296
ccggacccgt ccat                                                        14


<210>  2297
<211>  14
<212>  DNA
<213>  Artificial

<220>
<223>  Antisense nucleotide

<400>  2297
gcttgcttta ctgc                                                        14


<210>  2298
<211>  14
<212>  DNA
<213>  Artificial

<220>
<223>  Antisense nucleotide

<400>  2298
ggttgctctg agac                                                          14


<210>  2299
<211>  14
<212>  DNA
<213>  Artificial

<220>
<223>  Antisense nucleotide

<400>  2299
gccacagtca tgcc                                                          14


<210>  2300
<211>  14
<212>  DNA
<213>  Artificial

<220>
<223>  Antisense nucleotide

<400>  2300
cgggcatgct ggcg                                                          14


<210>  2301
<211>  18
<212>  DNA
<213>  Artificial

<220>
<223>  Antisense nucleotide

<400>  2301
gtgaagttca ggatgatc                                                      18


<210>  2302
<211>  14
<212>  DNA
<213>  Artificial

<220>
<223>  Antisense nucleotide

<400>  2302
ccagtgcctc atgg                                                          14


<210>  2303
<211>  15
<212>  DNA
<213>  Artificial

<220>
<223>  Antisense nucleotide

<400>  2303
cagtgttctc catgg                                                         15

<210>  2304
<211>  15
<212>  DNA
<213>  Artificial

<220>
<223>  Antisense nucleotide

<400>  2304
ctgtaccaga ccgag                                                        15


<210>  2305
<211>  15
<212>  DNA
<213>  Artificial

<220>
<223>  Antisense nucleotide

<400>  2305
gcatactgtt tcagc                                                        15


<210>  2306
<211>  15
<212>  DNA
<213>  Artificial

<220>
<223>  Antisense nucleotide

<400>  2306
gccatcagct ccttg                                                        15


<210>  2307
<211>  15
<212>  DNA
<213>  Artificial

<220>
<223>  Antisense nucleotide

<400>  2307
ccacaccata gatgg                                                        15


<210>  2308
<211>  15
<212>  DNA
<213>  Artificial

<220>
<223>  Antisense nucleotide

<400>  2308
gctggagcag tttcc                                                        15


<210>  2309
<211>  14
<212>  DNA
<213>  Artificial

<220>
<223>  Antisense nucleotide

<400>  2309
ctcgcttctg ctgc                                                         14


<210>  2310
<211>  14
<212>  DNA
<213>  Artificial

<220>
<223>  Antisense nucleotide

<400>  2310
accgtggcaa agcg                                                         14


<210>  2311
<211>  14
<212>  DNA
<213>  Artificial

<220>
<223>  Antisense nucleotide

<400>  2311
aggtgacacc gtgg                                                         14


<210>  2312
<211>  16
<212>  DNA
<213>  Artificial

<220>
<223>  Antisense nucleotide

<400>  2312
gacttgattc cttcag                                                       16


<210>  2313
<211>  17
<212>  DNA
<213>  Artificial

<220>
<223>  Antisense nucleotide

<400>  2313
ggatttgact tgattcc                                                      17


<210>  2314
<211>  14
<212>  DNA
<213>  Artificial

<220>
<223>  Antisense nucleotide

<400>  2314
gctgctgttc atgg                                                         14


<210>  2315
<211>  17
<212>  DNA
<213>  Artificial

<220>
<223>  Antisense nucleotide

<400>  2315
ccgtttcttt cagtagg                                                    17


<210>  2316
<211>  14
<212>  DNA
<213>  Artificial

<220>
<223>  Antisense nucleotide

<400>  2316
cttgaagtag gagc                                                       14


<210>  2317
<211>  14
<212>  DNA
<213>  Artificial

<220>
<223>  Antisense nucleotide

<400>  2317
cgctcctaca tggc                                                       14


<210>  2318
<211>  15
<212>  DNA
<213>  Artificial

<220>
<223>  Antisense nucleotide

<400>  2318
gatgaggtac aggcc                                                      15


<210>  2319
<211>  15
<212>  DNA
<213>  Artificial

<220>
<223>  Antisense nucleotide

<400>  2319
gtagatgagg tacag                                                      15


<210>  2320
<211>  15
<212>  DNA
<213>  Artificial

<220>
<223>  Antisense nucleotide

<400>  2320
gagtagatga ggtac                                                      15

<210>  2321
<211>  14
<212>  DNA
<213>  Artificial

<220>
<223>  Antisense nucleotide

<400>  2321
cctgggagta gatg                                                          14


<210>  2322
<211>  14
<212>  DNA
<213>  Artificial

<220>
<223>  Antisense nucleotide

<400>  2322
ggacctggga gtag                                                          14


<210>  2323
<211>  14
<212>  DNA
<213>  Artificial

<220>
<223>  Antisense nucleotide

<400>  2323
acatgggtgg aggg                                                          14


<210>  2324
<211>  14
<212>  DNA
<213>  Artificial

<220>
<223>  Antisense nucleotide

<400>  2324
gtgctcatgg tgtc                                                          14


<210>  2325
<211>  15
<212>  DNA
<213>  Artificial

<220>
<223>  Antisense nucleotide

<400>  2325
ctttcagtgc tcatg                                                         15


<210>  2326
<211>  15
<212>  DNA
<213>  Artificial

<220>
<223>  Antisense nucleotide

<400>  2326
tgctttcagt gctca                                                        15


<210>  2327
<211>  15
<212>  DNA
<213>  Artificial

<220>
<223>  Antisense nucleotide

<400>  2327
gatgatctga ctgcc                                                        15


<210>  2328
<211>  16
<212>  DNA
<213>  Artificial

<220>
<223>  Antisense nucleotide

<400>  2328
gttcgagaag atgatc                                                       16


<210>  2329
<211>  15
<212>  DNA
<213>  Artificial

<220>
<223>  Antisense nucleotide

<400>  2329
gggttcgaga agatg                                                        15


<210>  2330
<211>  16
<212>  DNA
<213>  Artificial

<220>
<223>  Antisense nucleotide

<400>  2330
ggtttgctac aacatg                                                       16


<210>  2331
<211>  15
<212>  DNA
<213>  Artificial

<220>
<223>  Antisense nucleotide

<400>  2331
cagcttgagg gtttg                                                        15


<210>  2332
<211>  14
<212>  DNA
<213>  Artificial

<220>
<223>  Antisense nucleotide

<400>  2332
tgcccctcag cttg                                                         14


<210>  2333
<211>  15
<212>  DNA
<213>  Artificial

<220>
<223>  Antisense nucleotide

<400>  2333
gacacacact atctc                                                        15


<210>  2334
<211>  16
<212>  DNA
<213>  Artificial

<220>
<223>  Antisense nucleotide

<400>  2334
gcagccatct ttattc                                                       16


<210>  2335
<211>  14
<212>  DNA
<213>  Artificial

<220>
<223>  Antisense nucleotide

<400>  2335
gttcagcagc catc                                                         14


<210>  2336
<211>  14
<212>  DNA
<213>  Artificial

<220>
<223>  Antisense nucleotide

<400>  2336
tggttcagca gcca                                                         14


<210>  2337
<211>  16
<212>  DNA
<213>  Artificial

<220>
<223>  Antisense nucleotide

<400>  2337
ctactggttc agcagc                                                       16

<210>  2338
<211>  14
<212>  DNA
<213>  Artificial

<220>
<223>  Antisense nucleotide

<400>  2338
tctactggtt cagc                                                          14


<210>  2339
<211>  16
<212>  DNA
<213>  Artificial

<220>
<223>  Antisense nucleotide

<400>  2339
gccacaaagt tgatgc                                                        16


<210>  2340
<211>  16
<212>  DNA
<213>  Artificial

<220>
<223>  Antisense nucleotide

<400>  2340
cattgccaca aagttg                                                        16


<210>  2341
<211>  16
<212>  DNA
<213>  Artificial

<220>
<223>  Antisense nucleotide

<400>  2341
gagaacttgg tcattc                                                        16


<210>  2342
<211>  16
<212>  DNA
<213>  Artificial

<220>
<223>  Antisense nucleotide

<400>  2342
ggtcaatgaa gagaac                                                        16


<210>  2343
<211>  14
<212>  DNA
<213>  Artificial

<220>
<223>  Antisense nucleotide

<400>  2343
cgatttcctt ggtc                                                          14


<210>  2344
<211>  15
<212>  DNA
<213>  Artificial

<220>
<223>  Antisense nucleotide

<400>  2344
ccgatttcct tggtc                                                         15


<210>  2345
<211>  18
<212>  DNA
<213>  Artificial

<220>
<223>  Antisense nucleotide

<400>  2345
caaatagagg ccgatttc                                                      18


<210>  2346
<211>  14
<212>  DNA
<213>  Artificial

<220>
<223>  Antisense nucleotide

<400>  2346
caaatagagg ccga                                                          14


<210>  2347
<211>  14
<212>  DNA
<213>  Artificial

<220>
<223>  Antisense nucleotide

<400>  2347
cctctaggct ggct                                                          14


<210>  2348
<211>  15
<212>  DNA
<213>  Artificial

<220>
<223>  Antisense nucleotide

<400>  2348
catacctcta ggctg                                                         15


<210>  2349
<211>  14
<212>  DNA
<213>  Artificial

<220>
<223>  Antisense nucleotide

<400>  2349
agccatacct ctag                                                          14


<210>  2350
<211>  15
<212>  DNA
<213>  Artificial

<220>
<223>  Antisense nucleotide

<400>  2350
cagccatacc tctag                                                         15


<210>  2351
<211>  17
<212>  DNA
<213>  Artificial

<220>
<223>  Antisense nucleotide

<400>  2351
cacagagata gttacag                                                       17


<210>  2352
<211>  17
<212>  DNA
<213>  Artificial

<220>
<223>  Antisense nucleotide

<400>  2352
gtcttcgttt tgaacag                                                       17


<210>  2353
<211>  18
<212>  DNA
<213>  Artificial

<220>
<223>  Antisense nucleotide

<400>  2353
ctagtcttcg ttttgaac                                                      18


<210>  2354
<211>  18
<212>  DNA
<213>  Artificial

<220>
<223>  Antisense nucleotide

<400>  2354
tagctagtct tcgttttg                                                      18

<210>  2355
<211>  13
<212>  DNA
<213>  Artificial

<220>
<223>  Antisense nucleotide

<400>  2355
gagccactgc gcc                                                          13


<210>  2356
<211>  14
<212>  DNA
<213>  Artificial

<220>
<223>  Antisense nucleotide

<400>  2356
cgtgagccac tgcg                                                         14


<210>  2357
<211>  15
<212>  DNA
<213>  Artificial

<220>
<223>  Antisense nucleotide

<400>  2357
cgtaacgatc actgg                                                        15


<210>  2358
<211>  15
<212>  DNA
<213>  Artificial

<220>
<223>  Antisense nucleotide

<400>  2358
gcactcgtaa cgatc                                                        15


<210>  2359
<211>  14
<212>  DNA
<213>  Artificial

<220>
<223>  Antisense nucleotide

<400>  2359
ggagcactcg taac                                                         14


<210>  2360
<211>  14
<212>  DNA
<213>  Artificial

<220>
<223>  Antisense nucleotide

<400>  2360
catcatcctg aggt                                                         14


<210>  2361
<211>  16
<212>  DNA
<213>  Artificial

<220>
<223>  Antisense nucleotide

<400>  2361
cagtatcatc atcctg                                                       16


<210>  2362
<211>  16
<212>  DNA
<213>  Artificial

<220>
<223>  Antisense nucleotide

<400>  2362
ctcagtatca tcatcc                                                       16


<210>  2363
<211>  18
<212>  DNA
<213>  Artificial

<220>
<223>  Antisense nucleotide

<400>  2363
ctaaaagtat gtgccatc                                                     18


<210>  2364
<211>  14
<212>  DNA
<213>  Artificial

<220>
<223>  Antisense nucleotide

<400>  2364
cacatcgcct ctct                                                         14


<210>  2365
<211>  18
<212>  DNA
<213>  Artificial

<220>
<223>  Antisense nucleotide

<400>  2365
gcttcacagt cacatcgc                                                     18


<210>  2366
<211>  16
<212>  DNA
<213>  Artificial

<220>
<223>  Antisense nucleotide

<400>  2366
ggaaggcttc acagtc                                                    16


<210>  2367
<211>  18
<212>  DNA
<213>  Artificial

<220>
<223>  Antisense nucleotide

<400>  2367
cctgtgactt gagaattg                                                  18


<210>  2368
<211>  14
<212>  DNA
<213>  Artificial

<220>
<223>  Antisense nucleotide

<400>  2368
ggaagacctg tgac                                                      14


<210>  2369
<211>  18
<212>  DNA
<213>  Artificial

<220>
<223>  Antisense nucleotide

<400>  2369
ctctgctcca catatttg                                                  18


<210>  2370
<211>  15
<212>  DNA
<213>  Artificial

<220>
<223>  Antisense nucleotide

<400>  2370
caacgaagat ctctg                                                     15


<210>  2371
<211>  14
<212>  DNA
<213>  Artificial

<220>
<223>  Antisense nucleotide

<400>  2371
caacaccaac gaag                                                      14

<210>  2372
<211>  14
<212>  DNA
<213>  Artificial

<220>
<223>  Antisense nucleotide

<400>  2372
ggtcttctgt ttgc                                                          14


<210>  2373
<211>  18
<212>  DNA
<213>  Artificial

<220>
<223>  Antisense nucleotide

<400>  2373
cgatgaagtg gtaggaag                                                      18


<210>  2374
<211>  14
<212>  DNA
<213>  Artificial

<220>
<223>  Antisense nucleotide

<400>  2374
ggttgcatgg aagc                                                          14


<210>  2375
<211>  15
<212>  DNA
<213>  Artificial

<220>
<223>  Antisense nucleotide

<400>  2375
ggtcacaaac ttgcc                                                         15


<210>  2376
<211>  17
<212>  DNA
<213>  Artificial

<220>
<223>  Antisense nucleotide

<400>  2376
ctgatttggt ccactag                                                       17


<210>  2377
<211>  16
<212>  DNA
<213>  Artificial

<220>
<223>  Antisense nucleotide

<400>  2377
catgttagca ctgttc                                                        16


<210>  2378
<211>  16
<212>  DNA
<213>  Artificial

<220>
<223>  Antisense nucleotide

<400>  2378
ggtcttgatg tactcc                                                        16


<210>  2379
<211>  17
<212>  DNA
<213>  Artificial

<220>
<223>  Antisense nucleotide

<400>  2379
ccacctaaag agagatc                                                       17


<210>  2380
<211>  16
<212>  DNA
<213>  Artificial

<220>
<223>  Antisense nucleotide

<400>  2380
cttgtactgc accatc                                                        16


<210>  2381
<211>  16
<212>  DNA
<213>  Artificial

<220>
<223>  Antisense nucleotide

<400>  2381
gccagttaag aagatg                                                        16


<210>  2382
<211>  17
<212>  DNA
<213>  Artificial

<220>
<223>  Antisense nucleotide

<400>  2382
gagatcatga tccatgg                                                       17


<210>  2383
<211>  17
<212>  DNA
<213>  Artificial

<220>
<223>  Antisense nucleotide

<400>  2383
gtagtgtccc aatagtg                                                    17


<210>  2384
<211>  16
<212>  DNA
<213>  Artificial

<220>
<223>  Antisense nucleotide

<400>  2384
cttcctcatc attccc                                                     16


<210>  2385
<211>  15
<212>  DNA
<213>  Artificial

<220>
<223>  Antisense nucleotide

<400>  2385
cacaagcttt tcgac                                                      15


<210>  2386
<211>  390
<212>  PRT
<213>  TGF-beta1

<400>  2386

Met Pro Pro Ser Gly Leu Arg Leu Leu Leu Leu Leu Leu Pro Leu Leu
1               5                   10                  15


Trp Leu Leu Val Leu Thr Pro Gly Arg Pro Ala Ala Gly Leu Ser Thr
            20                  25                  30


Cys Lys Thr Ile Asp Met Glu Leu Val Lys Arg Lys Arg Ile Glu Ala
        35                  40                  45


Ile Arg Gly Gln Ile Leu Ser Lys Leu Arg Leu Ala Ser Pro Pro Ser
    50                  55                  60


Gln Gly Glu Val Pro Pro Gly Pro Leu Pro Glu Ala Val Leu Ala Leu
65                  70                  75                  80


Tyr Asn Ser Thr Arg Asp Arg Val Ala Gly Glu Ser Ala Glu Pro Glu
                85                  90                  95


Pro Glu Pro Glu Ala Asp Tyr Tyr Ala Lys Glu Val Thr Arg Val Leu
            100                 105                 110


Met Val Glu Thr His Asn Glu Ile Tyr Asp Lys Phe Lys Gln Ser Thr

115                 120                 125

His Ser Ile Tyr Met Phe Phe Asn Thr Ser Glu Leu Arg Glu Ala Val
    130                 135                 140

Pro Glu Pro Val Leu Leu Ser Arg Ala Glu Leu Arg Leu Leu Arg Leu
145                 150                 155                 160

Lys Leu Lys Val Glu Gln His Val Glu Leu Tyr Gln Lys Tyr Ser Asn
                165                 170                 175

Asn Ser Trp Arg Tyr Leu Ser Asn Arg Leu Leu Ala Pro Ser Asp Ser
            180                 185                 190

Pro Glu Trp Leu Ser Phe Asp Val Thr Gly Val Val Arg Gln Trp Leu
        195                 200                 205

Ser Arg Gly Gly Glu Ile Glu Gly Phe Arg Leu Ser Ala His Cys Ser
    210                 215                 220

Cys Asp Ser Arg Asp Asn Thr Leu Gln Val Asp Ile Asn Gly Phe Thr
225                 230                 235                 240

Thr Gly Arg Arg Gly Asp Leu Ala Thr Ile His Gly Met Asn Arg Pro
                245                 250                 255

Phe Leu Leu Leu Met Ala Thr Pro Leu Glu Arg Ala Gln His Leu Gln
            260                 265                 270

Ser Ser Arg His Arg Arg Ala Leu Asp Thr Asn Tyr Cys Phe Ser Ser
        275                 280                 285

Thr Glu Lys Asn Cys Cys Val Arg Gln Leu Tyr Ile Asp Phe Arg Lys
    290                 295                 300

Asp Leu Gly Trp Lys Trp Ile His Glu Pro Lys Gly Tyr His Ala Asn
305                 310                 315                 320

Phe Cys Leu Gly Pro Cys Pro Tyr Ile Trp Ser Leu Asp Thr Gln Tyr
                325                 330                 335

Ser Lys Val Leu Ala Leu Tyr Asn Gln His Asn Pro Gly Ala Ser Ala
            340                 345                 350

Ala Pro Cys Cys Val Pro Gln Ala Leu Glu Pro Leu Pro Ile Val Tyr
        355                 360                 365

Tyr Val Gly Arg Lys Pro Lys Val Glu Gln Leu Ser Asn Met Ile Val
    370                 375                 380

Arg Ser Cys Lys Cys Ser

385                390

<210>  2387
<211>  20
<212>  PRT
<213>  TGF-beta1

<400>  2387

Ala Leu Asp Thr Asn Tyr Cys Phe Ser Ser Thr Glu Lys Asn Cys Cys
1               5                   10                  15

Val Arg Gln Leu
            20

<210>  2388
<211>  20
<212>  PRT
<213>  TGF-beta1

<400>  2388

Tyr Ile Asp Phe Arg Lys Asp Leu Gly Trp Lys Trp Ile His Glu Pro
1               5                   10                  15

Lys Gly Tyr His
            20

<210>  2389
<211>  20
<212>  PRT
<213>  TGF-beta1

<400>  2389

Ala Asn Phe Cys Leu Gly Pro Cys Pro Tyr Ile Trp Ser Leu Asp Thr
1               5                   10                  15

Gln Tyr Ser Lys
            20

<210>  2390
<211>  20
<212>  PRT
<213>  TGF-beta1

<400>  2390

Val Leu Ala Leu Tyr Asn Gln His Asn Pro Gly Ala Ser Ala Ala Pro
1               5                   10                  15

Cys Cys Val Pro
            20

<210>  2391
<211>  20
<212>  PRT
<213>  TGF-beta1

<210>  2391
<211>  20
<212>  PRT
<213>  TGF-beta1

<400>  2391

Gln Ala Leu Glu Pro Leu Pro Ile Val Tyr Tyr Val Gly Arg Lys Pro
1               5                   10                  15

Lys Val Glu Gln
            20

<210>  2392
<211>  12
<212>  PRT
<213>  TGF-beta1

<400>  2392

Leu Ser Asn Met Ile Val Arg Ser Cys Lys Cys Ser
1               5                   10

<210>  2393
<211>  20
<212>  PRT
<213>  TGF-beta1

<400>  2393

Thr Glu Lys Asn Cys Cys Val Arg Gln Leu Tyr Ile Asp Phe Arg Lys
1               5                   10                  15

Asp Leu Gly Trp
            20

<210>  2394
<211>  20
<212>  PRT
<213>  TGF-beta1

<400>  2394

Lys Trp Ile His Glu Pro Lys Gly Tyr His Ala Asn Phe Cys Leu Gly
1               5                   10                  15

Pro Cys Pro Tyr
            20

<210>  2395
<211>  19
<212>  PRT
<213>  TGF-beta1

<400>  2395

Trp Ser Leu Asp Thr Gln Tyr Ser Lys Val Leu Ala Leu Tyr Asn Gln
1               5                   10                  15

His Asn Pro

<210>  2396
<211>  20
<212>  PRT
<213>  TGF-beta1

<400>  2396

Gly Ala Ser Ala Ala Pro Cys Cys Val Pro Gln Ala Leu Glu Pro Leu
1               5                   10                  15

Pro Ile Val Tyr
            20

<210>  2397
<211>  22
<212>  PRT
<213>  TGF-beta1

<400>  2397

Tyr Val Gly Arg Lys Pro Lys Val Glu Gln Leu Ser Asn Met Ile Val
1               5                   10                  15

Arg Ser Cys Lys Cys Ser
            20

<210>  2398
<211>  40
<212>  PRT
<213>  TGF-beta1

<400>  2398

Gln Tyr Ser Lys Val Leu Ala Leu Tyr Asn Gln His Asn Pro Gly Ala
1               5                   10                  15

Ser Ala Ala Pro Cys Cys Val Pro Gln Ala Leu Glu Pro Leu Pro Ile
            20                  25                  30

Val Tyr Tyr Val Gly Arg Lys Pro
        35                  40

<210>  2399
<211>  40
<212>  PRT
<213>  TGF-beta1

<400>  2399

Gln Tyr Ser Lys Val Leu Ala Leu Tyr Asn Gln His Asn Pro Gly Ala
1               5                   10                  15

Ser Ala Ala Pro Cys Cys Val Pro Gln Ala Leu Glu Pro Leu Pro Ile
            20                  25                  30

Val Tyr Tyr Val Gly Arg Lys Pro

35                  40

<210>  2400
<211>  112
<212>  PRT
<213>  TGF-beta1

<400>  2400

Ala Leu Asp Thr Asn Tyr Cys Phe Ser Ser Thr Glu Lys Asn Cys Cys
1               5                   10                  15

Val Arg Gln Leu Tyr Ile Asp Phe Arg Lys Asp Leu Gly Trp Lys Trp
            20                  25                  30

Ile His Glu Pro Lys Gly Tyr His Ala Asn Phe Cys Leu Gly Pro Cys
        35                  40                  45

Pro Tyr Ile Trp Ser Leu Asp Thr Gln Tyr Ser Lys Val Leu Ala Leu
    50                  55                  60

Tyr Asn Gln His Asn Pro Gly Ala Ser Ala Ala Pro Cys Cys Val Pro
65                  70                  75                  80

Gln Ala Leu Glu Pro Leu Pro Ile Val Tyr Tyr Val Gly Arg Lys Pro
                85                  90                  95

Lys Val Glu Gln Leu Ser Asn Met Ile Val Arg Ser Cys Lys Cys Ser
            100                 105                 110

<210>  2401
<211>  30
<212>  PRT
<213>  TGF-beta1

<400>  2401

Ala Leu Asp Thr Asn Tyr Cys Phe Ser Ser Thr Glu Lys Asn Cys Cys
1               5                   10                  15

Val Arg Gln Leu Tyr Ile Asp Phe Arg Lys Asp Leu Gly Trp
            20                  25                  30

<210>  2402
<211>  30
<212>  PRT
<213>  TGF-beta1

<400>  2402

Lys Trp Ile His Glu Pro Lys Gly Tyr His Ala Asn Phe Cys Leu Gly
1               5                   10                  15

Pro Cys Pro Tyr Ile Trp Ser Leu Asp Thr Gln Tyr Ser Lys
            20                  25                  30

<210>  2403
<211>  30
<212>  PRT
<213>  TGF-beta1

<400>  2403

Val Leu Ala Leu Tyr Asn Gln His Asn Pro Gly Ala Ser Ala Ala Pro
1               5                   10                  15

Cys Cys Val Pro Gln Ala Leu Glu Pro Leu Pro Ile Val Tyr
            20                  25                  30

<210>  2404
<211>  22
<212>  PRT
<213>  TGF-beta1

<400>  2404

Tyr Val Gly Arg Lys Pro Lys Val Glu Gln Leu Ser Asn Met Ile Val
1               5                   10                  15

Arg Ser Cys Lys Cys Ser
            20

<210>  2405
<211>  30
<212>  PRT
<213>  TGF-beta1

<400>  2405

Cys Val Arg Gln Leu Tyr Ile Asp Phe Arg Lys Asp Leu Gly Trp Lys
1               5                   10                  15

Trp Ile His Glu Pro Lys Gly Tyr His Ala Asn Phe Cys Leu
            20                  25                  30

<210>  2406
<211>  30
<212>  PRT
<213>  TGF-beta1

<400>  2406

Gly Pro Cys Pro Tyr Ile Trp Ser Leu Asp Thr Gln Tyr Ser Lys Val
1               5                   10                  15

Leu Ala Leu Tyr Asn Gln His Asn Pro Gly Ala Ser Ala Ala
            20                  25                  30

<210>  2407
<211>  30
<212>  PRT
<213>  TGF-beta1

<400>  2407

Pro Cys Cys Val Pro Gln Ala Leu Glu Pro Leu Pro Ile Val Tyr Tyr

1               5                   10                  15

Val Gly Arg Lys Pro Lys Val Glu Gln Leu Ser Asn Met Ile
            20                  25                  30

<210>  2408
<211>  413
<212>  PRT
<213>  TGF-beta2

<400>  2408

Met His Tyr Cys Val Leu Ser Ala Phe Leu Ile Leu His Leu Val Thr
1               5                   10                  15

Val Ala Leu Ser Leu Ser Thr Cys Ser Thr Leu Asp Met Asp Gln Phe
            20                  25                  30

Met Arg Lys Arg Ile Glu Ala Ile Arg Gly Gln Ile Leu Ser Lys Leu
        35                  40                  45

Lys Leu Thr Ser Pro Pro Glu Asp Tyr Pro Glu Pro Glu Glu Val Pro
    50                  55                  60

Pro Glu Val Ile Ser Ile Tyr Asn Ser Thr Arg Asp Leu Leu Gln Glu
65                  70                  75                  80

Lys Ala Ser Arg Arg Ala Ala Ala Cys Glu Arg Glu Arg Ser Asp Glu
                85                  90                  95

Glu Tyr Tyr Ala Lys Glu Val Tyr Lys Ile Asp Met Pro Pro Phe Phe
            100                 105                 110

Pro Ser Glu Asn Ala Ile Pro Pro Thr Phe Tyr Arg Pro Tyr Phe Arg
        115                 120                 125

Ile Val Arg Phe Asp Val Ser Ala Met Glu Lys Asn Ala Ser Asn Leu
    130                 135                 140

Val Lys Ala Glu Phe Arg Val Phe Arg Leu Gln Asn Pro Lys Ala Arg
145                 150                 155                 160

Val Pro Glu Gln Arg Ile Glu Leu Tyr Gln Ile Leu Lys Ser Lys Asp
                165                 170                 175

Leu Thr Ser Pro Thr Gln Arg Tyr Ile Asp Ser Lys Val Val Lys Thr
            180                 185                 190

Arg Ala Glu Gly Glu Trp Leu Ser Phe Asp Val Thr Asp Ala Val His
        195                 200                 205

Glu Trp Leu His His Lys Asp Arg Asn Leu Gly Phe Lys Ile Ser Leu
    210                 215                 220

His Cys Pro Cys Cys Thr Phe Val Pro Ser Asn Asn Tyr Ile Ile Pro
225                 230                 235                 240

Asn Lys Ser Glu Glu Leu Glu Ala Arg Phe Ala Gly Ile Asp Gly Thr
                245                 250                 255

Ser Thr Tyr Thr Ser Gly Asp Gln Lys Thr Ile Lys Ser Thr Arg Lys
            260                 265                 270

Lys Asn Ser Gly Lys Thr Pro His Leu Leu Leu Met Leu Leu Pro Ser
        275                 280                 285

Tyr Arg Leu Glu Ser Gln Gln Thr Asn Arg Arg Lys Arg Ala Leu Asp
    290                 295                 300

Ala Ala Tyr Cys Phe Arg Asn Val Gln Asp Asn Cys Cys Leu Arg Pro
305                 310                 315                 320

Leu Tyr Ile Asp Phe Lys Arg Asp Leu Gly Trp Lys Trp Ile His Glu
                325                 330                 335

Pro Lys Gly Tyr Asn Ala Asn Phe Cys Ala Gly Ala Cys Pro Tyr Leu
            340                 345                 350

Trp Ser Ser Asp Thr Gln His Ser Arg Val Leu Ser Leu Tyr Asn Thr
        355                 360                 365

Ile Asn Pro Glu Ala Ser Ala Ser Pro Cys Cys Val Ser Gln Asp Leu
    370                 375                 380

Glu Pro Leu Thr Ile Leu Tyr Tyr Ile Gly Lys Thr Pro Lys Ile Glu
385                 390                 395                 400

Gln Leu Ser Asn Met Ile Val Lys Ser Cys Lys Cys Ser
                405                 410

<210>   2409
<211>   20
<212>   PRT
<213>   TGF-beta2

<400>   2409

Ala Leu Asp Ala Ala Tyr Cys Phe Arg Asn Val Gln Asp Asn Cys Cys
1               5                   10                  15

Leu Arg Pro Leu
            20

<210>   2410
<211>   20
<212>   PRT

<213>  TGF-beta2

<400>  2410

Tyr Ile Asp Phe Lys Arg Asp Leu Gly Trp Lys Trp Ile His Glu Pro
1               5                   10                  15

Lys Gly Tyr Asn
            20

<210>  2411
<211>  20
<212>  PRT
<213>  TGF-beta2

<400>  2411

Ala Asn Phe Cys Ala Gly Ala Cys Pro Tyr Leu Trp Ser Ser Asp Thr
1               5                   10                  15

Gln His Ser Arg
            20

<210>  2412
<211>  20
<212>  PRT
<213>  TGF-beta2

<400>  2412

Val Leu Ser Leu Tyr Asn Thr Ile Asn Pro Glu Ala Ser Ala Ser Pro
1               5                   10                  15

Cys Cys Val Ser
            20

<210>  2413
<211>  20
<212>  PRT
<213>  TGF-beta2

<400>  2413

Gln Asp Leu Glu Pro Leu Thr Ile Leu Tyr Tyr Ile Gly Lys Thr Pro
1               5                   10                  15

Lys Ile Glu Gln
            20

<210>  2414
<211>  20
<212>  PRT
<213>  TGF-beta2

<400>  2414

Gln Asp Leu Glu Pro Leu Thr Ile Leu Tyr Tyr Ile Gly Lys Thr Pro
1               5                   10                  15

Lys Ile Glu Gln
            20

<210>  2415
<211>  20
<212>  PRT
<213>  TGF-beta2

<400>  2415

Val Gln Asp Asn Cys Cys Leu Arg Pro Leu Tyr Ile Asp Phe Lys Arg
1               5                   10                  15

Asp Leu Gly Trp
            20

<210>  2416
<211>  20
<212>  PRT
<213>  TGF-beta2

<400>  2416

Lys Trp Ile His Glu Pro Lys Gly Tyr Asn Ala Asn Phe Cys Ala Gly
1               5                   10                  15

Ala Cys Pro Tyr
            20

<210>  2417
<211>  20
<212>  PRT
<213>  TGF-beta2

<400>  2417

Leu Trp Ser Ser Asp Thr Gln His Ser Arg Val Leu Ser Leu Tyr Asn
1               5                   10                  15

Thr Ile Asn Pro
            20

<210>  2418
<211>  20
<212>  PRT
<213>  TGF-beta2

<400>  2418

Glu Ala Ser Ala Ser Pro Cys Cys Val Ser Gln Asp Leu Glu Pro Leu
1               5                   10                  15

Thr Ile Leu Tyr
            20

<210>  2419
<211>  22
<212>  PRT
<213>  TGF-beta2

<400>  2419

Tyr Ile Gly Lys Thr Pro Lys Ile Glu Gln Leu Ser Asn Met Ile Val
1               5                   10                  15

Lys Ser Cys Lys Cys Ser
            20

<210>  2420
<211>  41
<212>  PRT
<213>  TGF-beta2

<400>  2420

Gln His Ser Arg Val Leu Ser Leu Tyr Asn Thr Ile Asn Pro Glu Ala
1               5                   10                  15

Ser Ala Ser Pro Cys Cys Val Ser Gln Asp Leu Glu Pro Leu Thr Ile
            20                  25                  30

Leu Tyr Tyr Ile Gly Lys Thr Pro Lys
        35                  40

<210>  2421
<211>  41
<212>  PRT
<213>  TGF-beta2

<400>  2421

Gln His Ser Arg Val Leu Ser Leu Tyr Asn Thr Ile Asn Pro Glu Ala
1               5                   10                  15

Ser Ala Ser Pro Cys Cys Val Ser Gln Asp Leu Glu Pro Leu Thr Ile
            20                  25                  30

Leu Tyr Tyr Ile Gly Lys Thr Pro Lys
        35                  40

<210>  2422
<211>  112
<212>  PRT
<213>  TGF-beta2

<400>  2422

Ala Leu Asp Ala Ala Tyr Cys Phe Arg Asn Val Gln Asp Asn Cys Cys
1               5                   10                  15

Leu Arg Pro Leu Tyr Ile Asp Phe Lys Arg Asp Leu Gly Trp Lys Trp
            20                  25                  30

Ile His Glu Pro Lys Gly Tyr Asn Ala Asn Phe Cys Ala Gly Ala Cys
        35                  40                  45

Pro Tyr Leu Trp Ser Ser Asp Thr Gln His Ser Arg Val Leu Ser Leu
    50                  55                  60

Tyr Asn Thr Ile Asn Pro Glu Ala Ser Ala Ser Pro Cys Cys Val Ser
65                  70                  75                  80

Gln Asp Leu Glu Pro Leu Thr Ile Leu Tyr Tyr Ile Gly Lys Thr Pro
                85                  90                  95

Lys Ile Glu Gln Leu Ser Asn Met Ile Val Lys Ser Cys Lys Cys Ser
            100                 105                 110

<210>  2423
<211>  30
<212>  PRT
<213>  TGF-beta2

<400>  2423

Ala Leu Asp Ala Ala Tyr Cys Phe Arg Asn Val Gln Asp Asn Cys Cys
1               5                   10                  15

Leu Arg Pro Leu Tyr Ile Asp Phe Lys Arg Asp Leu Gly Trp
            20                  25                  30

<210>  2424
<211>  29
<212>  PRT
<213>  TGF-beta2

<400>  2424

Lys Trp Ile His Glu Pro Lys Gly Tyr Asn Ala Asn Phe Cys Ala Gly
1               5                   10                  15

Ala Cys Pro Tyr Leu Trp Ser Ser Asp Thr Gln His Ser
            20                  25

<210>  2425
<211>  29
<212>  PRT
<213>  TGF-beta2

<400>  2425

Val Leu Ser Leu Tyr Asn Thr Ile Asn Pro Glu Ala Ser Ala Ser Pro
1               5                   10                  15

Cys Cys Val Ser Gln Asp Leu Glu Pro Leu Thr Ile Leu
            20                  25

<210>  2426
<211>  22
<212>  PRT
<213>  TGF-beta2

<400>  2426

Tyr Ile Gly Lys Thr Pro Lys Ile Glu Gln Leu Ser Asn Met Ile Val
1               5                   10                  15

Lys Ser Cys Lys Cys Ser
            20

<210>  2427
<211>  30
<212>  PRT
<213>  TGF-beta2

<400>  2427

Cys Leu Arg Pro Leu Tyr Ile Asp Phe Lys Arg Asp Leu Gly Trp Lys
1               5                   10                  15

Trp Ile His Glu Pro Lys Gly Tyr Asn Ala Asn Phe Cys Ala
            20                  25                  30

<210>  2428
<211>  30
<212>  PRT
<213>  TGF-beta2

<400>  2428

Gly Ala Cys Pro Tyr Leu Trp Ser Ser Asp Thr Gln His Ser Arg Val
1               5                   10                  15

Leu Ser Leu Tyr Asn Thr Ile Asn Pro Glu Ala Ser Ala Ser
            20                  25                  30

<210>  2429
<211>  30
<212>  PRT
<213>  TGF-beta2

<400>  2429

Pro Cys Cys Val Ser Gln Asp Leu Glu Pro Leu Thr Ile Leu Tyr Tyr
1               5                   10                  15

Ile Gly Lys Thr Pro Lys Ile Glu Gln Leu Ser Asn Met Ile
            20                  25                  30

<210>  2430
<211>  412
<212>  PRT
<213>  TGF-beta3

<400>  2430

Met Lys Met His Leu Gln Arg Ala Leu Val Val Leu Ala Leu Leu Asn
1               5                   10                  15

Phe Ala Thr Val Ser Leu Ser Leu Ser Thr Cys Thr Thr Leu Asp Phe
            20                  25                  30

Gly His Ile Lys Lys Lys Arg Val Glu Ala Ile Arg Gly Gln Ile Leu
        35                  40                  45

Ser Lys Leu Arg Leu Thr Ser Pro Pro Glu Pro Thr Val Met Thr His
    50                  55                  60

Val Pro Tyr Gln Val Leu Ala Leu Tyr Asn Ser Thr Arg Glu Leu Leu
65                  70                  75                  80

Glu Glu Met His Gly Glu Arg Glu Glu Gly Cys Thr Gln Glu Asn Thr
                85                  90                  95

Glu Ser Glu Tyr Tyr Ala Lys Glu Ile His Lys Phe Asp Met Ile Gln
            100                 105                 110

Gly Leu Ala Glu His Asn Glu Leu Ala Val Cys Pro Lys Gly Ile Thr
        115                 120                 125

Ser Lys Val Phe Arg Phe Asn Val Ser Ser Val Glu Lys Asn Arg Thr
    130                 135                 140

Asn Leu Phe Arg Ala Glu Phe Arg Val Leu Arg Val Pro Asn Pro Ser
145                 150                 155                 160

Ser Lys Arg Asn Glu Gln Arg Ile Glu Leu Phe Gln Ile Leu Arg Pro
                165                 170                 175

Asp Glu His Ile Ala Lys Gln Arg Tyr Ile Gly Gly Lys Asn Leu Pro
            180                 185                 190

Thr Arg Gly Thr Ala Glu Trp Leu Ser Phe Asp Val Thr Asp Thr Val
        195                 200                 205

Arg Glu Trp Leu Leu Arg Arg Glu Ser Asn Leu Gly Leu Glu Ile Ser
    210                 215                 220

Ile His Cys Pro Cys His Thr Phe Gln Pro Asn Gly Asp Ile Leu Glu
225                 230                 235                 240

Asn Ile His Glu Val Met Glu Ile Lys Phe Lys Gly Val Asp Asn Glu
                245                 250                 255

Asp Asp His Gly Arg Gly Asp Leu Gly Arg Leu Lys Lys Gln Lys Asp
            260                 265                 270

His His Asn Pro His Leu Ile Leu Met Met Ile Pro Pro His Arg Leu
        275                 280                 285

Asp Asn Pro Gly Gln Gly Gly Gln Arg Lys Lys Arg Ala Leu Asp Ala
    290                 295                 300

Ala Tyr Cys Phe Arg Asn Val Gln Asp Asn Cys Cys Leu Arg Pro Leu
305                 310                 315                 320

Tyr Ile Asp Phe Lys Arg Asp Leu Gly Trp Lys Trp Ile His Glu Pro
                325                 330                 335

Lys Gly Tyr Asn Ala Asn Phe Cys Ala Gly Ala Cys Pro Tyr Leu Trp
            340                 345                 350

Ser Ser Asp Thr Gln His Ser Arg Val Leu Ser Leu Tyr Asn Thr Ile
        355                 360                 365

Asn Pro Glu Ala Ser Ala Ser Pro Cys Cys Val Ser Gln Asp Leu Glu
    370                 375                 380

Pro Leu Thr Ile Leu Tyr Tyr Ile Gly Lys Thr Pro Lys Ile Glu Gln
385                 390                 395                 400

Leu Ser Asn Met Ile Val Lys Ser Cys Lys Cys Ser
                405                 410

<210>  2431
<211>  20
<212>  PRT
<213>  TGF-beta3

<400>  2431

Ala Leu Asp Thr Asn Tyr Cys Phe Arg Asn Leu Glu Glu Asn Cys Cys
1               5                   10                  15

Val Arg Pro Leu
            20

<210>  2432
<211>  20
<212>  PRT
<213>  TGF-beta3

<400>  2432

Tyr Ile Asp Phe Arg Gln Asp Leu Gly Trp Lys Trp Val His Glu Pro
1               5                   10                  15

Lys Gly Tyr Tyr
            20

<210>  2433
<211>  20
<212>  PRT
<213>  TGF-beta3

<400>  2433

Ala Asn Phe Cys Ser Gly Pro Cys Pro Tyr Leu Arg Ser Ala Asp Thr
1               5                   10                  15

Thr His Ser Thr
            20

<210>  2434
<211>  20
<212>  PRT
<213>  TGF-beta3

<400>  2434

Val Leu Gly Leu Tyr Asn Thr Leu Asn Pro Glu Ala Ser Ala Ser Pro
1               5                   10                  15

Cys Cys Val Pro
            20

<210>  2435
<211>  20
<212>  PRT
<213>  TGF-beta3

<400>  2435

Gln Asp Leu Glu Pro Leu Thr Ile Leu Tyr Tyr Val Gly Arg Thr Pro
1               5                   10                  15

Lys Val Glu Gln
            20

<210>  2436
<211>  12
<212>  PRT
<213>  TGF-beta3

<400>  2436

Leu Ser Asn Met Val Val Lys Ser Cys Lys Cys Ser
1               5                   10

<210>  2437
<211>  20
<212>  PRT
<213>  TGF-beta3

<400>  2437

Asn Leu Glu Glu Asn Cys Cys Val Arg Pro Leu Tyr Ile Asp Phe Arg
1               5                   10                  15

Gln Asp Leu Gly
            20

<210>  2438
<211>  20
<212>  PRT
<213>  TGF-beta3

<400>  2438

Trp Lys Trp Val His Glu Pro Lys Gly Tyr Tyr Ala Asn Phe Cys Ser
1               5                   10                  15

Gly Pro Cys Pro
            20

<210>  2439
<211>  20
<212>  PRT
<213>  TGF-beta3

<400>  2439

Tyr Leu Arg Ser Ala Asp Thr Thr His Ser Thr Val Leu Gly Leu Tyr
1               5                   10                  15

Asn Thr Leu Asn
            20

<210>  2440
<211>  20
<212>  PRT
<213>  TGF-beta3

<400>  2440

Pro Glu Ala Ser Ala Ser Pro Cys Cys Val Pro Gln Asp Leu Glu Pro
1               5                   10                  15

Leu Thr Ile Leu
            20

<210>  2441
<211>  23
<212>  PRT
<213>  TGF-beta3

<400>  2441

Tyr Tyr Val Gly Arg Thr Pro Lys Val Glu Gln Leu Ser Asn Met Val
1               5                   10                  15

Val Lys Ser Cys Lys Cys Ser
            20

<210>  2442
<211>  41
<212>  PRT
<213>  TGF-beta3

<400>  2442

Thr His Ser Thr Val Leu Gly Leu Tyr Asn Thr Leu Asn Pro Glu Ala
1               5                   10                  15

Ser Ala Ser Pro Cys Cys Val Pro Gln Asp Leu Glu Pro Leu Thr Ile
            20                  25                  30

Leu Tyr Tyr Val Gly Arg Thr Pro Lys
        35                  40

<210>  2443
<211>  41
<212>  PRT
<213>  TGF-beta3

<400>  2443

Thr His Ser Thr Val Leu Gly Leu Tyr Asn Thr Leu Asn Pro Glu Ala
1               5                   10                  15

Ser Ala Ser Pro Cys Cys Val Pro Gln Asp Leu Glu Pro Leu Thr Ile
            20                  25                  30

Leu Tyr Tyr Val Gly Arg Thr Pro Lys
        35                  40

<210>  2444
<211>  112
<212>  PRT
<213>  TGF-beta3

<400>  2444

Ala Leu Asp Ala Ala Tyr Cys Phe Arg Asn Val Gln Asp Asn Cys Cys
1               5                   10                  15

Leu Arg Pro Leu Tyr Ile Asp Phe Lys Arg Asp Leu Gly Trp Lys Trp
            20                  25                  30

Ile His Glu Pro Lys Gly Tyr Asn Ala Asn Phe Cys Ala Gly Ala Cys
        35                  40                  45

Pro Tyr Leu Trp Ser Ser Asp Thr Gln His Ser Arg Val Leu Ser Leu
    50                  55                  60

Tyr Asn Thr Ile Asn Pro Glu Ala Ser Ala Ser Pro Cys Cys Val Ser
65                  70                  75                  80

Gln Asp Leu Glu Pro Leu Thr Ile Leu Tyr Tyr Ile Gly Lys Thr Pro
                85                  90                  95

Lys Ile Glu Gln Leu Ser Asn Met Ile Val Lys Ser Cys Lys Cys Ser
            100                 105                 110

<210>  2445
<211>  30
<212>  PRT
<213>  TGF-beta3

<400>  2445

Ala Leu Asp Ala Ala Tyr Cys Phe Arg Asn Val Gln Asp Asn Cys Cys
1               5                   10                  15

Leu Arg Pro Leu Tyr Ile Asp Phe Lys Arg Asp Leu Gly Trp
            20                  25                  30

<210>  2446
<211>  30
<212>  PRT
<213>  TGF-beta3

<400>  2446

Lys Trp Ile His Glu Pro Lys Gly Tyr Asn Ala Asn Phe Cys Ala Gly
1               5                   10                  15

Ala Cys Pro Tyr Leu Trp Ser Ser Asp Thr Gln His Ser Arg
            20                  25                  30

<210>  2447
<211>  30
<212>  PRT
<213>  TGF-beta3

<400>  2447

Val Leu Ser Leu Tyr Asn Thr Ile Asn Pro Glu Ala Ser Ala Ser Pro
1               5                   10                  15

Cys Cys Val Ser Gln Asp Leu Glu Pro Leu Thr Ile Leu Tyr
            20                  25                  30

<210>  2448
<211>  22
<212>  PRT
<213>  TGF-beta3

<400>  2448

Tyr Ile Gly Lys Thr Pro Lys Ile Glu Gln Leu Ser Asn Met Ile Val
1               5                   10                  15

Lys Ser Cys Lys Cys Ser
            20

<210>  2449
<211>  30
<212>  PRT
<213>  TGF-beta3

<400>  2449

Cys Leu Arg Pro Leu Tyr Ile Asp Phe Lys Arg Asp Leu Gly Trp Lys
1               5                   10                  15

Trp Ile His Glu Pro Lys Gly Tyr Asn Ala Asn Phe Cys Ala
            20                  25                  30

<210>  2450
<211>  30
<212>  PRT

<213>  TGF-beta3

<400>  2450

Gly Ala Cys Pro Tyr Leu Trp Ser Ser Asp Thr Gln His Ser Arg Val
1               5                   10                  15

Leu Ser Leu Tyr Asn Thr Ile Asn Pro Glu Ala Ser Ala Ser
            20                  25                  30

<210>  2451
<211>  30
<212>  PRT
<213>  TGF-beta3

<400>  2451

Pro Cys Cys Val Ser Gln Asp Leu Glu Pro Leu Thr Ile Leu Tyr Tyr
1               5                   10                  15

Ile Gly Lys Thr Pro Lys Ile Glu Gln Leu Ser Asn Met Ile
            20                  25                  30

## Description

The present invention is directed to oligonucleotide-polymer conjugates.

Polymers have been widely used in biomaterial, biotechnology and medicine primarily because they are nonimmunogenic and water-soluble (Zhao 1997). In the area of drug delivery, polymer derivatives have been widely used in covalent attachment to proteins to reduce immunogenicity, proteolysis, kidney clearance and to enhance solubility. Similarly, polyethylene glycol has been attached to low molecular weight, relatively hydrophobic drugs to enhance solubility, reduce toxicity and alter biodistribution.

Polyethylene glycol is used as carrier or linked covalently to different therapeutics to enhance the cellular uptake. Another aim of linking polymers to therapeutics is to enlarge the molecular weight and increase the body halflife time. Reasons for this linkage are that those conjugates are described to show advantages such as reduced immunogenicity (Chirila 2001).
Though there are a couple of advantages there is no teaching, that polymers could enhance the activity of therapeutics.

Surprisingly immunostimulators linked with polymers of a certain weight show increased activity as well as pharmacological advantages despite of their higher molecular weight. One aspect of this invention are immunostimulators linked with at least one polymer.

A further aspect of this invention is the synthesis of these compounds and their use for the preparation of a pharmaceutical composition as well as a method of the treatment of cancer, fibrosis and/or HIV with this compound.

The immunostimulator linked with at least one polymer (also referred to as "compound" in the context of this invention) has reduced toxicity especially in kidney and liver. This compound shows further advantages such as at least one of reduced lymphozytopenia as well as reduced leukocytopenia, reactivation of reduced clotting, reduction of thrombocytopenia, reduced immunogenicity, and antigenicity as well as a prolonged circulation half life time, decreased kidney excretion, modified organ uptake, reduced uptake in organs with reticuloendothelial system, decreased plasma protein binding, increased drug stability compared to unconjugated oligonucleotides. A further important advantage of the compound is at least one of reduced coagulation, reduced complement activation, especially reduced C5 complement activation, recovering of the decreased CH₅₀ concentration and higher cardiovascular safety.

Further advances of the compound are at least one of enhanced cellular uptake, enhanced affinity for nucleic acid target, altered intracellular localization, enhanced safety, enhanced efficacy and increased stability in the presence of nucleases.

These beneficial properties of the modified immunostimulator make them very useful in a variety of therapeutic applications.

### Figures

### Figure 1:

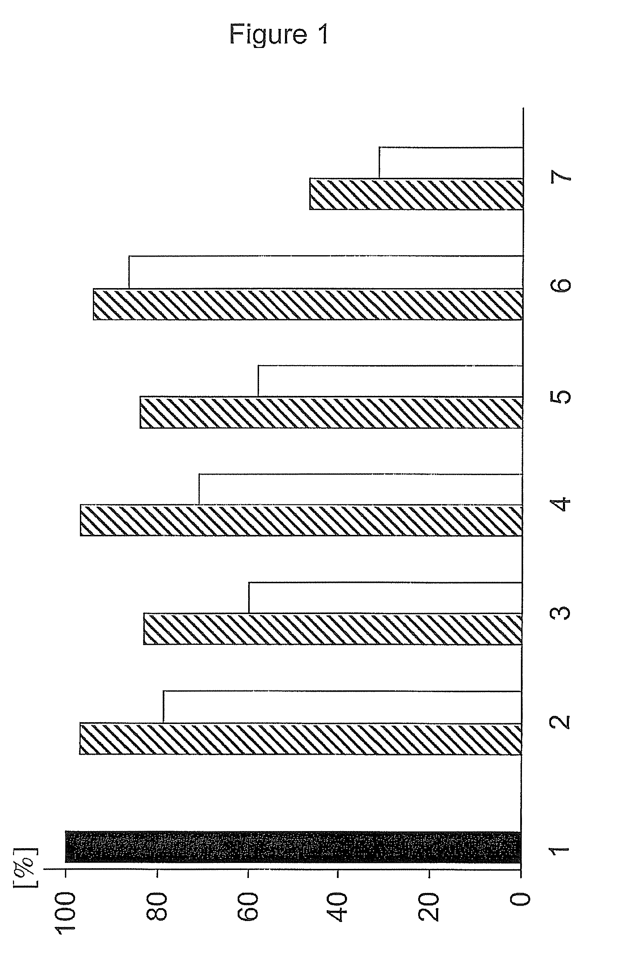

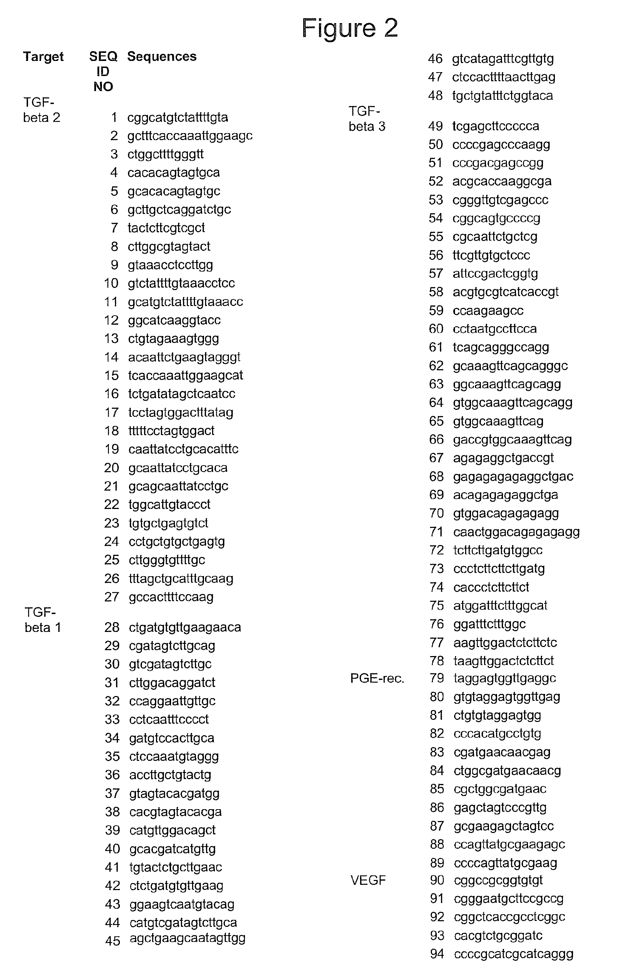

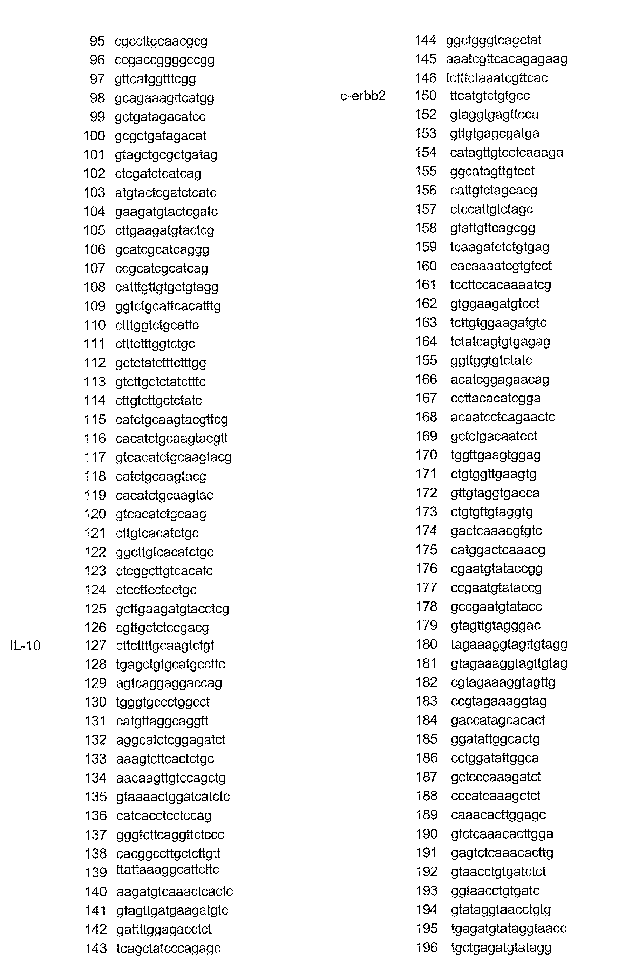

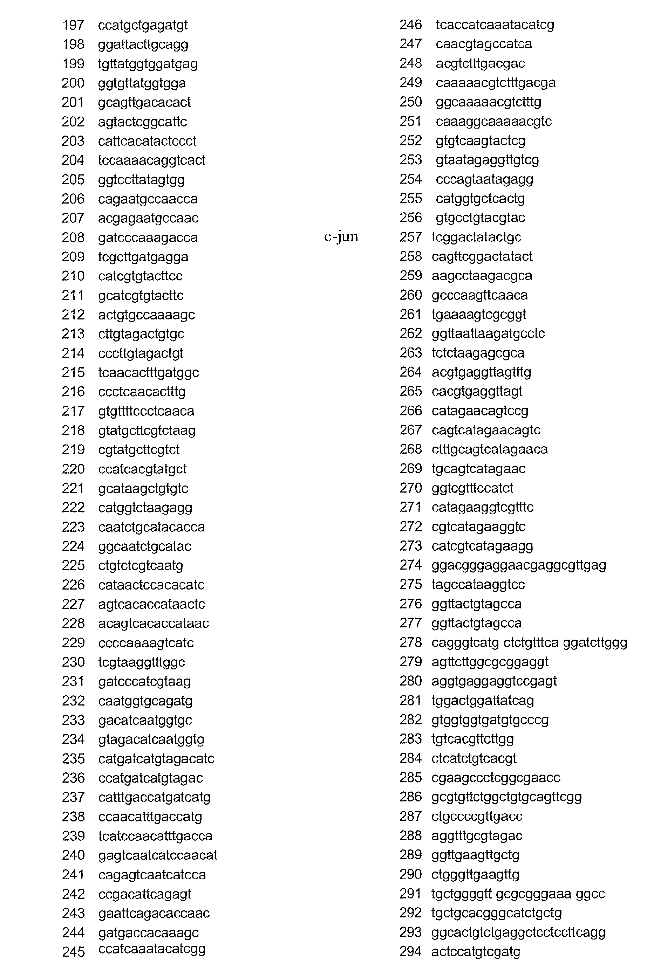

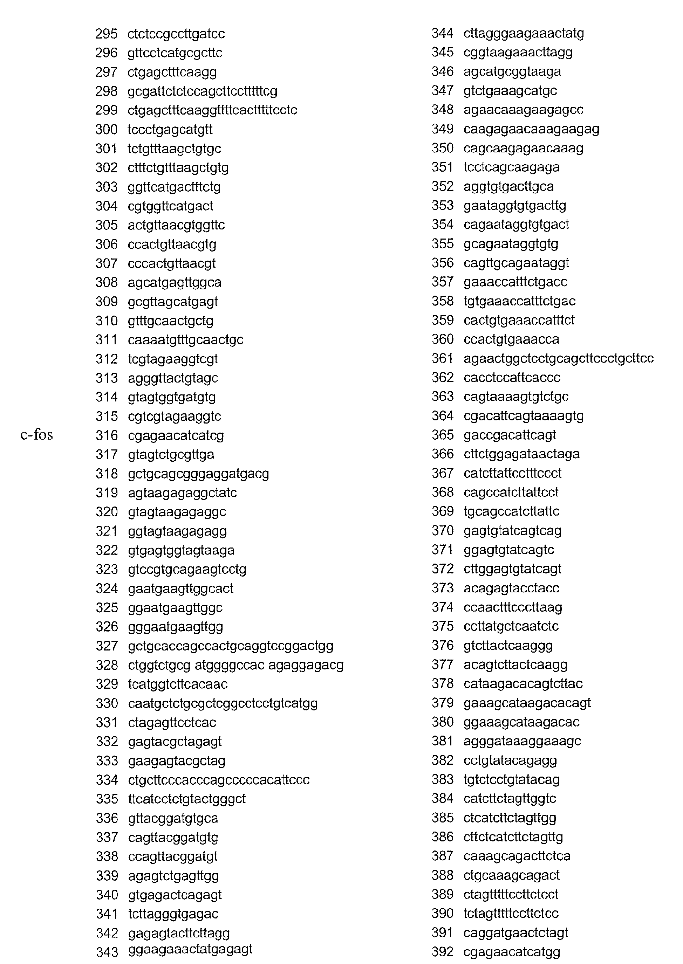

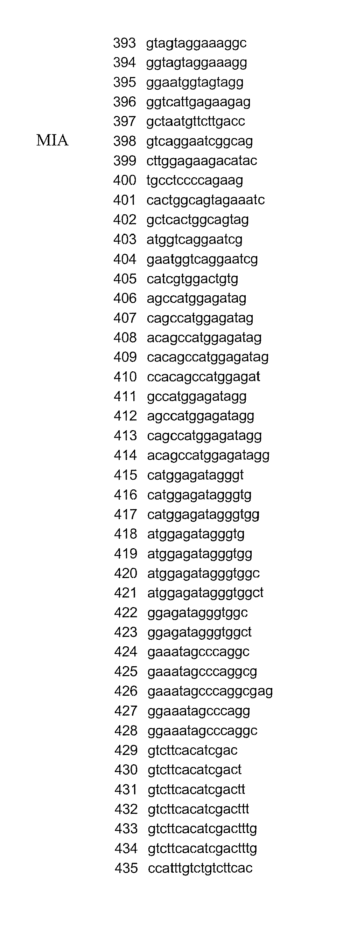

The figure depicts the inhibition of the immunosuppressor TGF-beta2 in a cell culture incubated with different concentrations of the respective immunostimulator, respectively the immunostimulator linked with at least one polymer. The experiment was performed according to the description in example 6. The results are given from the oligonucleotide with SEQ ID NO 1 phoshorothioate compared to phosphorotioate linked with at least one polyethylene glycol, reference is control. The vertical axis indicates the percentage of inhibition of suppression of the immunosuppressor TGF-beta2 (pg/Mio cells in % of untreated control). The black column (1) is the control (no inhibition), adjusted at 100 %. The striped columnes indicate the relative inhibition of TGF-beta2 by phosphorothioate of SEQ ID NO 1 compa. The white columns indicate the inhibition of TGF-beta2 by the phosphorothioate of SEQ ID NO 1 linked with at least one polyethylene glycol. Concentrations and weight of the linked polymers are shown in the table below. It can be clearly seen that the oligonucleotide linked with at least on polymer shows increased inhibition of TGF-beta2 compared to an oligonucleotide not linked to a polymer.

| Column | Colour of the column | Oligonucleotide (phosphorothioate) | linked polymer*) | concentration | TGF-beta2 [%] |
|---|---|---|---|---|---|
| 1 | Black | --- | --- | --- | 100 |
| 2 | striped | SEQ ID NO1 | --- | 0.2 microM | 97.1 |
| 2 | white | SEQ ID NO1 | PEG 400 | 0.2 microM | 78.8 |
| 3 | striped | SEQ ID NO1 | --- | 1.0 microM | 84.0 |
| 3 | white | SEQ ID NO1 | PEG 400 | 1.0 microM | 60.0 |
| 4 | striped | SEQ ID NO1 | --- | 0.2 microM | 97.0 |
| 4 | white | SEQ ID NO1 | PEG 800 | 0.2 microM | 71.0 |
| 5 | striped | SEQ ID NO1 | --- | 1.0 microM | 84.0 |
| 5 | white | SEQ ID NO1 | PEG 800 | 1.0 microM | 58.0 |
| 6 | striped | SEQ ID NO1 | --- | 0.2 microM | 94.1 |
| 6 | white | SEQ ID NO1 | PEG 5000 | 0.2 microM | 86.5 |
| 7 | striped | SEQ ID NO1 | --- | 40 microM | 46.5 |
| 7 | white | SEQ ID NO1 | PEG 5000 | 40 microM | 31.6 |

| | | | | | |
|---|---|---|---|---|---|
| *) the average total weight of the linked polymer is indicated in g/mol | | | | | |

Before the invention is described in further detail, it is to be understood that the invention is not limited to the particular embodiments of the invention described below, as variations of the particular may be made and still fall within the scope of the appended claims. It is also to be understood that the terminology employed is for the purpose of describing particular embodiments, and is not intended to be limiting. Instead, the scope of the present invention will be established by the appended claims.

### Definitions

### Branched:

The at least one polymer, more preferred the polyalkylene oxide, even more preferred the polyethylen glycol, in one embodiment is linear in other embodiments it is branched. Branched means that there is at least one branching in the respective polymer comprising that there are several branches in the molecule. Branches in the polymer have the advantage that the molecule is more compact and disadvantages of long linear polymers, such as masking the oligonucleotide, are compensated.

### Cancer:

As used herein, the term cancer, or carcinoma means new and abnormal growth or formation of tissue and/or blood cells in the body of a organism also comrised by the term neoplasm. The cancers or carcinomas include, but are not limited to, solid tumors, blood born tumors such as leukemias, such as acute or chronic myelotic or lymphoblastic leukemia, tumor metastasis, benign tumors, hemangiomas, acoustic neuromas, neurofibromas, trachomas, and pyogenic granulomas, pre-malignant tumors, rheumatoid arthritis, psoriasis, astrocytoma, acoustic neuroma, blastoma, craniopharyngloma, ependymoma, Ewing's tumor, medulloblastoma, glioma, hemangloblastoma, Hodgkins-lymphoma, medullablastoma, leukaemia, mesothelioma, neuroblastoma, neurofibroma, non-Hodgkins lymphoma, pinealoma, retinoblastoma, retinoblastoma, sarcoma (including angiosarcoma, chondrosarcoma, endothelialsarcoma, fibrosarcoma, leiomyosarcoma, liposarcoma, lymphangioandotheliosarcoma, lyphangiosarcoma, melanoma, meningioma, myosarcoma, oligodendroglioma, osteogenic sarcoma, osteosarcoma), seminoma, trachomas, Wilm's tumor, or is selected from the group of bile duct carcinoma, bladder carcinoma, brain tumor, breast cancer, bronchogenic carcinoma, carcinoma of the kidney, cervical cancer, choriocarcinoma, cystadenocarcinome, embrional carcinoma, epithelial carcinoma, esophageal cancer, cervical carcinoma, colon carcinoma, colorectal carcinoma, endometrial cancer, gallbladder cancer, gastric cancer, head cancer, liver carcinoma, lung carcinoma, medullary carcinoma, neck cancer, non-small-cell bronchogenic/lung carcinoma, ovarian cancer, pancreas carcinoma, papillary carcinoma, papillary adenocarcinoma, prostata cancer, small intestine carcinoma, rectal cancer, renal cell carcinoma, skin cancer, small-cell bronchogenic/lung carcinoma, squamous cell carcinoma, sebaceous gland carcinoma, testicular carcinoma, and uterine cancer,

### Conjugate:

A conjugate or compound in the context of this application refers to an immunostimulator, more preferred an oligonucleotide linked with at least one polymer.

**PEGylating** means linking at least one polyethylene glycol (PEG) to another molecule, in the context of this invention to an immunostimulator, more preferred an oligonucleotide.

### Linkage:

A linkage also referred to as link between two molecules eg. polymer and oligonucleotide is any kind of covalent connection between at least two molecules comprising linkers.

### Linker:

Linker synonymiously also referred to as cross-linker in the context of this invention refers to any chemical substance able to bind at least two molecules, e.g. an oligonucleotide with at least one polymer. Linkers include zero length linkers, homobifunctional crosslinkers, heterobifunctional cross linkers and the like. A spacer can be understood as a linker as well.

### Nucleotide building block - modification

Oligonucleotides include oligonucleotides having non-naturally occurring oligonucleotide building blocks with similar function. Naturally occurring nucleotides as well as non-naturally occurring nucleotides, modifications of these nucleotides at the base, the sugar or the backbone as well as spacers instead of a at least one nucleotide are also referred to as nucleotide building block.

### Spacer

Spacer in the context of this invention is any molecule that connects two molecules. In some embodiments the linker used in this invention is also an spacer, but the spacer might be used additionally with a linker in the molecule. In certain embodiments the spacer substitutes at least one nucleotide builing block or parts of the nucleotide building block. In other embodiments the spacer substitutes at least one monomer of a polymer block or parts of that monomer. Compounds comprising spacers are also within the scope of this invention.

### Solid phase

In the context of this invention a solid phase is any inert material on which the polymerization of an oligonucleotide or a polymer can be performed. A preferred embodiment of a solid phase is a controlled pore glass. Controlled means that reactive groups of the solid phase are neutralized.

### Immunstimulator

An immunostimulator according to this invention is any substance inducing the function of immune cells and/or the immune system to enhanced abilities directly or indirectly reducing or inhibiting the tumor cell growth and/or inducing cell death of unwanted neoplasms in a pharmaceutical acceptable carrier.

In one embodiment the immunostimulator is selected from the group of chemokines, including but not limited to lymphotactin, interleukin 1, interleukin 2, interleukin 6, interleukin 12, interferon gamma, and/or immune cell attracting substances.

In yet another embodiment the immunostimulator is selected from the group of viruses and/or parts of viruses, including retroviruses, adenoviruses, papillomaviruses, Epstein-Barr-viruses and viruses that are non-pathogenic including Newcastle-Disease virus, Cow-pox-virus.

In another embodiment the immunostimulator is selected from the group of autologous, heterologous MHC-Molecules, molecules involved in antigen processing, molecules involved in antigen presentation, molecules involved in mediating immune cell effects, molecules involved in mediating immune cell cytotoxic effects, molecules involved in antigen transportation, co-stimulatory molecules, peptides enhancing recognition by immune cells and/or cytotoxic effects of immune cells.

In yet another embodiment the immunostimulators are peptides enhancing the recognition of unwanted neoplasms by immune cells and/or cytotoxic effects of immune cells containing one or more mutations and/or amino acid substitutions of the ras proteins, the p53 protein, the EGF-receptor protein, fusion peptides and/or fusion proteins, the retinoblastoma protein, proteins coded by oncogenes and/or protooncogenes and/or proteins coded by anti-oncogenes and/or tumor suppressor genes.

In yet another embodiment the immunostimulators are peptides enhancing the recognition of unwanted neoplasms by immune cells and/or cytotoxic effects of immune cells containing one or more mutations and/or amino acid substitutions caused by gene rearrangements and/or gene translocations.

In yet another embodiment the immunostimulators are peptides enhancing the recognition of unwanted neoplasm by immune cells and/or cytotoxic effects of immune cells derived from proteins differing in the target cell by one or more amino acids from the proteins expressed by other cells in the same organism.

In yet another preferred embodiment the immunostimulators are peptides enhancing the recognition of unwanted neoplasm by immune cells and/or cytotoxic effects of immune cells derived from viral antigens and/or coded by viral nucleic acids.

In yet another embodiment the immunostimulators are peptides derived from proteins expressed in a diseased organ but not in the nervous system, muscle, hematopoetic system or other organs essential for survival. Diseased organs are e.g. prostate, ovary, breast, melanine producing cells and the like.

In yet another embodiment the immunostimulator is a peptide containing one or more amino acids differing between a protein in the target cell from the other cells within an organism, tumor cell extracts, tumor cell lysates and/or adjuvants.

In yet another embodiment the immunostimulator is fusion cell of dendritic and tumor cells or is dendritic cells. These fusion cells are hybridoma cells derived from a mixture of dendritic cells and tumor cells. Dendritic cells are generated e.g. by treatment of PBMC with GM-CSF and IL-4 or a mixture of GM-CSF, IL-4 and IFN-γ or FLT-3 ligand. Fusion of dendritic cells with tumor cells can be achieved e.g. using PEG (polyethylene glycol) or electrofusion (Hayashi, T., et al. 2002, Parkhust, M.R. 2003, Phan, V. 2003).

In yet another preferred embodiment the immunostimulator is an antagonist of factors negatively influencing the function of the immune system. These factors are e.g. TGF-beta (transorming growth factor beta), VEGF (vascular endothelial growth factor), PGE₂ (prostaglandin E₂), IL-10 (interleukin 10).

In yet another embodiment the immunostimulator is a vaccine.

Vaccines according to this invention comprise but are not limited to substance in a pharmaceutical acceptable carrier selected from the group of whole (irradiated) tumor cells, ASI (active specific immunization) with e.g. Newcastle Disease Virus (NDV) modified tumor cell vaccine (Schneider, T. et al. 2001), tumor cell lysates.

In one preferred embodiment the vaccines are peptides, peptides combined with cytokines (e.g. IL-2, IL-12, GM-CSF) or peptides combined with adjuvants (e.g. incomplete Freund's adjuvant, QS21).

In yet another embodiment of vaccination recombinant virus constructs that encode carcinoma antigen(s) are part of e.g. adenovirus, vaccinia, fowlpox and/or avipox.

In yet another embodiment the vaccine is naked DNA encoding carcinoma antigen(s).

In yet another embodiment the vaccines are dendritic cells, dendritic cells loaded with peptides derived from carcinoma antigens, dendritic cells transfected with recombinant viruses or RNA, DNA and/or cDNA encoding different tumor antigens, dendritic cells pulsed with tumor lysates and/or dendritic cells fused with whole tumor cells. For further vaccines see also Jager, E. et al. 2003.

In a preferred embodiment of this invention the immunostimulator is an antagonist of factors negatively influencing the function of the immune system.

An antagonist as used herein is any substance inhibiting the production of e.g. a cytokine and/or the effect of cytokines. Examples for cytokines negatively influencing the immune systems are e.g. TGF-beta, VEGF, IL-10, PGE-E₂. The inhibition in one embodiment works by binding the cytokine to a binding protein, to a receptor or to a part of this receptor, by binding the cytokine with an antibody, a low molecular substance inhibiting the cytokine or its production, or by inhibiting the signal pathway of said cytokine, e.g. by inhibiting the receptors of these cytokines or any other link downstream in the activation cascade of cytokines.

More details are given for the preferred embodiment of TGF-beta antagonists, which can be transferred to the cytokines described above as well.

Antagonist of the immune system as used herein is any substance or method inhibiting the activity of the immune system.

"Low molecular substances" or "small molecules" herein comprise substances with a molecular weight of less than about 10 kg/mol and more than about 1 g/mol of organic or anorganic origin.

In a preferred embodiment the immunostimulator of the pharmaceutical composition of this invention is a TGF-beta antagonist.

TGF-beta (transorming growth factor beta) in the context of this invention comprises all subclasses of TGF-beta, preferred subclasses are TGF-beta 1, TGF-beta 2 and TGF-beta 3.

In the context of this invention a TGF-beta antagonist is any substance inhibiting the function of TGF-beta in the meaning that any effect that is induced by TGF-beta is inhibited.

In preferred embodiments TGF-beta antagonists are substances inhibiting the production of TGF-beta, are substances binding TGF-beta and/or are substances in habiting the function of TGF-beta downstream its activation cascade. For more details about TGF-beta antagonists see also Wojtowicz-Praga (2003) herein incorporated by reference. Examples for TGF-beta antagonists are given in Example 7.

In one embodiment of TGF-beta antagonists inhibiting the production of TGF-beta are oligonucleotides and/or their active derivatives hybridising with an area of the messenger RNA (mRNA) of TGF-beta and/or the DNA encoding TGF-beta and by this inhibit the production of TGF-beta.

In yet another embodiment the substance inhibiting the production of TGF-beta is a peptide, a peptide of less than 100 kg/mol, peptides being part of TGF-beta, a protein, a protein that is not an antibody, and/or a small molecule, e.g. tranilast (N-[3,4-dimethoxycinnamoyl]-anthranilic acid) (Wilkenson, K.A. 2000).

In one embodiment the peptides being part of TGF-beta are amino acid sequences of TGF-beta 1, TGF-beta2 and TGF-beta3 also published in Mittl (1996) herein incorporated by reference.

In one preferred embodiment peptides comprise the 112 amino acids starting counting from the end of the TGF-beta1, TGF-beta2 or TGF-beta 3 peptide. The start of those peptides is after the RXXR motif ending 113 amino acid before the end of the TGF-beta1, TGF-beta2 or TGF-beta3 peptide, in which R is the amino acid arginin and XX represents any amino acid or is even no amino acid.

In yet other embodiments peptides being part of TGF-beta are part of these sequences presented in example 8 comprising one to all amino acids of this peptide, in other embodiments preferred peptides comprise about 1-100 amino acids, about 2-50 amino acids, about 3-30 amino acids or about 5-20 amino acids ot those peptides.

In yet other embodiments preferred amino acids are those presented in example 8 for TGF-beta1, TGF-beta2 and TGF-beta3 with the respective numbers 1-21.

Further preferred embodiments are parts of amino acids as described above with about 1-50 amino acids, about 1-40, about 2-30, about 3-25, about 4-18, about 5-15 or about 6-12 amino acids.

In yet other embodiments of the peptides described above at least one of the basic amino acid selected from the group of histidin (H), lysin (K) and/or arginine (R) is substituted by another basic amino acid selected from this group without loosing its TGF-beta antagonizing effects.

In yet other embodiments of the peptides described above at least one of the acid amino acid selected from the group of glutaminic acid (E) and/or asparaginic acid (D) is substituted by its counterpart of this group without loosing its TGF-beta antagonizing effects.

The peptides that are part of TGF-beta wherein some amino acids are replaced conservatively compared to their sequences presented in example 8 are also referred to as analogs of TGF-beta1, TGf-beta2 and/or TGF-beta3.

In some embodiments in the analogs of TGF beta1, TGF-beta2 and TGF-beta3 1 to about 30 %, about 2% to about 20%, about 3 % to about 15%, 4 % to about 12 % or about 5 % to about 10 % of the amino acids are replaced conservatively.

Amino acid replaced conservatively, also referred to as conservative analogs or active derivatives of peptides in the context of this invention means replacing at least one amino acid of a peptide or protein. Preferably at least one acid amino acid (glutaminic acid (E), asparaginic acid (D)) is replaced by the respective other acid amino acid, accordingly at least one basic amino acids is replaced by another basic amino acid, at least one amino acid with a polar group (-OH, -SH, -CONH₂) is replaced by another amino acid with a polar group and/or amino acids with pure carbon side chains are replaced by another amino acid with pure carbone side chain. Peptides and/or proteins conservatively replaced with amino acids are still in the scope of this invention.

In another embodiment the peptides described above are single and not in the combination with a chemotherapeutic agent. In yet another embodiment these peptides are used for preparing a pharmaceutical composition with a pharmaceutically acceptable carrier. In yet another embodiment these peptides are comprised by a pharmaceutical composition for the treatment of neoplasms and in yet another embodiment these peptides are used for a method treating neoplasms according to this invention, more preferred glioma, astrocytoma and/or glioblastoma.

In yet another embodiment TGF-beta antagonists are receptors and/or parts of it binding TGF-beta and in that way inhibiting the function of TGF-beta.

In yet another embodiment the TGF-beta antagonist is an antibody and/or parts of it binding TGF-beta and by this inhibiting the function of TGF-beta. Those antibodies are commercially available, see e.g. R & D Systems, Inc. The production of those antibodies is well known in the art. Animals such as e.g. chicken, mice, rabbits, goats, are immunized with purified human TGF-beta. IgY then is purified with e.g. affinity chromatography as described for example by Cooper, H.M. (1995). In yet other embodiments the TGF-beta antibodies are further modified e.g. biotinylated.

In a more preferred embodiment the TGF-beta antibodies are humanized antibodies. For more details about humanized antibodies see also Carrington (1998).

In yet another embodiment the TGF-beta antagonist is a protein and/or peptide binding to TGF-beta and by this inhibiting the function of TGF-beta. Preferred embodiments of these peptides are e.g. Latency-assosciated peptides and can inhibit all three isoforms of TGF-beta (TGF-beta 1, TGF-beta 2 and TGF-beta 3).

In another embodiment the TGF-beta inhibitor is a protein, peptide or a small molecule inhibiting the function of the TGF-beta receptor, acting extracellularly or intracellularly.

In yet other embodiments the TGF-beta antagonists comprise, proteins, peptides, antibodies and/or small molecules, which inhibit the TGF-beta activity by inhibiting any link downstream the TGF-beta cascade of activation.

In a preferred embodiment of this invention the antagonists of a peptide, cytokine and/or receptor is an oligonucleotide according to this invention.

In one embodiment the immunstimulator is an oligonucleotide, that is linked with at least one polymer, more preferred polyalkylene oxide, most preferred with polyethylene glycol.

Yet another aspect of this invention is a compound comprising an oligonucleotide linked with at least one polymer as described herein.

The conjugation with the polymer is anywhere within the molecule, preferably the linkage is at the 3'and/or 5' end of the oligonucleotide. In a preferred embodiment, polymers of same or different kind of weight are linked to the 3' and the 5' end of the oligonucleotide. The term 3' respectively 5' end refers to the carbon atom of the sugar moiety of the nucleotide building block. If the sugar is substituted by another molecule this term is used in an analogous way, which means that it is looked upon the nucleotide building block in that way that there would be a sugar moiety. Further possibilities for linking the at least one polymer with the oligonucleotide are for example linking it to the phosphorodiester group, to any other O-atom of the sugar portion or active group of the respective substitute. In other embodiments the polymer is linked with an active group of the base, its modification, its substitute or the respective spacer. The polymer can also be linked to a spacer within the compound.

One compound can comprise one or several polymers linked to the oligonucleotide. The linkage of several polymers can e.g. induce better pharmacological activity, less side effects, better cellular uptake, improve hybridization, improve halfe-time, and/or reduce toxicity.

The oligonucleotide linked with the at least one polymer has a length from about 8 to about 30 nucleotide building blocks. Preferred oligonucleotides are oligonucleotides that hybridize with mRNA coding for molecules relevant in the process of inhibiting the synthesis and/or the function of molecules suppressing and/or downregulating and/or negatively affecting the immune response.

In preferred embodiments these molecules are TGF-beta1, TGF-beta2, TGF-beta3, VEGF, interleukin-10, c-jun, c-fos, c-erbB2, their respective receptors and/or the prostaglandin E2 receptor. Further preferred embodiments of oligonucleotides are given in the sequence listing, in the examples 6 and 7 or are oligonucleotides published to in WO 94/25588, WO 95/17507, WO 95/02051, WO 98/33904, WO 99/63975, WO 01/68146, WO 01/68122, WO 03/06445, WO 2005/014812, WO 2005/059133, WO 2005/084712 herein incorporated by reference.

In the context of this invention, the term "oligonucleotide" refers to an oligomer of ribonucleic acid (RNA) or deoxyribonucleic acid (DNA) or mimetics thereof also referred to as nucleotide building block-polymers.

The oligonucleotide preferably comprises from 8 to 30 nucleotide building blocks, more preferably 12 to 28 nucleotide building blocks, even more preferred 16 to 26 nucleotide building blocks, most preferred 18, 20 or 22 nucleotide building blocks.

The term nucleotide building block comprises nucleotides composed of naturally-occuring nucleobases, sugars and covalent internucleoside (backbone) linkages, each of these also referred to as portions, as well as oligonucleotides having non-naturallly-occuring portions which function similarly, e.g. hybridizing with the same mRNA of a selected target. In one embodiment the base is modified or substituted by a similar molecule. Similar bases are those molecules that are also able to support the hybridization to the mRNA or at least do not affect the hybridization in a negative way. In some embodiments at least one base portion is substituted with a spacer.

In other embodiments the sugar moiety of the nucleotide building block is modified or substituted by another molecule with comparable chemical and physical properties. Examples for suggars are arabinose, xylose, lyxose, allose, altorse, glucose, mannnse, gulose, idose, galactose, talose or stabilized modifications of those sugars. In some embodiments the sugar is substituted by a spacer.

In other embodiments the internucleoside linkage, also referred to as linkage between two nucleotide building blocks is not an phosphorodiester but another molecule

Such oligonucleotides with at least one modified nucleotide building block are often preferred over native forms because of desirable properties such as enhanced cellular uptake, enhanced affinity for nucleic acid target, increased stability in the presence of nucleases and/or enhanced therapeutic effectiveness.

Preferred oligonucleotides that are linked with at least one polymer are those defined in the sequence listing with SEQ ID NO 1 to 435 or are those of examples 7 or 8.

Especially preferred are SEQ ID NO. 1, 2, 3, 28 and 37.

In one embodiment the nucleotide building block include for example sugar moieties that are covalently attached to low molecular weight organic groups other than a hydroxyl group at the 3' and/or 2' position and other than a phosphate group at the 5' position. Thus modified nucleic acids may include a 2'-O-alkylated sugar more preferred ribose group. The alkyl group can be describes as R1-R2. R1 is an alkyl with 1 to 20 carbon atoms and R2 is O-alkyl, S-alkyl, NH-alkyl, N-dialkyl, O-aryl, S-aryl, NH-aryl, O-aralkyl, S-aralkyl, NH-aralky. Preferred embodiments of 2'-O-alkyl groups are methoxy-, ethoxy-, propyloxy-, isopropyloxy-, methoxy-ethoxy or any other combined alky groups.

In yet other embodiments the oligonucleotide is a "chimeric" oligonucleotide. In the context of this invention chimeric oligonucleotides are oligonucleotides, which contain at least one chemically region wherein at least one portion of the nucleotide building block is modified. These oligonucleotides show for example increased resistance to nuclease degradation, increased cellular uptake, and/or increased binding affinity for the target nucleic acid.

An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Cleavage of the RNA target can be routinely detected by gel electrophoresis and, if necessary, associated nucleic acid hybridization techniques known in the art.

Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers. Representative United States patents that teach the preparation of such hybrid structures include, but are not limited to, U.S. Pat. Nos.: 5,013,830; 5,149,797; 5,220,007; 5,256,775; 5,366,878; 5,403,711; 5,491,133; 5,565,350; 5,623,065; 5,652,355; 5,652,356; and 5,700,922, each of which is herein incorporated by reference.

The nucleic acid molecules of the invention may include naturally-occurring or synthetic purine or pyrimidine heterocyclic bases. Purine or pyrimidine heterocyclic bases include, but are not limited to adenine, guanine, cytosine, thymidine, uracil, and inosine. Other representative heterocyclic bases are disclosed in U.S. Pat. No. 3,687,808. The terms "purines" or "pyrimidines" or "bases" are used herein to refer to both naturally-occurring or synthetic purines, pyrimidines or bases.

In other embodiment the oligonucleotides include non-ionic DNA analogs, such as alkyl- and arylphosphates (in which the charged phosphonate oxygen is replaced by an alkyl or aryl group), phosphodiester and alkylphosphotriesters, in which the charged oxygen moiety is alkylated. Nucleic acids which contain diol, such as tetraethyleneglycol or hexaethyleneglycol, at either or both termini have also been shown to be substantially resistant to nuclease degradation.

In yet another embodiment the base units are maintained for hybridization with an appropriate nucleic acid target compound. One such oligomeric compound, an oligonucleotide that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are bound directly or indirectly to aza nitrogen atoms of the amide portion of the backbone. Representative United States patents that teach the preparation of PNA compounds include, but are not limited to, U.S. Pat. Nos.: 5,539,082, 5,714,331, and 5,719,262, each of which is herein incorporated by reference. Further teaching of PNA compounds can be found in Nielsen et al. (1994).

In other embodiments the deoxyribose phosphate backbone is replaced with an achiral polyamide backbone. For example thymine-linked aminothioglycol units are prepared. The hybrids of these oligonucleotides have a very high stability compared to underivated oligonucleotides. For more details see Nielsen (1991).

In some embodiments at least one nucleotide building block of an oligonucleotide is modified as described in one of the modifications above. The modification can either cover the oligonucleotide continuously or irregularly.

In yet another embodiment at least two modifications as described above are combined within one oligonucleotide.

In yet other embodiments the compounds are complexed to biological or chemical carriers or are coupled to tissue-type or cell-type directed ligands or antibodies.

In some embodiments of the invention the active modifications of the compound comprises oligonucleotides as mentioned herein that have additional nucleotide building blocks. These nucleotide building blocks are chosen in the way, that they support the hybridization with the target region or at least do not influence the hybridization negatively. Those compounds with the respective antisense structure of the mRNA of said targets are still within the scope of this invention. The additionally nucleotides in one embodiment are according to the coding region of the mRNA, in yet another embodiment the additional nucleotides are also from the non coding part of the mRNA, including introns and exons. The additionally nucleotides can comprise from about 1 to about 10,000 nucleotides, from about 1 to about 5,000 nucleotides, from about 1 to about 3000 nucleotides, from about 1 to about 1,000 nucleotides, from about 1 to about 500 nucleotides, from about 1 to about 100 nucleotides, from about 1 to about 50 nucleotides, from about 1 to about 25 nucleotides, from about 1 to about 10 nucleotides, from about 1 to about 5 nucleotides or from about 1 to about 2 nucleotides bound to at least one of the 3' and/or 5' end, in another embodiment on at least one of the 2' or 5'end. In yet another embodiment some nucleotide building blocks of those oligonucleotides respectively polynucleotides may be modified or substituted by spacers as described herein.

For more details for derivatisation see for example Gualtieri (2000), R. Schlingensiepen (1997) or Hecht (1997) herein incorporated by reference.

Polymers in the context of this application comprise biocompatible materials, such as polyalkylen oxides, more preferred polyethylene glycol, biodegradable lactone-based polymers, e.g. polyacrylic acid, polylactide acid (PLA), poly(glycolic acid) (PGA), polypropylene, polystyrene, polyolefin, polyamide, polycyanoacrylate, polyimide, polyethylenterephthalat (PET, PETG), polyethylene terephthalate (PETE), polytetramethylene glycol (PTG), or polyurethane as well as mixtures thereof. Mixture refers to the use of different polymers within the same compound as well as it refers to block copolymers. Block copolymers are polymers wherein at least one section of a polymer is build up from monomers of another polymer.

Selection of such materials depends on a number of factors including the stability and toxicity of the polymer.

In one embodiment of an immunostimulator linked with at least one polymer, more preferred polyalkylen oxid, even more preferred polyethylene glycol has an average weight of from about 0.05 kg/mol to about 50 kg/mol, in a more preferred embodiment from about 0.05 kg/mol to about 5 kg/mol, or from about 5 kg/mol to about 20 kg/mol or from about 20 kg/mol to about 50 kg/mol. In other embodiments the polymer has a weight of about 0.05 kg/mol to about 1 kg/mol, from about 1 kg/mol to about 10 kg/mol, from about 10 kg/mol to about 25 kg/mol, from about 25 kg/mol to about 50 kg/mol. In yet other embodiments the polymer has ab weight of from about 0.05 kg/mol to about 0.15 kg/mol, about 0.15 kg/mol to about 0.25 kg/mol, about 0.25 kg/mol to about 0.35 kg/mol, about 0.35 kg/mol to about 0.45 kg/mol, about 0.45 kg/mol to about 0.55 kg/mol, about 0.55 kg/mol to about 0.65 kg/mol, about 0.65 kg/mol to about about 0.75 kg/mol, about 0.75 kg/mol to about 0.85 kg/mol, about 0.85 kg/mol to about 0.95 kg/mol, about 0.95 kg/mol to about 1.05 kg/mol, about 1.05 kg/mol to about 1150 kg/mol, about 1.15 kg/mol to about 1.25 kg/mol, about 1.25 kg/mol to about1.55 kg/mol, about 1.55 kg/mol to about 1.85 kg/mol, about 1.85 kg/mol to about 2.15 kg/mol, about 2.15 kg/mol to about 2.45 kg/mol, about 2.45 kg/mol to about 2.75 kg/mol, about 2.75 kg/mol to about 3.05 kg/mol, about 3.05 kg/mol to about 3.35 kg/mol, about 3.35 kg/mol to about 3.65 kg/mol, about 3.65 kg/mol to about 4.3 kg/mol, about 4.3 kg/mol to about 4.6 kg/mol, about 4.6 kg/mol to about 5.5 kg/mol, 5.5 kg/mol to about 6.5 kg/mol, 6.5 kg/mol to about 7.5 kg/mol, 7.5 kg/mol to about 8.5 kg/mol, 8.5 kg/mol to about 9.5 kg/mol, 9.5 kg/mol to about 10.5 kg/mol, 10.5 kg/mol to about 11.5 kg/mol, 11.5 kg/mol to about 12.5 kg/mol, 12.5 kg/mol to about 13.5 kg/mol, 13.5 kg/mol to about 14.5 kg/mol, 14.5 kg/mol to about 15.5 kg/mol, 15.5 kg/mol to about 16.5 kg/mol, 16.5 kg/mol to about 17.5 kg/mol, 17.5 kg/mol to about 18.5 kg/mol, 18.5 kg/mol to about 19.5 kg/mol, 19.5 kg/mol to about 20.5 kg/mol, 20.5 kg/mol to about 21.5 kg/mol, 21.5 kg/mol to about 22.5 kg/mol, 22.5 kg/mol to about 23.5 kg/mol, 23.5 kg/mol to about 24.5 kg/mol, 24.5 kg/mol to about 25.5 kg/mol, 25.5 kg/mol to about 26.5 kg/mol, 26.5 kg/mol to about 27.5 kg/mol, 27.5 kg/mol to about 28.5 kg/mol, 28.5 kg/mol to about 29.5 kg/mol, 29.5 kg/mol to about 30.5 kg/mol, 30.5 kg/mol to about 31.5 kg/mol, 30.5 kg/mol to about 31.5 kg/mol, 30.5 kg/mol to about 31.5 kg/mol, 31.5 kg/mol to about 32.5 kg/mol, 32.5 kg/mol to about 33.5 kg/mol, 33.5 kg/mol to about 34.5 kg/mol, 34.5 kg/mol to about 35.5 kg/mol, 35.5 kg/mol to about 36.5 kg/mol, 36.5 kg/mol to about 36.5 kg/mol, 36.5 kg/mol to about 37.5 kg/mol, 37.5 kg/mol to about 38.5 kg/mol, 38.5 kg/mol to about 39.5 kg/mol, 39.5 kg/mol to about 40.5 kg/mol, 40.5 kg/mol to about 41.5 kg/mol, 41.5 kg/mol to about 42.5 kg/mol, 42.5 kg/mol to about 43.5 kg/mol, 43.5 kg/mol to about 44.5 kg/mol, 44.5 kg/mol to about 45.5 kg/mol, 45.5 kg/mol to about 46.5 kg/mol, 46.5 kg/mol to about 46.5 kg/mol, 46.5 kg/mol to about 47.5 kg/mol, 47.5 kg/mol to about 48.5 kg/mol, 48.5 kg/mol to about 49.5 kg/mol, 49.5 kg/mol to about 50.5 kg/mol.

In further preferred embodiments the average molecular weight of the polymer is about 0.1 kg/mol, about 0.2 kg/mol, about 0.3 kg/mol, about 0.4 kg/mol, about 0.5 kg/mol, about 0.6 kg/mol, about 0.7 kg/mol, about 0.8 kg/mol, about 0.9 kg/mol, about 1 kg/mol, about 1.1 kg/mol, about 1.2 kg/mol, about 1.3 kg/mol, about 1.4 kg/mol, about 1.5 kg/mol, about 1.5 kg/mol, about 1.6 kg/mol, about 1.7 kg/mol, about 1.8 kg/mol, about 1.9 kg/mol, about 2 kg/mol, about 2.2 kg/mol, about 2.4 kg/mol, about 2.6 kg/mol, about 2.8 kg/mol, about 3 kg/mol about 3.1 kg/mol, about 3.2 kg/mol, about 3.3 kg/mol, about 3.4 kg/mol, about 3.5 kg/mol, about 3.6 kg/mol, about 3.7 kg/mol, about 3.8 kg/mol, about 3.9 kg/mol, about 4 kg/mol, about 4.1 kg/mol, about 4.2 kg/mol, about 4.3 kg/mol, about 4.4 kg/mol, about 4.5 kg/mol, about 4.6 kg/mol, about 4.7 kg/mol, about 4.8 kg/mol, about 4.9 kg/mol, about 5 kg/mol about 5.5 kg/mol, about 6 kg/mol, about 6.5 kg/mol, about 7 kg/mol, about 7.5 kg/mol, about 8 kg/mol, about 8.5 kg/mol, about 9 kg/mol, about 9.5 kg/mol, about 10 kg/mol, about 11 kg/mol, about 12 kg/mol, about 13 kg/mol, about 14 kg/mol, about 15 kg/mol, about 16 kg/mol, about 17 kg/mol, about 18 kg/mol, about 19 kg/mol, about 20 kg/mol, about 21 kg/mol, about 22 kg/mol, about 23 kg/mol, about 24 kg/mol, about 25 kg/mol, about 26 kg/mol, about 27 kg/mol, about 28 kg/mol, about 29 kg/mol, about 30 kg/mol, about 31 kg/mol, about 32 kg/mol, about 33 kg/mol, about 34 kg/mol, about 35 kg/mol, about 36 kg/mol, about 37 kg/mol, about 38 kg/mol, about 39 kg/mol, about 40 kg/mol, about 41 kg/mol, about 42 kg/mol, about 43 kg/mol, about 44 kg/mol, about 45 kg/mol, about 46 kg/mol, about 47 kg/mol, about 48 kg/mol, about 49 kg/mol, about 50 kg/mol.

The term about referes to the fact, that commercially available polymers are not completely homogenous in weight, but are fractions of polymers with about a certain weight not defined accurately by the mass, but differes around this mentioned average mass/weight dependent of the absolute mass and according to the way of synthesis and/or purification. The average molecular weight refers to the weight-average molecular weight.

In one embodiment one of two polymers is linked to the 3'-end and the second polymer is linked to the 5'-end or the respective portion of the nucleotide building block of the oligonucleotide.

In a further preferred embodiment the polymer linked to the 3'-end has a higher weight compared with the polymer linked to the 5'-end. In a further preferred embodiment the 3'-polymer has the 1.5 to 100 fold weight of the polymer linked to the 5'-end.

In another preferred embodiment the polymer linked to the 5'-end has a higher weight compared with the polymer linked to the 3'-end. In a further preferred embodiment the 3'-polymer has the 1.5 to 100 fold weight of the polymer linked to the 5'-end.

In further preferred embodiments the weight ratio of the polymers is 1 : 1.5, 1 : 2, 1 : 2,5, 1 : 3"1:3.5,1:4,1:4.5,1:5,1:5.5,1:6,1:7,1:7.5,1:8,1:8.5,1:9,1:9.5,1:10,1: 11, 1 : 12 , 1: 13, 1 : 14, 1 : 15, 1 : 16, 1 : 17, 1 :18, 1: 19, 1: 20, 1 :22, 1 : 24. 1 : 26, 1 : 28, 1 : 30, 1 : 32, 1 : 34, 1 : 36, 1 : 38, 1 : 40, 1 : 42, 1 : 44, 1 : 46, 1 : 48, 1 :50, 1: 55, 1 : 60, 1 : 65, 1 : 70, 1 : 75, 1 :80, 1 : 85, 1 : 90, 1 : 95, 1 : 100.
In further preferred embodiments the smaller polymer has an average molecular weigth of 0.4 kg/mol, 0,6 kg/mol, or 0,8 kg/mol and the other polymer has an average weight of 0.8 kg/mol, 1 kg/mol, 1,2 kg/mol, 1,4 kg/mol, 1,6 kg/mol, 1,8 kg/mol, 2 kg/mol, 3 kg/mol, 4 kg/mol or 5 kg/mol.

In one embodiment the at least one polymer of the conjugate is linear (single stranded). In yet another embodiment the polymer is branched. There can be one branch or several branches in the polymer, the polymer in one embodiment is polyoxyalkylene, more preferred polyethylene glycol.

The linkage between the oligonucleotide and the at least one polymer is by at least one linker. In some embodiments linkers are sensitive to enzymes, pH value and/or other parameters, which allow the oligonuclotide to be split of the polymer under specific conditions. Linkers are known in the art. For more details see also Hermanson (1996). Examples for homobifunctional linkers are Lomant's reagent dithiobis (succinimidyl-propionate) DSP, 3'3'-dithiobis(sulfosuccinimidyl proprionate (DTSSP), disuccinimidyl suberate (DSS), bis(sulfosuccinimidyl)suberate (BS), disuccinimidyl tartrate (DST), disulfosuccinimidyl tartrate (sulfo DST), ethylene glycobis(succinimidylsuccinate) (EGS), disuccinimidyl glutarate (DSG), N, N'-disuccinimidyl carbonate (DSC), dimethyl adipimidate (DMA), dimethyl pimelimidate (DMP), dimethyl suberimidate (DMS), dimethy-3,3'-dithiobispropionimidate (DTBP), 1,4-di-3'-(2'-pyridyldithio)propionamido)butane (DPDPB), bismaleimidohexane (BMH), aryl halide-containing compound (DFDNB, such as e.g. 1,5-difluoro-2,4-dinitrobenzene or 1,3-difluoro-4,6-dinitrobenzene, 4,4'-difluoro-3,3'-dinitrophenylsulfone (DFDNPS), bis-[β-(4-azidosalicylamido)ethyl]disulfide (BASED), formaldehyde, glutaraldehyde, 1,4-butanediol diglycidyl ether, adipic acid dihydrazide, carbohydrazide, o-toluidine, 3,3'-dimethylbenzidine, benzidine, α, α'-p-diaminodiphenyl, diiodo-p-xylene sulfonic acid, N,N'-ethylene-bis(iodoacetamide), N,N'-hexamethylene-bis(iodoacetamide).

The linkage with heterobifunctional liners is preferred since the linkage can be better controlled. Examples for heterobifunctional linkers are amine-reactive and sulfhydryl cross-linkers such as N-succinimidyl 3-(2-pyridyldithio)propionate (sPDP), long-chain N-succinimidyl 3-(2-pyridyldithio)propionate (LC-sPDP), water-soluble- long-chain N-succinimidyl 3-(2-pyridyldithio) propionate (sulfo-LC-sPDP), succinimidyloxycarbonyl-α-methyl-α-(2-pyridyldithio)toluene (sMPT), sulfosuccinimidyl-6-[α-methyl-α-( 2-pyridyldithio)toluamido]hexanoate (sulfo-LC-sMPT), succinimidyl-4-(N-maleimidomethyl)cyclohexane-1-carboxylate (sMCC), sulfosuccinimidyl-4-(N-maleimidomethyl)cyclohexane-1-carboxylate (sulfo-sMCC), m-maleimidobenzoyl-N-hydroxysuccinimide ester (MBs), m-maleimidobenzoyl-N-hydroxysulfosuccinimide ester (sulfo-MBs), N-succinimidyl(4-iodoacteyl)aminobenzoate (slAB), sulfosuccinimidyl(4-iodoacteyl)aminobenzoate (sulfo-slAB), succinimidyl-4-(p-maleimidophenyl)butyrate (sMPB), sulfosuccinimidyl-4-(p-maleimidophenyl)butyrate (sulfo-sMPB), N-(γ-maleimidobutyryloxy)succinimide ester (GMBs), N-(γ-maleimidobutyryloxy)sulfosuccinimide ester (sulfo-GMBs), succinimidyl 6-((iodoacetyl)amino)hexanoate (sIAX), succinimidyl 6-[6-(((iodoacetyl)amino)hexanoyl)amino]hexanoate (sIAXX), succinimidyl 4-(((iodoacetyl)amino)methyl)cyclohexane-1-carboxylate (sIAC), succinimidyl 6-((((4-iodoacetyl)amino)methyl)cyclohexane-1-carbonyl)amino)hexanoate (sIACX), p-nitrophenyl iodoacetate (NPIA), carbonyl-reactive and sulfhydryl-reactive cross-linkers such as 4-(4-N-maleimidophenyl)butyric acid hydrazide (MPBH), 4-(N-maleimidomethyl)cyclohexane-1-carboxyl-hydrazide -8 (M₂C₂H), 3-(2-pyridyldithio)propionyl hydrazide (PDPH), amine-reactive and photoreactive cross-linkers such as N-hydroxysuccinimidyl-4-azidosalicylic acid (NHs-AsA), N-hydroxysulfosuccinimidyl-4-azidosalicylic acid (sulfo-NHs-AsA), sulfosuccinimidyl-(4-azidosalicylamido)hexanoate (sulfo-NHs-LC-AsA), sulfosuccinimidyl-2-(p-azidosalicylamido)ethyl-1,3'-dithiopropionate (sAsD), N-hydroxysuccinimidyl-4-azidobenzoate (HsAB), N-hydroxysulfosuccinimidyl-4-azidobenzoate (sulfo-HsAB), N-succinimidyl-6-(4'-azido-2'-nitrophenylamino)hexanoate (sANPAH), sulfosuccinimidyl-6-(4'-azido-2'-nitrophenylamino)hexanoate (sulfo-sANPAH), N-5-azido-2-nitrobenzoyloxysuccinimide (ANB-NOs), sulfosuccinimidyl-2-(m-azido-o-nitrobenzamido)-ethyl-1,3'-dithiopropionate (sAND), N-succinimidyl-4(4-azidophenyl)1,3'-dithiopropionate (sADP), N-sulfosuccinimidyl(4-azidophenyl)-1,3'-dithiopropionate (sulfo-sADP), sulfosuccinimidyl 4-(p-azidophenyl)butyrate (sulfo-sAPB), sulfosuccinimidyl 2-(7-azido-4-methylcoumarin-3-acetamide)ethyl-1,3'-dithiopropionate (sAED), sulfosuccinimidyl 7-azido-4-methylcoumain-3-acetate (sulfo-sAMCA), p-nitrophenyl diazopyruvate (pNPDP), p-nitrophenyl-2-diazo-3,3,3-trifluoropropionate (PNP-DTP), sulfhydryl-reactive and photoreactive cross-linkers such as1-(ρ-Azidosalicylamido)-4-(iodoacetamido)butane (AsIB), N-[4-(ρ-azidosalicylamido)butyl]-3'-(2'-pyridyldithio)propionamide (APDP), benzophenone-4-iodoacetamide, benzophenone-4-maleimide carbonyl-reactive and photoreactive cross-linkers such as p-azidobenzoyl hydrazide (ABH), carboxylate-reactive and photoreactive cross-linkers such as 4-(p-azidosalicylamido)butylamine (AsBA), and arginine-reactive and photoreactive cross-linkers such as p-azidophenyl glyoxal (APG).

In embodiment the polyethylene oxide is linked with the oligonucleotide using carbodiimide coupling. For more details see Hermanson (1996).

### Synthesis

The synthesis respectively purification of immunostimulators, more preferred oligonucleotides are well known to those skilled in the art. For more details see for example Gualtieri (2000), R. Schlingensiepen (1997) or Hecht (1997) herein incorporated by reference.

For use in the instant invention, the nucleic acids can be synthesized de novo using any of a number of procedures well known in the art. Such compounds are referred to as synthetic nucleic acids, for example, the cyanoethyl phosphoramidite method (Beaucage, S. L., and Caruthers, M. H., Tet. Let. 22:1859, 1981); nucleoside H-phosphonate method (Garegg et al., Tet. Let. 27:4051-4054, 1986; Froehler et al., Nucl. Acid. Res. 14: 5399-5407, 1986, Garegg et al, Tet. Let. 27:4055-4058, 1986, Gaffney et al., Tet. Let. 29: 2619-2622, 1988). These chemistries can be performed by a variety of automated oligonucleotide synthesizers available on the market.

Alternatively, nucleic acids can be produced on a large scale in plasmids, (see, e.g., Sambrook, et al., Molecular Cloning: A Laboratory Manual, Cold Spring Harbor Laboratory Press, New York, 1989) and separated into smaller pieces or administered in the whole. Nucleic acids can be prepared from existing nucleic acid sequences (e.g. genomic or cDNA) using known techniques, such as those employing restriction enzymes, exonucleases or endonucleases. Nucleic acids prepared in this manner are referred to as isolated nucleic acids. The term oligonucleotide encompasses both synthetic and isolated nucleic acids.

One preferred embodiment for the synthesis of oligonucleotide-conjugates is solid phase synthesis using a first nucleotide coupled to a solid support, e.g. controlled pore glass (CPG) and nucleotide phosphoroamidites. The 5'-hydroxygroup of the nucleotide phosphoroamidites is protected by dimethoxytrityl (DMT) and the exocyclic nitrogen atoms of the base are protected e.g. with benozoyl preferable for adenosine and cytidine, and isobutyryl preferable for guanosine. A synthesis cycyle is done as follows:
The DMT protecting group of the terminal 5'-hydroxygroup of the support- bound nucleotide chain is removed and the hydroxy function is reacted with the phosporamidite group of the added nucleotide resulting in a phosphite triester linkage. This is stabilized by oxidation to a phosphate linkage. In a preferred embodiment this oxidation is done by a sulfurizing reagent e.g. 3H-1,2-benzodithiol-3-one, 1,1-dioxide (Beaucage reagent) resulting in thiophosphate linkage. Non-reacted 5-hydroxygroups are capped to prevent synthesis of failure sequences. This procedure is repeated until the desired number of nucleotides is added.
In one embodiment the polymer is linked to the 5'-terminus of the oligonucleotide by using a DMT-protected phosphoramidite-derivative of the polymer as educt in the last synthesis cycle. Cleaving from the support results in deprotection of the bases and gives the 5'-DMT-protected oligonucleotide or conjugate respectively. This DMT-on product preferably is purified before detritylation.
In yet another embodiment at least one polymer is linked to the oligonucleotide in solution. The linkage of at least one polymer with the oligonucleotide can be done by activating certain groups of the oligonucleotide and/or the polymer and then linking those molecules. Preferred for coupling of the polymer are the 3' and/or the 5'- end of the oligonucleotide. In yet other embodiments at least one polymer is coupled to another part of the oligonucleotide. In some embodiments this part of the oligonucleotide is the phosphate-group, in other embodiments the polymer is coupled to the 2'-O of the sugar moiety of the nucleotide or the respective moiety of the nucleotide building block. In yet other embodiments the polymer is coupled to an activated group of the base of the nucleotide. In another embodiment instead of at least one nucleotide a spacer is used. The at least one polymer can be coupled to this spacer as well. Several polymers can be coupled to the oligonucleotide at different sites of the above described parts of the olignucleotide.
Polymers are synthesized by polymerisation of the respective monomers or by natural sources. Most of them are commercially available or can be achieved by standard synthesis, see for example Braun (2005).
Covalent attachment of a polymer, more preferred polyoxyalkylene, even more preferred polyethylene glycol to an oligonucleotide is accomplished by known chemical synthesis techniques. For more details see Hermanson (1996).
Linking the immunostimulator with at least one polymer is achieved by the reactivity of the active groups of those molecules such as COOH, CHO, OH, NH₂, SH etc. In general one functional group is activated and then linked with another active group.
In some preferred embodiments the selective derivatisation of preferred functional groups is achieved by protecting other functional groups that should not be linked with protecting groups. After linkage of the immunostimulator with the at least one polymer those protection groups are removed.
In some embodiments the linkage of the immunostimulator with the at least on polymer is on a solid phase, in yet other embodiments the linkage is done in solution.

### 5'-PEGylation of oligonucleotides

In one preferred embodiment for 5'-polymer linkage, more preferred linking a polymer to an oligonucleotide (e.g. PEGylation) the polymer (e.g. PEG) is activated as phosphoramidate. In this embodiment DMT-protected-PEG is prepared by reacting an excess of the polymer with dimethoxytriphenylmethylchlorid in the presence of for example triethanolamine (TEA) and 4-dimethylaminopyridine (DMAP). Reaction of DMT-PEG with 2-cyanoethyl-diisopropylchlorophosphoramidite in the presence of diisopropylethylamine gives access to DMT-polymer-phosphoramidites which than can be used as educts in the final cycle of standard oligonucleotide synthesis on CPG-solid-support.

In one embodiment intermediates are purified by column chromatography. The conjugate can be purified by for example RP-HPLC as DMT-on product. After detritylation with acetic acid the final product can be desalted by gelfiltration.

### 3'-Pegylation of oligonucleotides

In one embodiment, for linking a polmer to the 3'-end of an oligonucleotide (e.g. 3'-PEGylation), the polymer is linked to a carboxy-functionalized CPG-support. This in one embodiment is prepared by succinylation of aminopropylated CPG-support using succinic anhydride in the presence of DMAP. The carboxyl groups of the succinylated CPG are activated by for example O-benzotriazole-N,N,N',N'-tetramethyl-uronium-hexafluorophosphate (HBTU) in the presence of for example DMAP and diisopropylethylamine and subsequently reacted with the polymer (e.g. PEG). The resulting polymer-modified support is used as solid support in standard oligonuclotide synthesis, resulting iin an oligonucleotide linked to the polymer via its 3'-hydroxy-group. Cleavage from the support using for example 32 % NH₃ results in the deprotected, PEG-modified DMT-on product which then is detritylated e.g. with acetic acid.

### 3',5'-di polymer linkage of oligonucleotides

In yet other embodiment the polymer (e.g.PEG) is linked to the 3'- and 5'-end of the oligonucleotide. In one embodiment these oligonucleotides linked with two polymers are prepared by combining the two above described embodiments using for example a DMT-polymer-phosphoramidite in the final cycle of the oligonucleotide synthesis on a polymer-modified CPG-support.

### 5'-polymer linkage of oligonucleotides'

In another embodiment a oligonucleotide is linked with at least one polymer (e.g. polyoxyethylene, PEG) at the 5'-end. The oligonucleotide is prepared by conventional oligonucleotide synthesis, using for example a phosphoramidite in the final cycle, for example resulting in a 5'-amino group (5'-amino-modifier) or a 5'-thiol group (5'thiol-modifier). A 5'-amino-modifier respectively a 5'thiol-modifier is a linker covalently binding to the 5'-hydroxy-group of the sugar portion of the nucleotide building block and adds a NH₂ respectively an SH group as active group for linking with the polymer. This group of the modifier is linked to a commercial available or custom synthesized functionalized PEG according to standard procedures as described for example by Hermanson (1996) after deprotection.

In one preferred embodiment where the functional group of the oligonucleotide is NH₂ suitable functionalized polymers (e.g. PEG) are for example NHS-ester, NHS-carbamates or NHS-carbonates, 4-nitrophenylester or otherwise activated carboxylic acids.

In yet other embodiments, where the functional group of the immunostimulator, more preferred the oligonucleotide is the SH-group, the functional groups of the polymer, more preferred the PEG, are for example maleimides or dithiodipyridyl-activated sulfhydryls.

### 3'-Peqylation of oligonucleotides

In yet another embodiment a 3'-modified oligonucleotide is prepared by standard solid phase oligonucleotide chemistry using commercially available or custom synthesized supports, for example a 3'-amino-modifier or 3'-thiol-modifier. A 3'-amino-modifier respectively a 3'-thiol-modifier in this context is a solid support where the 3'OH of a nuceotide is linked to the solid support by a liner that results in a 3'terminal NH₂ or SH function after cleavage the oligonucleotide of the colum. In one embodiment the resulting 3'-modified oligonucleotide is linked to a commercial available or custom synthesized functionalized PEG according to standard procedures as described for example by Hermanson (1996).

In further preferred embodiments the functional group for linking an polymer (e.g. PEG) to an aminomodified oligonucleotides are for example NHS-ester, NHS-carbamates or NHS-carbonates, 4-nitrophenylester or otherwise activated carboxylic acids. Functional groups suitable for polymer linkage (PEG conjugation to sulfhydryls are maleimids or dithiodipydriyl-activated sulfhydryls.

### 3',5'-diPEGylation of oligonucleotides

In yet other embodiments compounds comprising at least one polymer (e.g. PEG) linked to both ends, the 3'- and the 5'-end, of the oligonucleotide are prepared by combining the two embodiments of solution phase chemistry as described above using a 3'-modifier and a 5'-modifier. In yet another embodiment the linkage of an oligonucleotide with at least two polymers is done by a combination of solid phase chemistry and solution phase chemistry.

In yet another embodiment the immunostimulators has two identical functional groups (e.g. SH, OH, NH₂). This immunostimulator is suitable, to conjugate identical polymers.

In yet another embodiment the immunostimulators has at least two different functional groups (e.g. SH, OH, NH₂). This immunostimulator is suitable, to conjugate different polymers.

### Application

Besides being useful in human treatment, the present invention is also useful for other subjects including veterinary animals, reptiles, birds, exotic animals and farm animals, including mammals, rodents, and the like. Mammals include horses, dogs, pigs, cats, or primates (for example, a monkey, a chimpanzee, or a lemur). Rodents include rats, rabbits, mice, squirrels, or guinea pigs.

The oligonucleotide linked with at least one polymer according to this invention is administered by different routes. These routes of administration include, but are not limited to, electrocorporation, epidermal, impression into skin, intra-arterial, intra-articular, intracranial, intra-dermal, intra-lesional, intra-muscular, intranasal, intra-ocular, intra-peritoneal, intra-prostatic, intra-pulmonary, intra-spinal, intratracheal, intra-tumoral, intra-venous, intra-vesical, placement within cavities of the body, nasal inhalation, oral, pulmonary inhalation (e.g., by inhalation or insufflation of powders or aerosols, including by nebulizer), subcutaneous, subdermal, topical (including ophthalmic and to mucous membranes including vaginal and rectal delivery), transdermal.

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual oligonucleotides with at least one polymer, and can generally be estimated based on EC₅₀ values found to be effective in in vitro and in vivo animal models. In general, dosage is from 0.01 µ to 100 g per kg of body weight, and may be given once or more daily, weekly, monthly or yearly, or even once every 2 to 20 years. Persons of ordinary skill in the art can easily estimate repetition rates for dosing based on measured residence times and concentrations of the drug in body fluids or tissues. Following successful treatment, it may be desirable to have the patient undergo maintenance therapy to prevent the recurrence of the disease state, wherein the oligonucleotide with at least one polymer is administered in maintenance doses, ranging from 0.01 µ to 100 g per kg of body weight, once or more daily, to once every 20 years.

In one embodiment the at least one polymer linked with the oligonucleotide is in a pharmaceutically acceptable carrier. The compound in a pharmaceutically acceptable carrier is also referred to as pharmaceutical composition or composition.

A pharmaceutically acceptable carrier (excipient) is a pharmaceutically acceptable solvent, suspending emulsificant agent or any other pharmacologically inert vehicle for delivering the compound to an animal. The pharmaceutically acceptable carrier may be liquid or solid and is selected with the planned manner of administration in mind so as to provide for the desired bulk, consistency, etc., when combined with a nucleic acid and the other components of a given pharmaceutical composition. Typical pharmaceutically acceptable carriers include, but are not limited to, binding agents (e.g. pregelatinized maize starch, polyvinylpyrrolidone or hydroxypropyl methylcellulose, etc.); fillers (e.g. lactose and other sugars, microcrystalline cellulose, pectin, gelatin, calcium sulfate, ethyl cellulose, polyacrylates or calcium hydrogen phosphate, etc.); lubricants (e.g., magnesium stearate, talcum, silica, colloidal silicon dioxide, stearic acid, metallic stearates, hydrogenated vegetable oils, corn starch, polyethylene glycols, sodium benzoate, sodium acetate, etc.); disintegrates (e.g., starch, sodium starch glycolate, etc.); or wetting agents (e.g., sodium lauryl sulphate, etc.). Sustained release oral delivery systems and/or enteric coatings for orally administered dosage forms are described in U.S. Pat. No. 4,704,295; 4,556,552; 4,309,406; and 4,309,404. An adjuvant is included under these phrases.

Pharmaceutical compositions and formulations for topical administration may include transdermal patches, pastes, ointments, lotions, creams, gels, drops, suppositories, globuli, sprays, liquids, and powders. Conventional pharmaceutical carriers, aqueous, powder or oily bases, thickeners and the like may be necessary or desirable. Coated condoms, gloves and the like may also be useful.

Topical administration comprises administration to the skin, the eyes and the ears.

Compositions and formulations for oral administration include powders or granules, suspensions, emulsions, or solutions in water or non-aqueous media, capsules, sachets or tablets. In certain embodiments thickeners, flavoring agents, diluents, emulsifiers, dispersing aids or binders are used.

In other embodiments the pharmaceutical composition for parenteral, intrathecal, or intraventricular administration include sterile aqueous solutions which in some embodiments contains buffers, diluents and other suitable additives such as penetration enhancers, carrier compounds and other pharmaceutically acceptable carriers or excipients.

In other embodiments the pharmaceutical composition also includes penetration enhancers in order to enhance the alimentary delivery of the oligonucleotides. Penetration enhancers may be classified as belonging to one of five broad categories, i.e., fatty acids, bile salts, chelating agents, surfactants and non-surfactants (Lee (1991), Muranishi (1990)). One or more penetration enhancers from one or more of these broad categories may be included.

Various fatty acids and their derivatives which act as penetration enhancers include, for example, oleic acid, lauric acid, capric acid, myristic acid, palmitic acid, stearic acid, linoleic acid, linolenic acid, dicaprate, tricaprate, recinleate, monoolein (1-monooleoyl-rac-glycerol), dilaurin, caprylic acid, arichidonic acid, glyceryl 1-monocaprate, 1-dodecylazacycloheptan-2-one, acylcarnitines, acylcholines, mono- and di-glycerides and physiologically acceptable salts thereof (i.e., oleate, laurate, caprate, myristate, palmitate, stearate, linoleate, etc.) (Lee (1991), Muranishi (1990), El-Hariri (1992). Examples of some presently preferred fatty acids are sodium caprate and sodium laurate, used singly or in combination at concentrations of 0.5 to 5%.

The physiological roles of bile include the facilitation of dispersion and absorption of lipids and fat-soluble vitamins (Brunton 1996). Various natural bile salts, and their synthetic derivatives, act as penetration enhancers. Thus, the term "bile salt" includes any of the naturally occurring components of bile as well as any of their synthetic derivatives. A presently preferred bile salt is chenodeoxycholic acid (CDCA) (Sigma Chemical Company, St. Louis, Mo.), generally used at concentrations of 0.5 to 2%.

Complex formulations comprising one or more penetration enhancers may be used. For example, bile salts may be used in combination with fatty acids to make complex formulations. Preferred combinations include CDCA combined with sodium caprate or sodium laurate (generally 0.5 to 5%).

Chelating agents include, but are not limited to, disodium ethylenediaminetetraacetate (EDTA), citric acid, salicylates (e.g., sodium salicylate, 5-methoxysalicylate and homovanilate), N-acyl derivatives of collagen, laureth-9 and N-amino acyl derivatives of beta-diketones (enamines) (Lee (1991)), Muranishi (1990), Buur (1990)). Chelating agents have the added advantage of also serving as RNase inhibitors.

Surfactants include, for example, sodium lauryl sulfate, polyoxyethylene-9-lauryl ether and polyoxyethylene-20-cetyl ether (Lee (1991)), and perfluorochemical emulsions, such as FC-43 (Takahashi (1988)).

Non-surfactants include, for example, unsaturated cyclic ureas, 1-alkyl- and 1-alkenylazacyclo-alkanone derivatives (Lee (1991)) and non-steroidal anti-inflammatory agents such as diclofenac sodium, indomethacin and phenylbutazone (Yamashita (1987)).

Regardless of the method by which the compounds of the invention are introduced into a patient, colloidal dispersion systems may be used as delivery vehicles to enhance the in vivo stability of the compounds and/or to target the compounds to a particular organ, tissue or cell type. Colloidal dispersion systems include, but are not limited to, macromolecule complexes, nanocapsules, nanospheres, microcapsules, microspheres, beads and lipid-based systems including oil-in-water emulsions, micelles, mixed micelles, liposomes and lipid-compound complexes of uncharacterized structure. A preferred colloidal dispersion system is a plurality of liposomes. Liposomes are microscopic spheres having an aqueous core surrounded by one or more outer layer(s) made up of lipids arranged in a bilayer configuration (Chonn (1995)).

In some embodiments of a pharmamaceutical composition of this iinvention where an acid form can exist, salt and ester forms may preferably be formed from the acid, and all such forms are included within the meaning of the terms compound or oligonucleotid as used herein. Pharmaceutically acceptable salt shall mean non-toxic salts of the compounds employed in this invention which are generally prepared by reacting the free acid with a suitable organic or inorganic base, particulary those formed from cations such as sodium, potassium, aluminium, calcium, lithium, magnesium, zinc, and tetramethylammonium, as well as those salts formed from amines such as ammonia, ethylenediamine, N-methylglucamine, lysine, arginine, ornithine, choline, N,N'-dibenzylethylenediamine, chloroprocaine, diethanolamine, procain, N-benzylphenethylamine, 1-p-chlorobenzyl-2-pyrrolidine-1'-yl-methylbenzimidazole, diethylamine, piperazine, and tris(hydroxymethyl)aminomethane.

### Therapy

The immunostimulator or oligonucleotid linked with at least one polymer is useful for the prepartion of a pharmaceutical composition. In preferred embodiments this pharmaceutical composition is for therapy of unwanted neoplams such as cancer, carcinoma and/or fibrosis, as well as for the treatment of HIV. The pharmaceutical composition comprises at least one of the imunostimulator linked with at least one polymer.

The neoplasms, cancers or carcinomas with treated with immunostimulators comprising at least one immuostimulator linked with at least one polymer include, but are not limited to, solid tumors, blood born tumors such as leukemias, acute or chronic myelotic or lymphoblastic leukemia, tumor metastasis, benign tumors, for example hemangiomas, acoustic neuromas, neurofibromas, trachomas, pyogenic granulomas, pre-malignant tumors, rheumatoid arthritis, psoriasis, astrocytoma, acoustic neuroma, blastoma, craniopharyngloma, ependymoma, Ewing's tumor, medulloblastoma, glioma, hemangloblastoma, Hodgkins-lymphoma, medullablastoma, leukaemia, mesothelioma, neuroblastoma, neurofibroma, non-Hodgkins lymphoma, pinealoma, retinoblastoma, retinoblastoma, sarcoma (including angiosarcoma, chondrosarcoma, endothelialsarcoma, fibrosarcoma, leiomyosarcoma, liposarcoma, lymphangioandotheliosarcoma, lyphangiosarcoma, melanoma, meningioma, myosarcoma, oligodendroglioma, osteogenic sarcoma, osteosarcoma), seminoma, trachomas, Wilm's tumor, or is selected from the group of bile duct carcinoma, bladder carcinoma, brain tumor, breast cancer, bronchogenic carcinoma, carcinoma of the kidney, cervical cancer, choriocarcinoma, cystadenocarcinome, embrional carcinoma, epithelial carcinoma, esophageal cancer, cervical carcinoma, colon carcinoma, colorectal carcinoma, endometrial cancer, gallbladder cancer, gastric cancer, head cancer, liver carcinoma, lung carcinoma, medullary carcinoma, neck cancer, non-small-cell bronchogenic/lung carcinoma, ovarian cancer, pancreas carcinoma, papillary carcinoma, papillary adenocarcinoma, prostata cancer, small intestine carcinoma, prostate carcinoma, rectal cancer, renal cell carcinoma, skin cancer, small-cell bronchogenic/lung carcinoma, squamous cell carcinoma, sebaceous gland carcinoma, testicular carcinoma, uterine cancer.

The following examples will serve to further illustrate the present invention without, at the same time, however, constituting any limitation thereof. On the contrary, it is to be clearly understood that resort may be had to various other embodiments, modifications, and equivalents thereof which, after reading the description herein, may suggest themselves to those skilled in the art without departing from the spirit of the invention

### Examples

### Example 1

### Synthesis of 5'-PEGylated oligonucleotides

### 1) Synthesis of DMT-PEG 400:

### DMT = Dimethoxytrityl

PEG 400 (20,0 mmol) was evaporated twice from 5 ml pyridine (dried with molecular sieve) and dried in vacuo 4 days over P₄O₁₀. The PEG 400 was dissolved in dichloromethane (5 ml, refluxed and distilled with CaH₂) and triethylamine (7.21 mmol, refluxed and distilled with CaH₂) and 4-dimethyl-aminopyridine (0.21 mmol) were added. Subsequently 4,4'-dimethoxytriphenylmethyl chloride (4.5 mmol) was added within 1 hour and the mixture was stirred for 24 hours in the dark. The mixture was diluted with 50 ml dichloromethane and extracted twice with 25 ml 5% sodium hydrogencarbonate and once with 25 ml water. The organic phase was dried (Na₂SO₄ and concentrated by rotary evaporation.

The mixture was purified by column chromatography (silica gel 60 (diameter 0.063-0.100 mm) with the solvent: chloroform : methyl alcohol : triethylamine (96.5 : 3 : 0.5). The product was identified by ¹H-NMR

### 2) Synthesis of DMT-PEG 400-phosphoramidite:

PEG 400-DMT (0.41 mmol) was evaporated twice from 30 ml pyridine (dried with molecular sieve) / dichloromethane (refluxed and distilled with CaH₂) at a ratio of 1:10 and dried in vacuo 6 days over P₄O₁₀. The PEG 400-DMT was dissolved in dichloromethane (970 µl, refluxed and distilled with CaH₂) and diisopropylethylamine (1.65 mmol, refluxed and distilled with CaH₂) and 2-cyanoethyl-diisopropylchlorophosphoramidite (0.63 mmol) was added. The mixture was stirred for 30 minutes in the dark (reaction detection by thin layer chromatography (TLC): silica gel 60 F₂₅₄, dichloromethane / ethyl acetate / triethylamine, 45 / 45 / 10). Methyl alcohol (20 µl, distilled with magnesium) was added. The mixture was diluted with 6 ml dichloromethane and extracted twice with 10 ml 5 % sodium hydrogencarbonate and twice with 10 ml distilled water. The organic phase was dried (Na₂SO₄ and concentrated by rotary evaporation.

The mixture was purified by chromatography (silica gel 60 (0,040-0,063 mm); dichloromethan / ethyl acetate / triethylamine, 45/45/10). The product was idetiified with NMR.

### 3) Synthesis of 5'-PEG 400-oligonucleotide conjugates:

The oligonucleotide was synthesized by standard phosphoramidite chemistry on 1000Åadenosine-CPG (10 micromol, loeadiing 38 micromol/gsupport using a Expedite Synthesizer (PerSeptive Biosystems). The 5'-OH of the oligonucleotid was PEGylated by using the DMT-PEG 400-phosphoramidite (50 mg/ml acetonitrile) on the Synthesizer. In order to calculate the loading of DMT-groups, a aliquote was taken, 0,1 M toluene-4-sulfonic acid was added and the absorption of the cleavaged DMT-cations were determinated at 260 nm (loading: 11,2 micromol).The product was cleaved from the support and deprotected by using 32% NH₃ at 55°C for 16 h. The crude product was dried and purified by reversed-phase HPLC (TOSOHAAS, Amberchrom CG-300S (reversed phase), 35 µm; 125 x 4,7 mm; acetonitrile / 0.1 M triethylammonium acetate pH 7.5). The oligonucleotide was detritylated by using 80% acetic acid at room temperature for 15 min, desalted by using a NAP-10-Column (Amersham Biosciences) and lyophilized.

### Analysis:

a) MALDI-TOF: Matrix: 3-hydroxypicolinic acid, reflector-Mode, molecular weight was determined
b) capillary gel electrophoresis: capillary: fused silica capillary, 40 cm, inner diameter: 100 µm, external diameter: 360 µm, buffer: 10% buffer solution pH 2.5 for HPCE (0.1 M sodium phoshate-buffer)
90% 0.1 % (hydroxypropyl)methyl cellulose in water, sample: 10 pmol oligonucleotide / µl (in water), voltage: 8 kV, polarity: from - to +, run time: 30 min, run: capillary was rinsed under pressure, 60 sec with 0.1 M NaOH (filtered 0.2µm), 120 sec with buffer, sample injection (5 sec. with pressure), results: broad peak at 20.5 min

### Example 2

### Synthesis of 3'5'-PEGylated oligonucleotides

### 1) Succinylation of controlled pore glass (CPG) support:

Succinic anhydride (44 µmol) and 4-di(methyl)aminopyridine (22 µmol) were dissolved in pyridine (440 µl). The mixture was added to aminopropylated CPG-support (4.4 µmol, loading: 44 µmol) and mixed at room temperature. After 22 h the solution was removed and the CPG-support was washed five times with 1 ml dichlormethane and three times with 1 ml acetonitrile. For testing for unreacted amino groups, an aliquot (approx. 1 mg) of CPG-support was taken and 50 µl of a ninhydrin- solution (50 mg ninhydrin/ml ethanol) was added. After 10 minutes by 55 °C no colour was detected indicating no free amino groups.

### 2) PEGylation of the succinylated CPG support:

2-(1H-benzotriazole-1-yl)-1,1,3,3-tetramethyluronium hexafluorophosphate (HBTU, 3.0 mmol), 4-di(methyl)aminopyridine (3.0 mmol) and diisopropylethylamine (3.0 mmol) were dissolved in acetonitrile (22.5 ml). The mixture was added to the succinylated CPG-support (approx. 26.4 µmol). Subsequently, PEG 400 (12.7 mmol) was added and the mixture was mixed at room temperature. After 24 h the solvent was removed and the CPG support was washed three times with 10 ml dichloromethane and three times with 10 ml acetonitrile.

### 3) 3'PEGylated oligonucleotide synthesis:

Oligonucleotides were synthesized by phosphoramidite chemistry on theh PEGylated-CPG support using a Expedite Synthesizer. For more details see also R. Schlingensiepen (1997). In order to calculate the loading of DMT-groups, a aliquote was taken, 0,1 M toluene-4-sulfonic acid was added and the absorption of the cleavaged DMT-cations were determinated at 260 nm (loading: 16,8 µmol).

### 4) 5'-PEGylation of the oligonucleotides:

The oligonucleotides were PEGylated by using 58.6 mg DMT-PEG 400-phosphoramidite / ml acetonitrile (dry) on the Expedite Synthesizer. For calculating the loading of DMT-groups, a aliquote was taken, 0,1 M toluene-4-sulfonic acid was added and the absorption of the cleavaged DMT-cations were determinated at 260 nm (loading: 6,3 µmol).

The product was cleaved from the support and deprotected by using 32% NH₃ at 55°C for 16 h. The crude product was dried and purified by reversed-phase HPLC (TOSOHAAS, Amberchrom CG-300S (reversed phase), 35 µm; 125 x 4,7 mm; acetonitrile /0.1 M triethylammonium acetate pH 7.5). The oligonucleotide was detritylated by using 80% acetic acid at room temperature for 15 min, desalted by using a NAP-10-Column (Amersham Biosciences) and lyophilized.

### Analysis:

a) MALDI-TOF: Matrix: 3-hydroxypicolinic acid, reflector-Mode, molecular weight was determined
b) capillary gel electrophoresis: capillary: fused silica capillary, 40 cm, inner diameter: 100 µm, external diameter: 360 µm, buffer: 10% buffer solution pH 2.5 for HPCE (0.1 M sodium phoshate buffer), 90% 0.1% (hydroxypropyl)methyl cellulose in water; sample: 10 pmol oligonucleotide / µl (in water); voltage: 8 kV; polarity: from - to +; run time: 30 min; run: capillary was rinsed under pressure, 60 sec with 0.1 M NaOH (filtered 0,2µm), 120 sec with buffer, sample injection (5 sec with pressure), results: broad peak at 21 min

### Example 3

### 3'-PEGylated oligonucleotide

### 1) Succinylation of controlled pore glass (CPG) support:

Succinic anhydride (44 µmol) and 4-di(methyl)aminopyridine (22 µmol) were dissolved in pyridine (440 µl). The mixture was added to aminopropylated CPG support (4,4 µmol) and mixed at room temperature. After 20 h the solvent was removed and the CPG-support was washed five times with 1ml dichloromethane and three times with 1ml acetonitrile. For testing of unreacted amino group, a aliquot (approx. 1 mg) of CPG-support was taken and 50 µl of a ninhydrin-solution (50 mg ninhydrin / ml ethanol) was added. After 10 minutes by 55°C no colour was detected.

### 2) PEGylation of the succinylated CPG support:

2-(1H-benzotriazole-1-yl)-1,1,3,3-tetramethyluronium hexafluorophosphate (HBTU, 1,5 mmol), 4-di(methyl)aminopyridine (1,5 mmol) and diisopropylethylamine (1.5 mmol) were dissolved in acetonitrile (11.25 ml). The mixture was added to the succinylated CPG support (combined, see 1); approx. 13,2 µmol). Subsequently PEG 400 (6.36 mmol) was added and the mixture was mixed at room temperature. After 20 h the solution was removed and the CPG-support was washed three times with 15 ml dichloromethane and three times with 15 ml acetonitrile.

### 3) Oligonucleotide synthesis:

Oligonucleotides were synthesized by phosphoramidite chemistry on th ePEGylated-CPG support using a Expedite Synthesizer. The product was cleaved from the support and deprotected by using 32% NH₃ at 55°C for 16 h. The crude product was dried and purified by reversed-phase HPLC (TOSOHAAS, Amberchrom CG-300S (reversed phase), 35 µm; 125 x 4,7 mm; acetonitrile / 0, 1 M triethylammonium acetate pH 7.5). The oligonucleotid was detritylated by using 80% acetic acid at room temperature for 15 min, desalted by using a NAP-10-Column (Amersham Biosciences) and lyophilized.

### Analysis:

a) MALDI-TOF: Matrix: 3-hydroxypicolinic acid, reflector mode, molecular weight was determined
b) capillary gel electrophoresis: capillary: fused silica capillary, 40 cm, inner diameter: 100 µm, external diameter: 360 µm, buffer: 10 % buffer solution pH 2.5 for HPCE (0.1 M sodium phoshate buffer), 90 %, 0.1 % (hydroxypropyl)methyl cellulose in water voltage: 8 kV; polarity: from - to +; run time: 30 min; concentration of the sample: about 10 pmol oligonucleotide / µl (in water), Methode: I) capillary preparation: capillary was rinsed under pressure: 360 sec with water (destilled, filtereed pore diameter 0.2 µm), 120 sec with 0.1 M NaOH (filtrated pore diameter 0,2µm), 360 sec with water (destilled, filtrated pore diameter 0.2µm) II) run: capillary was rinsed under pressure: 60 sec. with 0,1 M NaOH (filtrated 0.2µm) 120 sec with buffer; sample injection (5 sec. with pressure); result: broad peak at 20 min

### Example 4

### Synthesis of oligonucleotide-PEG conjugate in single reactor unit

The oligonucleotide was synthesized by standard phosphoramidite chemistry on 1000Å-CPG support using a Expedite Synthesizer (PerSeptive Biosystems). The 5'-OH of the oligonucleotide was aminomodified by a 2-[2-(4-monomethoxytrityl)aminoethoxy]ethyl-(2-cyanoethyl)-N,N-diisopropyl)-phosphoramidite (5'-amino-modifier 5, Glen Research, 0.067M in acetonitril) on the Synthesizer. The product was cleaved from the support and deprotected by using 32% NH₃ at 40°C for 17 h. The crude product was dried and purified by reversed-phase HPLC (Phenomenex, Polymerx, 10 µm, RP-1, 100 Å, 250 x 10mm; 0.1M trietylammonium acetate pH 7.5 /acetonitrile). The oligonucleotide was detritylated by using 80% acetic acid at room temperature for 1 h and eluated over a NAP-10-column (Amersham Biosciences).

The 5'-aminomodified oligonucleotide was dissolved in 0.3M NaHCO₃, pH 8.5, 40% DMF and heated to 37°C. The MPEG-SPA-5K (NEKTAR, 24 equivalents) was added stepwise. After 18 h the mixture was dried and the crude product was separated from the excess of PEG by ion-exchange chromatography (TOSOHAAS, Toyopearl, Super Q-650S, 250 x 4 mm; 0.01 M triethylammonium acetate pH 7.5/0.01M triethylammonium acetate pH 7.5, 2 M NaCl). Subsequently, sodium chloride was removed by using Amicon Ultra Centrifugal Filter Devices (Millipore). The unreacted oligonucleotid was removed by reversed-phase HPLC (Phenomenex, Polymerx, 10 µm, RP-1, 100 Å, 250 x 10 mm; 0.1M trietylammonium acetate pH 7.5 /acetonitrile). The purified product was desalted by using a NAP 10 column (Amersham Biosciences) and lyophilized.

Analysis: MALDI-TOF: Matrix: 3-hydroxypicolinic acid, reflector mode, Peak at approximately 11.5 kg/mol.

### Example 5

### Cell culture experiments: Suppression of TGF-beta2 secretion

Cell culture:
The human glioblastoma cell line A-172 (Accession no. CRL-1620) was established from a 53-year old man with a glioblastoma. These cells were grown in monolayers.
The A-172 tumor cells were cultivated in DMEM medium with 4.5 g/l glucose and 10% (v/v) FCS at 37°C, 5% CO₂, and 95% relative humidity. To investigate TGF-beta2 suppression as well as tumor cell proliferation, 10⁴ A-172 cells/well were seeded into 12-well tissue culture plates. 24 hours after seeding the supernatants were removed and replaced by a treatment solution consisting of cell culture medium containing the respective amount of oligonucleotide with linkers with at least one polymer. The exchange of the medium was repeated after 3 days (schedule signed as 2*3 days). Medium of the negative control (= untreated cells) and the medium control (= medium without cells) were changed without addition of oligonucleotide. After 6 days of treatment the supernatants were collected for quantification of secreted TGF-beta2 by using an Enzyme-Linked Immunosorbent Assay (ELISA) from R&D Systems. In parallel, the number of tumor cells was investigated by cell counting using an electronic cell counter.

### Methods:

TGF-beta2 in cell culture supernatants was quantified by using the Quantikine® Human TGF-beta2 Immunoassay according to the manufacturer's instructions (R&D Systems). The supernatants were cleared of cellular components by centrifugation (850 g, 5 min). Optical density (OD) quantification and calculations were performed with the Multiskan Ascent Type 354 200-240V plate reader using a 4 parameter logistic.

Tumor cell proliferation was investigated by cell counting using the Coulter Counter Z2 from Beckmann. This method is based on the principle that particles (cells resuspended in an electrolyte) passing through an electric field alter the electrical resistance. The cell number was analyzed according to the manufacturer's instructions. Briefly, the cell suspension (i.e. trypsinized tumor cells) was mixed with the counter solution (Isotone 1®) in a defined volume (9.5 ml counter solution Isotone 1® and 500 µl sample, dilution factor 20) and counted for three times in the Coulter Counter Z2.

### Example 6

### Enzymatic stability testing of oligonucleotides

### 3'-exonuclease digestion

Oligonucleotides (1 OD) were dissolved in 98 µl 0.05 M Tris-HCl buffer, pH 8.5. Two µl (0.083 U) phosphodiesterase I (from Crotalus adamanteus venom, 42 U/mg, 1mg/ml, dissolved in 110 mM Tris-HCl, 110 mM NaCl, 15 mM MgCl₂, 50% glycerol, pH 8.9) was added. The mixtures were incubated at 37°C (Thermocycler, Hybaid, PCR Sprint).

The unmodified phosphodiester oligonucleotide was analysed at 0 min, 5 min, 10 min, 20 min, 30 min, 1 h, 3 h, 6 h, and 24 h. Unmodified thiophosphate oligonucleotide, 3'-PEGylated thiophosphate, 5'-PEGylated thiophosphate, and 3'-5'-PEGylated thiophosphate oligonucleotide were analyzed at 0 min, 10 min, 30 min, 1 h, 3 h, 6 h, 24 h, 48 h, and 72 h.

For analysis, a 10 µl aliquot was drawn, heated to 95°C for 10 min to destroy the phosphodiesterase, and centrifuged (5 min, 16060 g, room temperature). Nine µl of the 10 µl aliquot were taken, diluted with 11 µl 0.04 M KH₂PO₄, 0.002% H₃PO₄, pH 3.6, and analyzed by HPLC using a Waters 600 Pump, Waters 600 Controller, and Waters 2487 Dual-Absorbance Detector (254 nm). Separation was performed with a Merck LiChrospher 100 Reversed-Phase C18 column (endcapped, pore size: 100 Å, particle size: 5 µm, 125 x 4 mm) with a flow-rate of 1 ml/min in a gradient of 0, 13.5 and 50% acetonitrile /100 and 86.5, 50% 0.04 M KH₂PO₄, 0.002% H₃PO₄, pH 3.6, respectively.

### 5'-exonuclease digestion

Oligonucleotides (1 OD) were dissolved in 50 µl 0.2 M triethylammonium acetate buffer, pH 6.5. Fifty µl (0.22 U) phosphodiesterase II (Type I-SA, from bovine spleen, dissolved in water (4.4 U/ml)) were added. The mixtures were incubated at 37°C (Thermocycler, Hybaid, PCR Sprint). The unmodified phosphodiester oligonucleotide was analysed at 0 min, 5 min, 10 min, 20 min, 30 min, 1 h, 3 h, 6 h, and 24 h. Unmodified thiophosphate oligonucleotide, 3'-PEGylated thiophosphate, 5'-PEGylated thiophosphate, and 3'-5'-PEGylated thiophosphate oligonucleotide were analyzed at 0 min, 10 min, 30 min, 1 h, 3 h, 6 h, 24 h, 48 h, and 72 h.

For analysis, a 10 µl aliquot was drawn, heated to 95°C for 10 min to destroy the phosphodiesterase, and centrifuged (5 min, 16060 g, room temperature). Nine µl of the 10 µl aliquot were taken, diluted with 11 µl 0.04 M KH2PO4, 0.002% H3PO4, pH 3.6, and analyzed by HPLC using a Waters 600 Pump, Waters 600 Controller, Waters 2487 Dual-Absorbance Detector (254 nm). Separation was performed with a Merck LiChrospher 100 Reversed-Phase C18 column (endcapped, pore size: 100 Å, particle size: 5 µm, 125 x 4 mm) with a flow-rate of 1 ml/min in a gradient of 0, 13.5 and 50% acetonitrile / 100, 86.5 and 50% 0.04 M KH2PO4, 0.002% H3PO4, pH 3.6, respectively.

### Results:

Surprisingly the immunostimulators linked with at least one polymer showed increased nuclease stability over those not linked with polymers. Oligonucleotides linked with polymers at the 3'- and 5'- end were surprisingly superior over those linked with a polymer of the same size/weight as the two polymers linked at only the 3'-end or the 5'-end of the oligonucleotide. Best results were reached with those linked with a smaller polymer at the 3'-end compared with the polymer at the 5'-end.

### Example 7

### ISIS Sequenzen

The TGF-beta 1, TGF-beta2 and TGF-beta3 oligonucleotidesof this example linked with at least one polymer are also part of the invention. They are further embodiments for polymer-oligonucleotide conjugates inhibiting formation of TGF-beta 1,2 and 3 "in vitro" and "in vivo" and at least one of these conjugates can be used in pharmaceutical acceptable carriers as a pharmaceutical composition for the treatment of unwanted neoplasms, fibrosis and HIV as described in this invention.

TGF-beta1, 2 and 3 antisense oligonucleotides:

gtgccatcaatacctgcaaa, catcagttacatcgaaggag, tcttgggacacgcagcaagg,
gaaatcaatgtaaagtggac, catgaactggtccatatcga, gaggttctaaatcttgggac,
gcactctggcttttgggttc, tagctcaatccgttgttcag, ccctagatccctcttgaaat,
accaaggctctcttatgttt, tcgagtgtgctgcaggtaga, tgaacagcatcagttacatc,
gctgggttggagatgttaaa, agaggttctaaatcttggga, cgccggttggtctgttgtga,
ctgctttcaccaaattggaa, aagtatagatcaaggagagt, tgctcaggatctgcccgcgg,
gtgctgttgtagatggaaat, agggcggcatgtctattttg, taagcttattttaaatccca,
tagctgcatttgcaagactt, tctgttgtgactcaagtctg, aagcaataggccgcatccaa,
tcaatgtaaagtggacgtag, attttagctgcatttgcaag, tgtagatggaaatcacctcc,
ttaacactgatgaaccaagg, attgtaccctttgggttcgt, agatccctcttgaaatcaat,
tgtaaagtggacgtaggcag, ccattcgccttctgctcttg, tgttaaatctttggacttga,
gaagggcggcatgtctattt, gaccctgctgtgctgagtgt, gaactagtaccgccttttca,
cgatcctcttgcgcatgaac, ccggccaaaagggaagagat, aaagagacgagtggctatta,
aagtggaaatattaatacgg, agatcaaggagagttgtttg, agttgtttttaaaagtcaga,

tgtaacaactgggcagacag, ggtgttgtaacaactgggca, tacccacagagcacctggga,
gggatggcatcaaggtaccc, tcgtcatcatcattatcatc, aagggtgcctattgcatagc,
ctcactgttaactctaagag, gcaaagtatttggtctccac, caagttccttaagccatcca,
ttatcttaatgcagactttc, cttacaagaagcttccttag, actggtgagcttcagcttgc,
acttgagaatctgatatagc, aggttcctgtctttatggtg, gtgtatccatttccaccta,
cagcacagaagttggcattg, gcaaggagaagcagatgctt, agcaaggagaagcagatgct,
ttttccaagaatttagctg, ttcttgttacaagcatcatc, ttaaagaaggagcggttcgg,
ctgggctgaaatttatatat, gggcagacagctaggagttt, gtgtactcaccaaggtaccc,
cccagcactttgggaggccg, ggctcacgcctgtaatccca, tgaccgtgaactcactattt,
atagtggtgatggctataca, ttttggttacctgcaaatct, gaacactcaccctgctgtgc,
gaatggctctttaaacccta, gaagaaatggagttcagtgt, tttctcctggaagggagagg,
aaatgcaacgcgttcccaac, aatacgaaacttttgcaaag, actagtaattctcagagcgg,
aagaaactagtaattctcag, agtgcatgtttttaaaagga, cagtagtgcatgtttttaaa,
ctcagcacacagtagtgcat, agatgcaggagcaaaaaggt, caggtagacagactgagcgc,
gcctcgatcctcttgcgcat, gcggatggcctcgatcctct, ctcaggatctgcccgcggat,
gctccggatagtcttccggg, agatggaaatcacctccggg, gttgtagatggaaatcacct,
ctggtactgttgtagatgga, aggcggctgccctccggctt, aacctccttggcgtagtact,
attttataaacctccttggc, cggcatgtcgattttataaa, cgggatggcattttcggagg,
gtagggtctgtagaaagtgg, tgaagtagggtctgtagaaa, attctgaagtagggtctgta,
aagcggacgattctgaagta, cccaggttcctgtctttgtg, ggcagtgtaaacttatttta,
ccatcaatacctgcaaatct, aggtgccatcaatacctgca, agttttctgatcaccactgg,
ttatagttttctgatcacca, cctagtggactttatagttt, acattagcaggagatgtggg,
agggcaacaacattagcagg, actccagtctgtaggagggc, tcctgcacatttctaaagca,
cagcaattatcctgcacatt, atgtaaagagggcgaaggca, ctcttaaaatcaatgtaaag,
ccaagatccctcttaaaatc, cctttgggttcatggatcca, gcattgtaccctttgggttc,
gcacagaagttagcattgta, ctgaggactttggtgtgttg, tcctgggacacacagcaagg,
tttagctgcatttacaagac, caaggactttagctgcattt, gtcattgtcaccgtgatttt,
ccagttttaacaaacagaac, agatgccagttttaacaaac, gttcattatatagtaacaca,
atgaaaggttcattatatag, ttccaagggtaatgaaaggt, cttaagccatccatgagttt,
cctggcttatttgagttcaa, ttagtcctataacaactcac, gcaaagaaccatttacaatt,
cttgcttaaactggcaaaga, acatgtaaagtagttactgt, acacattacatgtaaagtag,
taagatctacacattacatg, attcaaaggtactggccagc, tttgtagtgcaagtcaaaat,
catgtcattaaatggacaat, cctacatttgtgcgaacttc, ttcccccttgaaaaactca,
tttttaatcagcctgcaaag, actgggcagacagtttcgga, taacaactgggcagacagtt,
tgttgtaacaactgggcaga, cacagagcacctgggactgt, gtacccacagagcacctggg,
tcaaggtacccacagagcac, tggcatcaaggtacccacag, ggcgggatggcatcaaggta,
tttgcaggtattgatggcac, ctccttcgatgtaactgatg, ccttgctgcgtgtcccaaga,
tcgatatggaccagttcatg, gtcccaagatttagaacctc, gaacccaaaagccagagtgc,
atttcaagagggatctaggg, aaacataagagagccttggt, tctacctgcagcacactcga,
tcacaacagaccaaccggcg, ttccaatttggtgaaagcag, actctccttgatctatactt,
ccgcgggcagatcctgagca, caaaatagacatgccgccct, tgggatttaaaataagctta,
cagacttgagtcacaacaga, ttggatgcggcctattgctt, ctacgtccactttacattga,
ggaggtgatttccatctaca, ccttggttcatcagtgttaa, acgaacccaaagggtacaat,
attgatttcaagagggatct, ctgcctacgtccactttaca, caagagcagaaggcgaatgg,
tcaagtccaaagatttaaca, aaatagacatgccgcccttc, acactcagcacagcaggtc,
tgaaaaggcggtactagttc, gttcatgcgcaagaggatcg, atctcttccctttggccgg,
taatagccactcgtctcttt, ccgtattaatatttccactt, caaacaactctccttgatct,
ctgtctgcccagttgttaca, tgcccagttgttacaacacc, tcccaggtgctctgtgggta,
gatgataatgatgatgacga, gctatgcaataggcaccctt, ctcttagagttaacagtgag,
gtggagaccaaatactttgc, gaaagtctgcattaagataa, ctaaggaagcttcttgtaag,
gcaagctgaagctcaccagt, tagggtggaaatggatacac, caatgccaacttctgtgctg,
aagcatctgcttctccttgc, cagctaaaattcttggaaaa, gatgatgcttgtaacaagaa,
aaactcctagctgtctgccc, gggtaccttggtgagtacac, cggcctcccaaagtgctggg,
tgggattacaggcgtgagcc, tgtatagccatcaccactat, acactgaactccatttcttc,
cctctcccttccaggagaaa, gttgggaacgcgttgcattt, ccgctctgagaattactagt,
ctgagaattactagtttctt, tttaaaaacatgcactactg, atgcactactgtgtgctgag,
gcgctcagtctgtctacctg, atgcgcaagaggatcgaggc, agaggatcgaggccatccgc,
atccgcgggcagatcctgag, cccggaagactatccggagc, cccggaggtgatttccatct,
aggtgatttccatctacaac, tccatctacaacagtaccag, aagccggagggcagccgcct,
agtactacgccaaggaggtt, gccaaggaggtttataaaat, tttataaaatcgacatgccg,
cctccgaaaatgccatcccg, ccactttctacagaccctac, tttctacagaccctacttca,
tacagaccctacttcagaat, tacttcagaatcgtccgctt, cacaaagacaggaacctggg,
taaaataagtttacactgcc, tgcaggtattgatggcacct, ccagtggtgatcagaaaact,

tggtgatcagaaaactataa, cctgctaatgttgttgccct, gccctcctacagactggagt,
tgctttagaaatgtgcagga, tgccttcgccctctttacat, gattttaagagggatcttgg,
tggatccatgaacccaaagg, gaacccaaagggtacaatgc, tacaatgctaacttctgtgc,
ccttgctgtgtgtcccagga, gtcttgtaaatgcagctaaa, aaatgcagctaaagtccttg,
aaaatcacggtgacaatgac, gttctgtttgttaaaactgg, gtttgttaaaactggcatct,
tgtgttactatataatgaac, acctttcattacccttggaa, aaactcatggatggcttaag,
aattgtaaatggttctttgc, tctttgccagtttaagcaag, acagtaactactttacatgt,
ctactttacatgtaatgtgt, catgtaatgtgtagatctta, gctggccagtacctttgaat,
ctttgcaggctgattaaaaa, aactgtctgcccagttgtta, tctgcccagttgttacaaca,
acagtcccaggtgctctgtg, cccaggtgctctgtgggtac, gtgctctgtgggtaccttga,
ctgtgggtaccttgatgcca, taccttgatgccatcccgcc, ttccaccattagcacgcggg,
ccgtgaccagatgcaggatc

### Example 8

The oligonucleotides of this example linked with at least one polymer are further embodiments for polymer-oligonucleotide conjugates inhibiting the effect of a substance negatively effecting an immune response. The pharmaceutical composition with at least one of these conjugates in pharmaceutical acceptable carriers in preferred embodiments are used for the treatment of unwanted neoplasms, fibrosis and HIV.

cccggagggcggcatgggga, cctcagggagaagggcgc, gtaggagggcctcgaggg,
ctgcaggggctgggggtc, agggctggtgtggtgggg, ggcatgggggaggcggcg,
ccggagggcggcatgggg, ggggggctggcgagccgc, ggacaggatctggccgcggatgg,
ccccctggctcgggggc, gggccgggcggcacctcc, gggcagcgggccgggcgg,
acggcctcgggcagcggg, gggtgctgttgtacaggg, gggtttccaccattagcacgcggg,
tcatagatttcgtt, ttgtcatagattt, aagaacatatatatg, aagaacatatatat,
ttgaagaacatatata, ccgggagagcaacacggg, acttttaacttga, attgttgctgtattt,
attgttgctgtatt, aattgttgctgtatt, aattgttgctgtat,
ggcgagtcgctgggtgccagcagccgg, ggcgagtcgctggg, acatcaaaagataa,
tgacatcaaaagat, gggccctctccagcgggg, gggctcggcggtgccggg, ggggcagggcccgaggca,
ggctccaaatgtaggggc, cgggttatgctggttgtacagggc, cggcgccgccgaggcgcccggg,
ggggcggggcgggacc, gggcggggcggggcgggg, gggcggggtggggccggg,
gggcaaggcagcgggggcgggg, cggtagcagcagcg, ccagtagccacagc, gcaggtggatagtcc,
cttgcaggtggatag, cgatagtcttgcagg, ccatgtcgatagtcttgc, ctcgatgcgcttccg,
cctcgatgcgcttcc, ggatggcctcgatgc, ggacaggatctggcc, cgcagcttggacagg,
gagccgcagcttgg, cgagccgcagcttg, acctccccctggct, ccaccattagcacg,
gaacttgtcatagatttc, gctgtgtgtactctgc, gctccacgtgctgc, gaattgttgctgtatttc,
gccaggaattgttgc, gtgacatcaaaagataac, ggctcaaccactgcc, gctgtcacaggagc,
cctgctgtcacagg, gcagtgtgttatccctgc, gcagtgtgttatccc, ccaggtcacctcgg,
gccatgaatggtggc, gccatgaatggtgg, ccatgagaagcagg, ggaagtcaatgtacagc,
ccacgtagtacacgatgg, gcacttgcaggagc, ccatggcagtgacc, ggctcctccatggc,
gctaggatctgactgc, cctgactcagaggg, ggtctgaaaatgtttcc, ccattgcttgggacgg,
gcatcaaatcatcc, ccattgttcaatatcg, ggtcttcagtgaacc, ggagcttcatctggacc,
cctctggcattctgg, agggacagaagatg, gttttctgggaagg, ggttttctgggaag,
aggttttctgggaag, gtaggttttctggg, ggtaggttttctgg, ccagaatgcaagaagcc,
gctgtcccagaatgc, gcaagtcacagacttggc, ccacagctgcacagg, ggtgtggaatcaacc,
gtcatgtgctgtga, cgctatctgagcagcg, ccagtgtgatgatgg, ccagtagattaccactgg,
ggcacaaacacgcacc, ccacggatctgaagg, cggaacatctcgaagcg, cctcattcagctctcgg,
ccttgagttccaagg, cctttttggacttcagg, ggaggtagactgaccc, aaaatgtttcct,
tgaaaatgtttc, ctgaaaatgttt, tctgaaaatgttt, tctgaaaatgtt, aaatcatccatt,
ttgttcaatatc, attgttcaatatc, attgttcaatat, cattgttcaatat, cattgttcaata,
aaaagtgtttct, acatgagtttttat, aacatgagtttttat, acatgagtttttta,
aacatgagtttttta, aacatgagtttttt, aaaacatcttgtt, cagagggggctcgacgc,
ctgactcagagggggctc, agggggacagaacg, ttgggacggcaagggggacagaa,
tgggacggcaaggggga, gccacgggggagca, gcaggggccacgggggag, aggggccacggggg,
caggggccacgggg, ggtgcaggggccacg, tggtgcaggggccgccgg, ggggctggtgcaggggcc,
agggggctggtgcagggg, gggctggtgcaggg, gagggggctggtgcag, aggagggggctggtg,

gggccaggagggggctgg, aggggccaggagggggct, ggggccaggagggg, caggggccaggaggg,
tctgggaagggacaga, tgagggcaggggagta, ttgagggcaggggag, cgggtgccggcgggggtg,
cggacgcgggtgccggcggggt, cgggtgccggcggg, ggacgcgggtgccggcg,
tgggggcagcgcctcaca, ggtgggggcagcgcct, ccattttagtgcacatccgg,
ccattttagtgcacatcc, gctgttccattttagtgc, gtagtcgtgtagag, gtttgtagtcgtgtag,
gtttcaggagtttgtag, ccagctccgaagagg, cgtcgtcgtgatcacg, ggtaaaagtactgtcc,
ggctttgacaaagcc, cttgtgcagatcgtccag, cgtggttcatcttgtgc, cacgtggttcatcttgtg,
cctccttgaaggtgg, cgctccactttgatgcg, ccttgtcctccagg, ggtactcgacagcc,
ctgacgtgggtcatg, ccgttgctgacgtgg, catcctccgcctcc, gtttccatcctccg,
ggtgtttccatcctcc, ggtgtttccatcctc, gctcagcgcctcatc, ccttcttcatcatgctgc,
ccttcttcatcatgctg, ccttcttcatcatgc, gcgtccttcttcatcatgc, cctgctcactcagg,
cgcaggcttgagcg, gccagcttcagcagc, ggtggtgaccagcc, cctcggcgaactcc,
gcttgtgtaaatcc, ggttctgcttgtgtaaatcc, gctgctcaggttcgc, gaaggcgaccgtcg,
cgaaggcgaccgtc, gcaccgtctgtggc, cgtgtccatgtcgatgg, cgtgtccatgtcgatg,
gcgtgtccatgtcg, ccagcttgcgcttgc, cgctccagcttgcg, cgtgttctgactcttgag,
cgtgttctgactcttg, gctgttgacgtggc, cgactcagtacgcc, gccatgcccgactc,
cccttggaggtggc, ttttagtgcacat, tgttccattttagt, aaaaaaagtggaag,
tacaaaaaaaagtg, atacaaaaaaaagt, catacaaaaaaaagt, catacaaaaaaaag,
gaaaaaaaacatac, cagaaaaaaaacatac, cagaaaaaaaacat, ttcaatatgaatcg,
tattcaatatgaatcg, tattcaatatgaatc, tattcaatatgaat, tatattcaatatgaa,
ttatattcaatatga, tattatattcaatatga, ttatattcaatatg, tattatattcaatatg,
attatattcaatat, tattatattcaatat, atatattatattcaatat, aaatatattatattcaatat,
tattatattcaata, atatattatattcaata, caaatatattatattcaata, tatattatattcaat,
aatatattatattcaat, tatattatattcaa, caaatatattatattcaa, caaatatattatattca,
caaatatattatattc, cacaaatatattatattc, aaatatattatatt, caaatatattatatt,
caaatatattatat, cacaaatatattatat, cacaaatatattat, tacacaaatatattat,
tacacaaatatatta, taaatacacaaatatatt, aatacacaaatata, gttaaatacacaaata,
tgttaaatacacaa, tttagagactaagt, ataaactctttaga, taaaataaactctttag,
taaaataaactcttta, ttaaaataaactcttt, cttaaaataaactc, taaaaagaacaaaca,
taaaaagaacaaac, caataaaaagaacaa, tcaataaaaagaacaa, tcaataaaaagaac,
ttcaataaaaagaa, tagattcaataaaaaga, tggcgcgggcgggtagc, gggctggcgcgggcgggtag,
tcgggggctggcgcgggcggg, tgggtgcctggtcgcgcgttctcggg, agggtccctgcggggccg,
gggagggtccctgcgggg, gggagggtccctgcgg, tgggccgggtccgc, tcccgggggtgtag,
agtactgtcccgggggtgt, gggacacgttggggggtg, gccgggggcccccggtagc,
cgggcccagccgggggc, cgggcccagccggg, gggaggtggctccgggccgg,
agggcggcgcgtgtggga, gggtggccaccggcgaaggg, aggggcaggggacgt,
taaaggggcaggggacgt, agggggtgtccgtaaagggg, ggggacgcgaacgtgccgccg,
cggggaacaagcggcccgggg, ggccgtcgggggcg, gcggccgtcgggggc, agggggtaggaggcggg,
gcgctgggggcgcc, ggccgtcggggggt, ggggaggccagcttc, ggccgccaccttgggg,
gcggccgccgccgggg, gggcgcggccgccgccgggg, ggggtggcggcggcgg, gggggtggcggcggc,
tggggcagcagctggcag, cggggcgcccacgacacc, cggggcgcccacgacac,
gggccgcaccctctccaagtccgggg, gcagcagtcagtgg, ccattgtctagcacgg,
ggtctccattgtctagc, ggtggtattgttcagc, gctggatcaagaccc, ccacaaaatcgtgtcc,
ccttccacaaaatcgtgtcc, ggttgttcttgtgg, cctcttggttgtgc, ccagagtctcaaacacttgg,
ggtaacctgtgatctcttcc, cctgcagtactcgg, ggcattcacatactcc, gcaaacagtgcctggc,
cgcatcgtgtacttccg, gcacgttccgagcg, ggtaccagatactcc, ccagtggagacctgg,
cctgaggacacatcagg, cctcacttggttgtgagc, ggaagatgtccttcc, gcacactgctcatggc,
gctgtcacctcttgg, cctctgctgtcacc, ccacacatcactctgg, cctcctcttcagagg,
ccttctggttcacactgg, catggtgctcactgcg, cttggttgtgagcg, ggacaggcagtcac,
gtcacctcttggttgtgc, ccagagtctcaaacac, cacatactccctgg, gaccagcacgttccg,
gttggtgtctatcagtg, ccctggtagaggtg, ctcaaacacttggagc, cacacatcactctggtgg,
gcacagacagtgcgc, catggcagcagtcag, ctgctcatggcagcag, catctggaaacttccagatg,
ctggaaacttccag, cataactccacacatcactc, caccataactccacacatc, ctggtgggtgaacc,
cggattacttgcagg, cgctaggtgtcagcg, gccatcacgtatgc, gcatacaccagttcagc,
ccatcaaatacatcgg, ccagcagaagtcagg, gcttcatgtctgtgc, ggtgagttccaggtttcc,
ccacaaaatcgtgtcctgg, cccttacacatcgg, gcagctcacagatgc, gcactggtaactgc,
cctggatattggcactgg, ccagcaaactcctgg, gcagaaatgccaggc, ccattgtgcagaattcg,
ccctgcagtactcgg, ggcattcacatactccc, ggtcaggtttcacacc, ccaggtccacacagg,
ccttgtcatccagg, ggatcccaaagacc, cctcaacactttgatgg, gctgtgtcaccagc,
ggtctaagaggcagcc, ggcaatctgcatacacc, cctgtgtacgagcc, ccatccacttgatgg,
cccacacagtcacacc, ccatcgtaaggtttgg, ccttttccagcagg, ggagaattcagacacc,
ccaagtcctcattctgg, ccatcagtctcagagg, cctttgaaggtgctgg, ggcatggcaggttcc,
cctggcatggcagg, agatgtataggtaa, attttcacattctc, aattttcacattctc,

aattttcacattct, gaattttcacattc, ggaattttcacatt, agatttctttgttg,
aagatttctttgttg, aagatttctttgtt, taagatttctttgtt, ctaagatttctttgtt,
taagatttctttgt, ctaagatttctttgt, ctaagatttctttg, tctaagatttcttt,
gtctaagatttcttt, gtctaagatttctt, ttcgtctaagattt, attttgacatggtt,
aattttgacatggtt, aattttgacatggt, taattttgacatggt, taattttgacatgg,
gtaattttgacatg, tgtaattttgacatg, tgtaattttgacat, tctgtaattttgacat,
ctgtaattttgaca, tctgtaattttgaca, tctgtaattttgac, gtctgtaattttga,
aagtctgtaattttga, agtctgtaattttg, aagtctgtaattttg, aagtctgtaatttt,
gaagtctgtaatttt, gaagtctgtaattt, atgtagacatcaat, atcatccaacattt,
aatcatccaacattt, aatcatccaacatt, accatcaaatacat, aaaaacgtctttga,
ttttgttcttagaca, ttttgttcttagac, taaacagaaaagca, actaaacagaaaag,
aaactaaacagaaaag, aactaaacagaaaa, aaactaaacagaaaa, aaactaaacagaaa,
taaaaactaaacagaaa, aaaactaaacagaa, gtaaaaactaaacagaa, aaaaactaaacaga,
taaaaactaaacaga, taaaaactaaacag, gtaaaaactaaaca, aaaaagtaaaaactaaaca,
agtaaaaactaaac, aaaaaaagtaaaaactaaac, aagtaaaaactaaa, aaaaaaagtaaaaactaaa,
aaagtaaaaactaa, aaaagtaaaaacta, aaaaaaagtaaaaacta, aaaaagtaaaaact,
aaaaaaagtaaaaact, aaaaaaagtaaaaac, caaaaaaagtaaaaac, aaaaaaagtaaaaa,
caaaaaaagtaaaa, aacaaaacaaaaaaagtaaa, aaacaaaaaaagta, caaaacaaaaaaagta,
caaaacaaaaaaagt, caaaacaaaaaaag, ctttaaaaaaacaaaac, tctttaaaaaaacaaa,
gtctttaaaaaaacaaa, gtctttaaaaaaaca, gtctttaaaaaaac, tttatttcgtcttt,
tctttatttcgtct, tatttgcaaatgga, tatatttgcaaatgg, tatatttgcaaatg,
caaaatatatttgcaaatg, caaaatatatttgcaaat, caaaatatatttgca, caaaatatatttgc,
ttccaaaatatatttg, ttttccaaaatatattt, gttttccaaaatatatt, gttttccaaaatat,
ggttaggcaaagcc, ccgagaacatcatcgtgg, ccgagaacatcatcgtg, ccgagaacatcatcg,
cgtagtctgcgttgaagc, ccatgctggagaagg, ccgtgcagaagtcc, ggaatgaagttggc,
tgaccgtgggaatg, tggcagtgaccgtg, agatggcagtgacc, cgagatggcagtgacc,
ccagccactgcagg, gcaccagccactgc, ccctggagtaagcc, ggagataactgttccacc,
ggagataactgttcc, cttctagttggtctg, catcttctagttgg, tctcatcttctagttgg,
ctgcaaagcagacttctc, ccttcagcaggttgg, cccaggtcatcagg, ccagtcagatcaagg,
ggtgaaggcctcctc, cagggtgaaggcctc, cctggatgatgctgg, ccactgtgcagagg,
ggagtacaggtgacc, gctcattgctgctgc, ggaaggctcattgctgc, ttttctcttcttct,
atcttattcctttc, catcttattccttt, tagtttttccttct, tctagtttttcctt,
aactctagtttttc, gaactctagttttt, tgaactctagttttt, atgaactctagttttt,
tgaactctagtttt, atgaactctagtttt, atgaactctagttt, gcacacagtagtgc,
gcaggatcagaaaagc, gcaggtagacaggc, gcttgctcaggatctgc, gcaagtccctggtgc,
cctggagcaagtcc, cgtagtactcttcgtcg, cgtagtactcttcg, gtaaacctccttgg,
gtctattttgtaaacctcc, gcatgtctattttgtaaacc, ggcatcaaggtaccc, ggcatcaaggtacc,
gctttcaccaaattggaagc, gagaatctgatatagctc, ggagatgttaaatctttgg,
gctgtcgatgtagc, ccaggttcctgtctttatgg, cagcagggacagtg, cttgcttctagttcttcac,
gccatcaatacctgc, ggtgccatcaatacc, ccactggtatatgtgg, ggactttatagttttctg,
ctcaagtctgtaggag, ggtctgttgtgactc, caattatcctgcacatttc, gcagcaattatcctgc,
ggcagcaattatcc, ggttcgtgtatccatttcc, gcacagaagttggc, ccagcacagaagttgg,
gtgctgagtgtctg, cctgctgtgctgagtg, gctcaggaccctgc, gcagcaaggagaagc,
ccaatgtagtagagaatgg, gctgcatttgcaag, aaaaaagaaatcaa, aaaaaaagaaatcaa,
aaaaaaaagaaatcaa, taaaaaaaagaaatcaa, ataaaaaaaagaaatcaa,
aataaaaaaaagaaatcaa, gaataaaaaaaagaaat, agaataaaaaaaagaaat, cagaataaaaaaaa,
tcagaataaaaaaa, ttgtttttaaaagt, agttgtttttaaaa, aagttgtttttaaaa,
aaagttgtttttaaaa, aaaagttgtttttaaaa, aaaaagttgtttttaaaa,
aaaaaaagttgtttttaaaa, aaaaaaagttgtttttaaaa, aaaaaaaagttgtttttaaa,
tttttaaaaaagtg, ttttttaaaaaagtg, attttttaaaaaagtg, cattttttaaaaaagt,
gcattttttaaaaaa, tgcattttttaaaaaa, agcttattttaaat, aagcttattttaaat,
taagcttattttaaat, tgtaattattagat, atgtaattattagat, tgatgtaattatta,
atgatgtaattatta, atggtattatataa, tatggtattatataa, ttatggtattatataa,
tttatggtattatataa, atttatggtattatataa, aatcatattagaaa, ttacaatcatatta,
tttacaatcatatta, ggcatgacgcctttcc, gcatgacgcctttc, gcctgacgagaggc,
ctcaagcctgacgag, ccacagttcctttttc, gctgcaataaagatacag, gctgcaataaagatac,
ggacactgatttctatg, gcattatcaactttgg, acttttagcaccaatg, ccaagaaacttttagcacc,
ccagatcatcttcc, agtcaaggacacatag, tctttgagcaacatgg, gggtataacagctg,
gaggtgaaccattaatgg, tcttcgtatcgtttag, tgttggatagtgttc, gttgatcacttgctg,
ggattccattactcg, gacatatgaaaaatgttgtc, gccaataaagacatatg,
ccagaatcaagattctg, ctgttccagaatcaag, gacaaatctgttccagaatc,
ggaaagacaaatctgttcc, gattaagaggacaagc, ggaagattaagagg, gcagtgtgattattctgg,
ggagaaagatacatatctg, ggagatcttacagg, gcatttgcagtagaatttac, cagtgaaagagagg,

gctagccgatacac, ggaagatccttgtatgc, gcatgaggaagatcc, ggagtcattttgttg,
ccaattgatactaagattc, tcttttgagcacacg, ccttcagcacttcttttg, ggttgcttccttcagc,
cagtggtttaggag, cctgagatcctcatttc, ccaaggtcctgagatcc, ggtgtacacagtgtcc,
tatctttaatttct, tcttttgaatataa, ttcttttgaatataa, tttcttttgaatataa,
ttttcttttgaatataa, tttttcttttgaatataa, atttctatgttttt, ttaaagaatttatg,
gttaaagaatttat, agttaaagaatttat, aagttaaagaatttat, taagttaaagaatttat,
tttagtaagttaaa, ttttagtaagttaaa, atttcttttagtaa, aatttcttttagtaa,
atcaatttctttta, tatcaatttctttta, aatatataagttca, aaatatataagttca,
caaatatataagtt, tcaaatatataagtt, tgtcaaatatataa, aatttatttcagta,
aataaaaatgtgat, taataaaaatgtgat, tagctaataaaaat, ttagctaataaaaat,
tttagctaataaaaat, aataaaatagtcaa, taataaaatagtcaa, ttaataaaatagtcaa,
tttaataaaatagtcaa, gtttaataaaatagt, agtttaataaaatagt, gagtttaataaaata,
agagtttaataaaata, aataattcttgtat, tatattacattcat, atctatattacatt,
ataaacatttttca, aataaacatttttca, aaataaacatttttca, gaaataaacattttt,
tgaaataaacattttt, ttgaaataaacattttt, tttgaaataaacattttt,
ttttgaaataaacattttt, tttttgaaataaacattttt, attttgaaataaacatttt,
aattttgaaataaacatt, aaattttgaaataaacatt, aaaattttgaaataaacat,
taaaattttgaaataaaca, ataaaattttgaaataaac, tataaaattttgaaataaa,
gtataaaattttgaaat, ggtataaaattttt, aggtataaaattttt, aaggtataaaattttt,
aaaggtataaaattttt, aaaaggtataaaattttt, taaaaggtataaaattttt,
ataaaaggtataaaattttt, tttagaaagatttt, aagataaatttctt, taagataaatttctt,
ttaagataaatttctt, tttaagataaatttctt, ttttaagataaatttctt,
tttttaagataaatttctt, atttttaagataaatttctt, tatttttaagataaatttct,
ttatttttaagataaatt, tttatttttaagataaatt, ctttatttttaagataaat,
tctttatttttaagataaat, atctttatttttaagataaa, atctttatttttaa,
gatctttatttttaa, agatctttatttttaa, tagatctttatttttaa, aatcatcattaatt,
aaatcatcattaatt, aaaatcatcattaatt, taaaatcatcattaatt, ttaaaatcatcattaatt,
tttaaaatcatcattaatt, atttaaaatcatcattaatt, aatttaaaatcatcattaa,
gaatttaaaatcat, tgaatttaaaatcat, ttaaaataggaaat, aatttctctttaaa,
aaatttctctttaaa, taaaattttgaatg, ctaaaattttgaat, tttgctaaaatttt,
atatgaaaaatgtt, ttttaaattaagca, ttgtaaaaatcaaa, tttgtaaaaatcaaa,
tttgataaaacttt, atgttttatcattt, aatgttttatcattt, aaatgttttatcattt,
taaatgttttatcattt, tctaaatgttttat, ttctaaatgttttat, taagatcaaataaa,
ataagatcaaataaa, aataagatcaaataaa, taataagatcaaataaa, ttaataagatcaaataaa,
tttaataagatcaaataaa, ttgtttaataagat, attgtttaataagat, tgattgtttaataa,
ttgattgtttaataa, tttgattgtttaataa, ttttataaaacagt, tttttataaaacagt,
ttttttataaaacagt, ctttttttataaaaca, actttttttataaaaca, cactttttttataaaa,
acactttttttataaaa, tacactttttttataaaa, atacactttttttataaaa, attttgaatttaag,
gattttgaatttaa, tgattttgaatttaa, atgattttgaatttaa, aatgattttgaatttaa,
ataatagaatcata, tataatagaatcata, tataatagaatcat, tactataatagaat,
atactataatagaat, aatactataatagaat, agaatactataata, tagaatactataata,
atagaatactataata, tatagaatactataata, ttatagaatactataata, aatatttgttttca,
aaatatttgttttca, aaaatatttgttttca, caaaatatttgtttt, aaattttatatgga,
tgaaattttatatg, ctgaaattttatat, tctgaaattttatat, ttctgaaattttatat,
atctgatttatttt, aagatattaaatgt, tgaagatattaaat, ataaataacaatga,
tataaataacaatga, gtataaataacaat, tgtataaataacaat, ttgtataaataacaat,
tcttgtataaataa, atcttgtataaataa, aatcttgtataaataa, acaactttttaaat,
tacaactttttaaat, tacaactttttaaa, cggggggttttgggcggcatg,
ttttcggggggttttgggcggca, tcggggggttttgggcggc, ggtggcggccgtttttcggggggt,
ccggggggttccgcggcggcagcg, cggggggttccgcggcgg, ggcggcggtgccggggggttccgc,
ggagggggcggcggcggcggtg, gggggcggcggcggcgg, ggggcggcggcggcg,
agggggcctggtggaag, tagggggcctggtg, gtagggggcctggt,
gaggtattggtgacaaggtagggggc, tcttcaggggtgaaatatagatgttc, ggactcttcaggggtg,
tcggactatactgc, cagttcggactatact, aagcctaagacgca, gcccaagttcaaca,
tgaaaagtcgcggt, ggttaattaagatgcctc, tctctaagagcgca, acgtgaggttagtttg,
cacgtgaggttagt, catagaacagtccg, cagtcatagaacagtc, ctttgcagtcatagaaca,
tgcagtcatagaac, ggtcgtttccatct, catagaaggtcgtttc, cgtcatagaaggtc,
catcgtcatagaagg, ggacgggaggaacgaggcgttgag, tagccataaggtcc, ggttactgtagcca,
ggttactgtagcca, agttcttggcgcggaggt, aggtgaggaggtccgagt, tggactggattatcag,
gtggtggtgatgtgcccg, tgtcacgttcttgg, ctcatctgtcacgt, cgaagccctcggcgaacc,
gcgtgttctggctgtgcagttcgg, ctgccccgttgacc, aggtttgcgtagac, ggttgaagttgctg,
ctgggttgaagttg, tgctgcacgggcatctgctg, ggcactgtctgaggctcctccttcagg,
actccatgtcgatg, ctctccgccttgatcc, gttcctcatgcgcttc, ctgagctttcaagg,

gcgattctctccagcttccttttcg, ctgagctttcaaggttttcacttttcctc, tccctgagcatgtt,
tctgtttaagctgtgc, ctttctgtttaagctgtg, ggttcatgactttctg, cgtggttcatgact,
actgttaacgtggttc, ccactgttaacgtg, cccactgttaacgt, agcatgagttggca,
gcgttagcatgagt, gtttgcaactgctg, caaaatgtttgcaactgc, tccattttagtgcacatc,
ctgttccattttagtgca, gtgtatgagtcgtc, ctgtgtatgagtcg, cgtagctgtgtatg,
tcgtgtagagagag, agtttgtagtcgtgtaga, gtttgtagtcgtgtag, agtttgtagtcgtg,
ggagtttgtagtcg, tcaggagtttgtagtc, gtttcaggagtttgtagt, tcggtttcaggagt,
ttgagactccggta, accagaaaagtagctg, cctgaccagaaaag, attcaggcgttcca,
ggtaaaagtactgtcc, gggtaaaagtactgtc, gcacctccaccgctgcca, ctcctgctcctcggtgac,
gctttgacaaagcc, cttgtgcagatcgt, tcatcttgtgcagatc, gttcatcttgtgcaga,
cgtggttcatcttg, tcacgtggttcatc, ggttggtgtaaacg, tacgagctcccggtcccgac,
tagctgatggtggt, tccttgaaggtgga, tcttccatgttgatgg, ctttgatgcgctct,
ctccactttgatgc, gctccagcttccgcttccggcacttggtgg,
ggccttgagcgtcttcaccttgtcctccag, tgaccttctgtttgag, catgaccttctgtttg,
gtcatgaccttctg, cgagaacatcatcg, gtagtctgcgttga, gctgcagcgggaggatgacg,
agtaagagaggctatc, gtagtaagagaggc, ggtagtaagagagg, gtgagtggtagtaaga,
gtccgtgcagaagtcctg, gaatgaagttggcact, ggaatgaagttggc, gggaatgaagttgg,
gctgcaccagccactgcaggtccggactgg, tcatggtcttcacaac,
caatgctctgcgctcggcctcctgtcatgg, ctagagttcctcac, gagtacgctagagt,
gaagagtacgctag, ctgcttcccacccagcccccacattccc, ttcatcctctgtactgggct,
gttacggatgtgca, cagttacggatgtg, ccagttacggatgt, agagtctgagttgg,
gtgagactcagagt, tcttagggtgagac, gagagtacttcttagg, ggaagaaactatgagagt,
cttagggaagaaactatg, cggtaagaaacttagg, agcatgcggtaaga, gtctgaaagcatgc,
agaacaaagaagagcc, caagagaacaaagaagag, cagcaagagaacaaag, tcctcagcaagaga,
aggtgtgacttgca, gaataggtgtgacttg, cagaataggtgtgact, gcagaataggtgtg,
cagttgcagaataggt, gaaaccatttctgacc, tgtgaaaccatttctgac, cactgtgaaaccatttct,
ccactgtgaaacca, agaactggctcctgcagcttccctgcttcc, cacctccattcaccc,
cagtaaaagtgtctgc, cgacattcagtaaaagtg, gaccgacattcagt, cttctggagataactaga,
catcttattcctttccct, cagccatcttattcct, tgcagccatcttattc, gagtgtatcagtcag,
ggagtgtatcagtc, cttggagtgtatcagt, acagagtacctacc, ccaactttcccttaag,
ccttatgctcaatctc, gtcttactcaaggg, acagtcttactcaagg, cataagacacagtcttac,
gaaagcataagacacagt, ggaaagcataagacac, agggataaaggaaagc, cctgtatacagagg,
tgtctcctgtatacag, catcttctagttggtc, ctcatcttctagttgg, cttctcatcttctagttg,
caaagcagacttctca, ctgcaaagcagact, ctagtttttccttctcct, tctagtttttccttctcc,
caggatgaactctagt, tcgtagaaggtcgt, agggttactgtagc, gtagtggtgatgtg,
cgtcgtagaaggtc, tttcgtgcacatcc, agtttgtagtcgtgaaga, cgagaacatcatgg,
gtagtaggaaaggc, ggtagtaggaaagg, ggaatggtagtagg, ggtcattgagaagag,
gctaatgttcttgacc, gccaaggtcctcat, ggagtctatctcca, ccaaagaatcctgact,
cacatgcttagtgg, ctcgtaaatgaccg, aggaatctcgtaaatgac, cagcagcgattcat,
ggagatcatcaaagga, ctcagcaatggtca, gatctcgaacacct, cacaatctcgatctttct,
ccttcttaaagattggct, cacataccaactgg, agcttgatgtgagg, gaagttgtagcttgatgt,
gcttgaagttgtagct, ctgcttgaagttgtag, gacacaactcctct, tcctttgatagacacaac,
ctcgtttgatagacac, ggttagcacacact, ggtaacggttagca, cgtaacacatttagaagc,
ctcatccgtaacac, ccggtaagtattgtagtt, ggtgtatttccttgac, acataccaactggtgt,
gtccctatacgaac, ttcatgtctgtgcc, gtaggtgagttcca, gttgtgagcgatga,
catagttgtcctcaaaga, ggcatagttgtcct, cattgtctagcacg, ctccattgtctagc,
gtattgttcagcgg, tcaagatctctgtgag, cacaaaatcgtgtcct, tccttccacaaaatcg,
gtggaagatgtcct, tcttgtggaagatgtc, tctatcagtgtgagag, ggttggtgtctatc,
acatcggagaacag, ccttacacatcgga, acaatcctcagaactc, gctctgacaatcct,
tggttgaagtggag, ctgtggttgaagtg, gttgtaggtgacca, ctgtgttgtaggtg,
gactcaaacgtgtc, catggactcaaacg, cgaatgtataccgg, ccgaatgtataccg,
gccgaatgtatacc, gtagttgtagggac, tagaaaggtagttgtagg, gtagaaaggtagttgtag,
cgtagaaaggtagttg, ccgtagaaaggtag, gaccatagcacact, ggatattggcactg,
cctggatattggca, gctcccaaagatct, cccatcaaagctct, caaacacttggagc,
gtctcaaacacttgga, gagtctcaaacacttg, gtaacctgtgatctct, ggtaacctgtgatc,
gtataggtaacctgtg, tgagatgtataggtaacc, tgctgagatgtatagg, ccatgctgagatgt,
ggattacttgcagg, tgttatggtggatgag, ggtgttatggtgga, gcagttgacacact,
agtactcggcattc, cattcacatactccct, tccaaaacaggtcact, ggtccttatagtgg,
cagaatgccaacca, acgagaatgccaac, gatcccaaagacca, tcgcttgatgagga,
catcgtgtacttcc, gcatcgtgtacttc, actgtgccaaaagc, cttgtagactgtgc,
cccttgtagactgt, tcaacactttgatggc, ccctcaacactttg, gtgttttccctcaaca,
gtatgcttcgtctaag, cgtatgcttcgtct, ccatcacgtatgct, gcataagctgtgtc,
catggtctaagagg, caatctgcatacacca, ggcaatctgcatac, ctgtctcgtcaatg,

cataactccacacatc, agtcacaccataactc, acagtcacaccataac, ccccaaaagtcatc,
tcgtaaggtttggc, gatcccatcgtaag, caatggtgcagatg, gacatcaatggtgc,
gtagacatcaatggtg, catgatcatgtagacatc, ccatgatcatgtagac, catttgaccatgatcatg,
ccaacatttgaccatg, tcatccaacatttgacca, gagtcaatcatccaacat, cagagtcaatcatcca,
ccgacattcagagt, gaattcagacaccaac, gatgaccacaaagc, ccatcaaatacatcgg,
tcaccatcaaatacatcg, caacgtagccatca, acgtctttgacgac, caaaaacgtctttgacga,
ggcaaaaacgtctttg, caaaggcaaaaacgtc, gtgtcaagtactcg, gtaatagaggttgtcg,
cccagtaatagagg, catggtgctcactg, gtgcctgtacgtac, tgcaggtggatagt,
catgtcgatagtcttgca, gtcgatagtcttgc, ccatgtcgatagtc, ctccatgtcgatag,
cttggacaggatct, tgctgttgtacagg, gtgctgttgtacag, ttggcgtagtagtc,
tccaccattagcac, gatttcgttgtggg, gtcatagatttcgttgtg, tgtactctgcttgaac,
gtgtactctgcttg, tgctgtgtgtactc, ctgatgtgttgaagaaca, ctctgatgtgttgaag,
gctctgatgtgttg, gagctctgatgtgt, cacttttaacttgagcct, ctccacttttaacttgag,
tgctgtatttctggtaca, ccaggaattgttgc, ttgctgaggtatcg, gataaccactctgg,
caaaagataaccactctg, cggtgacatcaaaag, cctcaatttcccct, gttatccctgctgt,
gcagtgtgttatcc, gatgtccacttgca, tagtgaacccgttg, tgccatgaatggtg,
gttcatgccatgaatg, catgagaagcagga, gctttgcagatgct, gagctttgcagatg,
tagttggtgtccag, ctgaagcaatagttgg, agctgaagcaatagttgg, ggagctgaagcaat,
caatgtacagctgc, ggaagtcaatgtacag, cggaagtcaatgtac, gcggaagtcaatgt,
agttggcatggtag, gcagaagttggcat, ctccaaatgtaggg, accttgctgtactg,
tgctggttgtacag, ggttatgctggttg, gtagtacacgatgg, cgtagtacacgatg,
cacgtagtacacga, catgttggacagct, gcacgatcatgttg, cacacagtagtgca,
gatcagaaaagcgc, accgtgaccagatg, gtagacaggctgag, tatcgagtgtgctg,
ttgcgcatgaactg, ttgctcaggatctg, actggtgagcttca, gctcaggatagtct,
tgtagatggaaatcacct, tggtgctgttgtag, ttctcctggagcaa, tactcttcgtcgct,
cttggcgtagtact, cggcatgtctattttgta, cgggatggcatttt, ctgtagaaagtggg,
acaattctgaagtagggt, attgctgagacgtcaaat, tctccattgctgag, tcaccaaattggaagcat,
ctctgaactctgct, aacgaaagactctgaact, tgggttctgcaaac, ctggcttttgggtt,
gttgttcaggcact, tctgatatagctcaatcc, tctttggacttgagaatc, tgggttggagatgt,
tgctgtcgatgtag, acaactttgctgtcga, attcgccttctgct, gaaggagagccatt,
tcagttacatcgaagg, tgaagccattcatgaaca, tcctgtctttatggtg, aaatcccaggttcc,
ggacagtgtaagcttatt, gtacaaaagtgcagca, tagatggtacaaaagtgc,
cacttttatttgggatgatg, gcaaatcttgcttctagt, gtgccatcaatacc, ggtatatgtggagg,
tctgatcaccactg, tcctagtggactttatag, tttttcctagtggact, caataacattagcagg,
aagtctgtaggagg, tctgttgtgactcaag, gttggtctgttgtg, caaagcacgcttct,
tttctaaagcaataggcc, gcaattatcctgcaca, acgtaggcagcaat, atcaatgtaaagtggacg,
ctagatccctcttg, ccatttccaccccta, tgggttcgtgtatc, tggcattgtaccct,
tccagcacagaagt, ataaatacgggcatgc, agtgtctgaactcc, tgtgctgagtgtct,
ataagctcaggacc, aggagaagcagatg, agcaaggagaagca, aatcttgggacacg,
tagagaatggttagaggt, gttttgccaatgtagtag, cttgggtgttttgc, gcaagactttacaatc,
gcatttgcaagactttac, tttagctgcatttgcaag, gccacttttccaag, ttggtcttgccact,
cagcacacagtagt, cgatagtcttgcag, ctttcaccaaattggaag, caccaaattggaagc,
tcaccaaattggaagc, ctctggcttttggg, cggcatgtctattttg, cactacagacgagc,
cgtgcactacagacg, ggaacagttcgtcc, gaacagttcgtccatg, ccagagtttcggttc,
ctaggactgggacag, cgcacttgtagcg, ctcgcacttgtagc, gcacttgtagc,
gcgcactgtccctg, ccagggagatgcgc, gccggtgaggagg, ccggtgaggaggg,
cggttcactcggc, gagtttcggttcactc, ggcacgattgtcaaag, caggcgtcaccccc,
gcaggcgtcaccc, ctccctcctaagc, ccctcctaagcgg, cgagtccgcgttcg,
catcttctgccattc, gtgttttcccaccag, ggttttggttcactag, gcatcttcacgtctcc,
cttcacgtctcctgtc, gtcaccgcgtagtc, caaataggcaaggtc, cttgcaaataggcaag,
tgcttgcaaatagg, ctgcttgcaaatagg, gcaggtggatattt, ctgctgttggcag,
cactagtttccaagt, gttttggttcactag, ctttgatttcaggatag, gcacttcttctttatct,
ccaagtcagatttcc, gtttccaagtcagatttc, ggttcactagtttcc, ggttttggttcactag,
ccgaaaaattgggca, ccgaaaaattggg, ctatccgaaaaattgg, gttgataatgtcatcag,
ctcatgttgataatgtc, ctgtcaccgcgtag, cgtctcctgtcaccg, cttcacgtctcctg,
gagaactttatcatgtc, gctatatgcaggg, ccagctgctatatgcagg, aggctaaattttgcct,
ggctaaattttgcc, ggctaaattttgccttc, gcaggctaaattttgcc, gagttacccaagcg,
cagagttacccaagcg, cagagttacccaag, acagagttacccaag, ggtgcaaaacagag,
ctaggtgcaaaacag, gagaactttatcatgtcc, gctagatgaatggc, gcaaacatggcaggc,
cagcaaacatggca, gcagcaaacatggc, agcagcaaacatgg, cagcagcaaacatg,
agcagcagcaaaca, cagcagcagcaaaca, cagcagcagcaaac, caccagcagcagca,
gcattgacgtcagc, gatgttgtcgtgctc, tgagatgttgtcgtgct, tgagatgttgtcgtg,
gccaatgagatgttg, ctgccaatgagatg, cacatgggcatcac, tgtccacatgggca,

gtactgtccacatg, cagctgctatatgc, gttctccaccaggg, agttctccaccagg,
caaagttctccaccag, ccaagagtcatccagg, cccaagagtcatcc, cctgcattttcccaag,
tcctgcattttccc, gccatatctagaggc, tcacatcttcagcc, gcttcacatcttcagc,
cagcttcacatcttc, gtaacttatacagctgc, ccagtttttgtctgg, ccatttgtctcagg,
gtgtagcccatttg, gcttcggtgtagcc, gatcacttcaattgcttc, cttgtggaggcagg,
gctgccttgtggag, ctatttgctgccttgtgg, ggatgtctccacgc, ggaaggatgtctcc,
tgcggaaggatgtc, gtttgcggaaggatgtc, gctgagtttgcgga, ggtaaagctgagtttg,
tcggtaaagctgag, gactcggtaaagctg, agagactcggtaaagc, gaaattgtcagcaggc,
gaaattgtcagcagg, ggaaattgtcagcagg, ggaaattgtcagcag, gggaaattgtcagc,
gtgtgggaaattgtc, ggtttacacggtgtg, gctttggtttacacg, gcacctttgggatgc,
ccaggttctgcttcc, gctctgtctagtggc, actctccatgtctc, caactctccatgtctc,
caactctccatgtc, agcaactctccatg, gtagcaactctccatg, gtagcaactctcca,
ggttgtagcaactctcc, cgggcagtcctcca, gcaccgggcagtc, aggcaccgggcag,
gtgtgttaccaggtc, tgtgtgttaccaggt, tgggtcactgtgtg, cagactgtgggcatg,
cccaccagactgtggg, ccaccagactgtgg, tgcccaccagactg, cggcttcctcccc,
ccttgtcttccacc, accgaggctgccac, ggaagaaaccgagg, gggaagaaaccgag,
ggccatctgcgcc, gcggccatctgcg, gtggcggccatctg, accgtggcggccat,
gccgctcaatcttcatc, cttcatcttgtgatagg, gctcaatcttcatcttg, cagaaacactgttacag,
cagttgcagaaacactg, gtttcagttgcagaaac, cttccaccagaggg, gtcttccaccagag,
cttgtcttccaccagag, tccttgtcttccac, cttccttgtcttccac, catcttgtgataggg,
gctaggtgcagtggt, gatggctaggtgca, gtggatgatggctag, cccgtggatgatgg,
ctgcccgtggatga, agagcctccaccca, gttgtactctcgagc, cgttgtactctcg,
cgcgttgtactctc, gagtctccatgccg, ctgagtctccatgc, catggctgagtctc,
tgcatggctgagtc, gcgttcacgttggc, gtgcgagcgttcac, aggtgcgagcgttc,
gcaaaggtgcgagc, cctggtggctcagg, gtcagtcacctgag, caggtcagtcacctg,
cagcaggtcagtcac, gcagcaggtcagtc, catttagcagcaaggtc, gcagcatttagcagc,
ctgagcagcatttag, cccatgagaatcct, ccttcccatgagaatcc, tcctccccttccca,
gcctccagtagacc, gtcagacagggcct, ccatgtcagacagg, ggcccatgtcagac,
gctattcctgaaatcac, cctcttgtcttcttacc, ggagaagaaacctcttg, ccttgctgaagtttctt,
ccaagactccttgc, cccttcatggagc, cctcttggtgtgac, gactaaggatgccg,
gtggcaggactaagg, agacgtggcaggac, cttccagcaggcag, gttcctctgcctgg,
gatgttcctctgcctg, gagatgttcctctgcc, gtgagatgttcctctg, cagagagtgagatgttcc,
ccagagagtgagatgttc, ggtccagagagtgag, gaggtccagagagtg, ggtcctgtagtgcc,
gattttatgatgcaggc, gacctgcatcccttattg, tagttgattttccagcag,
gaatctcacgttttgc, cagagaaagaatctcacg, tttcaccatcagagaaag,
catttggacatttcacc, ccttcatttggacatttc, caatgtgcttgatgatcc, cgcatcggatttctc,
caaaccgcatcggatttc, gaactgcaaaccgc, gcagagaagaactgc, gcaagtaaacatggg,
ggtccacgttttgg, gcaagggtccacgttt, tggcttcttcttcaggg, tcctgctggcttcttc,
gtcctgctggcttc, ggtagtctaggaattgg, cttgcaggtagtctagg, gaaactcttgcaggtag,
caccaagaaactcttgc, cattacaccaagaaactc, ctcggtgttcattacacc,
ctttctattatccactcg, ccagtttagtctcaactt, aaccagtttagtctcaac,
acaaaccagtttagtctc, ctcgcgaaaaagtttctt, ccctcgcgaaaaagtttc,
gtccctcgcgaaaaag, cagttgaaccgtccc, gctttcgaagtttcagtt, gatgctttcgaagtttc,
ctgtctctgcaaataatg, cacttattacattcaccc, ttttcctccagttcctc,
ggacaatatgtacaaaactc, gttgatgaacatttggac, gtgttgatgaacatttgg,
caaaatttggccaggg, gcccaaaatttggcc, cccagcccaaaatttgg, gtccccagcccaaaatt,
aaatcgccagaggctg, accaaatcgccagagg, catcaccaaatcgccag, taggagtggttgaggc,
gtgtaggagtggttgag, ctgtgtaggagtgg, cccacatgcctgtg, cgatgaacaacgag,
ctggcgatgaacaacg, cgctggcgatgaac, gagctagtcccgttg, gcgaagagctagtcc,
ccagttatgcgaagagc, ccccagttatgcgaag, cacatgcttggcgc, gatcacatgcttggcg,
gacaaagagcatgatcac, gagtcacagggacaaag, gagagtcacagggac, gcagagagtcacagg,
ccatgcagagagtc, ccaccatgcagagag, tagccacgaccacc, gattagctgcccatcctt,
ggtatagattagctgcc, gtatcttctgtgaatggg, ctggcccacagtct, ctctggcccacagt,
tgcagggctctctg, agtgcagggctctc, cactgatcatgatggc, gacactgatcatgatggc,
acaatgacactgatcatg, gaaccaccaggaggat, gacacaaaacagccact, gtggacctttcggac,
caaccagcatacgaagt, tccctctgggcttc, actgtccctctggg, gactgtccctctgg,
cctagatgactgtccc, cagcgaggatactgc, cttcaccagcgaggat, tttcctctgggtcttcac,
ctttcctctgggtcttc, ctcccaatccaagtttt, ttcatcccggagcc, ttcttcatcccggagc,
gctcagccagttcttc, gacagagagggcac, cttcacctccgacag, gaaaagtctgggcagg,
gaccctggaacagaaaag, ctgaccctggaacag, actacaggctgaccct, attcactacaggctgacc,
cgattcactacagg, ggccgattcactac, cgaacgtctgttggtc, cgcgaacgtctgttg,
cttctgtttgtcgaggat, ttcaccaccttctgtttg, aggatgcgcttttcattc,
agcttgcaggatgcg, gttgacagcttgcaggat, ggaacggaaagttgacag,

aactcgagtttgacgagg, tgtccttgaaggagaac, cgtactccatgaccatgt,
gcacgtactccatgac, gattctccggcttcag, tcaatgagcagattctcc, ggtcaatgagcagattc,
ccctgctggtcaatg, tagccctgctggtc, cgcttggcgaaacc, ccttcacgcgcttg,
aaggtccaagtgcg, tgccgcacaaggtc, ggtgaggaccaccattt, gggtgtcacaggtg,
ataccatcttcttcaggg, ggtgataccatcttcttc, ccaggtgataccatcttc,
cctcactgctctggt, taagacctcactgc, cagagcctaagacctc, ccagagcctaagacc,
tcttccttttgtgaagc, gaccaaattccatcttcc, atcagtggaccaaattcc,
ggttctttctggtccttt, tttttgggttctttctgg, ggtcttattttgggttc,
aatgggcagactctcct, tccaccatgacctcaatg, aacggcatccaccatg, gtgaacggcatccac,
acttgagcttgtgaacgg, ttcatacttgagcttgtg, ctggtgtagttttcatac,
agctgctggtgtagtttt, aggaggaccagggt, aggtggtccaggag, tttctggccaaactgagg,
ggaggtttctggcc, tctggagtggccac, cttctggagcatgttgct, gccttctggagcatg,
gtttgtctggccttctg, gagtttgtctggccttct, ctagagtttgtctggcct,
gcaagggtaaaattctag, agtgcaagggtaaaattc, aaacaggcctccact,
cttggttaattccaatgg, aggcaactcccattagtt, tactactaaggcacaggg,
aatactactaaggcacag, gtacatcttcaagtcttc, ggagtggacatgat, aagaagatgaagcctttg,
ccgtcttactcttcttgg, ccgatacaattccaagg, ccttttccttctgag, ctgttgcaagtacg,
cagaagcagagggc, cctcagaagcagagg, ctcctcagaagcag, acaggctggtggca,
ccactctcaaacaggc, acggtagccgaagc, gacggtagccgaagc, ggccagacggtagc,
gtgtagggccagacggta, ccgaagccattttcagg, ccccgaagccatttttc, ggttgatgtcgtcc,
gcttgagacactcgc, ccggacccgtccat, gcttgctttactgc, ggttgctctgagac,
gccacagtcatgcc, cgggcatgctggcg, gtgaagttcaggatgatc, ccagtgcctcatgg,
cagtgttctccatgg, ctgtaccagaccgag, gcatactgtttcagc, gccatcagctccttg,
ccacaccatagatgg, gctggagcagtttcc, ctcgcttctgctgc, accgtggcaaagcg,
aggtgacaccgtgg, gacttgattccttcag, ggatttgacttgattcc, gctgctgttcatgg,
ccgtttctttcagtagg, cttgaagtaggagc, cgctcctacatggc, gatgaggtacaggcc,
gtagatgaggtacag, gagtagatgaggtac, cctgggagtagatg, ggacctgggagtag,
acatgggtggaggg, gtgctcatggtgtc, ctttcagtgctcatg, tgctttcagtgctca,
gatgatctgactgcc, gttcgagaagatgatc, gggttcgagaagatg, ggtttgctacaacatg,
cagcttgagggtttg, tgcccctcagcttg, gacacacactatctc, gcagccatctttattc,
gttcagcagccatc, tggttcagcagcca, ctactggttcagcagc, tctactggttcagc,
gccacaaagttgatgc, cattgccacaaagttg, gagaacttggtcattc, ggtcaatgaagagaac,
cgatttccttggtc, ccgatttccttggtc, caaatagaggccgatttc, caaatagaggccga,
cctctaggctggct, catacctctaggctg, agccatacctctag, cagccatacctctag,
cacagagatagttacag, gtcttcgttttgaacag, ctagtcttcgttttgaac,
tagctagtcttcgttttg, gagccactgcgcc, cgtgagccactgcg, cgtaacgatcactgg,
gcactcgtaacgatc, ggagcactcgtaac, catcatcctgaggt, cagtatcatcatcctg,
ctcagtatcatcatcc, ctaaaagtatgtgccatc, cacatcgcctctct, gcttcacagtcacatcgc,
ggaaggcttcacagtc, cctgtgacttgagaattg, ggaagacctgtgac, ctctgctccacatatttg,
caacgaagatctctg, caacaccaacgaag, ggtcttctgtttgc, cgatgaagtggtaggaag,
ggttgcatggaagc, ggtcacaaacttgcc, ctgatttggtccactag, catgttagcactgttc,
ggtcttgatgtactcc, ccacctaaagagagatc, cttgtactgcaccatc, gccagttaagaagatg,
gagatcatgatccatgg, gtagtgtcccaatagtg, cttcctcatcattccc, cacaagcttttcgac

### Example 8

### TGF-beta Protein

Presented are the amino acid sequences of TGF-beta1, TGF-beta2 and TGF-beta 3 with the international one letter abbrevation for amino acids.
RXXR: cleavage site of the mature (active) part (XX may be anything)
ASPC: the C of this motif is the C for the intermolecular cystine bridge that links the two monomers into a functional dimer
C C C: intramolecular cystein bridges (cystein knot motif) mature protein of TGF-beta 1, 2 and 3 contains 112 amino acids from the end of this listing
TGF-beta 1 MPPSGLRLLLLLLPLLWLLVLTPGRPAAGLSTCKTIDMELVKRKRIEAIRGQILSKLRLASPPSQGEVPPGPLPE 
AVLALYNSTRDRVAGESAEPEPEPEADYYAKEVTRVLMVETHNEIYDKFKQSTHSIYMFFNTSELREAVPEPVLL
SRAELRLLRLKLKVEQHVELYQKYSNNSWRYLSNRLLAPSDSPEWLSFDVTGVVRQWLSRGGEIEGFRLSAHCSC
DSRDNTLQVDINGFTTGRRGDLATIHGMNRPFLLLMATPLERAQHLQSSRHRR
ALDTNYCFSSTEKNCCVRQLYIDFRKDLGWKWIHEPKGYHANFCLGPCPYIWSLDTQYSKVLALYNQHNPGASAA
PCCVPQALEPLPIVYYVGRKPKVEQLSNMIVRSCKCS
 preferred amino acid sequences of TGF-betal:
1) ALDTNYCFSSTEKNCCVRQL
2) YIDFRKDLGWKWIHEPKGYH
3) ANFCLGPCPYIWSLDTQYSK
4) VLALYNQHNPGASAAPCCVP
5) QALEPLPIVYYVGRKPKVEQ
6) LSNMIVRSCKCS
7) TEKNCCVRQLYIDFRKDLGW
8) KWIHEPKGYHANFCLGPCPY
9) WSLDTQYSKVLALYNQHNP
10) GASAAPCCVPQALEPLPIVY
11) YVGRKPKVEQLSNMIVRSCKCS
12) QYSKVLALYNQHNPGASAAPCCVPQALEPLPIVYYVGRKP
13) 
QYSKVLALYNQHNPGASAAPCCVPQALEPLPIVYYVGRKP
I
QYSKVLALYNQHNPGASAAPCCVPQALEPLPIVYYVGRKP
 (dimer of the TGF-beta1 amino acid sequence No.12 coupled by an S-S bridge at the Cytosins of the AAPC motif)
14) ALDTNYCFSSTEKNCCVRQLYIDFRKDLGWKWIHEPKGYHANFCLGPCPYIWSLDTQYSKVLALYNQHNPGA SAAPCCVPQALEPLPIVYYVGRKPKVEQLSNMIVRSCKCS
15) ALDTNYCFSSTEKNCCVRQLYIDFRKDLGW
16) KWIHEPKGYHANFCLGPCPYIWSLDTQYSK
17) VLALYNQHNPGASAAPCCVPQALEPLPIVY
18) YVGRKPKVEQLSNMIVRSCKCS
19) CVRQLYIDFRKDLGWKWIHEPKGYHANFCL
20) GPCPYIWSLDTQYSKVLALYNQHNPGASAA
21) PCCVPQALEPLPIVYYVGRKPKVEQLSNMI
TGF-beta 2

MHYCVLSAFLILHLVTVALSLSTCSTLDMDQFMRKRIEAIRGQILSKLKLTSPPEDYPEPEEVPPEVISIYNSTR
DLLQEKASRRAAACERERSDEEYYAKEVYKIDMPPFFPSENAIPPTFYRPYFRIVRFDVSAMEKNASNLVKAEFR
VFRLQNPKARVPEQRIELYQILKSKDLTSPTQRYIDSKVVKTRAEGEWLSFDVTDAVHEWLHHKDRNLGFKISLH
CPCCTFVPSNNYIIPNKSEELEARFAGIDGTSTYTSGDQKTIKSTRKKNSGKTPHLLLMLLPSYRLESQQTNRRK
R
ALDAAYCFRNVQDNCCLRPLYIDFKRDLGWKWIHEPKGYNANFCAGACPYLWSSDTQHSRVLSLYNTINPEASAS
PCCVSQDLEPLTILYYIGKTPKIEQLSNMIVKSCKCS

Preferred amino acid sequences of TGF-beta2
1) ALDAAYCFRNVQDNCCLRPL
2) YIDFKRDLGWKWIHEPKGYN
3) ANFCAGACPYLWSSDTQHSR
4) VLSLYNTINPEASASPCCVS
5) QDLEPLTILYYIGKTPKIEQ
6) LSNMIVKSCKCS
7) VQDNCCLRPLYIDFKRDLGW
8) KWIHEPKGYNANFCAGACPY
9) LWSSDTQHSRVLSLYNTINP
10) EASASPCCVSQDLEPLTILY
11) YIGKTPKIEQLSNMIVKSCKCS
12) QHSRVLSLYNTINPEASASPCCVSQDLEPLTILYYIGKTPK
13) 
QHSRVLSLYNTINPEASASPCCVSQDLEPLTILYYIGKTPK
I
QHSRVLSLYNTINPEASASPCCVSQDLEPLTILYYIGKTPK
 (dimer of the TGF-beta2 amino acid sequence No.12 coupled by an S-S bridge at the Cytosins of the ASPC motif)
14)ALDAAYCFRNVQDNCCLRPLYIDFKRDLGWKWIHEPKGYNANFCAGACPYLWSSDTQHSRVLSLYNTINPEA SASPCCVSQDLEPLTILYYIGKTPKIEQLSNMIVKSCKCS
15) ALDAAYCFRNVQDNCCLRPLYIDFKRDLGW
16) KWIHEPKGYNANFCAGACPYLWSSDTQHSR
17) VLSLYNTINPEASASPCCVSQDLEPLTILY
18) YIGKTPKIEQLSNMIVKSCKCS
19) CLRPLYIDFKRDLGWKWIHEPKGYNANFCA
20) GACPYLWSSDTQHSRVLSLYNTINPEASAS
21) PCCVSQDLEPLTILYYIGKTPKIEQLSNMI
TGF-beta3 
MKMHLQRALVVLALLNFATVSLSLSTCTTLDFGHIKKKRVEAIRGQILSKLRLTSPPEPTVMTHVPYQVLALYNS
TRELLEEMHGEREEGCTQENTESEYYAKEIHKFDMIQGLAEHNELAVCPKGITSKVFRFNVSSVEKNRTNLFRAE
FRVLRVPNPSSKRNEQRIELFQILRPDEHIAKQRYIGGKNLPTRGTAEWLSFDVTDTVREWLLRRESNLGLEISI
HCPCHTFQPNGDILENIHEVMEIKFKGVDNEDDHGRGDLGRLKKQKDHHNPHLILMMIPPHRLDNPGQGGQRKKR
ALDAAYCFRNVQDNCCLRPLYIDFKRDLGWKWIHEPKGYNANFCAGACPYLWSSDTQHSRVLSLYNTINPEASAS
PCCVSQDLEPLTILYYIGKTPKIEQLSNMIVKSCKCS
 preferred amino acid sequences of TGF-beta3:
1) ALDTNYCFRNLEENCCVRPL
2) YIDFRQDLGWKWVHEPKGYY
3) ANFCSGPCPYLRSADTTHST
4) VLGLYNTLNPEASASPCCVP
5) QDLEPLTILYYVGRTPKVEQ
6) LSNMVVKSCKCS
7 NLEENCCVRPLYIDFRQDLG
8 WKWVHEPKGYYANFCSGPCP
9) YLRSADTTHSTVLGLYNTLN
10) PEASASPCCVPQDLEPLTIL
11) YYVGRTPKVEQLSNMVVKSCKCS
12) THSTVLGLYNTLNPEASASPCCVPQDLEPLTILYYVGRTPK
13) 
THSTVLGLYNTLNPEASASPCCVPQDLEPLTILYYVGRTPK
I
THSTVLGLYNTLNPEASASPCCVPQDLEPLTILYYVGRTPK
 (dimer of the TGF-beta3 amino acid sequence No.12 coupled by an S-S bridge at the cytosins of the ASPC motif)
14)ALDAAYCFRNVQDNCCLRPLYIDFKRDLGWKWIHEPKGYNANFCAGACPYLWSSDTQHSRVLSLYNTINPEA SASPCCVSQDLEPLTILYYIGKTPKIEQLSNMIVKSCKCS
15) ALDAAYCFRNVQDNCCLRPLYIDFKRDLGW
16) KWIHEPKGYNANFCAGACPYLWSSDTQHSR
17) VLSLYNTINPEASASPCCVSQDLEPLTILY
18) YIGKTPKIEQLSNMIVKSCKCS
19) CLRPLYIDFKRDLGWKWIHEPKGYNANFCA
20) GACPYLWSSDTQHSRVLSLYNTINPEASAS
21) PCCVSQDLEPLTILYYIGKTPKIEQLSNMI

It must be noted that as used in this specification and the appended claims, the singular forms "a", "an" and "the" include plural references unless the context clearly dictates otherwise. Unless defined otherwise all technical and scientific terms used herein have the same meaning as commonly understood to one of ordinary skill in the art to which this invention belongs.

### References

Abuchowski, A. et al.; Cancer Therapy with Chemically Modified Enzymes. I. Antitumor Properties of Polyethylene Glycol-Asparaginase Conjugates, Cancer Biochem. Biophys., 7; 175-186, Gordon and Breach Science Publishers, Inc. (1984)
Braun, D., Cherdron, H., Rehahn, M., Ritter, H., Voit, B.; Polymer Synthesis: Theory and Practice, Fundamentals, Methods, Experiments,Springer New York, London, Berlin 4th ed., 2005.
Brunton, Chapter 38 In: Goodman & Gilman's The Pharmacological Basis of Therapeutics, 9th Ed., Hardman et al., eds., McGraw-Hill, New York, N.Y., pages 934-935 (1996)
Buur et al., J. Control Rel. 14, 43-51 (1990)
Chirila, T.V., Rakoczy, P.E., Garetti, K.L., Lou, S., Constable, I. J.; Review: The use of synthetic polymers for delivery of therapeutic antisense oligodeoxynucleotides, Biomaterial 23 321-342 (2001)
Chonn et al., Current Op. Biotech., 6, 698-708 (1995)
El-Hariri et al., J. Pharm. Pharmacol., 44, 651-654 (1992)
Gualtieri, F.; New Trends in Synthetic Medicinal Chemistry: Vol. 7 Methods and Principles in Medicinal Chemistry, Chapter), Chemistry of Antisense oligonucleotides, edited by R. Mannhold, H Kuinyi, H. Timmerman, Wiley-VCH, Weinheim (Germany)New York Toronto
Hayashi, T., et al. 2002, Parkhust, M.R. 2003, Phan, V. 2003
Hecht; M.; Bioorganic Chemistry: Nucleic acid, Chapter 2; The chemical Synthesis of DNA/RNA, Oxford University Press, New York, Oxford (1996)
Hermanson, G.T.; Bioconjugate Techniques, Academic Press, New York, Boston, London (1996)
Hooftman, G. et al.,;Review Poly(ethylene glycol)s with Reactive Endgroups. II. Practical Consideration for the Preparation of Protein-PEG Conjugates, J. Bioactive Compat. Polymers, 11; 135-159, Technomic Publishing Co., Inc. (April 1996)
Ladd, D., Snow, R.; Reagents for the Preparation of Chromophorically Labeled Polyethylene Glyos-Protein Conjugates, Anal. Biochem., 210; 258-261, Academic Press, Inc. (1993)
Lee et al.; Critical Reviews in Therapeutic Drug Carrier Systems 8, 91-192 (1991) Muranishi; Critical Reviews in Therapeutic Drug Carrier Systems 7, 1-33 (1990)
Nathan, A. et al., Copolymers of Lysine and Polyethylene Glycol : A New Family of Functionalized Drug Carriers, Bioconj. Chem. 4; 54-62, American Chemical Society (1993)
Nielsen, P.E. Engholm, M., Berg R.H. Buchardt, O.; Sequence-selective recognition of DNA by strand displacement with a thymine-substituted polyamide, Science 1991, 254(5037), 1497-1500 (1991)
Nielsen p. E.; Review: A DNA mimic with a peptide backbone, Bioconjug. Chem. 5(1) 3-7 (1994)
Schlingensiepen, R., Brysch, W., Schlingensiepen, K.-H.; Antisense-From Technology to Therapy, Blackwell Science, Berlin, Vienna (1997)
Takahashi et al., J. Pharm. Pharmacol., 40, 252-257 (1988)
Zalipsky, S. et al., Chemistry of polyethylene glycol conjugates with biologically active molecules, Adv. Drug Del. Reviews., 16; 157-182, Elsevier Science B. V. (1995)
Zalipsky, S. et al., Peptide Attachment to Extremities of Liposomal Surface Grafted PEG Chains: Preparation of the Long-Circulating Form of Laminin Pentapeptide, YIGSR, Bioconj. Chem., 6; 705-708, American Chemical Society (1995)
Zhao and Harris, ACS Symposium Series 680, 458-72 (1997)
Yamashita et al., J. Pharm. Pharmacol., 39, 621-626 (1987)

### Patente und Publikationen:

U.S. Pat. No.: 5,719,262
U.S. Pat. No.: 5,714,331
U.S. Pat. No.: 5,700,922
U.S. Pat. No.: 5,652,356
U.S. Pat. No.: 5,652,355
U.S. Pat. No.: 5,623,065
U.S. Pat. No.: 5,565,350
U.S. Pat. No.: 5,491,133
U.S. Pat. No.: 5,403,711
U.S. Pat. No.: 5,366,878
U.S. Pat. No.: 5,539,082
U.S. Pat. No.: 5,256,775
U.S. Pat. No.: 5,220,007
U.S. Pat. No.: 5,149,797
U.S. Pat. No.: 5,013,830
U.S. Pat. No.: 3,687,808

WO 2005 / 084712
WO 2005 / 059133
WO 2005 / 014812
WO 03 / 06445
WO 01 / 68122
WO 01 / 68146
WO 99/63975
WO 98 / 33904
WO 95 / 17507
WO 95/02051
WO 94/25588

## Claims

1. A compound comprising an immunostimulator linked with at least one polymer.

2. A compound according to claim 1 wherein the polymer is branched or linear.

3. A compound according to claim 1 or 2 wherein the at least one polymer has an average molecular weight of 0.05 kg/mol to about 50 kg/mol.

4. A compound according to any of the claims 1 to 3 wherein the at least one polymer has an average molecular weight of about 0.05 kg/mol to about 5 kg/mol.

5. A compound according to claim 4 wherein the at least one polymer has a molecular weight of about 0.1 kg/mol to about 1 kg/mol.

6. A compound according to any of the claims 1 to 5 wherein the immunostimulator is a substance inducing the function of immune cells and/or the immune system to enhance abilities directly or indirectly reducing or inhibiting tumor cell growth and/or inducing cell death of unwanted neoplasms such as cancer or fibrosis or is useful in the treatment of HIV.

7. A compound according to any of claims 1 to 6 wherein the immunostimulator is selected from the group of chemokines, immune cell attracting substances, viruses, parts of viruses, autologous MHC-molecules, heterologous MHC-molecules, molecules involved in antigen processing, molecules involved in antigen presentation, molecules involved in mediating immune cell effects, molecules involved in mediating immune cell cytotoxic effects, molecules involved in antigen transportation, co-stimulatory molecules, peptides enhancing recognition by immune cells, peptides enhancing the cytotoxic effects of immune cells, peptides enhancing the recognition of unwanted neoplasms by immune cells, peptides enhancing cytotoxic effects of immune cells containing, fusion peptides, fusion proteins, retinoblastoma protein, proteins coded by oncogenes, protein coded by protooncogenes, proteins coded by anti-oncogenes, tumor suppressor genes, peptides derived from proteins expressed in a diseased organ but not in the nervous system, muscle, hematopoetic system or other organs essential for survival.

8. A compound according to any of claims 1 to 7 wherein the immunostimulator is an oligonucleotide.

9. A compound according to claim 8 wherein the oligonucleotide has a length of 8 to 30 nucleotide building blocks.

10. A compound according to claim 8 or 9 wherein the oligonucleotide hybridizes with the mRNA of a molecule negatively influencing the immune response and/or the immune system or hybridizes with the mRNA of the receptor of a molecule negatively influencing the immune response and/or the immunsystem.

11. A compound according to claim 10 wherein the oligonucleotide hybridizes with the mRNA of TGF-beta1, TGF-beta2, TGF-beta3, VEGF, IL-10, c-jun, c-fos, Her-2 their respective receptors or hybridizes with the mRNA of the PGE-receptor.

12. A compound according to any of claims 8 to 11 wherein the oligonucleotide is identified in the sequence listing with SEQ ID NO 1 to 435, in the examples 7 or in the example 8.

13. A compound according to any of the claims 8 to 12 wherein the at least one polymer is linked with the oligonucletotide at the 3'-end or at the 5'end or at least one polymer is linked at the 3'and 5'end or at least one polymer is linked with the 3' and one polymer is linked with the 5' end.

14. A compound according to claim 13 wherein one polymer is linked at the 3'-end of the oligonucleotide, another polymer is linked at the 5'-end of the polymer and the polymer at the 3'-end has the 1,5- to 100-fold weight of the polymer linked at the 5'-end.

15. A compound according to claim 13 wherein one polymer is linked at the 3'-end of the oligonucleotide and another polymer is linked at the 5'-end of the polymer and the polymer at the 5'-end has the 1,5- to 100-fold weight of the polymer linked at the 3'-end.

16. A compound according to any of claims 1 to 15 wherein the polymer is a polyalkylene oxide.

17. A compound according to claim 16 wherein the polymer oxide is polyethylene glycol.

18. Synthesis of the compound according to any of claim 1 to 17 wherein
- the immunostimulator is sythesized in a per se known manner
- the at least one polymer is synthesized in a per se known manner
- the at least one polymer and the immunostimulator are linked by activation of a functional group and/or by a linker

19. A pharmaceutical composition comprising a compound according to any of the claims 1 to 17.

20. Use of a compound of at least one of claims 1 to 17 for the preperation of a pharmaceutical composition for the treatment of cancer, fibrosis and/or HIV.

21. Use according to claim 20 wherein the cancer is selected from the group of solid tumors, blood born tumors, leukemias, tumor metastasis, hemangiomas, acoustic neuromas, neurofibromas, trachomas, pyogenic granulomas, psoriasis, astracytoma, acoustic neuroma, blastoma, Ewing's tumor, craniopharyngloma, ependymoma, medulloblastoma, glioma, hemangloblastoma, Hodgkins-lymphoma, medullablastoma, leukaemia, mesothelioma, neuroblastoma, neurofibroma, non-Hodgkins lymphoma, pinealoma, retinoblastoma, sarcoma, seminoma, trachomas, Wilm's tumor, or is selected from the group of bile duct carcinoma, bladder carcinoma, brain tumor, breast cancer, bronchogenic carcinoma, carcinoma of the kidney, cervical cancer, choriocarcinoma, cystadenocarcinome, embrional carcinoma, epithelial carcinoma, esophageal cancer, cervical carcinoma, colon carcinoma, colorectal carcinoma, endometrial cancer, gallbladder cancer, gastric cancer, head cancer, liver carcinoma, lung carcinoma, medullary carcinoma, neck cancer, non-small-cell bronchogenic/lung carcinoma, ovarian cancer, pancreas carcinoma, papillary carcinoma, papillary adenocarcinoma, prostata cancer, small intestine carcinoma, prostate carcinoma, rectal cancer, renal cell carcinoma, skin cancer, small-cell bronchogenic/lung carcinoma, squamous cell carcinoma, sebaceous gland carcinoma, testicular carcinoma, and uterine cancer.

22. Method of treating cancer, fibrosis and/or HIV comprising the administration of a compound according to any of the claims 1 to 17 in a pharmaceutically acceptable carrier.

23. Methode according to claim 22 wherein the cancer is selected from the group of solid tumors, blood born tumors, leukemias, tumor metastasis, hemangiomas, acoustic neuromas, neurofibromas, trachomas, pyogenic granulomas, psoriasis, astracytoma, acoustic neuroma, blastoma, Ewing's tumor, craniopharyngloma, ependymoma, medulloblastoma, glioma, hemangloblastoma, Hodgkins-lymphoma, medullablastoma, leukaemia, mesothelioma, neuroblastoma, neurofibroma, non-Hodgkins lymphoma, pinealoma, retinoblastoma, sarcoma, seminoma, trachomas, Wilm's tumor, or is selected from the group of bile duct carcinoma, bladder carcinoma, brain tumor, breast cancer, bronchogenic carcinoma, carcinoma of the kidney, cervical cancer, choriocarcinoma, cystadenocarcinome, embrional carcinoma, epithelial carcinoma, esophageal cancer, cervical carcinoma, colon carcinoma, colorectal carcinoma, endometrial cancer, gallbladder cancer, gastric cancer, head cancer, liver carcinoma, lung carcinoma, medullary carcinoma, neck cancer, non-small-cell bronchogenic/lung carcinoma, ovarian cancer, pancreas carcinoma, papillary carcinoma, papillary adenocarcinoma, prostata cancer, small intestine carcinoma, prostate carcinoma, rectal cancer, renal cell carcinoma, skin cancer, small-cell bronchogenic/lung carcinoma, squamous cell carcinoma, sebaceous gland carcinoma, testicular carcinoma, and uterine cancer.
